# EUROPEAN PATENT SPECIFICATION

(11) **EP 3 033 098 B1**
(45) Date of publication and mention of the grant of the patent: **22.06.2022**
(21) Application number: 14835927.6
(22) Date of filing: 14.08.2014
(51) Int. Cl.: A61K 38/36, A61K 38/37, A61K 35/14, C07K 1/00, C07K 14/745, C07K 14/755, C07K 7/08, C07K 14/49, C07K 14/76, C07K 14/705, C07K 14/59, C07K 14/00, A61K 38/00, C07K 14/79, A61P 7/04, A61K 47/60, A61K 47/61, A61K 47/54

(54) **RECOMBINANT FACTOR VIII PROTEINS**
REKOMBINANTE FAKTOR-VIII-PROTEINE
PROTÉINES DE FACTEUR VIII DE RECOMBINAISON

(30) Priority: 14.08.2013 US 201361866017 P
(43) Date of publication of application: 22.06.2016
(60) Divisional application: 21212101.6
(73) Proprietor: Bioverativ Therapeutics Inc., Waltham, MA 02451 (US)
(72) Inventor: PETERS, Robert T., Needham, Massachusetts 02492 (US); KULMAN, John, Belmont, Massachusetts 02478 (US)
(74) Representative: Zwicker, Jörk
(86) International application number: PCT/US2014/051147
(87) International publication number: WO 2015/023894

(56) References cited:
- WO-A1-2013/122617
- WO-A1-2013/123457
- US-A1- 2004 126 856
- US-A1- 2004 248 785
- US-A1- 2006 293 238
- US-A1- 2009 118 184
- US-A1- 2013 017 997
- LIU T ET AL: "Identification of structurally permissive regions in coagulation factor VIII suitable for the insertion of exogenous peptidyl elements", JOURNAL OF THROMBOSIS AND HAEMOSTASIS, BLACKWELL PUBLISHING, OXFORD, GB, vol. 11, no. Suppl.2, 1 July 2013 (2013-07-01), page 974, XP009195129, ISSN: 1538-7933
- NESHEIM ET AL.: 'The Effect of Plasma von Willebrand Factor othne Binding of Human Factor VI11 to Thrombin-activated Human Platelets.' J BIOL CHEM vol. 266, no. 27, 25 September 1991, pages 17815 - 17820, XP055318775

## Description

### BACKGROUND OF THE INVENTION

Hemophilia is a bleeding disorder in which blood clotting is disturbed by a lack of certain plasma clotting factors. Hemophilia A and Hemophilia B are two different types of hemophilia that are caused by deficiencies in Factor VIII (FVIII) and Factor IX, respectively.

Hemophilia A is characterized by spontaneous hemorrhage and excessive bleeding after trauma. Over time, the repeated bleeding into muscles and joints, which often begins in early childhood, results in hemophilic arthropathy and irreversible joint damage. This damage is progressive and can lead to severely limited mobility of joints, muscle atrophy and chronic pain (Rodriguez-Merchan, E.C., Semin.Thromb. Hemost. 29:87-96 (2003.

Hemophilia B (also known as Christmas disease) is one of the most common inherited bleeding disorders in the world. It results in decreased *in vivo* and *in vitro* blood clotting activity and requires extensive medical monitoring throughout the life of the affected individual. In the absence of intervention, the afflicted individual will suffer from spontaneous bleeding in the joints, which produces severe pain and debilitating immobility; bleeding into muscles results in the accumulation of blood in those tissues; spontaneous bleeding in the throat and neck may cause asphyxiation if not immediately treated; renal bleeding; and severe bleeding following surgery, minor accidental injuries, or dental extractions also are prevalent.

Treatment of hemophilia is by replacement therapy targeting restoration of FVIII and Factor IX activity. Treatment of hemophilia A is by replacement therapy targeting restoration of FVIII activity to 1 to 5 % of normal levels to prevent spontaneous bleeding (Mannucci, P.M., et al., N. Engl. J. Med. 344:1773-1779 (2001. There are plasma-derived and recombinant FVIII products available to treat bleeding episodes on-demand or to prevent bleeding episodes from occurring by treating prophylactically. Based on the half-life of these products, treatment regimens require frequent intravenous administration. Such frequent administration is painful and inconvenient. Liu *et al.* relates to the identification of structurally permissive regions in coagulation factor VIII suitable for the insertion of exogenous peptidyl elements ("Identification of structurally permissive regions in coagulation factor VIII suitable for the insertion of exogenous peptidyl elements", Journal of Thrombosis and Haemostasis, Blackwell Publishing, Oxford, GB, vol. 11, no. Suppl.2, July 1, 2013, page 974). WO 2013/123457 A1 relates to recombinant Factor VIII proteins, e.g., human Factor VIII proteins with heterologous moieties inserted into flexible permissive loops located in the Factor VIII A domains, while retaining the procoagulant activity of Factor VIII. WO 2013/122617 A1 relates to compositions comprising factor VIII coagulation factors linked to extended recombinant polypeptide (XTEN), isolated nucleic acids encoding the compositions and vectors and host cells containing the same, and methods of making and using such compositions in treatment of factor VIII-related diseases, disorders, and conditions. US 2006/293238 A1 relates to purified and isolated nucleic acid sequences encoding procoagulant-active FVIII proteins. US 2013/017997 A1 relates to compositions comprising factor VIII coagulation factors linked to extended recombinant polypeptide (XTEN), isolated nucleic acids encoding the compositions and vectors and host cells containing the same, and methods of making and using such compositions in treatment of factor VIII -related diseases, disorders, and conditions. US 2004/126856 A1 relates to agents that inhibit the interaction of factor VIIIa with factor IXa in newly discovered regions of interaction such as region 2 and region 3. Nesheim *et al.* disclose the effect of plasma von Willebrand factor on the binding of human factor VIII to thrombin-activated human platelets ("The Effect of Plasma von Willebrand Factor on the binding of human Factor Vl11 to Thrombin-activated Human Platelets. ", Journal of Biological Chemistry, vol. 266, no. 27, September 25, pages 17815-17820). US 2004/248785 A1 relates to methods of increasing the half-life factor VIII. US 2009/118184 A1 relates to a recombinant factor VIII that includes one or more mutations that result in enhanced stability of both factor VIII and factor VIIIa.

### BRIEF SUMMARY OF THE INVENTION

The present invention is directed to a recombinant FVIII protein comprising: a first polypeptide comprising Formula I: (A1) - a1 - (A2) - a2 - [B]; and a second polypeptide comprising Formula II: a3 - (A3) - (C1); wherein the first polypeptide and the second polypeptide are fused or associated as a heterodimer; wherein, a) A1 is an A1 domain of FVIII; b) A2 is an A2 domain of FVIII; c) [B] is optionally present and is a B domain of FVIII, a fragment thereof; d) A3 is an A3 domain of FVIII; e) C1 is a C1 domain of FVIII; f) a1, a2, and a3 are acidic spacer regions; wherein one or more amino acids in a permissive loop-1 region in the A1 domain (A1-1)are substituted or deleted; and wherein the recombinant FVIII protein exhibits procoagulant activity and wherein a heterologous moiety is inserted in at least one of A1-1region of the recombinant FVIII protein and wherein the heterologous moiety is a polypeptide and wherein the one or more amino acid substituted or deleted in A1-1 comprise amino acids 19-22, amino acids 19-26 or amino acids 19-40, corresponding to SEQ ID NO: 1.

In another embodiment, the first polypeptide and the second polypeptide form a single polypeptide chain comprising the formula (A1) - a1 - (A2) - a2 - [B] - [a3] - (A3) - (C1). In other embodiments, the second polypeptide further comprises the formula [a3] - (A3) - (C1) - (C2), wherein (C2) is a C2 domain of FVIII.

In one aspect, A1-1 corresponds to a region in native mature human FVIII from about amino acid 15 to about amino acid 45 of SEQ ID NO:1. In another aspect, A1-1 corresponds to a region in native mature human FVIII from about amino acid 18 to about amino acid 41 of SEQ ID NO: 1. In other aspects, the one or more amino acids substituted or deleted are in A1-1. In still other aspects, the one or more amino acids substituted or deleted in A1-1 comprise amino acids 19 to 22, amino acids 19 to 26, amino acids 19 to 32, amino acids 19 to 40, amino acids 23 to 26, amino acids 23 to 32, amino acids 23 to 40, amino acids 27 to 32, amino acids 27 to 40, or amino acids 33 to 40 corresponding to native mature human FVIII. In yet other aspects, the recombinant FVIII protein having a substitution or deletion in A1-1 comprises a heterologous moiety inserted in at least one of A1-1, A1-2, A2-1, A2-2, A3-1, A3-2, or the a3 region.

In certain aspects, A1-2 corresponds to a region in native mature human FVIII from about amino acid 201 to about amino acid 232 of SEQ ID NO: 1. In other aspects, A1-2 corresponds to a region in native mature human FVIII from about amino acid 218 to about amino acid 229 of SEQ ID NO: 1. In some aspects, the one or more amino acids substituted or deleted in the FVIII protein are in A1-2. In other aspects, the recombinant FVIII protein having a deletion in A1-2 comprises a heterologous moiety inserted in at least one of A1-1, A1-2, A2-1, A2-2, A3-1, A3-2, or the a3 region.

In some aspects, A2-1 corresponds to a region in native mature human FVIII from about amino acid 395 to about amino acid 421 of SEQ ID NO: 1. In other aspects, A2-1 corresponds to a region in native mature human FVIII from about amino acid 397 to about amino acid 418 of SEQ ID NO: 1. In certain aspects the one or more amino acids substituted or deleted in the FVIII protein are in A2-1. In certain aspects, the one or more amino acids substituted or deleted in A2-1 comprise amino acids 400 to 403 corresponding to native mature human FVIII in A2-1. In other aspects, the recombinant FVIII protein having a deletion in A1-2 comprises a heterologous moiety inserted in at least one of A1-1, A1-2, A2-1, A2-2, A3-1, A3-2, or the a3 region. For example, a heterologous moiety can be inserted immediately downstream of amino acid 399 corresponding to native human FVIII in A2-1.

In certain aspects A2-2 corresponds to a region in native mature human FVIII from about amino acid 577 to about amino acid 635 of SEQ ID NO: 1. In some aspects, A2-2 corresponds to a region in native mature human FVIII from about amino acid 595 to about amino acid 607 of SEQ ID NO: 1. In other aspects, the one or more amino acids substituted or deleted in the FVIII protein are in A2-2. In certain aspects the recombinant FVIII protein having a deletion in A1-2 comprises a heterologous moiety inserted in at least one of A1-1, A1-2, A2-1, A2-2, A3-1, A3-2, or the a3 region.

In certain aspects A3-1 corresponds to a region in native mature human FVIII from about amino acid 1705 to about amino acid 1732 of SEQ ID NO:1. In some aspects, A3-1 corresponds to a region in native mature human FVIII from about amino acid 1711 to about amino acid 1725 of SEQ ID NO:1. In other aspects, the one or more amino acids substituted or deleted in a FVIII protein are in A3-1. In still other aspects, the one or more amino acids substituted or deleted in A3-1 comprise amino acids 1712 to 1720, amino acids 1712 to 1725, or amino acids 1721 to 1725 corresponding to native mature human FVIII. In yet other aspects, a recombinant FVIII protein having a deletion in A2-1 comprises a heterologous moiety inserted in at least one of A1-1, A1-2, A2-1, A2-2, A3-1, A3-2, or the a3 region. In some aspects, the heterologous moiety is inserted immediately downstream of amino acid 1711 or amino acids 1720 corresponding to native human FVIII in A3-1.

In other aspects, A3-2 corresponds to a region in native mature human FVIII from about amino acid 1884 to about amino acid 1917 of SEQ ID NO:1. In some aspects, A3-2 corresponds to a region in native mature human FVIII from about amino acid 1899 to about amino acid 1911 of SEQ ID NO: 1. It is disclosed that the one or more amino acids substituted or deleted in the FVII protein are in A3-2. It is disclosed that the one or more amino acids substituted or deleted in A3-2 comprise amino acids 1901 to 1905, amino acids 1901 to 1910, amino acids 1906 to 1910, amino acids 1901 to 1905, amino acids 1901 to 1910, or amino acids 1906 to 1910 corresponding to native mature human FVIII. In other embodiments, a recombinant FVIII protein having a deletion in A3-2 comprises a heterologous moiety inserted in at least one of A1-1, A1-2, A2-1, A2-2, A3-1, A3-2, or the a3 region. In some embodiments, the heterologous moiety is inserted immediately downstream of amino acid 1900 or amino acid 1905 corresponding to native human FVIII in A3-2.

In certain aspects, the one or more amino acids substituted or deleted in the FVIII protein are in the a3 region. In some aspects, the one or more amino acids substituted or deleted in the a3 region comprise amino acids 1649 to 1689 corresponding to native mature human FVIII. In other aspects, a recombinant FVIII protein having a deletion or substitution in the a3 region comprises a heterologous moiety is inserted in at least one of A1-1, A1-2, A2-1, A2-2, A3-1, A3-2, or the a3 region. In some aspects, the heterologous moiety is inserted immediately downstream of amino acid 1645 corresponding to native mature human FVIII.

In certain aspects, one or more of the A1-1, A1-2, A2-1, A2-2, A3-1, A3-2, and a3 regions are completely substituted or deleted and a heterologous moiety is inserted at the point of deletion. In other aspects, one or more of the A1-1, A1-2, A2-1, A2-2, A3-1, A3-2, and a3 regions are completely replaced by one or more heterologous moiety, e.g., XTENs. In one particular aspect, one or more of the regions A1-1, A1-2, A2-1, A2-2, A3-1, A3-2, and a3 are completely replaced by one or more heterologous moieties. In another aspect, one or more of the A1-1, A1-2, A2-1, A2-2, A3-1, A3-2, and a3 regions are deleted, and a heterologous moiety is inserted immediately downstream of the amino acid immediately upstream of the deletion and immediately upstream of the amino acid immediately downstream of the deletion.

In certain aspects, the recombinant FVIII protein of the present invention can have all or part of the B domain deleted. In other aspects, the one or more amino acids substituted or deleted in the FVIII protein are in the B domain. In some aspects, the one or more amino acids substituted or deleted in the B domain comprise from about amino acids 741 to about amino acid 1648 corresponding to native mature human FVIII. In one particular aspect, the B-domain is deleted, having an amino acid sequence of SEQ ID NO:2. In other aspects, a recombinant FVIII protein having a deletion or substitution in the B domain comprises a heterologous moiety inserted in at least one of A1-1, A1-2, A2-1, A2-2, A3-1, A3-2, or the a3 region.

In certain aspects, the one or more amino acids in at least two, at least three, at least four, at least five, at least six, or seven of the regions of A1-1, A1-2, A2-1, A2-2, A3-1, A3-2, and the a3 region are substituted or deleted.

The invention also includes a fusion protein, a nucleic acid encoding the recombinant FVIII protein or the fusion protein, a vector comprising the nucleic acid, a host cell comprising the vector, a composition comprising the fusion protein, the recombinant FVIII protein, the nucleic acid, the vector, or the host cell.

The invention further includes, a method of preventing, treating, ameliorating, or managing a clotting disease or condition in a patient in need thereof.

### BRIEF DESCRIPTION OF THE DRAWINGS/FIGURES

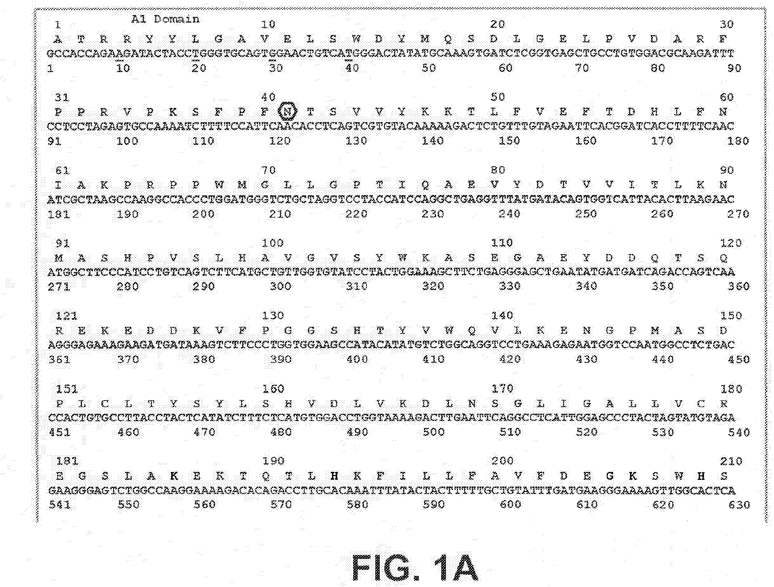

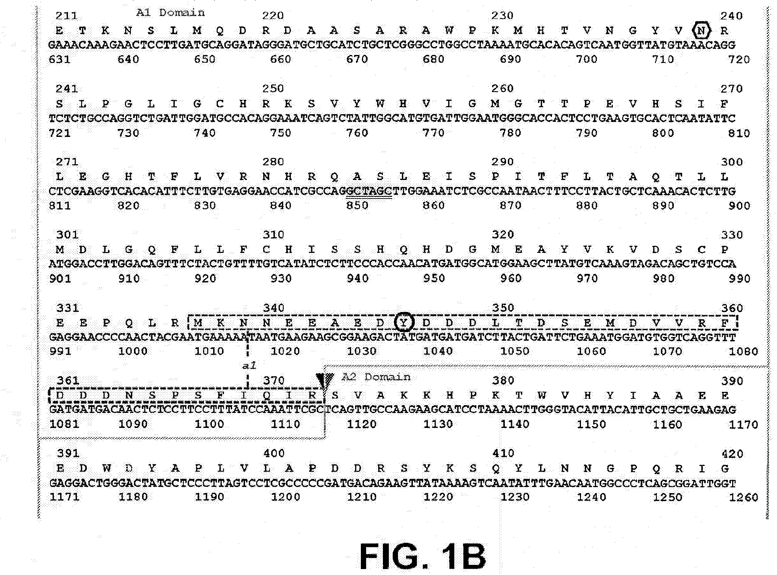

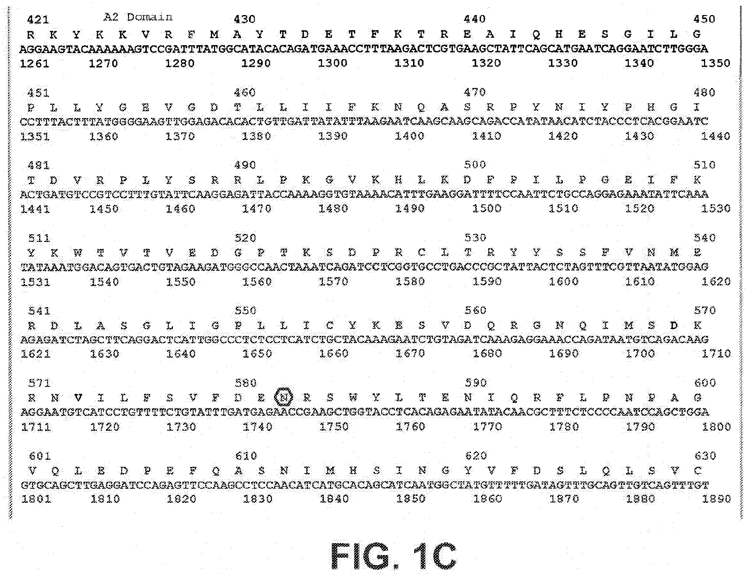

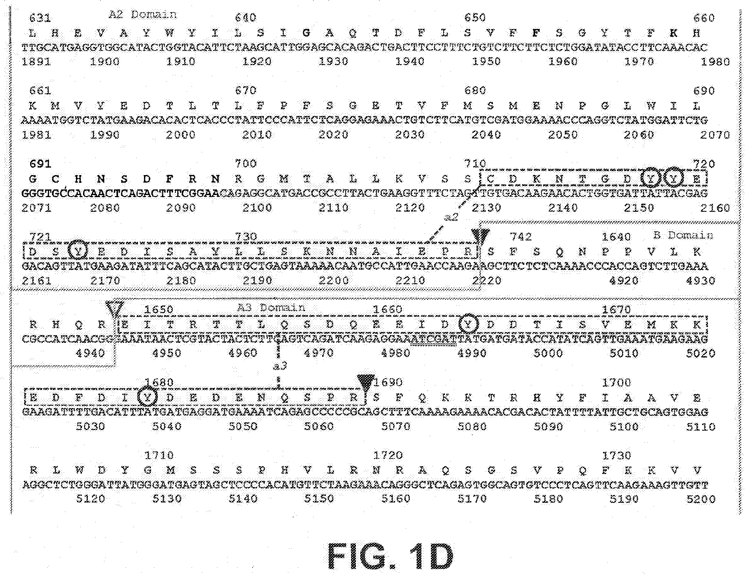

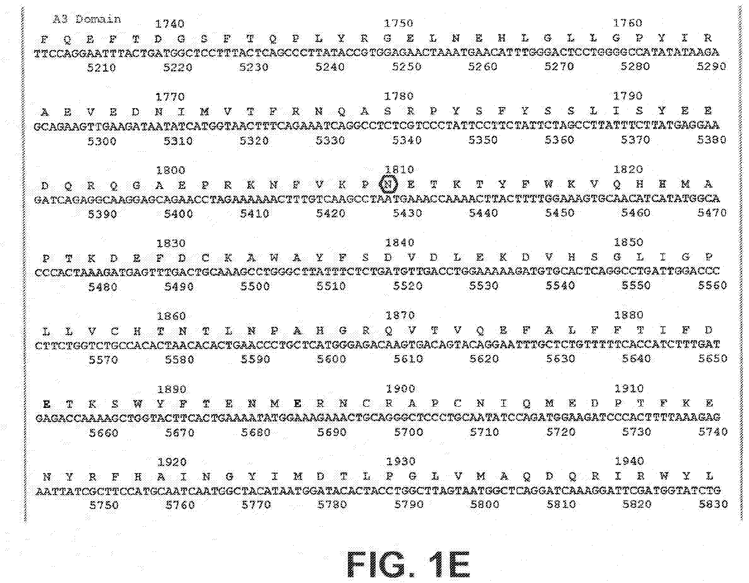

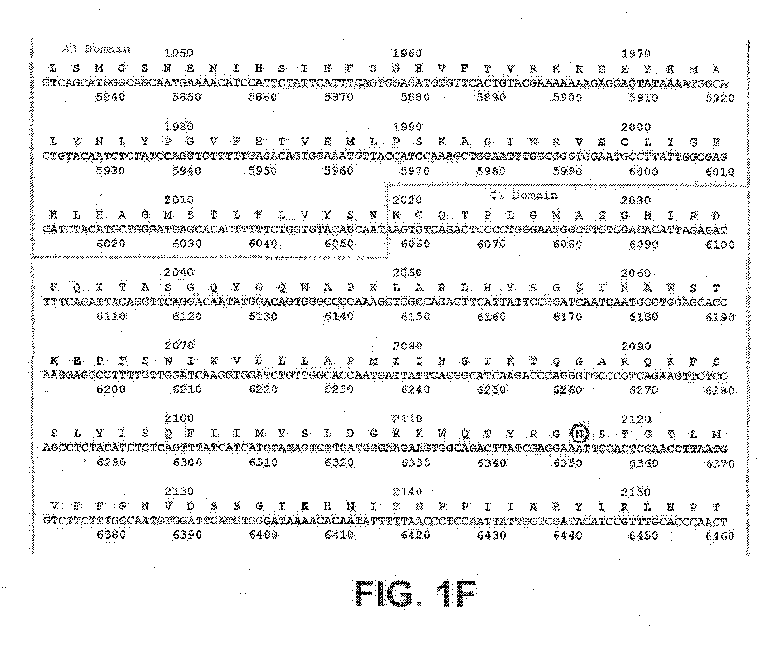

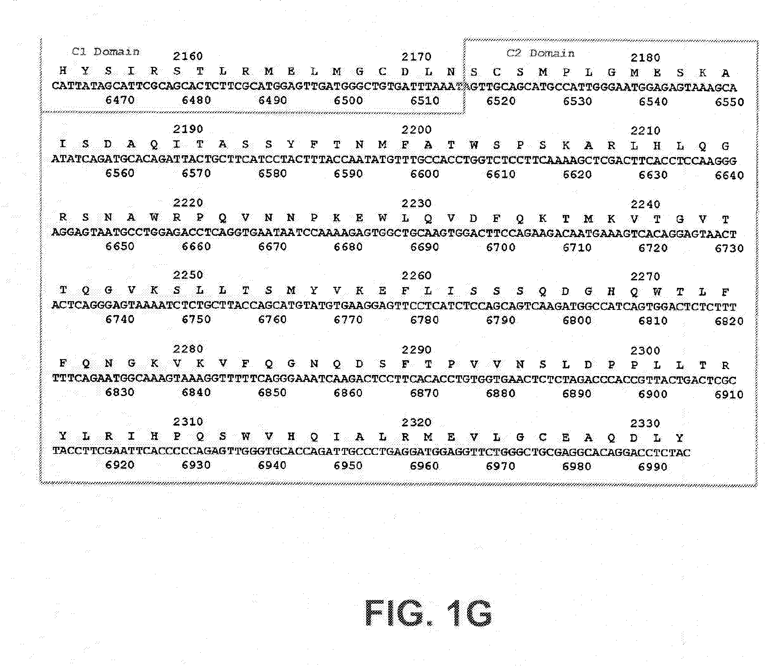

FIG. 1 (panels 1A to 1G) depicts the primary sequence and domain structure of mature B-domain deleted (BDD) human FVIII construct, presented as SEQ ID NO:2. The location of the introduced *NheI* and *ClaI* restriction sites is shown. Note that the amino acid numbering corresponds to the amino acid positions in the primary sequence of mature FVIII (SEQ ID NO: 1). Individual domains are bounded by gray lines/boxes with domain identification in gray text. Acidic regions (a1, a2, a3) are indicated with dashed boxes. Solid wedges/triangles indicate sites of thrombin cleavage in the activation of FVIII to FVIIIa. Unfilled wedges/triangle indicates the site of intracellular proteolytic processing to the two-chained form of FVIII. Hexagons indicate sites of N-linked glycosylation. Circles indicate sites of Tyr sulfation. Unique non-native restriction sites (*NheI,* gctagc; *ClaI,* atcgat) introduced into cDNA to facilitate XTEN insertion/recombination are highlighted in gray with double underline.

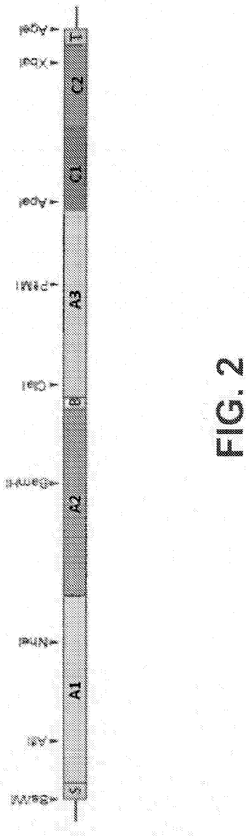

FIG. 2 provides graphical representation of the FVIII construct described in FIG. 1, indicating the domain organization and the location of native and non-native restriction sites.

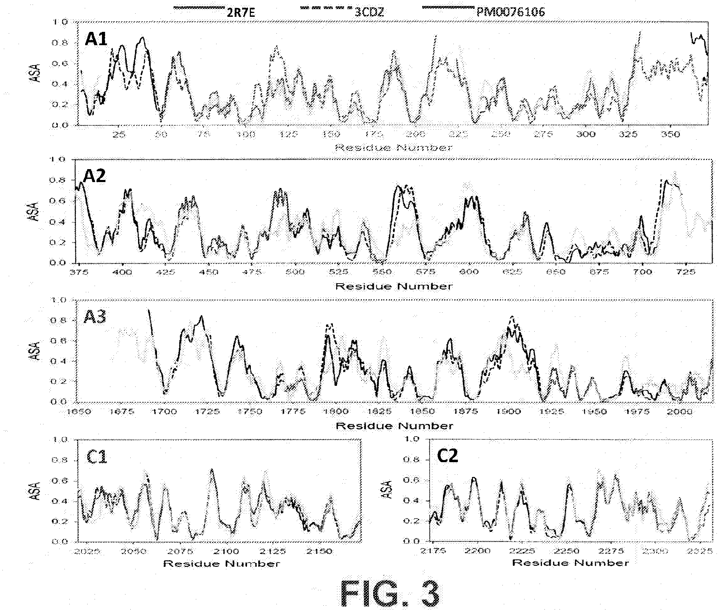

FIG. 3 shows the graphical ASAView outputs for structural datasets 2R7E, 3CDZ, and PM0076106. Solvent Accessible Surface Areas (ASA) for the amino acids in domains A1, A2, A3, C1 and C2 are shown.

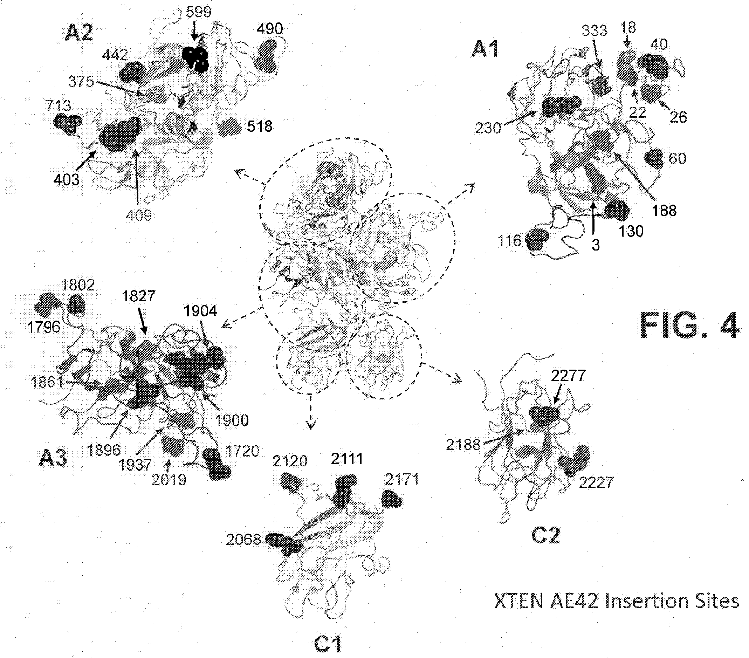

FIG. 4 shows a structural representation of the location of XTEN AE42 insertion sites. The central drawing corresponding to the crystal structure of FVIII (PDB: 2R7E) is surrounded by detailed view of domains A1, A2, A3, C1 and C2. Beta strands and alpha helices are shown as ribbon representation. Loops are shown as alpha carbon pipes. The amino acids at insertion sites are shown as CPK sphere representation. The number in each graph indicates the location of the insertion sites according to the numbering in FIG. 1.

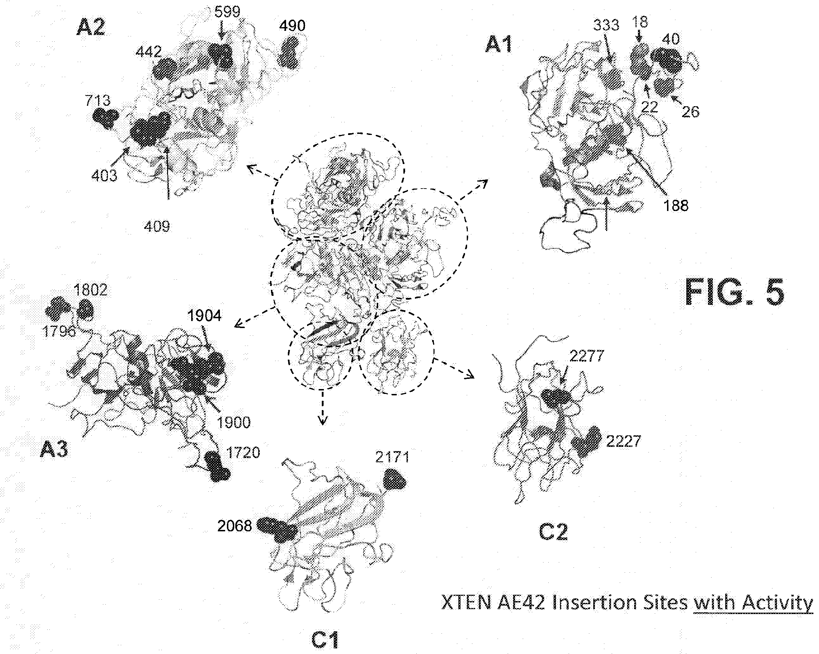

FIG. 5 shows a structural representation of the location of insertion sites shown in FIG. 4 wherein the resulting recombinant FVIII protein displays FVIII activity.

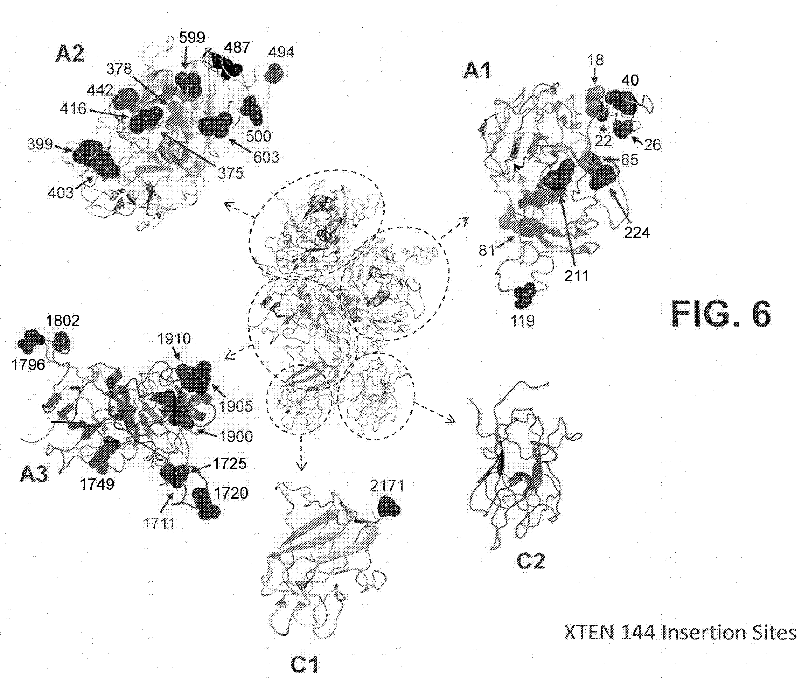

FIG. 6 shows a structural representation of the location of XTEN 144 insertion sites.

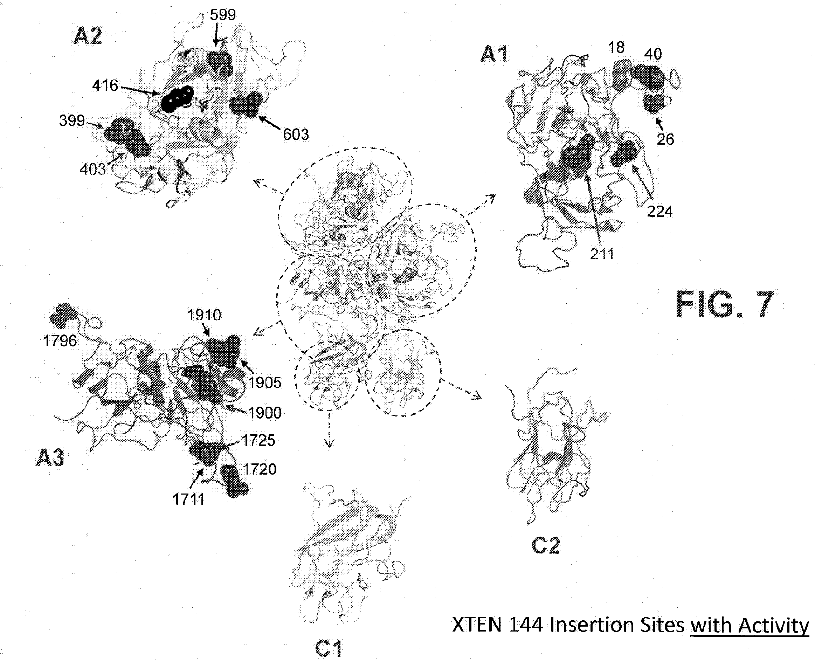

FIG. 7 shows a structural representation of the location of insertion sites shown in FIG. 6 wherein the resulting recombinant FVIII protein displays FVIII activity.

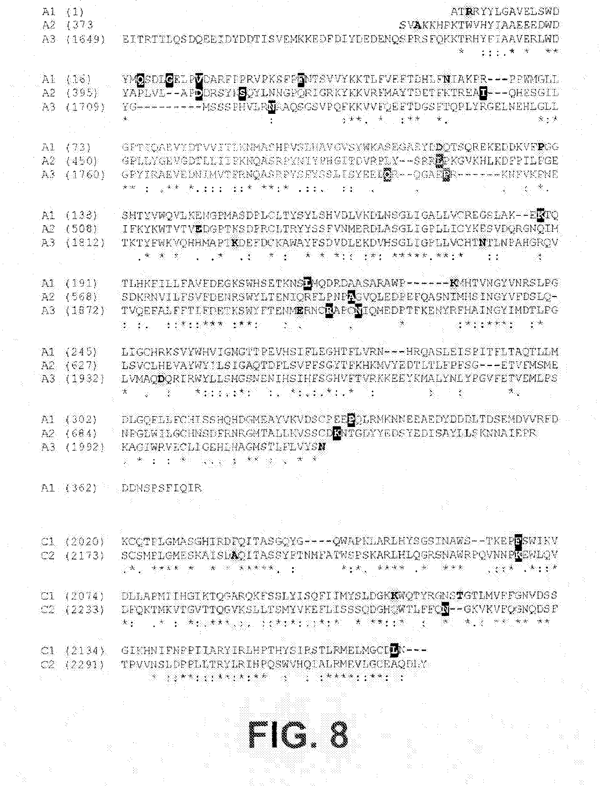

FIG. 8 shows a ClustalW multiple sequence alignment of domains A1, A2, A3, C1 and C2 of FVIII showing the location of XTEN AE42 insertions resulting in recombinant FVIII proteins displaying FVIII activity (black box, white text) or displaying no FVIII activity (grey box, bold text).

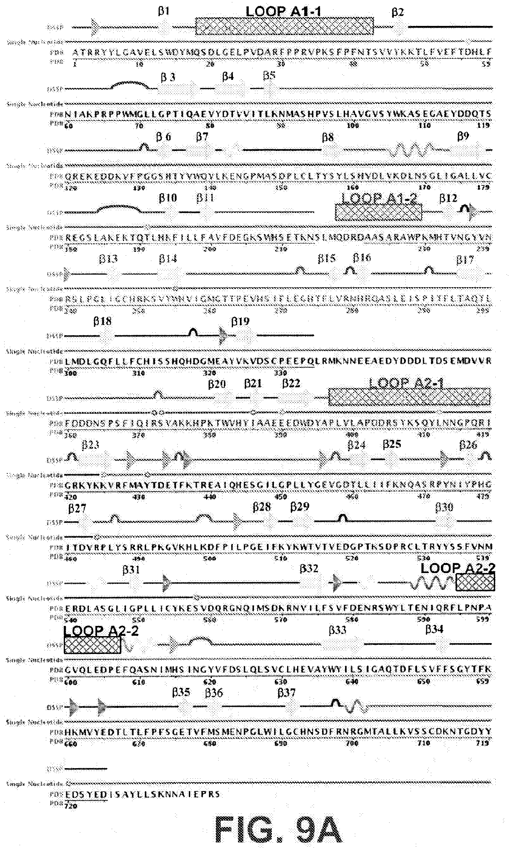

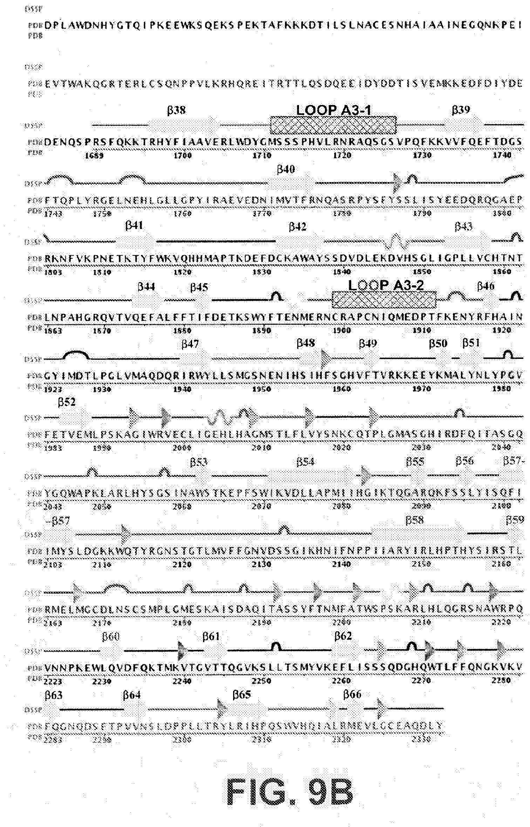

FIG. 9 (panels 9A and 9B) shows a DSSP graphical representation of the secondary structure of the two polypeptide chains in a native active human FVIII crystal structure deposited under the identifier 2R7E at the Protein Data Bank. Amino acid sequence numbering is the same as in the protein sequence in FIG. 1 and in SEQ ID NO: 1. The beta sheet regions are shown as filled arrows and are designated β1 to β66. The location of the permissive loops is denoted by crosshatched boxes. Domain A1 permissive loops are designated Loop A1-1 and Loop A1-2. Domain A2 permissive loops are designated Loop A2-1 and Loop A2-2. Domain A3 permissive loops are designated Loop A3-1 and Loop A3-2.

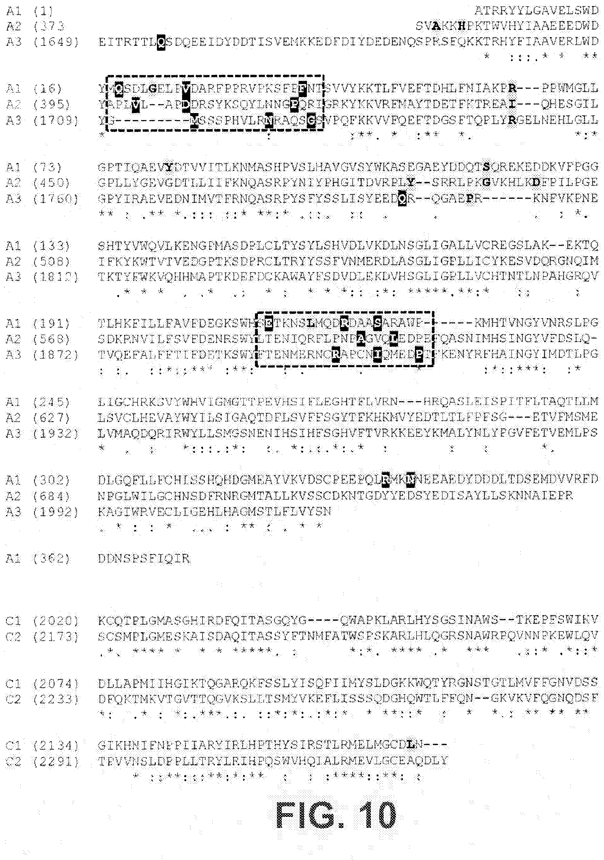

FIG. 10 shows a ClustalW multiple sequence alignment of domains A1, A2, A3, C1 and C2 of FVIII showing the location of XTEN 144 insertions resulting in recombinant FVIII proteins displaying FVIII activity (black box, white text) or displaying no FVIII activity (grey box, bold text). The locations of the permissive loops are indicated by dashed rectangles.

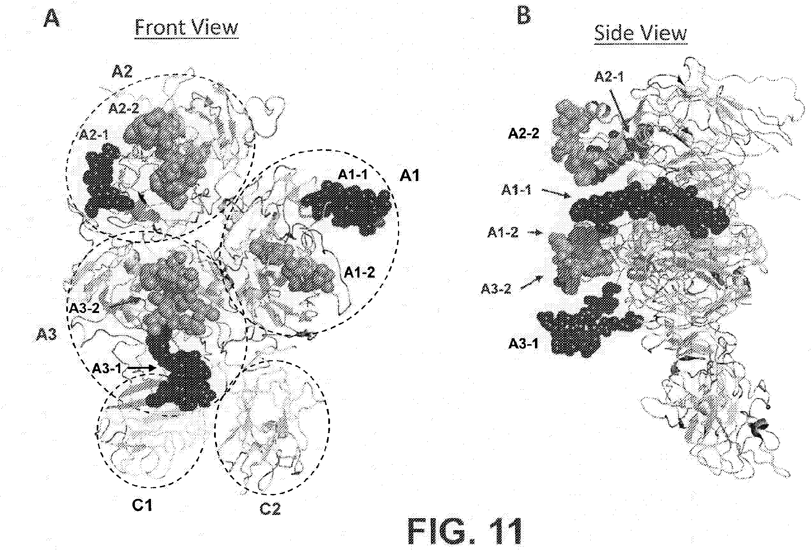

FIG. 11A presents a front view structural representation of human FVIII (PDB:2R7E) showing the location of domains A1, A2, A3, C1 and C2 (circled in dashed lined) and the locations of permissive loops A1-1, A1-2, A2-1, A2-2, A3-1 and A3-2 highlighted as CPK sphere representations.
FIG. 11B presents a side view structural representation of human FVIII (PDB:2R7E) showing the location of domains A1, A2, A3, C1 and C2 (circled in dashed lined) and the locations of permissive loops A1-1, A1-2, A2-1, A2-2, A3-1 and A3-2 highlighted as CPK sphere representations.

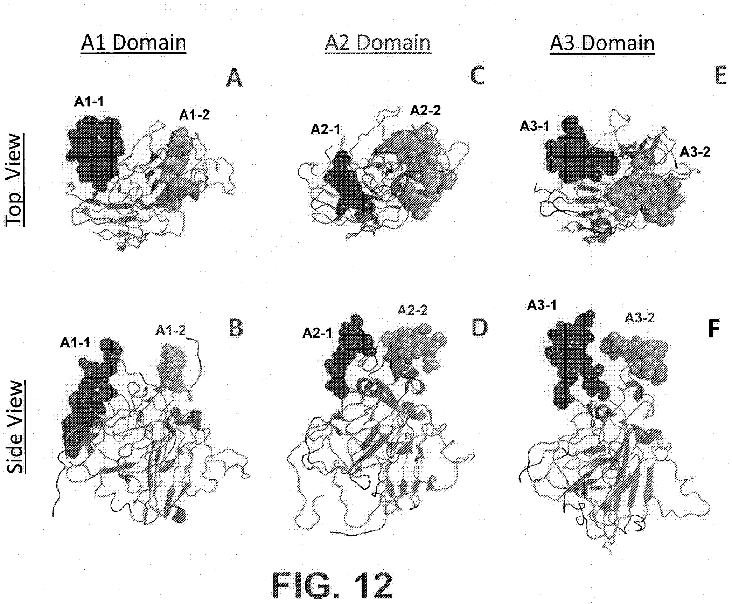

FIG. 12A, 12C and 12E show top view structural representations of isolated human FVIII (PDB:2R7E) A domains showing the location of permissive loops highlighted as CPK sphere representations. FIG. 12B, 12D and 12F show side view structural representations of isolated human FVIII (PDB:2R7E) A domains showing the location of permissive loops highlighted as CPK sphere representations.

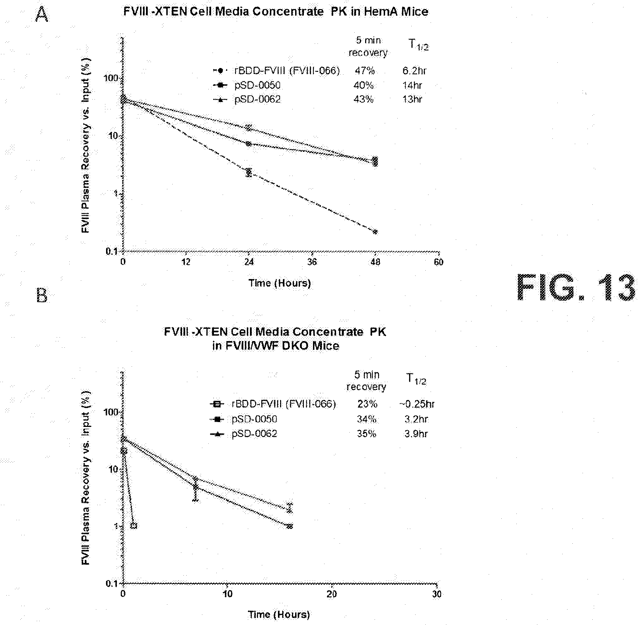

FIG. 13 shows the PK profile of two FVIII variants with intra domain insertions (pSD0050 and pSD0062, see TABLE 3) compared with B domain-deleted (BDD)-FVIII using a cell culture PK assay in HemA mice (FIG. 13, panel A) and FVIII/vWF double knock out (DKO) mice (FIG. 13, panel B). Five-minute recovery, and half-life (t_{1/2}) are shown.

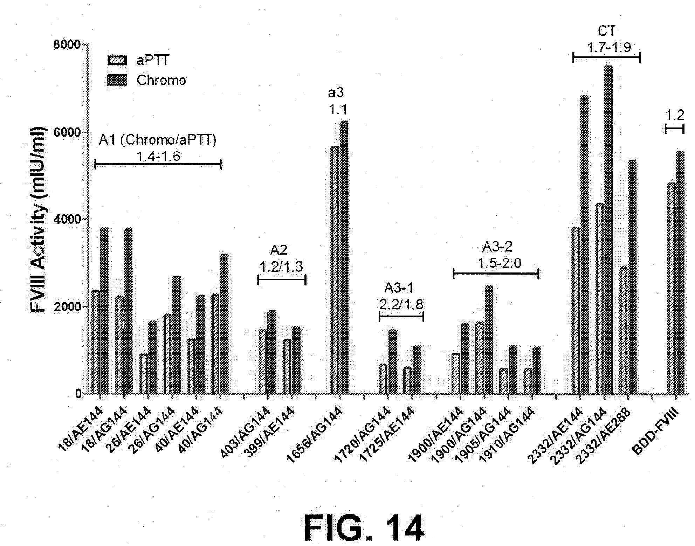

FIG. 14 is a bar graph of chromogenic and aPTT assay activity data of various FVIII variants with single XTEN insertions. The data presented correspond to single insertions of XTENs, e.g., AE144, AG144 or AE288, in permissive loop A1-1 (amino acid 18, 26 or 40 corresponding to SEQ ID NO: 1), permissive loop A2-1 (amino acid 403 or 399 corresponding to SEQ ID NO: 1), a3 region (amino acid 1656 corresponding to SEQ ID NO: 1), permissive loop A3-1 (amino acid 1720 or 1725 corresponding to SEQ ID NO: 1), permissive loop A3-2 (amino acid 1900, 1905 or 1910 corresponding to SEQ ID NO: 1), or the carboxy terminus (CT; amino acid 2332 corresponding to SEQ ID NO: 1). Also shown are aPTT and chromogenic activity assay activity data for BDD-FVII control. Also indicated in the drawing as the ratios or range of ratios *(e.g.,* 1.1 for a3 or the 1.4-1.6 range for A1-1 insertions) between the activity as determined by the chromogenic assay and the activity as detected by the aPTT assay (Chromo/aPTT ratio).

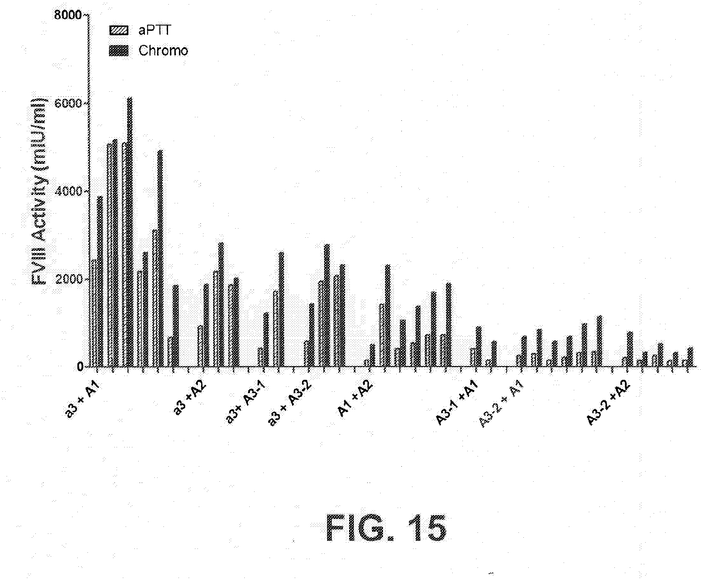

FIG. 15 is a bar graph of chromogenic and aPTT assay activity data of various FVIII variants with two XTEN insertions. The data presented correspond to double insertions of XTENs in permissive loop A1-1 and a3 region, permissive loop A2-1 and a3 region, permissive loop A3-1 and a3 region, permissive loop A3-2 and a3 region, permissive loops A1-1 and A2-1, permissive loops A1-1 and A3-1, permissive loops A1-1 and A3-2, and permissive loops A2-1 and A3-2, respectively.

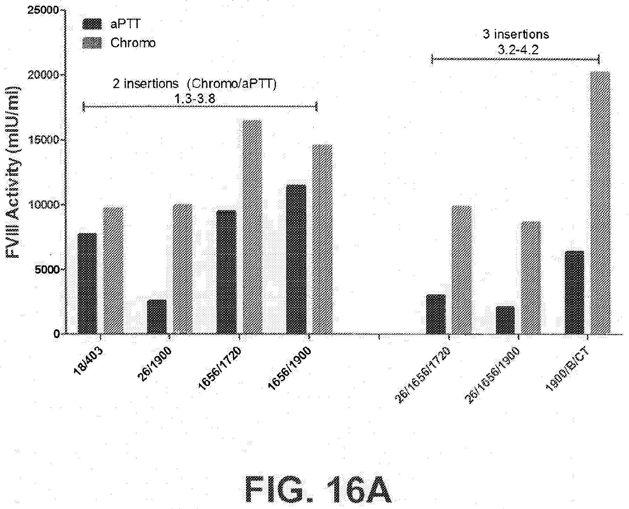

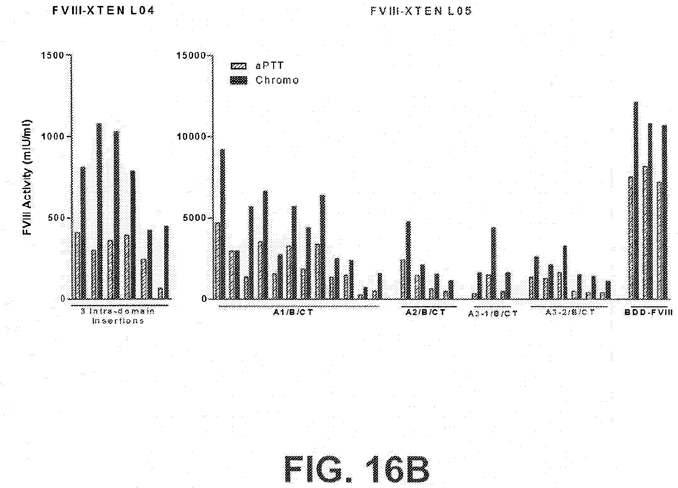

FIGS. 16A and 16B are bar graphs of chromogenic and aPTT assay activity data of various FVIII variants with two or three XTEN insertions. FIG. 16A presents data corresponding to double or triple insertions of XTENs in permissive loop A1-1 (amino acid 18 or 26 corresponding to SEQ ID NO: 1), permissive loop A2-1 (amino acid 403 corresponding to SEQ ID NO: 1), a3 region (amino acid 1656 corresponding to SEQ ID NO: 1), permissive loop A3-1 (amino acid 1720 corresponding to SEQ ID NO: 1), permissive loop A3-2 (amino acid 1900 corresponding to SEQ ID NO: 1), or the carboxy terminus (CT; amino acid 2332 corresponding to SEQ ID NO: 1). The graph also shows data corresponding to a construct with XTEN inserted at position 1900, the B domain, and the CT. Also indicated in the drawing as the ratios or range of ratios *(e.g.,* 3.2-4.2 for 3 XTEN insertions) between the activity as determined by the chromogenic assay and the activity as detected by the aPTT assay (Chromo/aPTT ratio).
FIG. 16B presents data corresponding to triple insertions of XTENs in permissive loops. The constructs shown in the left panel graph (left to right) correspond to insertions in amino acids 26, 403, and 1656; 26, 1656, and 1720; 26, 1656, and 1900; 403, 1656, and 1720; 403, 1656, and 1900; and, 1656, 1720 and 1900 corresponding to SEQ ID NO: 1, respectively. The constructs shown in the right panel graph correspond to three XTEN insertion constructs with one XTEN inserted in permissive loop A1-1, permissive loop A2-1, permissive loop A3-1, or permissive loop A3-2, and two XTEN non-permissive loop insertions, namely a second XTEN insertion in the B domain, and a third XTEN insertion in the carboxy terminus (CT). Also shown are aPTT and chromogenic activity assay data for BDD-FVII control.

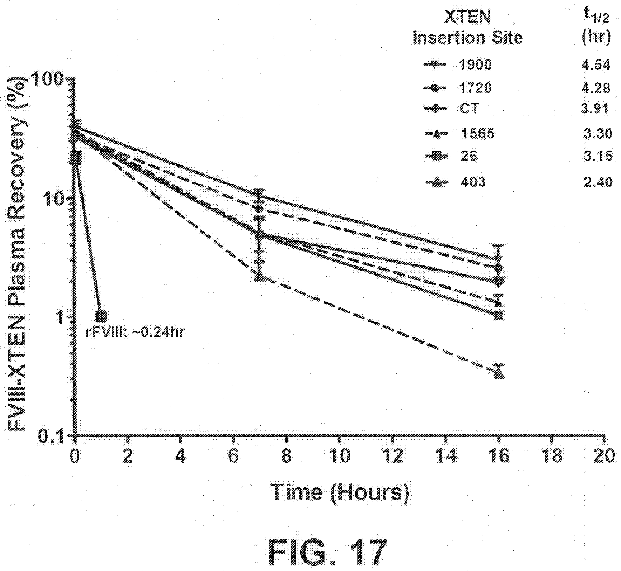

FIG. 17 shows plasma levels in DKO mice of various administered FVIII variants with single XTEN insertions compared to a BDD-FVIII control. The XTEN were inserted at amino acid 26, 403, 1565, 1720, 1900 or the carboxy terminus (CT) corresponding to SEQ ID NO: 1.

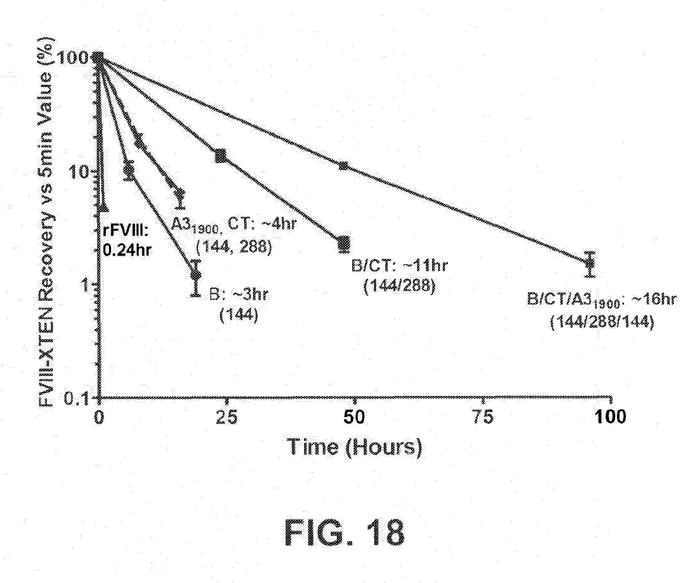

FIG. 18 shows plasma levels in DKO mice of various administered FVIII variants with one insertion (XTEN144 in B domain), two insertions (XTEN144 in A3-2 permissive loop at amino acid 1900 corresponding to SEQ ID NO: 1 and XTEN288 in carboxy terminus; or, XTEN144 in B domain and XTEN288 in carboxy terminus) and three XTEN insertions (XTEN144 in B domain, XTEN288 in carboxy terminus and XTEN144 in A3-2 permissive loop at amino acid 1900 corresponding to SEQ ID NO: 1) compared to a BDD-FVIII control (rFVIII).

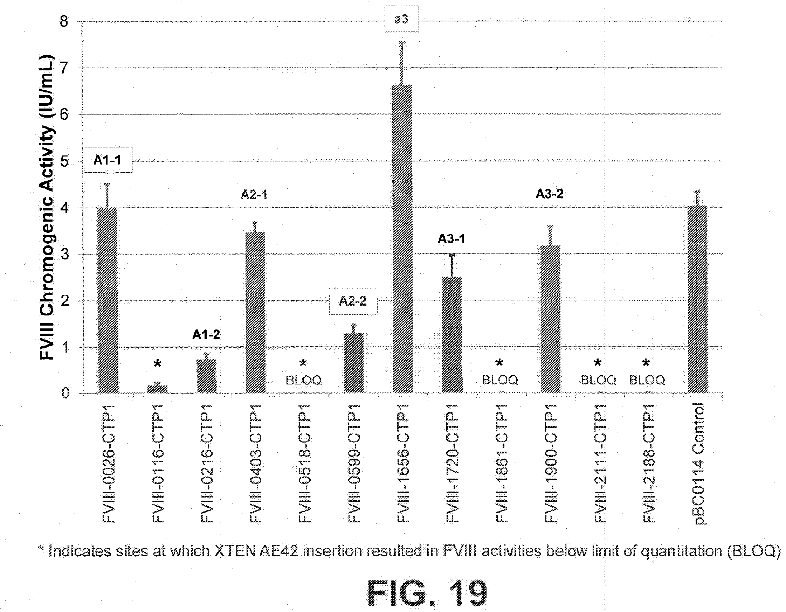

FIG. 19 shows a bar graph of chromogenic activity data of various FVIII variants with single CTP1 insertions. The data presented correspond to single insertions of a 45 amino acid long peptide encompassing a 29 amino acid long peptide derived from the carboxy terminus of human chorionic gonadotropin (CTP1, SEQ ID NO:81) at different locations in FVIII. The numeral in the construct designation shown in the x-axis corresponds to the amino acid position immediately after which the peptide is inserted. Permissive loop (and a3 region) locations of the insertions are indicated above the bars. Also shown is chromogenic activity assay data for a FVIII control.

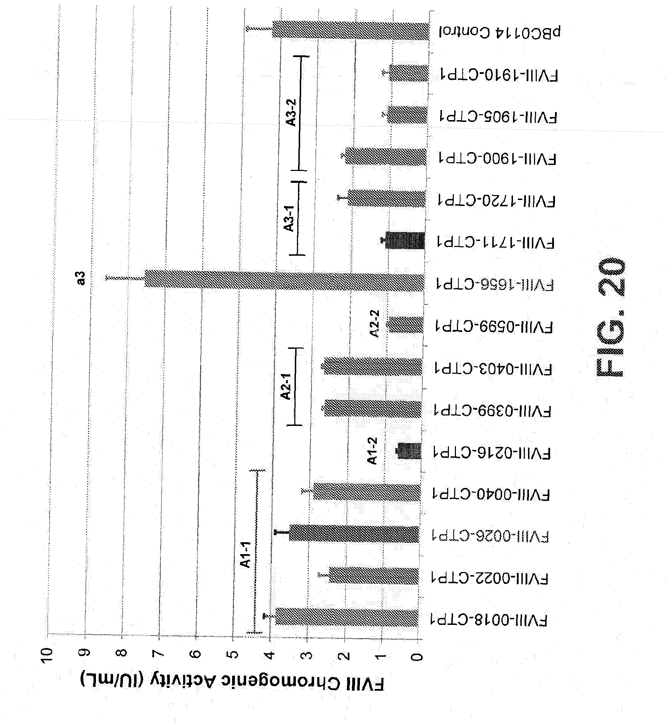

FIG. 20 shows a bar graph of chromogenic activity data of various FVIII variants with single CTP1 insertions. The data presented correspond to single insertions of a 45 amino acid long peptide encompassing a 29 amino acid long peptide derived from the carboxy terminus of human chorionic gonadotropin (CTP1, SEQ ID NO:81) at different locations in FVIII. The numeral in the construct designation shown in the x-axis corresponds to the amino acid position immediately after which the peptide is inserted. Permissive loop (and a3 region) locations of the insertions are indicated above the bars. Also shown is chromogenic activity assay data for a FVIII control.

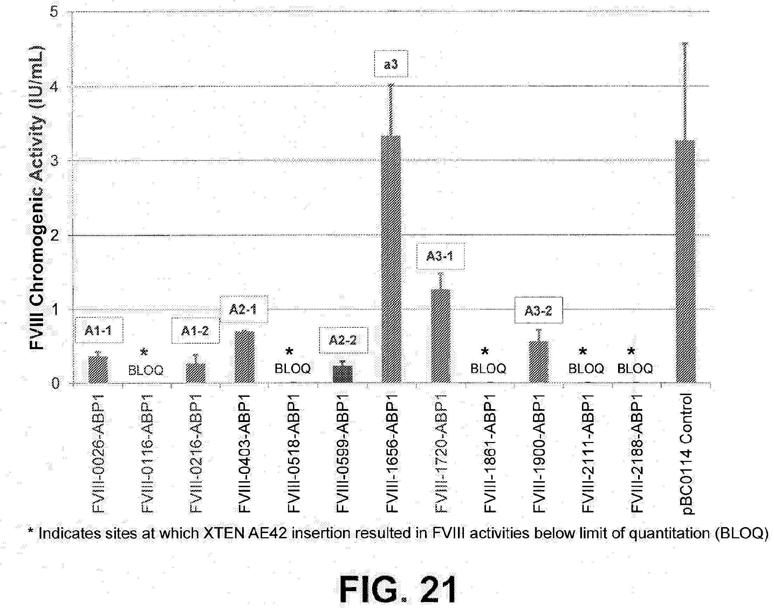

FIG. 21 shows a bar graph of chromogenic activity data of various FVIII variants with single albumin-binding peptide (ABP1, SEQ ID NO: 83) insertions. The data presented correspond to single insertions of a 44 amino acid long peptide encompassing an 18 amino acid long ABP1, SEQ ID NO: 83 at different locations in FVIII. The numeral in the construct designation shown in the x-axis corresponds to the amino acid position after which the peptide is inserted. Permissive loop (and a3 region) locations of the insertions are indicated above the bars. Also shown is chromogenic activity assay data for a FVIII control.

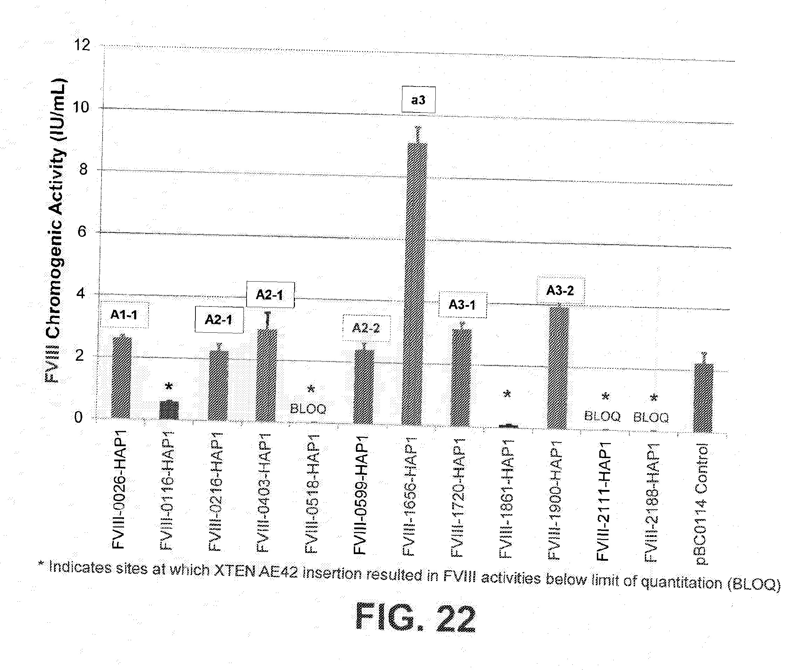

FIG. 22 shows a bar graph of chromogenic activity data of various FVIII variants with single Gly-Ser repeat (HAP1, SEQ ID NO: 85) insertions. The data presented correspond to single insertions of a 41 amino acid long peptide encompassing a 35 amino acid HAP1 at different locations in FVIII. The numeral in the construct designation shown in the x-axis corresponds to the amino acid position after which the peptide is inserted. Permissive loop (and a3 region) locations of the insertions are indicated above the bars. Also shown is chromogenic activity assay activity data for a FVIII control.

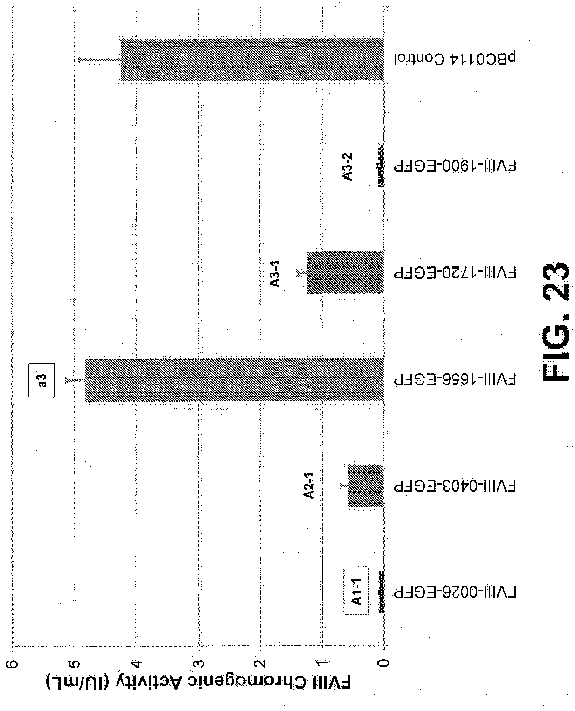

FIG. 23 shows a bar graph of chromogenic activity data of various FVIII variants with single enhanced green fluorescent protein (EGFP1, SEQ ID NO: 87) insertions. The data presented correspond to single insertions of a 265 amino acid long polypeptide encompassing the 239 amino acid residue sequence of EGFP1 flanked by two tandem repeats of the amino acid sequence Gly-Gly-Gly-Gly-Ser (SEQ ID NO:191) at different locations in FVIII. The numeral in the construct designation shown in the x-axis corresponds to the amino acid position after which the peptide is inserted. Permissive loop (and a3 region) locations of the insertions are indicated above the bars. Also shown is chromogenic activity assay data for a FVIII control.

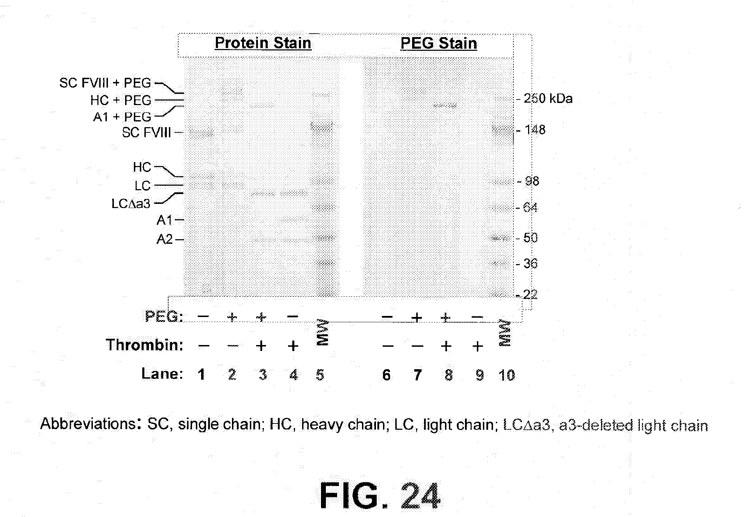

FIG. 24 shows protein-specific (left panel) and PEG-specific staining (right panel) of an SDS-PAGE gel of purified FVIII variant FVIII-0026-CCP1 before and after chemical PEGylation, and with and without thrombin treatment. FVIII-0026-CCP1 is a variant in which a cysteine-containing peptide (CCP1; SEQ ID NO: 90) is inserted immediately after residue 26.

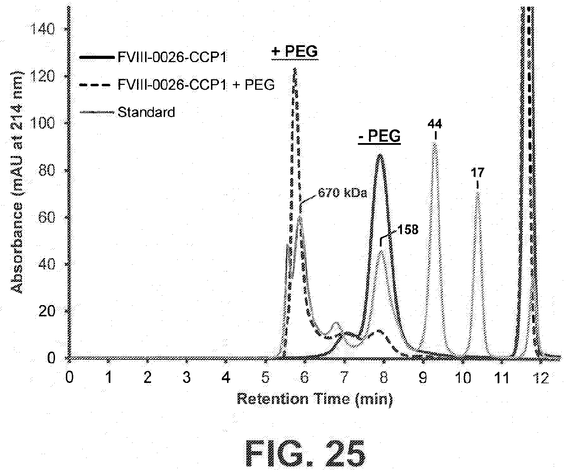

FIG. 25 shows overlaid chromatograms of FVIII-0026-CCP1 (solid black trace) and PEGylated FVIII-0026-CCP1 (dashed black trace) resolved by size-exclusion chromatography on a Tosoh G3000 SWxl column with UV monitoring at 214 nm. The elution profiles of molecular weight standards (gray trace) are overlaid with molecular weights of components indicated in units of kilodaltons (kDa)

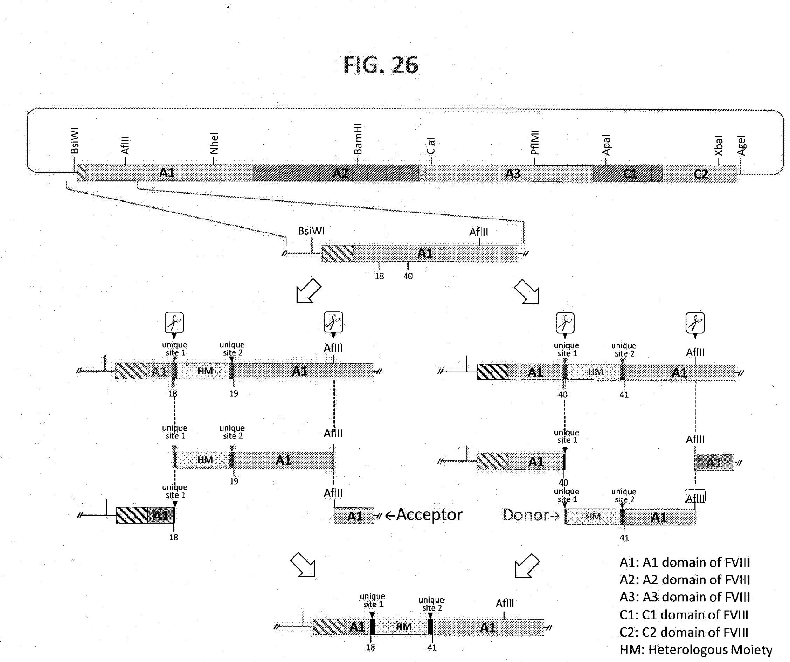

FIG. 26 shows a schematic diagram of a method used to replace all or part of a permissive loop in FVIII with a heterologous moiety utilizing two plasmids each with an insertion in the same loop. In this example, residues 19 through 40 in FVIII permissive loop A1-1 are replaced with a heterologous moiety. A first plasmid (acceptor) contains a heterologous moiety flanked by unique restriction sites immediately after the site corresponding to residue 18 of FVIII. A second plasmid (donor) contains the same heterologous moiety immediately after the site corresponding to residue 40 of native mature human FVIII. Both plasmids can then be digested with restriction enzymes, one of which being the unique restriction site that is nearest to the A1-1 loop in the 3' direction. The linearized plasmid from the digestion of the first plasmid (Acceptor) and the insert from the digestion of the second plasmid (Donor) can then be ligated together to generate a third plasmid in which the segment encoding FVIII residues 19-40 is replaced by the heterologous moiety sequence flanked by the restriction sites. Examples of the restriction sites are shown in Table 26.

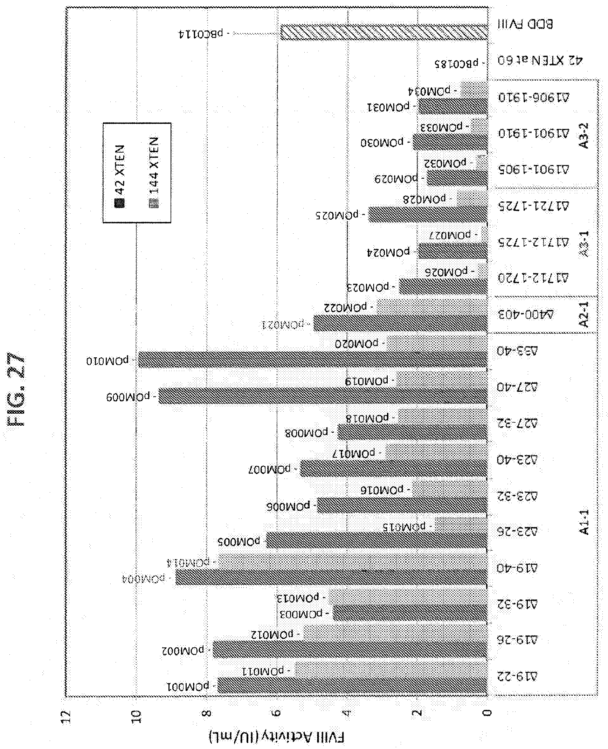

Fig. 27 shows a bar graph of activity data of various FVIII proteins with XTEN permissive loop replacements. The data presented correspond to FVIII proteins in which permissive loop sequences were replaced with 42 (dark grey bars) or 144 (light grey bars) amino acid XTEN sequences. The amino acid sequence that was replaced in each construct is indicated along the x-axis and denoted with delta (Δ), and the corresponding permissive loop is indicated below as A1-1, A2-1, A3-1, or A3-2. The corresponding DNA constructs, which encode the FVIII proteins, are indicated above each bar. Transfection with a negative control plasmid, pBC0185, which encodes a FVIII protein in which a 42-residue XTEN was inserted after residue 60, yielded no activity. Transfection with a positive control plasmid, pBC0114, which encodes B domain-deleted (BDD) FVIII bearing a carboxy terminal epitope tag but no XTEN insertion, was repeated in triplicate, and the mean FVIII activity +/- standard deviation is shown with the hatched bar.

### DETAILED DESCRIPTION OF THE INVENTION

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein.

The invention is directed to certain recombinant FVIII proteins with improved properties, e.g., improved half-life or improved stability, which have the procoagulant activity of FVIII and can be expressed in host cells. Such recombinant FVIII proteins can be used, *e.g.,* as a therapeutic treatment for hemophilia.

The term "polynucleotide" or "nucleotide" is intended to encompass a singular nucleic acid as well as plural nucleic acids, and refers to an isolated nucleic acid molecule or construct, e.g., messenger RNA (mRNA) or plasmid DNA (pDNA). In certain aspects, a polynucleotide comprises a conventional phosphodiester bond or a non-conventional bond *(e.g.,* an amide bond, such as found in peptide nucleic acids (PNA)).

The term "nucleic acid" refers to any one or more nucleic acid segments, e.g., DNA or RNA fragments, present in a polynucleotide. By "isolated" nucleic acid or polynucleotide is intended a nucleic acid molecule, DNA or RNA, which has been removed from its native environment. For example, a recombinant polynucleotide encoding a FVIII polypeptide contained in a vector is considered isolated for the purposes of the present invention. Further examples of an isolated polynucleotide include recombinant polynucleotides maintained in heterologous host cells or purified (partially or substantially) from other polynucleotides in a solution. Isolated RNA molecules include *in vivo* or *in vitro* RNA transcripts of polynucleotides of the present invention. Isolated polynucleotides or nucleic acids according to the present invention further include such molecules produced synthetically. In addition, a polynucleotide or a nucleic acid can include regulatory elements such as promoters, enhancers, ribosome binding sites, or transcription termination signals.

As used herein, a "coding region" or "coding sequence" is a portion of polynucleotide which consists of codons translatable into amino acids. Although a "stop codon" (tag, tga, or taa) is typically not translated into an amino acid, it may be considered to be part of a coding region, but any flanking sequences, for example promoters, ribosome binding sites, transcriptional terminators, introns, and the like, are not part of a coding region. The boundaries of a coding region are typically determined by a start codon at the 5' terminus, encoding the amino terminus of the resultant polypeptide, and a translation stop codon at the 3'terminus, encoding the carboxyl terminus of the resulting polypeptide. Two or more coding regions of the present invention can be present in a single polynucleotide construct, e.g., on a single vector, or in separate polynucleotide constructs, e.g., on separate (different) vectors. It follows, then, that a single vector can contain just a single coding region, or comprise two or more coding regions, *e.g.,* a single vector can separately encode a binding domain-A and a binding domain-B as described below. In addition, a vector, polynucleotide, or nucleic acid of the invention can encode heterologous coding regions, either fused or unfused to a nucleic acid encoding a binding domain of the invention. Heterologous coding regions include without limitation specialized elements or motifs, such as a secretory signal peptide or a heterologous functional domain.

Certain proteins secreted by mammalian cells are associated with a secretory signal peptide which is cleaved from the mature protein once export of the growing protein chain across the rough endoplasmic reticulum has been initiated. Those of ordinary skill in the art are aware that signal peptides are generally fused to the N-terminus of the polypeptide, and are cleaved from the complete or "full-length" polypeptide to produce a secreted or "mature" form of the polypeptide. In certain embodiments, a native signal peptide, *e.g.,* an immunoglobulin heavy chain or light chain signal peptide is used, or a functional derivative of that sequence that retains the ability to direct the secretion of the polypeptide that is operably associated with it. Alternatively, a heterologous mammalian signal peptide, *e.g.,* a human tissue plasminogen activator (TPA) or mouse β-glucuronidase signal peptide, or a functional derivative thereof, can be used.

As used herein, the term "polypeptide" is intended to encompass a singular "polypeptide" as well as plural "polypeptides," and refers to a molecule composed of monomers (amino acids) linearly linked by amide bonds (also known as peptide bonds). The term "polypeptide" refers to any chain or chains of two or more amino acids, and does not refer to a specific length of the product. Thus, peptides, dipeptides, tripeptides, oligopeptides, "amino acid chain," or any other term used to refer to a chain or chains of two or more amino acids, are included within the definition of "polypeptide," and the term "polypeptide" can be used instead of, or interchangeably with any of these terms. As used herein the term "protein" is intended to encompass a molecule comprised of one or more polypeptides, which can in some instances be associated by bonds other than amide bonds. For example, a heterodimer such as a native active FVIII protein is a heterodimer of a heavy chain polypeptide and a light chain polypeptide associated by disulfide bonds. On the other hand, a protein can also be a single polypeptide chain. In this latter instance the single polypeptide chain can in some instances comprise two or more polypeptide subunits fused together to form a protein. The terms "polypeptide" and "protein" are also intended to refer to the products of post-expression modifications, including without limitation glycosylation, acetylation, phosphorylation, amidation, derivatization by known protecting/blocking groups, proteolytic cleavage, or modification by non-naturally occurring amino acids. A polypeptide or protein can be derived from a natural biological source or produced by recombinant technology, but is not necessarily translated from a designated nucleic acid sequence. It can be generated in any manner, including by chemical synthesis.

A "recombinant" polypeptide or protein refers to a polypeptide or protein produced via recombinant DNA technology. Recombinantly produced polypeptides and proteins expressed in host cells are considered isolated for the purpose of the invention, as are native or recombinant polypeptides which have been separated, fractionated, or partially or substantially purified by any suitable technique.

As used herein, the term "host cell" refers to a cell or a population of cells harboring or capable of harboring a recombinant nucleic acid. Host cells can be a prokaryotic cells (*e.g., E. coli*)*,* or alternatively, the host cells can be eukaryotic, for example, fungal cells *(e.g.,* yeast cells such as *Saccharomyces cerevisiae, Pichia pastoris,* or *Schizosaccharomyces pombe*)*,* and various animal cells, such as insect cells *(e.g.,* Sf-9) or mammalian cells *(e.g.,* HEK293F, CHO, COS- 7, NIH-3T3).

Also described herein are fragments, variants, or derivatives of polypeptides, and any combination thereof. The term "fragment" or "variant" when referring to polypeptides and proteins disclosed herein include any polypeptides or proteins which retain at least some of the properties (*e*.*g*., procoagulant activity) of the reference polypeptide or protein. Fragments of polypeptides include proteolytic fragments, as well as deletion fragments. Variants of polypeptides or proteins disclosed herein include fragments as described above, and also polypeptides or proteins with altered amino acid sequences due to amino acid substitutions, deletions, or insertions. Variants can be naturally or non-naturally occurring. Non-naturally occurring variants can be produced using art-known mutagenesis techniques. Variant polypeptides can comprise conservative or non-conservative amino acid substitutions, deletions or additions. "Derivatives" of polypeptides or proteins of the invention are polypeptides or proteins which have been altered so as to exhibit additional features not found on the native polypeptide or protein, and have procoagulant activity. An example of a "derivative" is an Fc fusion protein.

The term "percent sequence identity" between two polynucleotide or polypeptide sequences refers to the number of identical matched positions shared by the sequences over a comparison window, taking into account additions or deletions *(i.e.,* gaps) that must be introduced for optimal alignment of the two sequences. A matched position is any position where an identical nucleotide or amino acid is presented in both the target and reference sequence. Gaps presented in the target sequence are not counted since gaps are not nucleotides or amino acids. Likewise, gaps presented in the reference sequence are not counted since target sequence nucleotides or amino acids are counted, not nucleotides or amino acids from the reference sequence.

The percentage of sequence identity is calculated by determining the number of positions at which the identical amino-acid residue or nucleic acid base occurs in both sequences to yield the number of matched positions, dividing the number of matched positions by the total number of positions in the window of comparison and multiplying the result by 100 to yield the percentage of sequence identity. The comparison of sequences and determination of percent sequence identity between two sequences may be accomplished using readily available software both for online use and for download. Suitable software programs are available from various sources, and for alignment of both protein and nucleotide sequences. One suitable program to determine percent sequence identity is bl2seq, part of the BLAST suite of programs available from the U.S. government's National Center for Biotechnology Information BLAST web site (blast.ncbi.nlm.nih.gov). Bl2seq performs a comparison between two sequences using either the BLASTN or BLASTP algorithm. BLASTN is used to compare nucleic acid sequences, while BLASTP is used to compare amino acid sequences. Other suitable programs are, *e.g.,* Needle, Stretcher, Water, or Matcher, part of the EMBOSS suite of bioinformatics programs and also available from the European Bioinformatics Institute (EBI) at www.ebi.ac.uk/Tools/psa.

Different regions within a single polynucleotide or polypeptide target sequence that aligns with a polynucleotide or polypeptide reference sequence can each have their own percent sequence identity. It is noted that the percent sequence identity value is rounded to the nearest tenth. For example, 80.11, 80.12, 80.13, and 80.14 are rounded down to 80.1, while 80.15, 80.16, 80.17, 80.18, and 80.19 are rounded up to 80.2. It also is noted that the length value will always be an integer.

One skilled in the art will appreciate that the generation of a sequence alignment for the calculation of a percent sequence identity is not limited to binary sequence-sequence comparisons exclusively driven by primary sequence data. Sequence alignments can be derived from multiple sequence alignments. One suitable program to generate multiple sequence alignments is ClustalW2, available from www.clustal.org. Another suitable program is MUSCLE, available from www.drive5.com/muscle/. ClustalW2 and MUSCLE are alternatively available, e.g., from the EBI.

It will also be appreciated that sequence alignments can be generated by integrating sequence data with data from heterogeneous sources such as structural data (*e.g.,* crystallographic protein structures), functional data (*e*.*g*., location of mutations), or phylogenetic data. A suitable program that integrates heterogeneous data to generate a multiple sequence alignment is T-Coffee, available at www.tcoffee.org, and alternatively available, e.g., from the EBI. It will also be appreciated that the final alignment used to calculate percent sequence identity may be curated either automatically or manually.

As used herein, "an amino acid which corresponds to an amino acid in mature native human FVIII" is an amino acid in any FVIII fragment, variant, or derivative; which falls at the same position as a corresponding amino acid in native human FVIII. For example, chimeric or hybrid FVIII proteins or fragments thereof such as those disclosed in PCT Publication Nos. WO 2011/069164 A2, WO 2012/006623 A2, WO 2012/006635 A2, or WO 2012/006633 A2 can be aligned with mature native human FVIII (SEQ ID NO: 1), and an amino acid in region of the chimeric or hybrid protein which aligns with a region of SEQ ID NO:1 "corresponds" to the amino acid number it aligns with in SEQ ID NO: 1. Similarly, any fragment of FVIII, *e.g.,* the light chain of a FVIII heterodimer (*e*.*g*., A3, C1 and C2 domains) can be aligned with SEQ ID NO: 1 to determine the corresponding region of SEQ ID NO:1, and the amino acids in the fragment corresponding to an amino acid in mature native human FVIII would be numbered based on the amino acids they align with in SEQ ID NO: 1. Aligned FVIII regions need not be 100% identical to the corresponding region of SEQ ID NO: 1, as long as the similarity between the regions can be readily identified by a person of ordinary skill in the art. Thus, aligned regions in a FVIII fragment, variant, derivative or analog can be 70%, 75%, 80%, 85%, 90%, 95%, 96%, 97%, 98%, 99%, or 100% identical to the corresponding region in SEQ ID NO: 1.

As used herein, the term "insertion site" refers to a position in a FVIII polypeptide, or fragment, variant, or derivative thereof, which is immediately upstream of the position at which a heterologous moiety can be inserted. An "insertion site" is specified as a number, the number being the number of the amino acid in mature native FVIII (SEQ ID NO:1) to which the insertion site corresponds, which is immediately N-terminal to the position of the insertion. For example, the phrase "a3 comprises a heterologous moiety at an insertion site which corresponds to amino acid 1656 of SEQ ID NO: 1" indicates that the heterologous moiety is located between two amino acids corresponding to amino acid 1656 and amino acid 1657 of SEQ ID NO: 1.

The phrase "immediately downstream of an amino acid" as used herein refers to position right next to the terminal carboxyl group of the amino acid. Similarly, the phrase "immediately upstream of an amino acid" refers to the position right next to the terminal amine group of the amino acid.

The terms "inserted," "is inserted," "inserted into" or grammatically related terms, as used herein refers to the position of a heterologous moiety in a recombinant FVIII polypeptide, relative to the analogous position in native mature human FVIII. As used herein the terms refer to the characteristics of the recombinant FVIII polypeptide relative to native mature human FVIII, and do not indicate, imply or infer any methods or process by which the recombinant FIII polypeptide was made. For example, in reference to a recombinant FVIII polypeptide provided herein, the phrase "a heterologous moiety is inserted into A1-2" means that the recombinant FVIII polypeptide comprises a heterologous moiety in a region which corresponds to the A1-2 region in native mature human FVIII (from about amino acids 218 to about amino acid 229 of native mature human FVIII), e.g., bounded by amino acids corresponding to amino acids 218 and 219, amino acids 219 and 220, amino acids 220 and 221, amino acids 221 and 222, amino acids 222 and 223, amino acids 223 and 224, amino acids 224 and 225, amino acids 225 and 226, amino acids 226 and 227, amino acids 227 and 228, or amino acids 228 and 229 of native mature human FVIII.

The term "substitution," "is substituted," "substitute," or grammatically related terms as used herein means that an amino acid in a recombinant FVIII polypeptide relative to the analogous position in native mature human FVIII is replaced with another amino acid residue. As used herein the terms refer to the characteristics of the recombinant FVIII polypeptide relative to native mature human FVIII, and do not indicate, imply or infer any methods or process by which the recombinant FVIII polypeptide was made.

A "conservative amino acid substitution" is one in which the amino acid residue is replaced with an amino acid residue having a similar side chain. Families of amino acid residues having similar side chains have been defined in the art, including basic side chains *(e.g.,* lysine, arginine, histidine), acidic side chains *(e.g.,* aspartic acid, glutamic acid), uncharged polar side chains (*e*.*g*., glycine, asparagine, glutamine, serine, threonine, tyrosine, cysteine), nonpolar side chains (*e*.*g*., alanine, valine, leucine, isoleucine, proline, phenylalanine, methionine, tryptophan), beta-branched side chains (*e*.*g*., threonine, valine, isoleucine) and aromatic side chains (*e*.*g*., tyrosine, phenylalanine, tryptophan, histidine). Thus, if an amino acid in a polypeptide is replaced with another amino acid from the same side chain family, the substitution is considered to be conservative. In another embodiment, a string of amino acids can be conservatively replaced with a structurally similar string that differs in order and/or composition of side chain family members.

The term "deletion," "delete," "deleted," or grammatically related terms as used herein mean that an amino acid residue corresponding to a wild type sequence is removed from the sequence. In some aspects, deleted amino acid residues can be replaced with the same number of the amino acid residues, amino acid residues shorter than the deleted amino acid residues, or amino acid residues longer than the deleted amino acid residues. As used herein these terms refer to the characteristics of the recombinant FVIII polypeptide relative to native mature human FVIII, and do not indicate, imply or infer any methods or process by which the recombinant FVIII polypeptide was made.

A "fusion" protein comprises a first polypeptide linked via amide bonds to a second polypeptide, e.g., where the second polypeptide is not naturally linked in nature to the first polypeptide. Polypeptides which normally exist in separate proteins can be brought together in the fusion polypeptide, or polypeptides which normally exist in the same protein can be placed in a new arrangement in the fusion polypeptide, e.g., fusion of a FVIII domain of the invention with an immunoglobulin Fc domain, or fusion of the A1 and A2 regions of FVIII directly to the A3 region of FVIII through deletion of the B domain. A fusion protein is created, for example, by chemical synthesis, or by creating and translating a polynucleotide in which the peptide regions are encoded in the desired relationship.

The terms "heterologous" and "heterologous moiety" mean that a polynucleotide, polypeptide, or other moiety is derived from a distinct entity from that of the entity to which it is being compared. For instance, a heterologous polypeptide can be synthetic, or derived from a different species, different cell type of an individual, or the same or different type of cell of distinct individuals. In one aspect, a heterologous moiety can be a polypeptide fused to another polypeptide to produce a fusion polypeptide or protein. In another aspect, a heterologous moiety can be a non-polypeptide such as PEG conjugated to a polypeptide or protein.

A linker which may be present in a polypeptide is herein referred to as a "cleavable linker" which comprises one or more heterologous protease-cleavage sites *(e.g.,* a factor XIa or thrombin cleavage site) that are not naturally occurring in the polypeptide and which may include additional linkers on either the N terminal of C terminal or both sides of the cleavage site. Exemplary locations for such sites are shown in the accompanying drawings and include, e.g., placement between a heavy chain of FVIII and a light chain of FVIII.

### 1. Factor VIII

"Factor VIII protein" or "FVIII protein" as used herein, means functional Factor VIII protein in its normal role in coagulation, unless otherwise specified. Thus, the term FVIII includes variant proteins that are functional. In one embodiment, the FVIII protein is the human, porcine, canine, rat, or murine FVIII protein. A functional FVIII protein can be a fusion protein, such as, but not limited to, a fusion protein comprising a fully or partially B domain-deleted FVIII, at least a portion of an immunoglobulin constant region, *e.g.,* an Fc domain, or both. Myriad functional FVIII variants have been constructed and can be used as recombinant FVIII proteins as described herein. *See* PCT Publication Nos. WO 2011/069164 A2, WO 2012/006623 A2, WO 2012/006635 A2, or WO 2012/006633 A2.

A great many functional FVIII variants are known. In addition, hundreds of nonfunctional mutations in FVIII have been identified in hemophilia patients. *See, e.g.,* Cutler et al., Hum. Mutat. 19:274-8 (2002. In addition, comparisons between FVIII from humans and other species have identified conserved residues that are likely to be required for function. *See, e.g.,* Cameron et al., Thromb. Haemost. 79:317-22 (1998) and US 6,251,632.

The human FVIII amino acid sequence was deduced from cDNA as shown in U.S. Pat. No. 4,965,199. Native mature human FVIII derived from the cDNA sequence (*i.e.,* without the secretory signal peptide but prior to other post-translational processing) is presented as SEQ ID NO:1. Partially or fully B domain-deleted FVIII is functional and has been used in commercial FVIII therapeutics. *See, e.g.,* EP506757B2.

"Native mature FVIII" comprises functional domains, which may or may not be necessary for procoagulant activity. The sequence of native mature human FVIII is presented as SEQ ID NO: 1. A native FVIII protein has the following formula: A1-a1-A2-a2-B-a3-A3-C1-C2, where A1, A2, and A3 are the structurally-related "A domains," B is optionally present and is the "B domain" or a fragment thereof, C1 and C2 are the structurally-related "C domains," and a1, a2 and a3 are acidic spacer regions. Referring to the primary amino acid sequence position in SEQ ID NO: 1, the A1 domain of human FVIII extends from Ala1 to about Arg336, the a1 spacer region extends from about Met337 to about Arg372, the A2 domain extends from about Ser373 to about Tyr719, the a2 spacer region extends from about Glu720 to about Arg740, the B domain extends from about Ser741 to about Arg 1648, the a3 spacer region extends from about Glu1649 to about Arg1689, the A3 domain extends from about Ser1690 to about Asn2019, the C1 domain extends from about Lys2020 to about Asn2172, and the C2 domain extends from about Ser2173 to Tyr2332 (Saenko et al., J. Thromb. Hemostasis 1:922-930 (2005)). Other than specific proteolytic cleavage sites, designation of the locations of the boundaries between the domains and regions of FVIII can vary in different literature references. The boundaries noted herein are therefore designated as approximate by use of the term "about."

A polypeptide comprising the a3, A3, C1, and C2 domains, *i.e.,* from about Ser1690 to Tyr2332, is cleaved from the polypeptide comprising the A1, a1, A2, a2, and B domains during normal FVIII processing resulting in a heavy chain and a light chain. The B domain is not required for procoagulant activity, and in certain aspects, including commercially available therapeutic compositions, some or all of the B domain of FVIII are deleted ("B domain-deleted factor VIII" or "BDD FVIII"). An example of a BDD FVIII is REFACTO^{®} or XYNTHA^{®} (recombinant BDD FVIII), which comprises a first polypeptide corresponding to amino acids 1 to 743 of SEQ ID NO:1, fused to a second polypeptide corresponding to amino acids 1638 to 2332 of SEQ ID NO:1. Exemplary BDD FVIII constructs which can be used to produce recombinant proteins of the invention include, but are not limited to FVIII with a deletion of amino acids corresponding to amino acids 747-1638 of mature human FVIII (SEQ ID NO:1) (Hoeben R.C., et al. J. Biol. Chem. 265 (13): 7318-7323 (1990) , and FVIII with a deletion of amino acids corresponding to amino acids 771-1666 or amino acids 868-1562 of mature human FVIII (SEQ ID NO:1) (Meulien P., et al. Protein Eng. 2(4): 301-6 (1988)).

In certain aspects a recombinant FVIII protein is provided, where the protein comprises a first polypeptide, *i*.*e*., an amino acid chain, comprising Formula I: (A1) - a1- (A2) - a2 - [B], and a second polypeptide, *i.e.,* an amino acid chain, comprising Formula II: a3 - (A3) - (C1). The first polypeptide and the second polypeptide can exist as a single amino acid chain, that is, fused through amide bonds, or can exist or associated as a heterodimer. In one embodiment, the recombinant FVIII protein comprises a FVIII heavy chain and a FVIII light chain in a single chain polypeptide. The single chain polypeptide can contain one or more substitutions, deletions, or mutations at the cleavage site between the FVIII heavy chain and the FVIII light chain. For example, a single chain FVIII polypeptide can contain one or more substitutions, deletions, or mutations at the arginine residue corresponding to residue 1645, residue 1648, or both residues of mature full-length FVIII protein, wherein the substitutions, deletions, or mutations prevent cleavage of the FVIII heavy chain and the FVIII light chain into a heterodimer. The substitutions or mutations can be any known amino acids, e.g., alanine. In another embodiment, the recombinant FVIII protein comprises a FVIII heavy chain and a FVIII light chain in a single chain polypeptide, wherein the FVIII heavy chain and the FVIII light chain are not processed (also referred to herein as "unprocessed" or "non-processed"). For example, a single chain polypeptide in the recombinant FVIII protein can still retain the arginine residues corresponding to residues 1645, 1648, or both residues of mature full-length FVIII protein, but the single chain polypeptide in the recombinant FVIII protein is not cleaved into the FVIII heavy chain and the FVIII light chain. In other embodiments, the recombinant FVIII protein composition comprises a mixture of the heterodimer FVIII and the unprocessed FVIII. In other embodiments, the recombinant FVIII protein composition comprises a mixture of the single chain FVIII, the unprocessed FVIII, and the heterodimer FVIII.

According to this aspect, A1 is an A1 domain of FVIII as described herein, A2 is an A2 domain of FVIII as described herein, [B] is an optional B domain of FVIII or a fragment thereof *(i.e.,* the B domain may or may not be part of the protein, and may be only partially present), A3 is an A3 domain of FVIII as described herein, C1 is a C1 domain of FVIII as described herein, and a1, a2, and a3 are acidic spacer regions. In certain aspects the second polypeptide further comprises a (C2) situated C-terminal to the (C1), where C2 is a C2 domain of FVIII. While the various FVIII domains of a recombinant polypeptide of the invention share primary sequence similarity with the corresponding regions of native mature FVIII, e.g., native mature human FVIII, the regions need not be identical provided that the recombinant polypeptide has procoagulant activity.

A recombinant FVIII protein of the invention can contain a substitution or deletion of one or more of permissive loops in A regions, a3 region, or any combinations thereof. The recombinant FVIII protein can further comprise at least one heterologous moiety inserted into the one or more permissive loops in A regions, a3 region, or any combinations thereof, in which one or more amino acids are substituted or deleted. In some embodiments, the recombinant FVIII protein has procoagulant activity, and can be expressed in a host cell. A "heterologous moiety" can be a heterologous polypeptide or a non-polypeptide entity, such as polyethylene glycol (PEG) or both. Exemplary heterologous moieties are described below. In certain aspects a recombinant FVIII protein of the invention comprises at least one heterologous moiety inserted into at least one permissive loop, in which one or more amino acids are substituted or deleted, wherein the heterologous moiety is not an XTEN sequence. In other aspects a recombinant FVIII protein of the invention comprises at least one heterologous moiety inserted into at least one permissive loop, or into the a3 region, or both, in which one or more amino acids are substituted or deleted, wherein the heterologous moiety increases the half-life of the protein, *e.g., in vivo* half-life, and wherein the heterologous moiety is not an XTEN sequence. Constructs comprising heterologous moieties (*e*.*g*., heterologous moieties that increase half-life of the protein) are described in the examples. The terms "insert" or "insert into" as applied to a permissive loop refer to the covalent or non-covalent attachment of heterologous moiety to a FVIII polypeptide by integrating it within the FVIII polypeptide chain, attaching it to the side chain of a native amino acid or a heterologous natural or non-natural amino acid *(e.g.,* a cysteine or another amino acid with a derivatizable side chain introduced in the FVIII sequence using molecular biology methods), or to a linker or other molecule covalently or non-covalently attached to the FVIII polypeptide. The term "insertion" when used in the context of a polypeptide sequence refers to the introduction of a heterologous sequence *(e.g.,* a polypeptide or a derivatizable amino acid such as cysteine ) between two contiguous amino acids in the amino acid sequence of a FVIII polypeptide, or the fusion, conjugation, or chemical attachment of a heterologous moiety to a FVIII polypeptide.

The recombinant FVIII protein of the invention can comprise one or more inserted heterologous moieties, wherein the heterologous moiety completely or partially replaces one or more permissive loops. In one particular embodiment, the heterologous moiety completely or partially replaces A1-1, A2-1, A3-1, A3-2, or any combination thereof. In further embodiments, the heterologous moiety is an XTEN, e.g., AE42 and/or AE144.

In certain aspects, a recombinant FVIII protein of the invention is chimeric. A "chimeric protein," or "chimeric polypeptide" as used herein, means a protein or polypeptide that includes within it at least two stretches of amino acids from different sources, *e.g.,* a FVIII protein comprising a heterologous polypeptide, e.g., within a permissive loop or within the a3 region of FVIII, as described in more detail below. Chimeric proteins or chimeric polypeptides can include two, three, four, five, six, seven, or more amino acid chains from different sources, such as different genes, different cDNAs, or different species. Exemplary heterologous polypeptides for use in recombinant polypeptides of the invention include, but are not limited to polypeptides which increase FVIII half-life or stability, for example, an immunoglobulin Fc region. Specific heterologous polypeptides which can be included in recombinant polypeptides of the invention are described elsewhere herein.

A chimeric protein or chimeric polypeptide can include one or more linkers joining the different subsequences. Thus, the subsequences can be joined directly or indirectly, via linkers, or both, within a single chimeric protein or chimeric polypeptide. Chimeric proteins or chimeric polypeptides described herein can include additional polypeptides such as signal sequences and sequences such as 6His and FLAG that aid in protein purification or detection. In addition, chimeric polypeptides can have amino acid or peptide additions to the N- and/or C-termini.

In certain embodiments, a recombinant FVIII protein of the invention is conjugated, *e.g.,* to comprise a non-polypeptide heterologous moiety. Conjugation may be through insertion of an acceptor amino acid (*e*.*g*., cysteine), peptide or polypeptide into a permissive loop, or into the a3 region, or both. As used herein, a conjugate refers to any two or more entities bound to one another by any physicochemical means, including, but not limited to, hydrophobic interaction, covalent interaction, hydrogen bond interaction, ionic interaction, or any combination thereof. Thus, in certain aspects, a conjugated recombinant FVIII protein of the invention refers to a recombinant FVIII protein with one or more entities bound to it by covalent or non-covalent interaction, which has procoagulant activity.

By "procoagulant activity" is meant the ability of the recombinant FVIII protein of the invention to participate in the clotting cascade in blood, substituting for native FVIII. For example, a recombinant FVIII protein of the invention has procoagulant activity when it can activate FIX as a cofactor to convert Factor X (FX) to activated Factor X (FXa), as tested, *e.g.,* in a chromogenic assay.

A recombinant FVIII protein of the invention need not exhibit 100% of the procoagulant activity of native mature human FVIII. In fact, in certain aspects a heterologous moiety inserted into a recombinant FVIII protein of the invention can increase the half-life or stability of the protein significantly, such that lower activity is perfectly acceptable. Thus, in certain aspects, a recombinant FVIII protein of the invention has at least about 10%, about 20%, about 30%, about 40%, about 50%, about 60%, about 70%, about 80%, about 90% or about 100% of the procoagulant activity of native FVIII.

Procoagulant activity can be measured by any suitable *in vitro* or *in vivo* assay. The activity of FVIII can be measured either downstream of the coagulation cascade by monitoring the generation of a clot (clotting assays), or upstream by measuring directly the enzymatic activity of FX following activation by the FVIII-FIX complex (chromogenic assays) (see, *e.g.,* Barrowcliffe et al., Semin. Thromb. Haemost. 28: 247-56 (2002); Lee et al., Thromb. Haemost. 82: 1644-47 (1999); Lippi et al., Clin. Chem. Lab. Med. 45: 2-12 (2007); Matsumoto et al., J. Thromb. Haemost. 4: 377-84 (2006)). Thus, procoagulant activity can be measured using a chromogenic substrate assay, a clotting assay *(e.g.,* a one stage or a two stage clotting assay), or both. The chromogenic assay mechanism is based on the principles of the blood coagulation cascade, where activated FVIII accelerates the conversion of FX into FXₐ in the presence of activated FIX, phospholipids and calcium ions. The FXₐ activity is assessed by hydrolysis of a p-nitroanilide (pNA) substrate specific to FXₐ. The initial rate of release of p-nitroaniline measured at 405 nM is directly proportional to the FXₐ activity and thus to the FVIII activity in the sample. The chromogenic assay is recommended by the Factor VIII and Factor IX Subcommittee of the Scientific and Standardization Committee (SSC) of the International Society on Thrombosis and Hemostasis (ISTH). Since 1994, the chromogenic assay has also been the reference method of the European Pharmacopoeia for the assignment of FVIII concentrate potency (Rosen et al., Thromb. Haemost. 54, 818-823 (1985); Lethagen et al., Scand. J. Haematol. 37, 448-453 (1986)).

Other suitable assays useful to determine pro-coagulant activity include those disclosed, *e.g.,* in U.S. Application Publication No. 2010/0022445 to Scheiflinger and Dockal.

In certain aspects the procoagulant activity of a recombinant FVIII protein of the invention is compared to native mature FVIII, in certain aspects it is compared to an international standard.

"Equivalent amount," as used herein, means the same amount of FVIII activity as expressed in International Units, which is independent of molecular weight of the polypeptide in question. One International Unit (IU) of FVIII activity corresponds approximately to the quantity of FVIII in one milliliter of normal human plasma. As described above, several assays are available for measuring FVIII activity, including the European Pharmacopoeia chromogenic substrate assay and a one stage clotting assay.

### 2. Factor VIII Permissive Loops

As described in detail elsewhere herein, the inventors have recognized that one or more permissive loops in FVIII, an a3 region in FVIII or a portion thereof can be substituted or deleted, and the FVIII containing the substitution or deletion has procoagulant activity. The one or more permissive loops can further comprise a heterologous moiety at the site immediately upstream of the substitution or deletion. As previously described, FVIII "A" domain comprise at least two "permissive loops" into which heterologous moieties can be inserted without eliminating procoagulant activity of the recombinant protein, or the ability of the recombinant proteins to be expressed *in vivo* or *in vitro* in a host cell. *See* PCT/US2013/026521, filed February 15, 2013. The permissive loops are regions with, among other attributes, high surface or solvent exposure and high conformational flexibility. Although "permissive sites" tend to cluster in permissive loops, there are other permissive sites outside of the identified permissive loops into which heterologous moieties can be inserted without eliminating procoagulant activity of the recombinant protein, or the ability of the recombinant proteins to be expressed *in vivo* or *in vitro* in a host cell. The term "permissive location" refers to both permissive loops and permissive sites. The A1 domain comprises a permissive loop-1 region (A1-1) and a permissive loop-2 region (A1-2), the A2 domain comprises a permissive loop-1 region (A2-1) and a permissive loop-2 region (A2-2), and the A3 domain comprises a permissive loop-1 region (A3-1) and a permissive loop-2 region (A3-2). *See* PCT/US2013/026521, filed February 15, 2013.

A recombinant FVIII protein of the invention can comprise a substitution or deletion in one or more of the permissive loops in each of the FVIII A domain regions or in an a3 region and can further allow insertion of a heterologous moiety while having procoagulant activity and still being able to be expressed *in vivo* or *in vitro* by a host cell. Various crystal structures of FVIII have been determined, of varying degrees of resolution. These structures of FVIII and FVIIIa, determined by X-ray crystallography and molecular dynamic simulation, were used to generate models of accessible surface area and conformational flexibility for FVIII. For example, the crystal structure of human FVIII has been determined by Shen et al. Blood 111: 1240-1247 (2008) and Ngo et al. Structure 16: 597-606 (2008). The data for these structures is available from the Protein Data Bank (pdb.org) under Accession Numbers 2R7E and 3CDZ, respectively.

The predicted secondary structure of the heavy and light chains of human FVIII according to the Shen *et al.* crystal structure is reproduced in FIGS. 9A and 9B. The various beta strands predicted from the Shen *et al.* crystal structure are numbered consecutively in FIGS. 9A and 9B. In certain embodiments, the permissive loops A1-1, A1-2, A2-1, A2-2, A3-1, and A3-2 are contained within surface-exposed, flexible loop structures in the A domains of FVIII. A1-1 is located between beta strand 1 and beta strand 2, A1-2 is located between beta strand 11 and beta strand 12, A2-1 is located between beta strand 22 and beta strand 23, A2-2 is located between beta strand 32 and beta strand 33, A3-1 is located between beta strand 38 and beta strand 39 and A3-2 is located between beta strand 45 and beta strand 46, according to the secondary structure of mature FVIII stored as Accession Number 2R7E of the PDB database (PDB:2R7E) and as shown in FIGS. 9A and 9B. The secondary structure of PDB Accession Number 2R7E shown in FIGS. 9A and 9B corresponds to the standardized secondary structure assignment according to the DSSP program (Kabsch and Sander, Biopolymers, 22:2577-2637 (1983)). The DSSP secondary structure of the mature FVIII stored as PDB Accession Number 2R7E can be accessed at the DSSP database, available at swift.cmbi.ru.nl/gv/dssp/ (Joosten *et al.,* 39(Suppl. 1): D411-D419 (2010)).

In certain aspects, a surface-exposed, flexible loop structure comprising A1-1 corresponds to a region in native mature human FVIII from about amino acid 15 to about amino acid 45 of SEQ ID NO: 1. In certain aspects, A1-1 corresponds to a region in native mature human FVIII from about amino acid 18 to about amino acid 41 of SEQ ID NO: 1. In certain aspects, the surface-exposed, flexible loop structure comprising A1-2 corresponds to a region in native mature human FVIII from about amino acid 201 to about amino acid 232 of SEQ ID NO: 1. In certain aspects, A1-2 corresponds to a region in native mature human FVIII from about amino acid 218 to about amino acid 229 of SEQ ID NO: 1. In certain aspects, the surface-exposed, flexible loop structure comprising A2-1 corresponds to a region in native mature human FVIII from about amino acid 395 to about amino acid 421 of SEQ ID NO: 1. In certain aspects, A2-1 corresponds to a region in native mature human FVIII from about amino acid 397 to about amino acid 418 of SEQ ID NO: 1. In certain aspects, the surface-exposed, flexible loop structure comprising A2-2 corresponds to a region in native mature human FVIII from about amino acid 577 to about amino acid 635 of SEQ ID NO: 1. In certain aspects, A2-2 corresponds to a region in native mature human FVIII from about amino acid 595 to about amino acid 607 of SEQ ID NO: 1. In certain aspects, the surface-exposed, flexible loop structure comprising A3-1 corresponds to a region in native mature human FVIII from about amino acid 1705 to about amino acid 1732 of SEQ ID NO:1. In certain aspects, A3-1 corresponds to a region in native mature human FVIII from about amino acid 1711 to about amino acid 1725 of SEQ ID NO: 1. In certain aspects, the surface-exposed, flexible loop structure comprising A3-2 corresponds to a region in native mature human FVIII from about amino acid 1884 to about amino acid 1917 of SEQ ID NO: 1. In certain aspects, A3-2 corresponds to a region in native mature human FVIII from about amino acid 1899 to about amino acid 1911 of SEQ ID NO: 1. In other aspects, the a3 region corresponds to a region in native mature human FVIII from about amino acid 1649 to about amino acid 1689 of SEQ ID NO: 1.

In certain aspects a recombinant FVIII protein of the invention comprises one or more heterologous moieties inserted into one or more permissive loops of FVIII, or into the a3 region, or both, wherein one or more amino acids in the one or more permissive loops of FVIII or the a3 region, or both are substituted or deleted and wherein the recombinant FVIII protein has procoagulant activity and can be expressed *in vivo* or *in vitro* in a host cell. Heterologous moieties to be inserted include, but are not limited to, (i) those that increase the half-life or the *in vivo* or *in vitro* stability of FVIII, (ii) a clearance receptor, or (iii) a moiety which aids in visualization or localization of the recombinant FVIII protein. Heterologous moieties are discussed in more detail below.

In some aspects, a recombinant FVIII protein comprises at least one heterologous moiety, wherein one or more amino acids from amino acid 745 to amino acid 1685 corresponding to SEQ ID NO: 1 or amino acid 745 to amino acid 1656 corresponding to SEQ ID NO: 1 are deleted or substituted.

In other aspects, a recombinant FVIII protein comprises at least one heterologous moiety, wherein the FVIII protein is not capable of binding to Von Willebrand Factor. In one aspect, the FVIII protein comprises a heterologous moiety and has a deletion at amino acids 745 to 1685 corresponding to native mature human FVIII.

In certain aspects one or more amino acids in the permissive loops A1-1, A1-2, A2-1, A2-2, A3-1, or A3-2 or an a3 region in a recombinant FVIII protein are substituted or deleted, wherein the recombinant FVIII protein has procoagulant activity. In some aspects, the recombinant FVIII protein that contains the substitution or deletion is expressed *in vivo* or *in vitro* in a host cell. In other aspects, one or more of the entire permissive loops can be substituted or deleted. In still other aspects, only a portion of the one or more permissive loops is substituted or deleted. In some aspects, any combinations of the substitution or deletion in one or more permissive loops or in the a3 region are possible for the purpose of preparing a recombinant FVIII protein.

In certain aspects, at least one heterologous moiety is inserted in the permissive loops or in the a3 region of the recombinant FVIII protein, e.g., upstream or downstream of the amino acids that are substituted or deleted. In certain aspects a recombinant FVIII protein as described above comprises at least two heterologous moieties inserted into a FVIII protein, wherein at least one of the two heterologous moieties is inserted in the permissive loops A1-1, A1-2, A2-1, A2-2, A3-1, or A3-2 or in the a3 region, or any combinations thereof, in which one or more amino acids are substituted or deleted, and wherein the recombinant FVIII protein has procoagulant activity and can be expressed *in vivo* or *in vitro* in a host cell. In one aspect, each of the two heterologous moieties is inserted in the permissive loops of the FVIII protein and/or in the a3 region, e.g., upstream or downstream of the substitution, deletion or a combination thereof. In another aspect, a first heterologous moiety is inserted in a permissive loop of a FVIII protein containing the substitution, deletion, or a combination thereof *(e.g.,* A1-1), e.g., upstream or downstream of a substitution, deletion or a combination thereof, and a second heterologous moiety is inserted in one of the other permissive loops *(e.g.,* A1-2, A2-1, A2-2, A3-1, A3-2) or in the a3 region. In other aspects, the other permissive loops and the a3 region do not contain any substitution or deletion. In some aspects, the first heterologous moiety and the second heterologous moiety can be the same or different. In still other aspects, one or more of the other permissive loops and the a3 region contain substitution, deletion, or a combination thereof. In certain aspects a recombinant FVIII protein as described above comprises at least three heterologous moieties inserted into a FVIII protein, wherein at least one of the three heterologous moieties is inserted into at least one of the permissive loops A1-1, A1-2, A2-1, A2-2, A3-1, or A3-2 or in a3 region, wherein one or more amino acids in the at least one of the permissive loops A1-1, A1-2, A2-1, A2-2, A3-1, or A3-2 or in a3 region are substituted or deleted and wherein the recombinant FVIII protein has procoagulant activity and can be expressed *in vivo* or *in vitro* in a host cell. The three heterologous moieties can be the same or different. In certain aspects a recombinant FVIII protein as described above comprises at least four heterologous moieties inserted into a FVIII protein, wherein at least one of the four heterologous moieties is inserted into at least one of the permissive loops A1-1, A1-2, A2-1, A2-2, A3-1, or A3-2 or in a3 region, wherein one or more amino acids in the at least one of the permissive loops A1-1, A1-2, A2-1, A2-2, A3-1, or A3-2 or in a3 region are substituted or deleted, and wherein the recombinant FVIII protein has procoagulant activity and can be expressed *in vivo* or *in vitro* in a host cell. The four heterologous moieties can be the same or different. In certain aspects a recombinant FVIII protein as described above comprises at least five heterologous moiety inserted into a FVIII protein, wherein at least one of the five heterologous moieties is inserted into at least one of the permissive loops A1-1, A1-2, A2-1, A2-2, A3-1, or A3-2 or in a3 region, wherein one or more amino acids in the at least one of the permissive loops A1-1, A1-2, A2-1, A2-2, A3-1, or A3-2 or in a3 region are substituted or deleted, and wherein the recombinant FVIII protein has procoagulant activity and can be expressed *in vivo* or *in vitro* in a host cell. The five heterologous moieties can be the same or different. In certain aspects a recombinant FVIII protein as described above comprises at least six heterologous moieties inserted into a FVIII protein, wherein at least one of the six heterologous moieties is inserted into at least one of the permissive loops A1-1, A1-2, A2-1, A2-2, A3-1, or A3-2 or in a3 region, one or more amino acids in the at least one of the permissive loops A1-1, A1-2, A2-1, A2-2, A3-1, or A3-2 or in the a3 region are substituted or deleted, and wherein the recombinant FVIII protein has procoagulant activity and can be expressed *in vivo* or *in vitro* in a host cell. The six heterologous moieties can be the same or different. In other aspects, at least one heterologous moiety can further be inserted in the a3 region. In still other aspects, at least one heterologous moiety can further be inserted in the B domain, e.g., amino acid 745 corresponding to SEQ ID NO: 1 or fused to the C-terminus of the FVIII protein, e.g., amino acid 2332 corresponding to SEQ ID NO: 1.

In certain aspects a recombinant FVIII protein as described above comprises at least two heterologous moieties inserted into FVIII, *e.g.,* into two different permissive loops A1-1, A1-2, A2-1, A2-2, A3-1, or A3-2 or in the a3 region, wherein one or more amino acids of each of the two different permissive loops are substituted or deleted and wherein the recombinant FVIII protein has procoagulant activity and can be expressed *in vivo* or *in vitro* in a host cell. Alternatively, a recombinant FVIII protein as described above can comprise two or more heterologous moieties inserted into a single permissive loop without heterologous moieties inserted into other permissive loops, wherein one or more amino acids in the single permissive loop are substituted and/or deleted and wherein the recombinant FVIII protein has procoagulant activity and can be expressed *in vivo* or *in vitro* in a host cell. In certain aspects a recombinant FVIII protein as described above can comprise at least one heterologous moiety inserted into at least one of the permissive loops as described above, and can further comprise one or more heterologous moieties inserted into a3, wherein the recombinant FVIII protein has procoagulant activity and can be expressed *in vivo* or *in vitro* in a host cell. In certain aspects, a recombinant FVIII protein of the invention can comprise three, four, five, six or more heterologous moieties inserted into one or more permissive loops or into a3, wherein one or more amino acids in the one or more permissive loops or in the a3 region are substituted or deleted and wherein the recombinant FVIII protein has procoagulant activity and can be expressed *in vivo* or *in vitro* in a host cell.

**TABLE 1A.**

| | Definition I | Deletion - Definition I | Definition II | Deletion - Definition II |
|---|---|---|---|---|
| A1-1 | 15-45 | 16-44 | 18 - 41 | 19-40 |
| A1-2 | 201 - 232 | 202 - 231 | 218 - 229 | 219 - 228 |
| A2-1 | 395 - 421 | 396 - 420 | 397 - 418 | 398-417 |
| A2-2 | 577 - 635 | 578 - 634 | 595 - 607 | 596 - 606 |
| A3-1 | 1705 - 1732 | 1706 - 1731 | 1711 - 1725 | 1712-1724 |
| A3-2 | 1884 - 1917 | 1885 - 1916 | 1899 - 1911 | 1900 - 1910 |
| a3 | | | | 1649-1689 |

The permissive loop regions that can be substituted or deleted (Definition I or Definition II) is identified in Table 1A. The amino acid residue coordinates of the one or more amino acids that can be substituted or deleted are listed in Table 1A (Deletion-Definition I and Deletion-Definition II). In some aspects, the one or more amino acids substituted or deleted in a recombinant FVIII protein are in amino acids 19 to 40 (A1-1), amino acids 219 to 228 (A1-2), amino acids 398 to 417 (A2-1), amino acids 596 to 606 (A2-2), amino acids 1712 to 1724 (A3-1), amino acids 1900 to 1910 (A3-2), amino acids 1649 to 1689 (a3 region), or any combinations thereof corresponding to native mature human FVIII. In other aspects, the one or more amino acids substituted or deleted are in amino acids 16 to 44 (A1-1), amino acids 202 to 231 (A2-2), amino acids 396 to 420 (A2-1), amino acids 578 to 634 (A2-2), amino acids 1706 to 1731 (A3-1), amino acids 1885 to 1916 (A3-2), or amino acids 1649 to 1689 (a3 region), or any combinations thereof corresponding to native mature human FVIII. In some aspects, the entire permissive loop of A1-1, A1-2, A2-1, A2-2, A3-1, or A3-2 is substituted or deleted. In other aspects, a portion of the permissive loops A1-1, A1-2, A2-1, A2-2, A3-1, or A3-2 is deleted or substituted. In certain embodiments, the recombinant FVIII protein comprises a fragment of the B domain deletion, a full length B domain, or the B-domain is not present.

In certain aspects, a recombinant FVIII protein of the invention comprises a heterologous moiety inserted anywhere in one or more permissive loops or in the a3 domain, *e.g.,* upstream or downstream of the one or more amino acids substituted or deleted, *e.g.,* immediately downstream of one or more amino acids corresponding to one or more amino acids in mature native human FVIII including, but not limited to: amino acid 18 of SEQ ID NO:1 with substitution or deletion of amino acids 19 to 40 of SEQ ID NO: 1 or a portion thereof; amino acid 22 of SEQ ID NO:1 with substitution or deletion of amino acids 23 to 40 of SEQ ID NO: 1 or a portion thereof; amino acid 26 of SEQ ID NO:1 with substitution or deletion of amino acids 27 to 40 of SEQ ID NO: 1 or a portion thereof, with substitution or deletion of amino acids 19 to 25 of SEQ ID NO: 1 or a portion thereof, or both; amino acid 40 of SEQ ID NO:1 with substitution or deletion of amino acids 19 to 39 of SEQ ID NO: 1 or a portion thereof; amino acid 216 of SEQ ID NO:1 with substitution or deletion of amino acids 217 to 228 of SEQ ID NO: 1 or a portion thereof; amino acid 220 of SEQ ID NO:1 with substitution or deletion of amino acids 221 to 228 of SEQ ID NO: 1 or a portion thereof, with substitution or deletion of amino acid 217 to 219 of SEQ ID NO: 1 or a portion thereof, or both; amino acid 224 of SEQ ID NO:1 with substitution or deletion of amino acids 225 to 228 of SEQ ID NO: 1 or a portion thereof, with substitution or deletion of amino acids 217 to 223 of SEQ ID NO: 1, or both; amino acid 399 of SEQ ID NO:1 with substitution or deletion of amino acids 400 to 417 of SEQ ID NO: 1 or a portion thereof, with substitution or deletion of amino acid 398 of SEQ ID NO: 1, or both; amino acid 403 of SEQ ID NO:1 with substitution or deletion of amino acids 404 to 417 of SEQ ID NO: 1 or a portion thereof, with substitution or deletion of amino acids 398 to 402 of SEQ ID NO: 1, or both; amino acid 409 of SEQ ID NO:1 with substitution or deletion of amino acids 410 to 417 of SEQ ID NO: 1 or a portion thereof, with substitution or deletion of amino acids 398 to 408 of SEQ ID NO: 1 or a portion thereof, or both; amino acid 599 of SEQ ID NO:1 with substitution or deletion of amino acids 600 to 606 of SEQ ID NO: 1 or a portion thereof, with substitution or deletion of amino acids 596 to 598 of SEQ ID NO: 1 or a portion thereof, or both; amino acid 603 of SEQ ID NO:1 with substitution or deletion of amino acids 603 to 606 of SEQ ID NO: 1 or a portion thereof, with substitution or deletion of amino acids 596 to 602 of SEQ ID NO: 1 or a portion thereof, or both; amino acid 1656 of SEQ ID NO: 1 with substitution or deletion of amino acids 1649 to 1655 of SEQ ID NO: 1 or a portion thereof, with substitution or deletion of amino acids 1657 to 1689 of SEQ ID NO: 1 or a portion thereof, or both; amino acid 1711 of SEQ ID NO:1 with substitution or deletion of amino acids 1712 to 1724 of SEQ ID NO: 1 or a portion thereof; amino acid 1720 of SEQ ID NO:1 with substitution or deletion of amino acids 1721 to 1724 of SEQ ID NO: 1 or a portion thereof, with substitution or deletion of amino acids 1712 to 1719 of SEQ ID NO: 1 or a portion thereof, or both; amino acid 1725 of SEQ ID NO:1 with substitution or deletion of amino acids 1712 to 1724 of SEQ ID NO: 1 or a portion thereof; amino acid 1900 of SEQ ID NO:1 with substitution or deletion of amino acids 1901 to 1910 of SEQ ID NO: 1 or a portion thereof; amino acid 1905 of SEQ ID NO:1 with substitution or deletion of amino acids 1906 to 1910 of SEQ ID NO: 1 or a portion thereof, with substitution or deletion of amino acids 1901 to 1904 of SEQ ID NO: 1 or a portion thereof, or both; amino acid 1910 of SEQ ID NO:1 with substitution or deletion of amino acids 1901 to 1909 of SEQ ID NO: 1 or a portion thereof; or any combination thereof. In certain aspects, a recombinant FVIII protein of the invention comprises a heterologous moiety inserted in the a3 region, *e.g.,* upstream or downstream of the one or more amino acids substituted, mutated, or deleted, which can be amino acids 1649 to 1689 corresponding to native mature human FVIII, *e.g.,* immediately downstream of amino acid 1656 of SEQ ID NO:1 with substitution or deletion of amino acids 1649 to 1648 of SEQ ID NO: 1 or a portion thereof, with substitution or deletion of amino acids 1650 to 1689 of SEQ ID NO: 1 or a portion thereof, or the combination thereof. In certain embodiments, the recombinant FVIII protein further comprises a full or partial deletion of the B domain.

In some aspects, both of the substitution or deletion of one or more amino acids and the insertion of a heterologous moiety are in the same permissive loop or in the a3 region. In other aspects, the substitution or deletion of one or more amino acids is in one permissive loop, and the insertion of a heterologous moiety is in another permissive loop or in the a3 region. In still other aspects the substitution or deletion of one or more amino acids is in the a3 region, and the insertion of a heterologous moiety is in a permissive loop. In other embodiments, the recombinant FVIII protein has one permissive loop replacement or substitution. In still other aspects, the recombinant FVIII protein has two or more of different permissive loop replacements or substitutions. The two or more of different permissive loop replacements or substitutions can be any possible combinations. In one embodiment, one FVIII protein has all of A1-1 replaced with a first heterologous moiety and A3-1 partially or totally replaced with a second heterologous moiety. It is disclosed that a FVIII protein comprises A3-1 partially replaced with a first heterologous moiety, while A3-2 is deleted and substituted with a second heterologous moiety and A1-1 is left intact. The first heterologous moiety and the second heterologous moiety can be the same or different. The present invention recognizes the possibility of any number of combinations of deletions and/or substitutions.

In certain aspects one or more amino acids in A1-1 in a recombinant FVIII protein are substituted or deleted, wherein the recombinant FVIII protein has procoagulant activity. In some aspects, the recombinant FVIII protein that contains the substitution or deletion is expressed *in vivo* or *in vitro* in a host cell. In other aspects, the one or more amino acids substituted or deleted are in amino acids 19 to 22, amino acids 19 to 26, amino acids 19 to 32, amino acids 19 to 40, amino acids 23 to 26, amino acids 23 to 32, amino acids 23 to 40, amino acids 27 to 32, amino acids 27 to 40, or amino acids 33 to 40 corresponding to native mature human FVIII. In certain aspects, at least one heterologous moiety is inserted immediately upstream of the one or more amino acids substituted or deleted in A1-1. In some aspects, at least one heterologous moiety is inserted immediately downstream of amino acid 18, amino acids 22, amino acids 26, or amino acids 32 corresponding to native human FVIII in the A1-1 region. In certain aspects a recombinant FVIII protein as described above comprises at least two heterologous moieties inserted into a FVIII protein, wherein at least one of the two heterologous moieties is inserted in A1-1 and wherein the recombinant FVIII protein has procoagulant activity and can be expressed *in vivo* or *in vitro* in a host cell. In one aspect, each of the two heterologous moieties is inserted in A1-1, e.g., upstream or downstream of the substitution or deletion in A1-1. In another aspect, a first heterologous moiety is inserted in A1-1, and a second heterologous moiety is inserted in one of the other permissive loops (*e.g.,* A1-2, A2-1, A2-2, A3-1, A3-2) or in an a3 region. In certain aspects, one of the other permissive loops does not contain a substitution or deletion. In other aspects, at least one heterologous moiety can be further inserted in the B domain or fused to the C-terminus of the FVIII protein.

In certain aspects one or more amino acids in A1-2 in a recombinant FVIII protein are substituted or deleted, wherein the recombinant FVIII protein has procoagulant activity. In some aspects, the recombinant FVIII protein that contains the substitution or deletion is expressed *in vivo* or *in vitro* in a host cell. In other aspects, the one or more amino acids substituted or deleted are in amino acids 218 to 229 corresponding to native mature human FVIII. In certain aspects, at least one heterologous moiety is inserted in A1-2, e.g., upstream or downstream of the one or more amino acids substituted or deleted. In some aspects, at least one heterologous moiety is inserted immediately downstream of amino acid 216 or 220 corresponding to native human FVIII in the A1-2 region. In certain aspects a recombinant FVIII protein as described above comprises at least two heterologous moieties inserted into a FVIII protein, wherein at least one of the two heterologous moieties is inserted in A1-2 and wherein the recombinant FVIII protein has procoagulant activity and can be expressed *in vivo* or *in vitro* in a host cell. In one aspect, each of the two heterologous moieties is inserted in A1-2, *e.g.,* upstream or downstream of the substitution or deletion in A1-2. In another aspect, a first heterologous moiety is inserted in A1-2 and a second heterologous moiety is inserted in one of the other permissive loops *(e.g.,* A1-1, A2-1, A2-2, A3-1, A3-2) or in an a3 region. In certain aspects, one of the other permissive loops does not contain a substitution or deletion. In other aspects, at least one heterologous moiety can further be inserted in the B domain, e.g., amino acid 745 of SEQ ID NO: 1, or fused to the C-terminus of the FVIII protein, e.g., amino acid 2332 of SEQ ID NO: 1.

In certain aspects one or more amino acids in A2-1 in a recombinant FVIII protein are substituted or deleted, wherein the recombinant FVIII protein has procoagulant activity. In some aspects, the recombinant FVIII protein that contains the substitution or deletion is expressed *in vivo* or *in vitro* in a host cell. In other aspects, the one or more amino acids substituted or deleted are in amino acids 400 to 403 corresponding to native mature human FVIII. In certain aspects, at least one heterologous moiety is inserted in A2-1, e.g., upstream or downstream of the one or more amino acids substituted or deleted in A2-1. In some aspects at least one heterologous moiety is inserted immediately downstream of amino acid 399 corresponding to native mature human FVIII. In certain aspects a recombinant FVIII protein as described above comprises at least two heterologous moieties inserted into a FVIII protein, wherein at least one of the two heterologous moieties is inserted in A2-1 and wherein the recombinant FVIII protein has procoagulant activity and can be expressed *in vivo* or *in vitro* in a host cell. In one aspect, each of the two heterologous moieties is inserted in A2-1, *e.g.,* upstream or downstream of the substitution, deletion or a combination thereof in A2-1. In another aspect, a first heterologous moiety is inserted in A2-1, e.g., upstream or downstream of a substitution, deletion or a combination thereof in A2-1, and a second heterologous moiety is inserted in one of the other permissive loops *(e.g.,* A1-1, A1-2, A2-2, A3-1, A3-2) or in an a3 region. In certain aspects, at least one of the other permissive loops does not contain a substitution or deletion. In other aspects, at least one heterologous moiety can be further inserted in the B domain, e.g., amino acid 745 of SEQ ID NO: 1, or fused to the C-terminus of the FVIII protein, e.g., amino acid 2332 of SEQ ID NO: 1.

In certain aspects one or more amino acids in A2-2 in a recombinant FVIII protein are substituted or deleted, wherein the recombinant FVIII protein has procoagulant activity. In some aspects, the recombinant FVIII protein that contains the substitution or deletion is expressed *in vivo* or *in vitro* in a host cell. In other aspects, the one or more amino acids substituted or deleted are in amino acids 595 to 607 corresponding to native mature human FVIII. In certain aspects, at least one heterologous moiety is inserted in A2-2, e.g., upstream or downstream of the one or more amino acids substituted or deleted in A2-2. In some aspects at least one heterologous moiety is inserted immediately downstream of amino acids 599 or 603 corresponding to native mature human FVIII. In certain aspects a recombinant FVIII protein as described above comprises at least two heterologous moieties inserted into a FVIII protein, wherein at least one of the two heterologous moieties is inserted in A2-2 and wherein the recombinant FVIII protein has procoagulant activity and can be expressed *in vivo* or *in vitro* in a host cell. In one aspect, each of the two heterologous moieties is inserted in A2-2, *e.g.,* upstream or downstream of the substitution, deletion or a combination thereof in A2-2. In another aspect, a first heterologous moiety is inserted in A2-2, e.g., upstream or downstream of a substitution, deletion or a combination thereof in A2-2, and a second heterologous moiety is inserted in one of the other permissive loops *(e.g.,* A1-1, A1-2, A2-1, A3-1, A3-2) or in an a3 region. In certain aspects, at least one of the other permissive loops does not contain a substitution or deletion. In other aspects, at least one heterologous moiety can be further inserted in the B domain, e.g., amino acid 745 of SEQ ID NO: 1, or fused to the C-terminus of the FVIII protein, e.g., amino acid 2332 of SEQ ID NO: 1.

In certain aspects one or more amino acids in A3-1 in a recombinant FVIII protein are substituted or deleted, wherein the recombinant FVIII protein has procoagulant activity. In some aspects, the recombinant FVIII protein that contains the substitution or deletion is expressed *in vivo* or *in vitro* in a host cell. In other aspects, the one or more amino acids substituted or deleted are in amino acids 1712 to 1720, amino acids 1712 to 1725, or amino acids 1721 to 1725 corresponding to native mature human FVIII. In certain aspects, at least one heterologous moiety is inserted in A3-1, e.g., upstream or downstream of the one or more amino acids substituted or deleted in A3-1. In some aspects, at least one heterologous moiety is inserted immediately downstream of amino acid 1711 or amino acids 1720 corresponding to native mature human FVIII. In certain aspects a recombinant FVIII protein as described above comprises at least two heterologous moieties inserted into a FVIII protein, wherein at least one of the two heterologous moieties is inserted in A3-1 and wherein the recombinant FVIII protein has procoagulant activity and can be expressed *in vivo* or *in vitro* in a host cell. In one aspect, each of the two heterologous moieties is inserted in A3-1, e.g., upstream or downstream of the substitution, deletion or a combination thereof in A3-1. In another aspect, a first heterologous moiety is inserted in A3-1, e.g., upstream or downstream of a substitution, deletion or a combination thereof in A3-1, and a second heterologous moiety is inserted in one of the other permissive loops *(e.g.,* A1-1, A1-2, A2-1, A2-2, or A3-2) or in an a3 region. In certain aspects, the one of the other permissive loops does not contain a substitution or deletion. In other aspects, at least one heterologous moiety can be further inserted in the B domain, e.g., amino acid 745 of SEQ ID NO: 1, or fused to the C-terminus of the FVIII protein, e.g., amino acid 2332 of SEQ ID NO: 1.

In certain aspects one or more amino acids in A3-2 in a recombinant FVIII protein are substituted or deleted, wherein the recombinant FVIII protein has procoagulant activity. In some aspects, the recombinant FVIII protein that contains the substitution or deletion is expressed *in vivo* or *in vitro* in a host cell. In other aspects, the one or more amino acids substituted or deleted are in amino acids 1901 to 1905, amino acids 1901 to 1910, amino acids 1906 to 1910, amino acids 1901 to 1905, amino acids 1901 to 1910, or amino acids 1906 to 1910 corresponding to native mature human FVIII. In certain aspects, at least one heterologous moiety is inserted in A3-2, e.g., upstream or downstream of the one or more amino acids substituted or deleted in A3-2. In some aspects, at least one heterologous moiety is inserted immediately downstream of amino acid 1900 or amino acids 1905 corresponding to native mature human FVIII. In certain aspects a recombinant FVIII protein as described above comprises at least two heterologous moieties inserted into a FVIII protein, wherein at least one of the two heterologous moieties is inserted in A3-2 and wherein the recombinant FVIII protein has procoagulant activity and can be expressed *in vivo* or *in vitro* in a host cell. In one aspect, each of the two heterologous moieties is inserted in A3-2, e.g., upstream or downstream of the substitution, deletion or a combination thereof in A3-2. In another aspect, a first heterologous moiety is inserted in A3-2, e.g., upstream or downstream of a substitution, deletion or a combination thereof in A3-2, and a second heterologous moiety is inserted in one of the other permissive loops *(e.g.,* A1-1, A1-2, A2-1, A2-2, or A3-1) or in an a3 region. In certain aspects, the one of the other permissive loops does not contain a substitution or deletion. In other aspects, at least one heterologous moiety can be further inserted in the B domain, e.g., amino acid 745 of SEQ ID NO: 1, or fused to the C-terminus of the FVIII protein, e.g., amino acid 2332 of SEQ ID NO: 1.

In certain aspects one or more amino acids in the a3 region in a recombinant FVIII protein are substituted or deleted, wherein the recombinant FVIII protein has procoagulant activity. In some aspects, the recombinant FVIII protein that contains the substitution or deletion is expressed *in vivo* or *in vitro* in a host cell. In other aspects, the one or more amino acids substituted or deleted are in amino acids 1649 to 1689 corresponding to native mature human FVIII. In certain aspects, at least one heterologous moiety is inserted in the a3 region, e.g., upstream or downstream of the one or more amino acids substituted or deleted in the a3 region. In some aspects, at least one heterologous moiety is inserted immediately downstream of amino acid 1656 corresponding to native mature human FVIII. In certain aspects a recombinant FVIII protein as described above comprises at least two heterologous moieties inserted into a FVIII protein, wherein at least one of the two heterologous moieties is inserted in the a3 region and wherein the recombinant FVIII protein has procoagulant activity and can be expressed *in vivo* or *in vitro* in a host cell. In one aspect, each of the two heterologous moieties is inserted in the a3 region, e.g., upstream or downstream of the substitution, deletion or a combination thereof in the a3 region. In another aspect, a first heterologous moiety is inserted in the a3 region, e.g., upstream or downstream of a substitution, deletion or a combination thereof in the a3 region, and a second heterologous moiety is inserted in one of the other permissive loops *(e.g.,* A1-1, A1-2, A2-1, A2-2, A3-1, or A3-1) or in an a3 region. In certain aspects, the one of the other permissive loops does not contain a substitution or deletion. In other aspects, at least one heterologous moiety can be further inserted in the B domain, e.g., amino acid 745 of SEQ ID NO: 1, or fused to the C-terminus of the FVIII protein, e.g., amino acid 2332 of SEQ ID NO: 1.

In certain aspects, the recombinant FVIII protein of the present invention comprises a full or partial deletion of the B domain. In other aspects, the one or more amino acids substituted or deleted in the FVIII protein are in the B domain. In some aspects, the one or more amino acids substituted or deleted in the B domain comprise from about amino acid 741 to about amino acid 1648 corresponding to native mature human FVIII. In one particular aspect, the B-domain is deleted, having an amino acid sequence of SEQ ID NO:2. In other aspects, a recombinant FVIII protein having a deletion or substitution in the B domain comprises a heterologous moiety inserted in at least one of A1-1, A1-2, A2-1, A2-2, A3-1, A3-2, or the a3 region.

In other aspects, a recombinant FVIII protein of the invention comprises a first heterologous moiety inserted into A1-1, A1-2, A2-1, A2-2, A3-1, or A3-2 or a3 region, in which one or more amino acids are substituted or deleted, and a second heterologous moiety inserted into B domain, e.g., immediately downstream of amino acid 745 corresponding to SEQ ID NO: 1.

In some aspects, a recombinant FVIII protein of the invention comprises a first heterologous moiety inserted immediately downstream of amino acid 403 of SEQ ID NO:1 and a second heterologous moiety inserted immediately downstream of amino acid 745 of SEQ ID NO:1, wherein one or more amino acids of amino acids 404 to 417 corresponding to native mature human FVIII are substituted or deleted. In other aspects, a recombinant FVIII protein of the invention comprises a first heterologous moiety inserted immediately downstream of amino acid 1900 corresponding to mature FVIII sequence *(i.e.,* SEQ ID NO: 1) and a second heterologous moiety inserted immediately downstream of amino acid 745 corresponding to SEQ ID NO: 1, wherein one or more amino acids of amino acids 1901 to 1910 corresponding to native mature human FVIII are substituted or deleted. In still other aspects, a recombinant FVIII protein of the invention comprises a first heterologous moiety inserted immediately downstream of amino acid 18 corresponding to SEQ ID NO: 1 and a second heterologous moiety inserted immediately downstream of amino acid 745 corresponding to SEQ ID NO: 1, wherein one or more amino acids of amino acids 19 to 22, amino acids 19 to 26, amino acids 19 to 32, amino acids 19 to 40, amino acids 23 to 26, amino acids 23 to 32, amino acids 23 to 40, amino acids 27 to 32, amino acids 27 to 40, or amino acids 33 to 40 corresponding to native mature human FVIII are substituted or deleted.

In yet other aspects, a recombinant FVIII protein of the invention comprises a first heterologous moiety inserted immediately downstream of amino acid 1656 corresponding to SEQ ID NO:1 and a second heterologous moiety inserted immediately downstream of amino acid 1900 corresponding to SEQ ID NO: 1, wherein one or more amino acids in amino acids 1901 to 1910 corresponding to native mature human FVIII are substituted or deleted. In certain aspects, a recombinant FVIII protein of the invention comprises a first heterologous moiety inserted immediately downstream of amino acid 26 corresponding to SEQ ID NO: 1, a second heterologous moiety inserted immediately downstream of amino acid 1656 corresponding to SEQ ID NO:1, and a third heterologous moiety inserted immediately downstream of amino acid 1900 corresponding to SEQ ID NO: 1, wherein one or more amino acids in amino acids 27 to 40 corresponding to native mature human FVIII or amino acids 1901 to 1910 corresponding to native mature human FVIII are substituted or deleted. In some aspects, the first and second heterologous moieties are identical. In other aspects, the first heterologous moieties are different.

In some embodiments, the FVIII protein of the invention can be a dual chain FVIII comprising the FVIII heavy chain (HC) and the FVIII light chain or a single chain FVIII.

In some aspects, the insertion of at least one heterologous moiety into the permissive loops of the A domains (*e*.*g*., A1-1, A1-2, A2-1, A2-2, A3-1, or A3-2 as described above) or the a3 region in addition to a substitution or deletion of one or more amino acids in the permissive loops or in the a3 region results in an increase in expression level when compared to the expression level of the recombinant FVIII protein without the at least one heterologous moiety inserted in the permissive loops or in the a3 region. In some aspects, the increase in expression level is determined by an activity assay.

In some aspects, the recombinant FVIII protein comprises two heterologous moieties, the first of the two heterologous moieties inserted into one or more of the permissive loops of the A domains (*e*.*g*., A1-1, A1-2, A2-1, A2-2, A3-1, or A3-2 as described above) or into the a3 region, wherein one or more amino acids in the one or more permissive loops and the a3 region are substituted or deleted, and the second of the two heterologous moieties inserted into the a3 region. In some aspects, the recombinant FVIII protein comprises three heterologous moieties, the first and the second of the three heterologous moieties inserted into one or more of the permissive loops of the A domains (*e*.*g*., A1-1, A1-2, A2-1, A2-2, A3-1, or A3-2 as described above) or into the a3 region, wherein one or more amino acids in the one or more permissive loops and the a3 region are substituted or deleted, and the third of the three heterologous moieties inserted into the a3 region. In other aspects, the recombinant FVIII protein comprises more than three heterologous moieties, the first of the more than three heterologous moieties inserted into the a3 region and the rest of the more than three heterologous moieties inserted into one or more of the permissive loops of the A domains (*e*.*g*., A1-1, A1-2, A2-1, A2-2, A3-1, or A3-2 as described above) or into an a3 region, wherein one or more amino acids in the one or more permissive loops and the a3 region are substituted or deleted.

In some aspects, the increase in expression level caused by the insertion of at least one heterologous moiety into the permissive loops of the A domains (*e*.*g*., A1-1, A1-2, A2-1, A2-2, A3-1, or A3-2 as described above) or into the a3 region, wherein one or more amino acids in the one or more permissive loops or the a3 region are substituted or deleted, is an increase of at least about 10%, at least about 20%, at least about 30%, at least about 40%, at least about 50%, at least about 60%, at least about 70%, at least about 80%, at least about 90% or at least about 100% when compared to the expression level of the recombinant FVIII protein without the at least one heterologous moiety inserted in the one or more permissive loops or in the a3 region. In some aspects, the increase in expression level caused by the insertion of at least one heterologous moiety inserted into the permissive loops of the A domains (*e*.*g*., A1-1, A1-2, A2-1, A2-2, A3-1, or A3-2 as described above) or into the a3 region, wherein one or more amino acids in the one or more permissive loops or the a3 region are substituted or deleted, is an increase of at least about 2-fold, at least about 3-fold, at least about 4-fold, at least about 5-fold, at least about 6-fold, at least about 7-fold, at least about 8-fold, at least about 9-fold, or at least about 10-fold when compared to the expression level of the recombinant FVIII protein without the additional heterologous moiety inserted in the one or more permissive loops or the a3 region.

In some aspects, the recombinant FVIII protein comprises multiple heterologous insertions, e.g., more than two, three, four, five, six, seven, eight, nine, or ten insertions, wherein the insertion sites include, but are not limited to, the sites listed in TABLES 10 to 18 or any combinations thereof, and wherein at least one of the insertion sites is located in a permissive loop, *e.g.,* A1-1, A1-2, A2-1, A2-2, A3-1, or A3-2, or in the a3 region, wherein one or more amino acids in the at least one of A1-1, A1-2, A2-1, A2-2, A3-1, or A3-2, or the a3 region are substituted or deleted.

In one aspect, a recombinant FVIII protein comprises two heterologous moieties, wherein at least one of the two heterologous moieties is inserted within a permissive loop or in an a3 region or both of the two heterologous moieties, wherein one or more amino acids in the at least one of A1-1, A1-2, A2-1, A2-2, A3-1, or A3-2, or the a3 region are substituted or deleted. The first and second heterologous moieties can be the same or different. Non-limiting examples of the recombinant FVIII protein comprising two heterologous moieties are listed in TABLE 11. In one example, the first heterologous moiety is inserted in permissive loop A1-1, and the second heterologous moiety is inserted in loop A2-1, wherein one or more amino acids in A1-1, A2-1, or both are substituted or deleted. In another aspect, the first heterologous moiety is inserted in permissive loop A1-1, and the second heterologous moiety is inserted in permissive loop A2-2, wherein one or more amino acids in A1-1, A2-2, or both are substituted or deleted. In another aspect, the first heterologous moiety is inserted in permissive loop A3-1, and the second heterologous moiety is inserted in permissive loop A3-2, wherein one or more amino acids in A3-1, A3-2, or both are substituted or deleted. In another aspect, the first heterologous moiety is inserted in permissive loop A1-1, and the second heterologous moiety is inserted in the a3 region, wherein one or more amino acids in A1-1 are substituted or deleted. In another aspect, the first heterologous moiety is inserted in permissive loop A2-1, and the second heterologous moiety is inserted in the a3 region, wherein one or more amino acids in A2-1 are substituted or deleted. In another aspect, the first heterologous moiety is inserted in permissive loop A2-2, and the second heterologous moiety is inserted in the a3 region, wherein one or more amino acids in A2-2 are substituted or deleted. In another aspect, the first heterologous moiety is inserted in permissive loop A3-1, and the second heterologous moiety is inserted in the a3 region, wherein one or more amino acids in A3-1 are substituted or deleted. In another aspect, the first heterologous moiety is inserted in permissive loop A1-1, and the second heterologous moiety is inserted in permissive loop A3-2, wherein one or more amino acids in A1-1, A3-2, or both are substituted or deleted. In another aspect, the first heterologous moiety is inserted in permissive loop A2-1, and the second heterologous moiety is inserted in permissive loop A3-2, wherein one or more amino acids in A2-1, A3-2, or both are substituted or deleted. In another aspect, the first heterologous moiety is inserted in permissive loop A3-2, and the second heterologous moiety is inserted in the a3 region, wherein one or more amino acids in A3-2 are substituted or deleted. In another aspect, the first heterologous moiety is inserted in permissive loop A1-1, and the second heterologous moiety is inserted in permissive loop A3-1, wherein one or more amino acids in A1-1, A3-1, or both are substituted or deleted. In another aspect, the first heterologous moiety is inserted in permissive loop A2-1, and the second heterologous moiety is inserted in permissive loop A3-1, wherein one or more amino acids in A2-1, A3-1, or both are substituted or deleted.

In another aspect, a recombinant FVIII protein comprises three heterologous moieties, wherein at least one of the three heterologous moieties is inserted in a permissive loop or in an a3 region, at least two of the three heterologous moieties are inserted in two permissive loops and/or in an a3 region, or any combinations thereof, or the three heterologous moieties are inserted in three permissive loops, in the a3 region, or any combinations thereof, and wherein one or more amino acids in A1-1, A1-2, A2-1, A2-2, A3-1, or A3-2, or in the a3 region are substituted or deleted. The first, second, or third heterologous moieties can be the same or different from each other. The first, second, and third heterologous moieties are the same or different. Non-limiting examples of the recombinant FVIII protein comprising three heterologous moieties are in TABLE 12 or 13. In one example, the first heterologous moiety is inserted in permissive loop A1-1, the second heterologous moiety is inserted in permissive loop A2-1, and the third heterologous moiety is inserted in the a3 region, wherein one or more amino acids in A1-1, A2-1, or both are substituted or deleted. In another example, the first heterologous moiety is inserted in permissive loop A1-1, the second heterologous moiety is inserted in permissive loop A2-1, and the third heterologous moiety is inserted in permissive loop A3-1, wherein one or more amino acids in A1-1, A2-1, A3-1, or any combinations thereof are substituted or deleted. In another example, the first heterologous moiety is inserted in permissive loop A1-1, the second heterologous moiety is inserted in permissive loop A3-1, and the third heterologous moiety is inserted in permissive loop A3-2, wherein one or more amino acids in A1-1, A3-1, A3-2, or any combinations thereof are substituted or deleted. In another example, the first heterologous moiety is inserted in permissive loop A2-1, the second heterologous moiety is inserted in the a3 region, and the third heterologous moiety is inserted in permissive loop A3-1, wherein one or more amino acids in A2-1, A3-1, or both are substituted or deleted. In another example, the first heterologous moiety is inserted in permissive loop A2-1, the second heterologous moiety is inserted in the a3 region, and the third heterologous moiety is inserted in permissive loop A3-2, wherein one or more amino acids in A2-1, A3-2, or both are substituted or deleted. In another aspect, the first heterologous moiety is inserted in the a3 region, the second heterologous moiety is inserted in permissive loop A3-1, and the third heterologous moiety is inserted in permissive loop A3-2, wherein one or more amino acids in A3-1, A3-2, or both are substituted or deleted. In another aspect, the first heterologous moiety is inserted in permissive loop A1-1, the second heterologous moiety is inserted in the B domain, and the third heterologous moiety is inserted at the carboxy terminus position (CT), wherein one or more amino acids in A1-1 are substituted or deleted. In another aspect, the first heterologous moiety is inserted in permissive loop A2-1, the second heterologous moiety is inserted in the B domain, and the third heterologous moiety is inserted at the CT, wherein one or more amino acids in A2-1 are substituted or deleted. In another aspect, the first heterologous moiety is inserted in permissive loop A3-1, the second heterologous moiety is inserted in the B domain, and the third heterologous moiety is inserted at the CT, wherein one or more amino acids in A3-1 are substituted or deleted. In another aspect, the first heterologous moiety is inserted in permissive loop A3-2, the second heterologous moiety is inserted in the B domain, and the third heterologous moiety is inserted at the CT, wherein one or more amino acids in A3-2 are substituted or deleted. In some embodiments, the FVIII protein comprising three heterologous moieties contains a deletion from amino acid 745 to amino acid 1685 corresponding to SEQ ID NO: 1 or amino acid 745 to amino acid 1656 corresponding to SEQ ID NO: 1 or a mutation or substitution at amino acid 1648 *(e.g.,* R1648A), 1680 (Y1680F), or both, wherein one or more amino acids in A1-1, A1-2, A2-1, A2-2, A3-1, or A3-2 or in the a3 region are substituted or deleted.

In another aspect, a recombinant FVIII protein comprises four heterologous moieties, wherein at least one of the four heterologous moieties is inserted within a permissive loop or in an a3 region, at least two of the four heterologous moieties are inserted within two permissive loop, in an a3 region, or any combinations thereof, at least three of the four heterologous moieties are inserted within three permissive loops, in an a3 region, or any combinations thereof, or all of the four heterologous moieties are inserted within four permissive loops, in an a3 region, or any combinations thereof, wherein one or more amino acids in at least one or more of the permissive loops or the a3 region are substituted or deleted. Non-limiting examples of the recombinant FVIII protein comprising four heterologous moieties are listed in TABLE 14 or 15. The first, second, third, or fourth heterologous moieties are the same or different. In one example, the first heterologous moiety is inserted in permissive loop A1-1, the second heterologous moiety is inserted in permissive loop A2-1, the third heterologous moiety is inserted in the a3 region, and the fourth heterologous moiety is inserted in permissive loop A3-1, wherein one or more amino acids in A1-1, A2-1, A3-1, or any combinations thereof are substituted or deleted. In another example, the first heterologous moiety is inserted in permissive loop A1-1, the second heterologous moiety is inserted in permissive loop A2-1, the third heterologous moiety is inserted in the a3 region, and the fourth heterologous moiety is inserted in permissive loop A3-2, wherein one or more amino acids in A1-1, A2-1, A3-2, or any combinations thereof are substituted or deleted. In another example, the first heterologous moiety is inserted in permissive loop A1-1, the second heterologous moiety is inserted in permissive loop A2-1, the third heterologous moiety is inserted in permissive loop A3-1, and the fourth heterologous moiety is inserted in permissive loop A3-2, wherein one or more amino acids in A1-1, A2-1, A3-1, A3-2, or any combinations thereof are substituted or deleted. In another aspect, the first heterologous moiety is inserted in permissive loop A1-1, the second heterologous moiety is inserted in the a3 region, the third heterologous moiety is inserted in permissive loop A3-1, and the fourth heterologous moiety is inserted in permissive loop A3-2, wherein one or more amino acids in A1-1, A3-1, A3-2, or any combinations thereof are substituted or deleted. In another aspect, the first heterologous moiety is inserted in permissive loop A2-1, the second heterologous moiety is inserted in the a3 region, the third heterologous moiety is inserted in permissive loop A3-1, and the fourth heterologous moiety is inserted in permissive loop A3-2, wherein one or more amino acids in A2-1, A3-1, A3-2, or any combinations thereof are substituted or deleted. In another aspect, the first heterologous moiety is inserted in permissive loop A1-1, the second heterologous moiety is inserted in permissive loop A2-1, the third heterologous moiety is inserted in the B domain, and the fourth heterologous moiety is inserted at the CT, wherein one or more amino acids in A1-1, A2-1, or both are substituted or deleted. In another aspect, the first heterologous moiety is inserted in permissive loop A1-1, the second heterologous moiety is inserted in permissive loop A3-1, the third heterologous moiety is inserted in the B domain, and the fourth heterologous moiety is inserted at the CT, wherein one or more amino acids in A1-1, A3-1, or both are substituted or deleted. In another aspect, the first heterologous moiety is inserted in permissive loop A1-1, the second heterologous moiety is inserted in permissive loop A3-2, the third heterologous moiety is inserted in the B domain, and the fourth heterologous moiety is inserted at the CT, wherein one or more amino acids in A1-1, A3-2, or both are substituted or deleted. In another aspect, the first heterologous moiety is inserted in permissive loop A1-1, the second heterologous moiety is inserted in permissive loop A3-2, the third heterologous moiety is inserted in the B domain, and the fourth heterologous moiety is inserted at the CT, wherein one or more amino acids in A1-1, A3-2, or both are substituted or deleted. In another aspect, the first heterologous moiety is inserted in permissive loop A2-1, the second heterologous moiety is inserted in permissive loop A3-1, the third heterologous moiety is inserted in the B domain, and the fourth heterologous moiety is inserted at the CT, wherein one or more amino acids in A2-1, A3-1, or both are substituted or deleted. In another aspect, the first heterologous moiety is inserted in permissive loop A2-1, the second heterologous moiety is inserted in permissive loop A3-2, the third heterologous moiety is inserted in the B domain, and the fourth heterologous moiety is inserted at the CT, wherein one or more amino acids in A2-1, A3-2, or both are substituted or deleted. In another aspect, the first heterologous moiety is inserted in permissive loop A3-1, the second heterologous moiety is inserted in permissive loop A3-2, the third heterologous moiety is inserted in the B domain, and the fourth heterologous moiety is inserted at the CT, wherein one or more amino acids in A3-1, A3-2, or both are substituted or deleted. In another aspect, the first heterologous moiety is inserted in permissive loop A2-1, the second heterologous moiety is inserted in the a3 region, the third heterologous moiety is inserted in permissive loop A3-1, and the fourth heterologous moiety is inserted at the CT, wherein one or more amino acids in A2-1, A3-1, or both are substituted or deleted. In another aspect, the first heterologous moiety is inserted in permissive loop A2-1, the second heterologous moiety is inserted in the a3 region, the third heterologous moiety is inserted in permissive loop A3-2, and the fourth heterologous moiety is inserted at the CT, wherein one or more amino acids in A2-1, A3-2, or both are substituted or deleted. In another aspect, the first heterologous moiety is inserted in the a3 region, the second heterologous moiety is inserted in permissive loop A3-1, the third heterologous moiety is inserted in permissive loop A3-2, and the fourth heterologous moiety is inserted at the CT, wherein one or more amino acids in A3-1, A3-2, or both are substituted or deleted. In another aspect, the first heterologous moiety is inserted in permissive loop A1-1, the second heterologous moiety is inserted in permissive loop A2-1, the third heterologous moiety is inserted in the a3 region, and the fourth heterologous moiety is inserted at the CT, wherein one or more amino acids in A1-1, A2-1, or both are substituted or deleted. In another aspect, the first heterologous moiety is inserted in permissive loop A1-1, the second heterologous moiety is inserted in permissive loop A2-1, the third heterologous moiety is inserted in permissive loop A3-1, and the fourth heterologous moiety is inserted at the CT, wherein one or more amino acids in A1-1, A2-1, A3-1, or any combinations thereof are substituted or deleted. In another aspect, the first heterologous moiety is inserted in permissive loop A1-1, the second heterologous moiety is inserted in permissive loop A2-1, the third heterologous moiety is inserted in permissive loop A3-2, and the fourth heterologous moiety is inserted at the CT, wherein one or more amino acids in A1-1, A2-1, A3-2, or any combinations thereof are substituted or deleted. In another aspect, the first heterologous moiety is inserted in permissive loop A1-1, the second heterologous moiety is inserted in the a3 region, the third heterologous moiety is inserted in permissive loop A3-1, and the fourth heterologous moiety is inserted at the CT, wherein one or more amino acids in A1-1, A3-1, or both are substituted or deleted. In another aspect, the first heterologous moiety is inserted in permissive loop A1-1, the second heterologous moiety is inserted in the a3 region, the third heterologous moiety is inserted in permissive loop A3-2, and the fourth heterologous moiety is inserted at the CT, wherein one or more amino acids in A1-1, A3-2, or both are substituted or deleted. In another aspect, the first heterologous moiety is inserted in permissive loop A1-1, the second heterologous moiety is inserted in permissive loop A3-1, the third heterologous moiety is inserted in permissive loop A3-2, and the fourth heterologous moiety is inserted at the CT, wherein one or more amino acids in A1-1, A3-1, A3-2, or any combinations thereof are substituted or deleted.

In another aspect, a recombinant FVIII protein comprises five heterologous moieties, wherein at least one of the five heterologous moieties is inserted within a permissive loop or in an a3 region, at least two of the five heterologous moieties are inserted within two permissive loops, in an a3 region, or any combinations thereof, at least three of the five heterologous moieties are inserted within three permissive loops, in an a3 region, or any combinations thereof, at least four of the five heterologous moieties are inserted within four permissive loops, in an a3 region, or any combinations thereof, or all of the five heterologous moieties are inserted within five permissive loops, in an a3 region, or any combinations thereof, wherein one or more amino acids in the at least one, at least two, at least three, at least four, or at least five of the permissive loops or the a3 region are substituted or deleted. The first, second, third, fourth, and fifth heterologous moieties are the same or different. Non-limiting examples of the recombinant FVIII protein comprising five heterologous moieties are in TABLE 16. In one example, the first heterologous moiety is inserted in permissive loop A2-1, the second heterologous moiety is inserted in the a3 region, the third heterologous moiety is inserted in permissive loop A3-1, the fourth heterologous moiety is inserted in permissive loop A3-2, and the fifth heterologous moiety is inserted at the CT, wherein one or more amino acids in A2-1, A3-1, A3-2, or any combinations thereof are substituted or deleted. In another aspect, the first heterologous moiety is inserted in permissive loop A1-1, the second heterologous moiety is inserted in permissive loop A2-1, the third heterologous moiety is inserted in the a3 region, the fourth heterologous moiety is inserted in permissive loop A3-1, and the fifth heterologous moiety is inserted at the CT, wherein one or more amino acids in A1-1, A2-1, A3-1, or any combinations thereof are substituted or deleted. In another aspect, the first heterologous moiety is inserted in permissive loop A1-1, the second heterologous moiety is inserted in permissive loop A2-1, the third heterologous moiety is inserted in the a3 region, the fourth heterologous moiety is inserted in permissive loop A3-2, and the fifth heterologous moiety is inserted at the CT, wherein one or more amino acids in A1-1, A2-1, A3-2, or any combinations thereof are substituted or deleted. In another aspect, the first heterologous moiety is inserted in permissive loop A1-1, the second heterologous moiety is inserted in permissive loop A2-1, the third heterologous moiety is inserted in permissive loop A3-1, the fourth heterologous moiety is inserted in permissive loop A3-2, and the fifth heterologous moiety is inserted at the CT, wherein one or more amino acids in A1-1, A2-1, A3-1, A3-2, or any combinations thereof are substituted or deleted. In another aspect, the first heterologous moiety is inserted in permissive loop A1-1, the second heterologous moiety is inserted in the a3 region, the third heterologous moiety is inserted in permissive loop A3-1, the fourth heterologous moiety is inserted in permissive loop A3-2, and the fifth heterologous moiety is inserted at the CT, wherein one or more amino acids in A1-1, A3-1, A3-2, or any combinations thereof are substituted or deleted. In another aspect, the first heterologous moiety is inserted in permissive loop A1-1, the second heterologous moiety is inserted in permissive loop A2-1, the third heterologous moiety is inserted in the B domain, the fourth heterologous moiety is inserted in permissive loop A3-1, and the fifth heterologous moiety is inserted at the CT, wherein one or more amino acids in A1-1, A2-1, A3-1, or any combinations thereof are substituted or deleted. In another aspect, the first heterologous moiety is inserted in permissive loop A1-1, the second heterologous moiety is inserted in permissive loop A2-1, the third heterologous moiety is inserted in the B domain, the fourth heterologous moiety is inserted in permissive loop A3-2, and the fifth heterologous moiety is inserted at the CT, wherein one or more amino acids in A1-1, A2-1, A3-2, or any combinations thereof are substituted or deleted. In another aspect, the first heterologous moiety is inserted in permissive loop A1-1, the second heterologous moiety is inserted in the B domain, the third heterologous moiety is inserted in permissive loop A3-1, the fourth heterologous moiety is inserted in permissive loop A3-2, and the fifth heterologous moiety is inserted at the CT, wherein one or more amino acids in A1-1, A3-1, A3-2, or any combinations thereof are substituted or deleted. In another aspect, the first heterologous moiety is inserted in permissive loop A2-1, the second heterologous moiety is inserted in the B domain, the third heterologous moiety is inserted in permissive loop A3-1, the fourth heterologous moiety is inserted in permissive loop A3-2, and the fifth heterologous moiety is inserted at the CT, wherein one or more amino acids in A2-1, A3-1, A3-2, or any combinations thereof are substituted or deleted.

In another aspect, a recombinant FVIII protein comprises six heterologous moieties, wherein at least one of the six heterologous moieties is inserted within a permissive loop or in an a3 region, at least two of the six heterologous moieties are inserted within two permissive loops, in an a3 region, or any combinations thereof, at least three of the six heterologous moieties are inserted within three permissive loops, in an a3 region, or any combinations thereof, at least four of the six heterologous moieties are inserted within four permissive loops, in an a3 region, or any combinations thereof, at least five of the six heterologous moieties are inserted within five permissive loops, in an a3 region, or any combinations thereof, or all of the six heterologous moieties are inserted within six permissive loops, in an a3 region, or any combinations thereof, wherein one or more amino acids in A1-1, A1-2, A2-1, A2-2, A3-1, or A3-2, or in the a3 region are substituted or deleted. The first, second, third, fourth, fifth, and sixth heterologous moieties are the same or different. Examples of the recombinant FVIII protein comprising six heterologous moieties include, but are not limited to, TABLE 17. In one example, the first heterologous moiety is inserted in permissive loop A1-1, the second heterologous moiety is inserted in permissive loop A2-1, the third heterologous moiety is inserted in the a3 region, the fourth heterologous moiety is inserted in permissive loop A3-1, the fifth heterologous moiety is inserted in permissive loop A3-2, and the sixth heterologous moiety is inserted at the CT, wherein one or more amino acids in A1-1, A2-1, A3-1, A3-2, or any combinations thereof are substituted or deleted. In another aspect, the first heterologous moiety is inserted in permissive loop A1-1, the second heterologous moiety is inserted in permissive loop A2-1, the third heterologous moiety is inserted in the B domain, the fourth heterologous moiety is inserted in permissive loop A3-1, the fifth heterologous moiety is inserted in permissive loop A3-2, and the sixth heterologous moiety is inserted at the CT, wherein one or more amino acids in A1-1, A2-1, A3-1, A3-2, or any combinations thereof are substituted or deleted.

In certain aspects, a recombinant FVIII protein comprises one heterologous moiety inserted immediately downstream of an amino acid selected from the group consisting of the amino acids in TABLE 10, wherein one or more amino acids in A1-1, A1-2, A2-1, A2-2, A3-1, or A3-2, or in the a3 region or any combinations thereof are substituted or deleted. In other aspects, a recombinant FVIII protein comprises two heterologous moieties inserted immediately downstream of two amino acids, each of the two amino acids selected from the group consisting of the amino acid in TABLE 10, wherein one or more amino acids in A1-1, A1-2, A2-1, A2-2, A3-1, or A3-2, or in the a3 region or any combinations thereof are substituted or deleted. In a particular embodiment, the two heterologous moieties are inserted in the two insertion sites selected from the group consisting of the insertion sites in TABLE 11, wherein one or more amino acids in A1-1, A1-2, A2-1, A2-2, A3-1, or A3-2, or in the a3 region or any combinations thereof are substituted or deleted. In still other aspects, a recombinant FVIII protein comprises three heterologous moieties inserted immediately downstream of three amino acids, each of the three amino acids selected from the group consisting of the amino acid in TABLE 10, wherein one or more amino acids in A1-1, A1-2, A2-1, A2-2, A3-1, or A3-2, or in the a3 region or any combinations thereof are substituted or deleted. In a specific embodiment, the three heterologous moieties are inserted in the three insertion sites selected from the group consisting of the insertion sites in TABLES 12 and 13, wherein one or more amino acids in A1-1, A1-2, A2-1, A2-2, A3-1, or A3-2, or in the a3 region or any combinations thereof are substituted or deleted. In yet other aspects, a recombinant FVIII protein comprises four heterologous moieties inserted immediately downstream of four amino acids, each of the four amino acids selected from the group consisting of the amino acid in TABLE 10, wherein one or more amino acids in A1-1, A1-2, A2-1, A2-2, A3-1, or A3-2, or in the a3 region or any combinations thereof are substituted or deleted. In a particular embodiment, the four heterologous moieties are inserted in the four insertion sites selected from the group consisting of the insertion sites in TABLES 14 and 15, wherein one or more amino acids in A1-1, A1-2, A2-1, A2-2, A3-1, or A3-2, or in the a3 region or any combinations thereof are substituted or deleted. In some aspects, a recombinant FVIII protein comprises five heterologous moieties inserted immediately downstream of five amino acids, each of the five amino acids selected from the group consisting of the amino acid in TABLE 10, wherein one or more amino acids in A1-1, A1-2, A2-1, A2-2, A3-1, or A3-2, or in the a3 region or any combinations thereof are substituted or deleted. In a particular embodiment, the five heterologous moieties are inserted in the five insertion sites selected from the group consisting of the insertion sites in TABLE 16, wherein one or more amino acids in A1-1, A1-2, A2-1, A2-2, A3-1, or A3-2, or in the a3 region or any combinations thereof are substituted or deleted. In certain aspects, a recombinant FVIII protein comprises six heterologous moieties inserted immediately downstream of six amino acids, each of the six amino acids selected from the group consisting of the amino acid in TABLE 10, wherein one or more amino acids in A1-1, A1-2, A2-1, A2-2, A3-1, or A3-2, or in the a3 region or any combinations thereof are substituted or deleted. In a particular embodiment, the six heterologous moieties are inserted in the six insertion sites selected from the group consisting of the insertion sites in TABLE 17, wherein one or more amino acids in A1-1, A1-2, A2-1, A2-2, A3-1, or A3-2, or in the a3 region or any combinations thereof are substituted or deleted.

In some aspects, a recombinant FVIII protein comprises one heterologous moiety inserted immediately downstream of an amino acid position corresponding to amino acid 26 of SEQ ID NO:1, amino acid 403 of SEQ ID NO:1, amino acid 1720 of SEQ ID NO:1, or amino acid 1900 of SEQ ID NO:1 in mature native human FVIII, and an additional heterologous moiety inserted immediately downstream of an amino acid corresponding to amino acid 1656 of SEQ ID NO:1, wherein one or more amino acids of amino acids 27 to 40, amino acids 404 to 417, amino acids 1721 to 1724, or any combinations thereof are substituted or deleted. In some aspects, a recombinant FVIII protein comprises two heterologous moieties inserted immediately downstream of two amino acid positions corresponding to amino acid 26 of SEQ ID NO:1, amino acid 403 of SEQ ID NO:1, amino acid 1720 of SEQ ID NO:1, or amino acid 1900 of SEQ ID NO:1 in mature native human FVIII, and an additional heterologous moiety inserted immediately downstream of an amino acid corresponding to amino acid 1656 of SEQ ID NO:1, wherein one or more amino acids of amino acids 27 to 40, amino acids 404 to 417, amino acids 1721 to 1724, amino acids 1901 to 1910, or any combinations thereof are substituted or deleted. In some aspects, a recombinant FVIII protein comprises three heterologous moieties inserted immediately downstream of three amino acid positions corresponding to amino acid 26 of SEQ ID NO: 1, amino acid 403 of SEQ ID NO: 1, amino acid 1720 of SEQ ID NO:1, or amino acid 1900 of SEQ ID NO:1 in mature native human FVIII, and an additional heterologous moiety inserted immediately downstream of an amino acid corresponding to amino acid 1656 of SEQ ID NO: 1, wherein one or more amino acids of amino acids 27 to 40, amino acids 404 to 417, amino acids 1721 to 1724, amino acids 1901 to 1910, or any combinations thereof are substituted or deleted.

### 3. Heterologous Moieties

A recombinant FVIII protein of the invention can comprise at least one heterologous moiety inserted into one or more permissive loops or into the a3 region, or both in which one or more amino acids are substituted or deleted, wherein the recombinant FVIII protein has procoagulant activity and can be expressed *in vivo* or *in vitro* in a host cell. A "heterologous moiety" can comprise a heterologous polypeptide, or a non-polypeptide moiety, or both. In certain aspects a recombinant FVIII protein of the invention comprises at least one heterologous moiety inserted into one or more permissive loops or into the a3 region, or both, in which one or more amino acids are substituted or deleted, wherein the heterologous moiety is not an XTEN sequence. In some aspects a recombinant FVIII protein comprises at least one heterologous moiety inserted into one or more permissive loops or into the a3 region, or both, wherein the heterologous moiety is a half-life extending moiety *(e.g.,* an *in vivo* half-life extending moiety), but is not an XTEN sequence.

Non-limiting examples of heterologous moieties (*e*.*g*., a half-life extending moiety) that can be inserted into a recombinant FVIII protein include albumin, albumin fragments, Fc fragments of immunoglobulins, the C-terminal peptide (CTP) of the β subunit of human chorionic gonadotropin, a HAP sequence, a transferrin, the PAS polypeptides of U.S. Pat Application No. 20100292130, polyglycine linkers, polyserine linkers, peptides and short polypeptides of 6-40 amino acids of two types of amino acids selected from glycine (G), alanine (A), serine (S), threonine (T), glutamate (E) and proline (P) with varying degrees of secondary structure from less than 50% to greater than 50%, amongst others, would be suitable for insertion in the identified active insertions sites of FVIII.

In certain aspects a heterologous moiety increases the *in vivo* or *in vitro* half-life of the recombinant FVIII protein. In other aspects a heterologous moiety facilitates visualization or localization of the recombinant FVIII protein. Visualization and/or location of the recombinant FVIII protein can be *in vivo, in vitro, ex vivo,* or combinations thereof. In other aspects a heterologous moiety increases stability of the recombinant FVIII protein. As used herein, the term "stability" refers to an art-recognized measure of the maintenance of one or more physical properties of the recombinant FVIII protein in response to an environmental condition (*e*.*g*., an elevated or lowered temperature). In certain aspects, the physical property can be the maintenance of the covalent structure of the recombinant FVIII protein *(e.g.,* the absence of proteolytic cleavage, unwanted oxidation or deamidation). In other aspects, the physical property can also be the presence of the recombinant FVIII protein in a properly folded state *(e.g.,* the absence of soluble or insoluble aggregates or precipitates). In one aspect, the stability of the recombinant FVIII protein is measured by assaying a biophysical property of the recombinant FVIII protein, for example thermal stability, pH unfolding profile, stable removal of glycans, solubility, biochemical function (*e*.*g*., ability to bind to another protein), etc., and/or combinations thereof. In another aspect, biochemical function is demonstrated by the binding affinity of the interaction. In one aspect, a measure of protein stability is thermal stability, *i.e.,* resistance to thermal challenge. Stability can be measured using methods known in the art, such as, HPLC (high performance liquid chromatography), SEC (size exclusion chromatography), DLS (dynamic light scattering), *etc.* Methods to measure thermal stability include, but are not limited to differential scanning calorimetry (DSC), differential scanning fluorometry (DSF), circular dichroism (CD), and thermal challenge assay.

In a specific aspect, a heterologous moiety inserted in one or more permissive loop, the a3 region, or both, in which one or more amino acids are substituted or deleted, retains the biochemical activity of the recombinant FVIII protein. In one embodiment, the biochemical activity is FVIII activity, which can be measured by chromogenic assay.

In some embodiments, heterologous moieties can be inserted indirectly in the position immediately upstream of the residue being substituted or deleted or in an insertion site via linkers located at the N-terminus, the C-terminus, or both the N-terminus and C-terminus of the heterologous moiety. The linkers at the N-terminus and C-terminus of the heterologous moiety can be the same or different. In some embodiments, several linkers can flank one or both termini of the heterologous moiety in tandem. In some embodiments, the linker is "Gly-Ser peptide linker." The term "Gly-Ser peptide linker" refers to a peptide that consists of glycine and serine residues.

An exemplary Gly/Ser peptide linker comprises the amino acid sequence (Gly₄Ser)ₙ (SEQ ID NO:60), wherein n is an integer that is the same or higher than 1, 2, 3, 4, 5, 6, 7, 8, 9, 10, 15, 20, 25, 30, 35, 40, 46, 50, 55, 60, 70, 80, 90, or 100. In one embodiment, n=1, *i.e.,* the linker is (Gly₄Ser) (SEQ ID NO: 191). In one embodiment, n=2, *i.e.,* the linker is (Gly₄Ser)₂ (SEQ ID NO: 192). In another embodiment, n=3, *i.e.,* the linker is (Gly₄Ser)₃ (SEQ ID NO: 193). In another embodiment, n=4, *i.e.,* the linker is (Gly₄Ser)₄ (SEQ ID NO: 194). In another embodiment, n=5, *i.e.,* the linker is (Gly₄Ser)₅ (SEQ ID NO:195). In yet another embodiment, n=6, *i.e.,* the linker is (Gly₄Ser)₆ (SEQ ID NO: 196). In another embodiment, n=7, *i.e.,* the linker is (Gly₄Ser)₇ (SEQ ID NO: 197). In yet another embodiment, n=8, *i.e.,* the linker is (Gly₄Ser)₈ (SEQ ID NO: 198). In another embodiment, n=9, *i.e.,* the linker is (Gly₄Ser)₉ (SEQ ID NO: 199). In yet another embodiment, n=10, *i.e.,* the linker is (Gly₄Ser)₁₀ (SEQ ID NO:200).

Another exemplary Gly/Ser peptide linker comprises the amino acid sequence Ser(Gly₄Ser)ₙ (SEQ ID NO: 201), wherein n is an integer that is the same or higher than 1, 2, 3, 4, 5, 6, 7, 8, 9, 10, 15, 20, 25, 30, 35, 40, 46, 50, 55, 60, 70, 80, 90, or 100. In one embodiment, n=1, *i.e.,* the linker is Ser(Gly₄Ser) (SEQ ID NO:202). In one embodiment, n=2, *i.e.,* the linker is Ser(Gly₄Ser)₂ (SEQ ID NO:203). In another embodiment, n=3, *i.e.,* the linker is Ser(Gly₄Ser)₃ (SEQ ID NO:204). In another embodiment, n=4, *i.e.,* the linker is Ser(Gly₄Ser)₄ (SEQ ID NO:205). In another embodiment, n=5, *i.e.,* the linker is Ser(Gly₄Ser)₅ (SEQ ID NO:206). In yet another embodiment, n=6, *i.e.,* the linker is Ser(Gly₄Ser)₆ (SEQ ID NO:207). In yet another embodiment, n=7, *i.e.,* the linker is Ser(Gly₄Ser)₇ (SEQ ID NO:208). In yet another embodiment, n=8, *i.e.,* the linker is Ser(Gly₄Ser)₈ (SEQ ID NO:209). In yet another embodiment, n=9, *i.e.,* the linker is Ser(Gly₄Ser)₉ (SEQ ID NO:210). In yet another embodiment, n=10, *i.e.,* the linker is Ser(Gly₄Ser)₁₀ (SEQ ID NO:211).

### 3.1 Half-Life Extension

In certain aspects, a recombinant FVIII protein of the invention comprises at least one heterologous moiety which increases the half-life of the protein, *e.g., in vivo* half-life of the protein. Half-life of a recombinant FVIII protein can be determined by any method known to those of skill in the art, *e.g.,* FVIII activity assays (chromogenic assay or one stage clotting aPTT assay) to detect plasma FVIII activity levels or FVIII ELISA to detect plasma FVIII antigen level. In a particular embodiment, half-life of the clotting activity of a recombinant FVIII protein is determined by one stage clotting assay. In a more particular embodiment, half-life of the clotting activity of a recombinant FVIII protein is determined in mice, either HemA mice or FVIII and von Willebrand Factor double knockout (DKO) mice.

In certain aspects, a heterologous moiety which increases half-life of the recombinant FVIII protein of the invention can comprise, without limitation, a heterologous polypeptide such as albumin, an immunoglobulin Fc region, an XTEN sequence, the C-terminal peptide (CTP) of the β subunit of human chorionic gonadotropin, a PAS sequence, a HAP sequence, a transferrin, albumin-binding moieties, or any fragments, derivatives, variants, or combinations of these polypeptides. In certain aspects the recombinant FVIII protein of the invention comprises a heterologous polypeptide which increases half-life, wherein the heterologous polypeptide is not an XTEN sequence. In other related aspects a heterologous moiety can include an attachment site for a non-polypeptide moiety such as polyethylene glycol (PEG), hydroxyethyl starch (HES), polysialic acid, or any derivatives, variants, or combinations of these moieties.

In other embodiments, a recombinant FVIII protein of the invention is conjugated to one or more polymers. The polymer can be water-soluble or non-water-soluble. The polymer can be covalently or non-covalently attached to FVIII or to other moieties conjugated to FVIII. Non-limiting examples of the polymer can be poly(alkylene oxide), poly(vinyl pyrrolidone), poly(vinyl alcohol), polyoxazoline, or poly(acryloylmorpholine). Additional types of polymer-conjugated FVIII are disclosed in U.S. Patent No. 7,199,223.

In certain aspects, a recombinant FVIII protein of the invention can comprise one, two, three or more heterologous moieties, which can each be the same or different molecules.

In other embodiments, the recombinant FVIII protein of the invention can comprise one or more inserted heterologous moieties, wherein the heterologous moiety completely or partially replaces one or more permissive loops. In one particular embodiment, the heterologous moiety completely or partially replaces A1-1, A2-1, A3-1, A3-2, or any combinations thereof. In further embodiments, the heterologous moiety is an XTEN, *e.g.,* AE42 and/or AE144. In still other embodiments, the recombinant FVIII protein comprises a full or partial deletion of the B domain.

### 3.1.1. Fc regions

In certain aspects, a recombinant FVIII protein of the invention comprises at least one Fc region inserted into a permissive loop, or into the a3 region, or both, wherein the recombinant FVIII protein has procoagulant activity and can be expressed *in vivo* or *in vitro* in a host cell. "Fc" or "Fc region" as used herein, means a functional neonatal Fc receptor (FcRn) binding partner comprising an Fc domain, variant, or fragment thereof, unless otherwise specified. An FcRn binding partner is any molecule that can be specifically bound by the FcRn receptor with consequent active transport by the FcRn receptor of the FcRn binding partner. Thus, the term Fc includes any variants of IgG Fc that are functional. The region of the Fc portion of IgG that binds to the FcRn receptor has been described based on X-ray crystallography (Burmeister et al., Nature 372:379 (1994)). The major contact area of the Fc with the FcRn is near the junction of the CH2 and CH3 domains. Fc-FcRn contacts are all within a single Ig heavy chain. FcRn binding partners include, but are not limited to, whole IgG, the Fc fragment of IgG, and other fragments of IgG that include the complete binding region of FcRn. An Fc can comprise the CH2 and CH3 domains of an immunoglobulin with or without the hinge region of the immunoglobulin. Also included are Fc fragments, variants, or derivatives which maintain the desirable properties of an Fc region in a chimeric protein, *e.g.,* an increase in half-life, *e.g., in vivo* half-life. Myriad mutants, fragments, variants, and derivatives are described, *e.g*., in PCT Publication Nos. WO 2011/069164 A2, WO 2012/006623 A2, WO 2012/006635 A2, or WO 2012/006633 A2.

### 3.1.2 Albumins

In certain aspects, a recombinant FVIII protein of the invention comprises at least one albumin polypeptide or fragment, variant, or derivative thereof inserted into a permissive loop or into the a3 region, or both, wherein the recombinant FVIII protein has procoagulant activity and can be expressed *in vivo* or *in vitro* in a host cell. Human serum albumin (HSA, or HA), a protein of 609 amino acids in its full-length form, is responsible for a significant proportion of the osmotic pressure of serum and also functions as a carrier of endogenous and exogenous ligands. The term "albumin" as used herein includes full-length albumin or a functional fragment, variant, derivative, or analog thereof. Examples of albumin or the fragments or variants thereof are disclosed in US Pat. Publ. Nos. 2008/0194481A1, 2008/0004206 A1, 2008/0161243 A1, 2008/0261877 A1, or 2008/0153751 A1 or PCT Appl. Publ. Nos. 2008/033413 A2, 2009/058322 A1, or 2007/021494 A2.

The albumin-binding polypeptides (ABPs) can compromise, without limitation, bacterial albumin-binding domains, albumin-binding peptides, or albumin-binding antibody fragments that can bind to albumin. Domain 3 from streptococcal protein G, as disclosed by Kraulis et al., FEBS Lett. 378:190-194 (1996) and Linhult et al., Protein Sci. 11:206-213 (2002) is an example of a bacterial albumin-binding domain. Examples of albumin-binding peptides include a series of peptides having the core sequence DICLPRWGCLW (SEQ ID NO:45). See, *e.g.,* Dennis et al., J. Biol. Chem. 2002, 277: 35035-35043 (2002). Examples of albumin-binding antibody fragments are disclosed in Muller and Kontermann, Curr. Opin. Mol. Ther. 9:319-326 (2007); Roovers et al., Cancer Immunol. Immunother. 56:303-317 (2007), and Holt et al., Prot. Eng. Design Sci., 21:283-288 (2008).

In certain aspects, a recombinant FVIII polypeptide of the invention comprises at least one attachment site for a non-polypeptide small molecule, variant, or derivative that can bind to albumin thereof inserted into a permissive loop or into the a3 region, or both, wherein the recombinant FVIII protein has procoagulant activity and can be expressed *in vivo* or *in vitro* in a host cell. For example, a recombinant FVIII protein of the invention can include one or more organic albumin-binding moieties attached in one or more permissive loops or in the a3 region, wherein the recombinant FVIII protein has procoagulant activity and can be expressed *in vivo* or *in vitro* in a host cell. An example of such albumin-binding moieties is 2-(3-maleimidopropanamido)-6-(4-(4-iodophenyl)butanamido)hexanoate ("Albu" tag) as disclosed by Trussel et al., Bioconjugate Chem. 20:2286-2292 (2009).

In some embodiments, the albumin-binding polypeptide sequence is flanked at the C-terminus, the N-terminus, or both termini, by a Gly-Ser peptide linker sequence. In some embodiments, the Gly-Ser peptide linker is Gly₄Ser (SEQ ID NO: 191). In other embodiments, the Gly-Ser peptide linker is (Gly₄Ser)₂ (SEQ ID NO: 192).

### 3.1.3 XTENs

In certain aspects, a recombinant FVIII protein of the invention comprises at least one XTEN polypeptide or fragment, variant, or derivative thereof inserted into a permissive loop or into the a3 region, or both, wherein the recombinant protein has procoagulant activity and can be expressed *in vivo* or *in vitro* in a host cell. As used here "XTEN sequence" refers to extended length polypeptides with non-naturally occurring, substantially non-repetitive sequences that are composed mainly of small hydrophilic amino acids, with the sequence having a low degree or no secondary or tertiary structure under physiologic conditions. As a chimeric protein partner, XTENs can serve as a carrier, conferring certain desirable pharmacokinetic, physicochemical and pharmaceutical properties, *e.g*., when inserted into a permissive loop or a3 region of a recombinant FVIII protein of the invention. Such desirable properties include but are not limited to enhanced pharmacokinetic parameters and solubility characteristics.

An XTEN sequence inserted into a recombinant FVIII protein of the invention can confer to the recombinant protein one or more of the following advantageous properties: conformational flexibility, enhanced aqueous solubility, high degree of protease resistance, low immunogenicity, low binding to mammalian receptors, or increased hydrodynamic (or Stokes) radii. In certain aspects, an XTEN sequence can increase pharmacokinetic properties such as longer half-life *(e.g., in vivo* half-life) or increased area under the curve (AUC), so that a recombinant FVIII protein of the invention stays *in vivo* and has procoagulant activity for an increased period of time compared to the native FVIII.

Examples of XTEN sequences that can be inserted into recombinant FVIII proteins of the invention are disclosed, *e.g.,* in U.S. Patent Publication Nos. 2010/0239554 A1, 2010/0323956 A1, 2011/0046060 A1, 2011/0046061 A1, 2011/0077199 A1, or 2011/0172146 A1, or International Patent Publication Nos. WO 2010091122 A1, WO 2010144502 A2, WO 2010144508 A1, WO 2011028228 A1, WO 2011028229 A1, or WO 2011028344 A2.

Exemplary XTEN sequences which can be inserted into recombinant FVIII proteins of the invention include XTEN AE42-4 (SEQ ID NO: 13), XTEN 144-2A (SEQ ID NO:15), XTEN A144-3B (SEQ ID NO:17), XTEN AE144-4A (SEQ ID NO:19), XTEN AE144-5A (SEQ ID NO:21), XTEN AE144-6B (SEQ ID NO:23), XTEN AG144-1 (SEQ ID NO:25), XTEN AG144-A (SEQ ID NO:27), XTEN AG144-B (SEQ ID NO:29), XTEN AG144-C (SEQ ID NO:31), and XTEN AG144-F (SEQ ID NO:33).

In certain aspects, a recombinant FVIII protein comprises at least one heterologous moiety inserted into the a3 region of FVIII *(e.g.,* an insertion site which corresponds to amino acid 1656 of SEQ ID NO:1), either alone or in combination with one or more heterologous moieties being inserted into the permissive loops of the A domains (*e.g*., A1-1, A1-2, A2-1, A2-2, A3-1, or A3-2 as described above) or in the a3 region, wherein one or more amino acids in the permissive loops of the A domains or in the a3 region are substituted or deleted and wherein at least one of the heterologous moieties is an XTEN sequence. In some aspects, two of the heterologous moieties are XTEN sequences. In some aspects, three of the heterologous moieties are XTEN sequences. In some aspects, four of the heterologous moieties are XTEN sequences. In some aspects, five of the heterologous moieties are XTEN sequences. In some aspects, six or more of the heterologous moieties are XTEN sequences.

In some aspects, a recombinant FVIII protein comprises one or more XTEN sequences in an insertion site within a permissive loop, *e.g.,* A1-1, A1-2, A2-1, A2-2, A3-1, A3-2, or a3 region, or any combinations thereof, wherein one or more amino acids in the permissive loop or in the a3 region are substituted or deleted. In one embodiment, the one or more XTEN sequences are inserted within A1-1, wherein one or more amino acids in A1-1 are substituted or deletedI is disclosed that the one or more XTEN sequences are inserted within A1-2, wherein one or more amino acids in A2-1 are substituted or deleted. It is disclosed that the one or more XTEN sequences are inserted within A2-1, wherein one or more amino acids in A2-1 are substituted or deleted. It is disclosed that the one or more XTEN sequences are inserted within A2-2, wherein one or more amino acids in A2-2 are substituted or deleted. It is further disclosed that the one or more XTEN sequences are inserted within A3-1, wherein one or more amino acids in A3-1 are substituted or deleted. It is disclosed that the one or more XTEN sequences are inserted within A3-2, wherein one or more amino acids in A3-1 are substituted or deleted. It is disclosed that the one or more XTEN sequences are inserted within the a3 region, wherein one or more amino acids in the a3 region are substituted or deleted.

In certain aspects, a recombinant FVIII protein comprises one XTEN sequence inserted at an insertion site listed in TABLE 10, wherein one or more amino acids in at least one of A1-1, A1-2, A2-1, A2-2, A3-1, A3-2, or the a3 region are substituted or deleted. In other aspects, a recombinant FVIII protein comprises two XTEN sequences inserted in two insertion sites listed in TABLE 10, wherein one or more amino acids in at least one of A1-1, A1-2, A2-1, A2-2, A3-1, A3-2, or the a3 region are substituted or deleted. In a particular embodiment, the two XTEN sequences are inserted in two insertion sites listed in TABLE 11, wherein one or more amino acids in at least one of A1-1, A1-2, A2-1, A2-2, A3-1, A3-2, or the a3 region are substituted or deleted. In still other aspects, a recombinant FVIII protein comprises three XTEN sequences inserted in three insertion sites listed in TABLE 10, wherein one or more amino acids in at least one of A1-1, A1-2, A2-1, A2-2, A3-1, A3-2, or the a3 region are substituted or deleted. In a specific aspect, the three XTEN sequences are inserted in three insertion sites listed in TABLE 12, TABLE 13 or both tables, wherein one or more amino acids in at least one of A1-1, A1-2, A2-1, A2-2, A3-1, A3-2, or the a3 region are substituted or deleted. In yet other aspects, a recombinant FVIII protein comprises four XTEN sequences inserted in four insertion sites listed in TABLE 10, wherein one or more amino acids in at least one of A1-1, A1-2, A2-1, A2-2, A3-1, A3-2, or the a3 region are substituted or deleted. In a particular aspect, the four XTEN sequences are inserted in four insertion sites listed in TABLE 14, TABLE 15, or both, wherein one or more amino acids in at least one of A1-1, A1-2, A2-1, A2-2, A3-1, A3-2, or the a3 region are substituted or deleted. In some aspects, a recombinant FVIII protein comprises five XTEN sequences inserted in five insertion sites listed in TABLE 10, wherein one or more amino acids in at least one of A1-1, A1-2, A2-1, A2-2, A3-1, A3-2, or the a3 region are substituted or deleted. In a particular aspect, the five XTEN sequences are inserted in five insertion sites listed in TABLE 16, wherein one or more amino acids in at least one of A1-1, A1-2, A2-1, A2-2, A3-1, A3-2, or the a3 region are substituted or deleted. In certain aspects, a recombinant FVIII protein comprises six XTEN sequences inserted in six insertion sites listed in TABLE 10, wherein one or more amino acids in at least one of A1-1, A1-2, A2-1, A2-2, A3-1, A3-2, or the a3 region are substituted or deleted. In a particular embodiment, the six XTEN sequences are inserted in six insertion sites listed in TABLE 17, wherein one or more amino acids in at least one of A1-1, A1-2, A2-1, A2-2, A3-1, A3-2, or the a3 region are substituted or deleted. In some aspects, all the inserted XTEN sequences are identical. In other aspects, at least one of the inserted XTEN sequences is different from the rest of inserted XTEN sequences.

In some aspects, a recombinant FVIII protein comprises one XTEN sequence inserted immediately downstream of an amino acid position corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional XTEN sequence inserted immediately downstream of an amino acid corresponding to amino acid 1656 of SEQ ID NO:1. In some aspects, a recombinant FVIII protein comprises two XTEN sequences inserted immediately downstream of two amino acid positions corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional XTEN sequence inserted immediately downstream of an amino acid corresponding to amino acid 1656 of SEQ ID NO:1. In some aspects, a recombinant FVIII protein comprises three XTEN sequences inserted immediately downstream of three amino acid positions corresponding to amino acid 26 of SEQ ID NO:1 with a substitution or deletion of amino acids 27 to 40 of SEQ ID NO: 1 or a portion thereof, amino acid 403 of SEQ ID NO:1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional XTEN sequence inserted immediately downstream of an amino acid corresponding to amino acid 1656 of SEQ ID NO:1.

### 3.1.4 CTP

In certain aspects, a recombinant FVIII protein of the invention comprises at least one C-terminal peptide (CTP) of the β subunit of human chorionic gonadotropin or fragment, variant, or derivative thereof inserted into a permissive loop or into the a3 region, or both, wherein the recombinant FVIII protein has procoagulant activity and can be expressed *in vivo* or *in vitro* in a host cell. One or more CTP peptides inserted into a recombinant protein is known to increase the half-life of that protein. *See, e.g.,* U.S. Patent No. 5,712,122, . Exemplary CTP peptides include DPRFQDSSSSKAPPPSLPSPSRLPGPSDTPIL (SEQ ID NO:35) or SSSSKAPPPSLPSPSRLPGPSDTPILPQ. (SEQ ID NO:36). *See, e.g.,* U.S. Patent Application Publication No. US 2009/0087411 A1. In some embodiments, the CTP sequence is flanked at the C-terminus, the N-terminus, or both termini, by a Gly-Ser peptide linker sequence. In some embodiments, the Gly-Ser peptide linker is Gly₄Ser (SEQ ID NO: 191). In other embodiments, the Gly-Ser peptide linker is (Gly₄Ser)₂ (SEQ ID NO:192).

In certain aspects, a recombinant FVIII protein comprises at least one heterologous moiety inserted into one or more heterologous moieties being inserted into the permissive loops of the A domains (*e.g.*, A1-1, A1-2, A2-1, A2-2, A3-1, or A3-2 as described above) or in the a3 region, wherein one or more amino acids in the permissive loops of the A domains or in the a3 region are substituted or deleted or wherein at least one of the heterologous moieties is a CTP sequence. In some aspects, two of the heterologous moieties are CTP sequences. In some aspects, three of the heterologous moieties are CTP sequences. In some aspects, four of the heterologous moieties are CTP sequences. In some aspects, five of the heterologous moieties are CTP sequences. In some aspects, six or more of the heterologous moieties are CTP sequences.

In some aspects, a recombinant FVIII protein comprises one or more CTP sequences in an insertion site within a permissive loop, *e.g.,* A1-1, A1-2, A2-1, A2-2, A3-1, A3-2, a3, or any combinations thereof, wherein one or more amino acids in the permissive loop or the a3 region are substituted or deleted. In one embodiment, the one or more CTP sequences are inserted within A1-1, wherein one or more amino acids in A1-1 are substituted or deleted. It is disclosed that the one or more CTP sequences are inserted within A1-2, wherein one or more amino acids in A1-2 are substituted or deleted. It is disclosed that the one or more CTP sequences are inserted within A2-1, wherein one or more amino acids in A2-1 are substituted or deleted. It is disclosed that the one or more CTP sequences are inserted within A2-2, wherein one or more amino acids in A2-2 are substituted or deleted. It is disclosed that the one or more CTP sequences are inserted within A3-1, wherein one or more amino acids in A3-1 are substituted or deleted. It is disclosed that the one or more CTP sequences are inserted within A3-2, wherein one or more amino acids in A3-2 are substituted or deleted. It is disclosed that the one or more CTP sequences are inserted within the a3 region, wherein one or more amino acids in the a3 region are substituted or deleted.

In certain aspects, a recombinant FVIII protein comprises one CTP sequence inserted at an insertion site listed in TABLE 10, wherein one or more amino acids in A1-1, A1-2, A2-1, A2-2, A3-1, or A3-2, or in the a3 region are substituted or deleted. In other aspects, a recombinant FVIII protein comprises two CTP sequences inserted in two insertion sites listed in TABLE 10, wherein one or more amino acids in A1-1, A1-2, A2-1, A2-2, A3-1, or A3-2, or in the a3 region are substituted or deleted. In a particular embodiment, the two CTP sequences are inserted in two insertion sites listed in TABLE 11, wherein one or more amino acids in A1-1, A1-2, A2-1, A2-2, A3-1, or A3-2, or in the a3 region are substituted or deleted. In still other aspects, a recombinant FVIII protein comprises three CTP sequences inserted in three insertion sites listed in TABLE 10, wherein one or more amino acids in A1-1, A1-2, A2-1, A2-2, A3-1, or A3-2, or in the a3 region are substituted or deleted. In a specific aspect, the three CTP sequences are inserted in three insertion sites listed in TABLE 12, TABLE 13 or both tables, wherein one or more amino acids in A1-1, A1-2, A2-1, A2-2, A3-1, or A3-2, or in the a3 region are substituted or deleted. In yet other aspects, a recombinant FVIII protein comprises four CTP sequences inserted in four insertion sites listed in TABLE 10, wherein one or more amino acids in A1-1, A1-2, A2-1, A2-2, A3-1, or A3-2, or in the a3 region are substituted or deleted. In a particular aspect, the four CTP sequences are inserted in four insertion sites listed in TABLE 14, TABLE 15, or both, wherein one or more amino acids in A1-1, A1-2, A2-1, A2-2, A3-1, or A3-2, or in the a31 region are substituted or deleted. In some aspects, a recombinant FVIII protein comprises five CTP sequences inserted in five insertion sites listed in TABLE 10, wherein one or more amino acids in A1-1, A1-2, A2-1, A2-2, A3-1, or A3-2, or in the a3 region are substituted or deleted. In a particular aspect, the five CTP sequences are inserted in five insertion sites listed in TABLE 16, wherein one or more amino acids in A1-1, A1-2, A2-1, A2-2, A3-1, or A3-2, or in the a3 region are substituted or deleted. In certain aspects, a recombinant FVIII protein comprises six CTP sequences inserted in six insertion sites listed in TABLE 10, wherein one or more amino acids in A1-1, A1-2, A2-1, A2-2, A3-1, or A3-2, or in the a3 region are substituted or deleted. In a particular embodiment, the six CTP sequences are inserted in six insertion sites listed in TABLE 17, wherein one or more amino acids in A1-1, A1-2, A2-1, A2-2, A3-1, or A3-2, or in the a3 region are substituted or deleted. In some aspects, all the inserted CTP sequences are identical. In other aspects, at least one of the inserted CTP sequences is different from the rest of inserted CTP sequences.

In some aspects, a recombinant FVIII protein comprises one CTP sequence inserted immediately downstream of an amino acid position corresponding to amino acid 26 of SEQ ID NO:1 with a substitution or deletion of amino acids 27 to 40 of SEQ ID NO: 1 or a portion thereof, amino acid 403 of SEQ ID NO:1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional CTP sequence inserted immediately downstream of an amino acid corresponding to amino acid 1656 of SEQ ID NO:1. In some aspects, a recombinant FVIII protein comprises two CTP sequences inserted immediately downstream of two amino acid positions corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO: 1 with a substitution or deletion of amino acids 1901 to 1910 of SEQ ID NO: 1 or a portion thereof, and an additional CTP sequence inserted immediately downstream of an amino acid corresponding to amino acid 1656 of SEQ ID NO:1. In some aspects, a recombinant FVIII protein comprises three CTP sequences inserted immediately downstream of three amino acid positions corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO: 1 with a substitution or deletion of amino acids 1901 to 1910 of SEQ ID NO: 1 or a portion thereof, and an additional CTP sequence inserted immediately downstream of an amino acid corresponding to amino acid 1656 of SEQ ID NO:1.

### 3.1.5 PAS

In certain aspects, a recombinant FVIII protein of the invention comprises at least one PAS peptide or fragment, variant, or derivative thereof inserted into a permissive loop or into the a3 region, or both, wherein the recombinant FVIII protein has procoagulant activity and can be expressed *in vivo* or *in vitro* in a host cell. A PAS peptide or PAS sequence, as used herein, means an amino acid sequence comprising mainly alanine and serine residues or comprising mainly alanine, serine, and proline residues, the amino acid sequence forming random coil conformation under physiological conditions. Accordingly, the PAS sequence is a building block, an amino acid polymer, or a sequence cassette comprising, consisting essentially of, or consisting of alanine, serine, and proline which can be used as a part of the heterologous moiety in the chimeric protein. An amino acid polymer also can form random coil conformation when residues other than alanine, serine, and proline are added as a minor constituent in the PAS sequence. By "minor constituent" is meant that that amino acids other than alanine, serine, and proline can be added in the PAS sequence to a certain degree, *e.g.,* up to about 12%, *i.e.,* about 12 of 100 amino acids of the PAS sequence, up to about 10%, up to about 9%, up to about 8%, about 6%, about 5%, about 4%, about 3%, *i.e.* about 2%, or about 1%, of the amino acids. The amino acids different from alanine, serine and proline cab be selected from the group consisting of Arg, Asn, Asp, Cys, Gln, Glu, Gly, His, Ile, Leu, Lys, Met, Phe, Thr, Trp, Tyr, and Val. Under physiological conditions, a PAS peptide forms a random coil conformation and thereby can mediate an increased *in vivo* and/or *in vitro* stability to a recombinant protein of the invention, and has procoagulant activity.

Non-limiting examples of the PAS peptides include ASPAAPAPASPAAPAPSAPA (SEQ ID NO: 37), AAPASPAPAAPSAPAPAAPS (SEQ ID NO:38), APSSPSPSAPSSPSPASPSS (SEQ ID NO:39), APSSPSPSAPSSPSPASPS (SEQ ID NO:40), SSPSAPSPSSPASPSPSSPA (SEQ ID NO:41), AASPAAPSAPPAAASPAAPSAPPA (SEQ ID NO:42), ASAAAPAAASAAASAPSAAA (SEQ ID NO:43) or any variants, derivatives, fragments, or combinations thereof. Additional examples of PAS sequences are known from, *e.g.,* US Pat. Publ. No. 2010/0292130 A1 and PCT Appl. Publ. No. WO 2008/155134 A1. European issued patent EP2173890. In some embodiments, the PAS sequence is flanked at the C-terminus, the N-terminus, or both termini, by a Gly-Ser peptide linker sequence. In some embodiments, the Gly-Ser peptide linker is Gly₄Ser (SEQ ID NO:191). In other embodiments, the Gly-Ser peptide linker is (Gly₄Ser)₂ (SEQ ID NO: 192).

In certain aspects, a recombinant FVIII protein comprises at least one heterologous moiety inserted into the permissive loops of the A domains *(e.g.,* A1-1, A1-2, A2-1, A2-2, A3-1, or A3-2 as described above) or into the a3 region, wherein one or more amino acids in at least one of the permissive loops of the A domains or the a3 region are substituted or deleted and wherein at least one of the heterologous moieties is a PAS sequence. In some aspects, two of the heterologous moieties are PAS sequences. In some aspects, three of the heterologous moieties are PAS sequences. In some aspects, four of the heterologous moieties are PAS sequences. In some aspects, five of the heterologous moieties are PAS sequences. In some aspects, six or more of the heterologous moieties are PAS sequences.

In some aspects, a recombinant FVIII protein comprises one or more PAS sequences in an insertion site within a permissive loop, *e.g.,* A1-1, A1-2, A2-1, A2-2, A3-1, A3-2, a3, or any combinations thereof, wherein one or more amino acids in the permissive loop or the a3 region are substituted or deleted. In one embodiment, the one or more PAS sequences are inserted within A1-1, wherein one or more amino acids in A1-1 are substituted or deleted. In another embodiment, the one or more PAS sequences are inserted within A1-2, wherein one or more amino acids in A1-2 are substituted or deleted. In other embodiments, the one or more PAS sequences are inserted within A2-1, wherein one or more amino acids in A2-1 are substituted or deleted. In still other embodiments, the one or more PAS sequences are inserted within A2-2, wherein one or more amino acids in A2-2 are substituted or deleted. In yet other embodiments, the one or more PAS sequences are inserted within A3-1, wherein one or more amino acids in A3-1 are substituted or deleted. In some embodiments, the one or more PAS sequences are inserted within A3-2, wherein one or more amino acids in A3-2 are substituted or deleted. In certain embodiments, the one or more PAS sequences are inserted within the a3 region, wherein one or more amino acids in the a3 region are substituted or deleted.

In certain aspects, a recombinant FVIII protein comprises one PAS sequence inserted at an insertion site listed in TABLE 10, wherein one or more amino acids in A1-1, A1-2, A2-1, A2-2, A3-1, or A3-2, or in the a3 region are substituted or deleted. In other aspects, a recombinant FVIII protein comprises two PAS sequences inserted in two insertion sites listed in TABLE 10, wherein one or more amino acids in A1-1, A1-2, A2-1, A2-2, A3-1, or A3-2, or in the a3 region are substituted or deleted. In a particular embodiment, the two PAS sequences are inserted in two insertion sites listed in TABLE 11, wherein one or more amino acids in A1-1, A1-2, A2-1, A2-2, A3-1, or A3-2, or in the a3 region are substituted or deleted. In still other aspects, a recombinant FVIII protein comprises three PAS sequences inserted in three insertion sites listed in TABLE 10, wherein one or more amino acids in A1-1, A1-2, A2-1, A2-2, A3-1, or A3-2, or in the a3 region are substituted or deleted. In a specific aspect, the three PAS sequences are inserted in three insertion sites listed in TABLE 12, TABLE 13 or both tables, wherein one or more amino acids in A1-1, A1-2, A2-1, A2-2, A3-1, or A3-2, or in the a3 region are substituted or deleted. In yet other aspects, a recombinant FVIII protein comprises four PAS sequences inserted in four insertion sites listed in TABLE 10, wherein one or more amino acids in A1-1, A1-2, A2-1, A2-2, A3-1, or A3-2, or in the a3 region are substituted or deleted. In a particular aspect, the four PAS sequences are inserted in four insertion sites listed in TABLE 14, TABLE 15, or both, wherein one or more amino acids in A1-1, A1-2, A2-1, A2-2, A3-1, or A3-2, or in the a3 region are substituted or deleted. In some aspects, a recombinant FVIII protein comprises five PAS sequences inserted in five insertion sites listed in TABLE 10, wherein one or more amino acids in A1-1, A1-2, A2-1, A2-2, A3-1, or A3-2, or in the a3 region are substituted or deleted. In a particular aspect, the five PAS sequences are inserted in five insertion sites listed in TABLE 16, wherein one or more amino acids in A1-1, A1-2, A2-1, A2-2, A3-1, or A3-2, or in the a3 region are substituted or deleted. In certain aspects, a recombinant FVIII protein comprises six PAS sequences inserted in six insertion sites listed in TABLE 10, wherein one or more amino acids in A1-1, A1-2, A2-1, A2-2, A3-1, or A3-2, or in the a3 region are substituted or deleted. In a particular embodiment, the six PAS sequences are inserted in six insertion sites listed in TABLE 17, wherein one or more amino acids in A1-1, A1-2, A2-1, A2-2, A3-1, or A3-2, or in the a3 region are substituted or deleted. In some aspects, all the inserted PAS sequences are identical. In other aspects, at least one of the inserted PAS sequences is different from the rest of inserted PAS sequences.

In some aspects, a recombinant FVIII protein comprises one PAS sequence inserted immediately downstream of an amino acid position corresponding to amino acid 26 of SEQ ID NO:1 with a substitution or deletion of amino acids 27 to 40 of SEQ ID NO: 1 or a portion thereof, amino acid 403 of SEQ ID NO:1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional PAS sequence inserted immediately downstream of an amino acid corresponding to amino acid 1656 of SEQ ID NO:1. In some aspects, a recombinant FVIII protein comprises two PAS sequences inserted immediately downstream of two amino acid positions corresponding to amino acid 26 of SEQ ID NO:1 with a substitution or deletion of amino acids 27 to 40 of SEQ ID NO: 1 or a portion thereof, amino acid 403 of SEQ ID NO:1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO: 1 with a substitution or deletion of amino acids 1901 to 1910 of SEQ ID NO: 1 or a portion thereof, and an additional PAS sequence inserted immediately downstream of an amino acid corresponding to amino acid 1656 of SEQ ID NO:1. In some aspects, a recombinant FVIII protein comprises three PAS sequences inserted immediately downstream of three amino acid positions corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional PAS sequence inserted immediately downstream of an amino acid corresponding to amino acid 1656 of SEQ ID NO:1.

### 3.1.6 HAP

In certain aspects, a recombinant FVIII protein of the invention comprises at least one homo-amino acid polymer (HAP) peptide or fragment, variant, or derivative thereof inserted into a permissive loop or into the a3 region, or both, wherein the recombinant FVIII protein has procoagulant activity and can be expressed *in vivo* or *in vitro* in a host cell. A HAP peptide can comprise a repetitive sequence of glycine, which has at least 50 amino acids, at least 100 amino acids, 120 amino acids, 140 amino acids, 160 amino acids, 180 amino acids, 200 amino acids, 250 amino acids, 300 amino acids, 350 amino acids, 400 amino acids, 450 amino acids, or 500 amino acids in length. A HAP sequence is capable of extending half-life of a moiety fused to or linked to the HAP sequence. Non-limiting examples of the HAP sequence includes, but are not limited to (Gly)ₙ, (Gly₄Ser)ₙ or S(Gly₄Ser)ₙ, wherein n is 1, 2, 3, 4, 5, 6, 7, 8, 9, 10, 11, 12, 13, 14, 15, 16, 17, 18, 19, or 20. In one embodiment, n is 20, 21, 22, 23, 24, 25, 26, 26, 28, 29, 30, 31, 32, 33, 34, 35, 36, 37, 38, 39, or 40. In another embodiment, n is 50, 60, 70, 80, 90, 100, 110, 120, 130, 140, 150, 160, 170, 180, 190, or 200. See, *e.g.,* Schlapschy M et al., Protein Eng. Design Selection, 20: 273-284 (2007).

In certain aspects, a recombinant FVIII protein comprises at least one heterologous moiety inserted into one or more heterologous moieties being inserted into the permissive loops of the A domains (*e.g*., A1-1, A1-2, A2-1, A2-2, A3-1, or A3-2 as described above) or into the a3 region, wherein one or more amino acids in at least one of the permissive loops of the A domains or the a3 region are substituted or deleted and wherein at least one of the heterologous moieties is a HAP sequence. In some aspects, two of the heterologous moieties are HAP sequences. In some aspects, three of the heterologous moieties are HAP sequences. In some aspects, four of the heterologous moieties are HAP sequences. In some aspects, five of the heterologous moieties are HAP sequences. In some aspects, six or more of the heterologous moieties are HAP sequences.

In some aspects, a recombinant FVIII protein comprises one or more HAP sequences in an insertion site within a permissive loop, *e.g.,* A1-1, A1-2, A2-1, A2-2, A3-1, A3-2, a3, or any combinations thereof, wherein one or more amino acids in the permissive loop or the a3 region are substituted or deleted. In one embodiment, the one or more HAP sequences are inserted within A1-1, wherein one or more amino acids in A1-1 are substituted or deleted. It is disclosed that, the one or more HAP sequences are inserted within A1-2, wherein one or more amino acids in A1-2 are substituted or deleted. It is disclosed that the one or more HAP sequences are inserted within A2-1, wherein one or more amino acids in A2-1 are substituted or deleted. It is further disclosed that the one or more HAP sequences are inserted within A2-2, wherein one or more amino acids in A2-2 are substituted or deleted. It is disclosed that the one or more HAP sequences are inserted within A3-1, wherein one or more amino acids in A3-1 are substituted or deleted. It is disclosed that the one or more HAP sequences are inserted within A3-2, wherein one or more amino acids in A3-2 are substituted or deleted. It is disclosed that, the one or more HAP sequences are inserted within the a3 region, wherein one or more amino acids in the a3 region are substituted or deleted.

In certain aspects, a recombinant FVIII protein comprises one HAP sequence inserted at an insertion site listed in TABLE 10, wherein one or more amino acids in A1-1, A1-2, A2-1, A2-2, A3-1, or A3-2, or in the a3 region are substituted or deleted. In other aspects, a recombinant FVIII protein comprises two HAP sequences inserted in two insertion sites listed in TABLE 10, wherein one or more amino acids in A1-1, A1-2, A2-1, A2-2, A3-1, or A3-2, or in the a3 region are substituted or deleted. In a particular embodiment, the two HAP sequences are inserted in two insertion sites listed in TABLE 11, wherein one or more amino acids in A1-1, A1-2, A2-1, A2-2, A3-1, or A3-2, or in the a3 region are substituted or deleted. In still other aspects, a recombinant FVIII protein comprises three HAP sequences inserted in three insertion sites listed in TABLE 10, wherein one or more amino acids in A1-1, A1-2, A2-1, A2-2, A3-1, or A3-2, or in the a3 region are substituted or deleted. In a specific aspect, the three HAP sequences are inserted in three insertion sites listed in TABLE 12, TABLE 13 or both tables, wherein one or more amino acids in A1-1, A1-2, A2-1, A2-2, A3-1, or A3-2, or in the a3 region are substituted or deleted. In yet other aspects, a recombinant FVIII protein comprises four HAP sequences inserted in four insertion sites listed in TABLE 10, wherein one or more amino acids in A1-1, A1-2, A2-1, A2-2, A3-1, or A3-2, or in the a3 region are substituted or deleted. In a particular aspect, the four HAP sequences are inserted in four insertion sites listed in TABLE 14, TABLE 15, or both, wherein one or more amino acids in A1-1, A1-2, A2-1, A2-2, A3-1, or A3-2, or in the a3 region are substituted or deleted. In some aspects, a recombinant FVIII protein comprises five HAP sequences inserted in five insertion sites listed in TABLE 10, wherein one or more amino acids in A1-1, A1-2, A2-1, A2-2, A3-1, or A3-2, or in the a3 region are substituted or deleted. In a particular aspect, the five HAP sequences are inserted in five insertion sites listed in TABLE 16. In certain aspects, a recombinant FVIII protein comprises six HAP sequences inserted in six insertion sites listed in TABLE , wherein one or more amino acids in A1-1, A1-2, A2-1, A2-2, A3-1, or A3-2, or in the a3 region are substituted or deleted. In a particular embodiment, the six HAP sequences are inserted in six insertion sites listed in TABLE 17, wherein one or more amino acids in A1-1, A1-2, A2-1, A2-2, A3-1, or A3-2, or in the a3 region are substituted or deleted. In some aspects, all the inserted HAP sequences are identical. In other aspects, at least one of the inserted HAP sequences is different from the rest of inserted HAP sequences.

In some aspects, a recombinant FVIII protein comprises one HAP sequence inserted immediately downstream of an amino acid position corresponding to amino acid 26 of SEQ ID NO:1 with a substitution or deletion of amino acids 27 to 40 of SEQ ID NO: 1 or a portion thereof, amino acid 403 of SEQ ID NO:1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional HAP sequence inserted immediately downstream of an amino acid corresponding to amino acid 1656 of SEQ ID NO:1. In some aspects, a recombinant FVIII protein comprises two HAP sequences inserted immediately downstream of two amino acid positions corresponding to amino acid 26 of SEQ ID NO:1 with a substitution or deletion of amino acids 27 to 40 of SEQ ID NO: 1 or a portion thereof, amino acid 403 of SEQ ID NO:1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional HAP sequence inserted immediately downstream of an amino acid corresponding to amino acid 1656 of SEQ ID NO:1. In some aspects, a recombinant FVIII protein comprises three HAP sequences inserted immediately downstream of three amino acid positions corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional HAP sequence inserted immediately downstream of an amino acid corresponding to amino acid 1656 of SEQ ID NO:1.

### 3.1.7 Transferrin

In certain aspects, a recombinant FVIII protein of the invention comprises at least one transferrin peptide or fragment, variant, or derivative thereof inserted into a permissive loop or into the a3 region, or both, wherein the recombinant FVIII protein has procoagulant activity and can be expressed *in vivo* or *in vitro* in a host cell. Any transferrin can be into a recombinant FVIII protein of the invention. As an example, wild-type human Tf (Tf) is a 679 amino acid protein, of approximately 75 kDa (not accounting for glycosylation), with two main domains, N (about 330 amino acids) and C (about 340 amino acids), which appear to originate from a gene duplication. *See* GenBank accession numbers NM001063, XM002793, M12530, XM039845, XM 039847 and S95936 (www.ncbi.nlm.nih.gov).

Transferrin transports iron through transferrin receptor (TfR)-mediated endocytosis. After the iron is released into an endosomal compartment and Tf-TfR complex is recycled to cell surface, the Tf is released back extracellular space for next cycle of iron transporting. Tf possesses a long half-life that is in excess of 14-17 days (Li et al., Trends Pharmacol. Sci. 23:206-209 (2002)).Transferrin fusion proteins have been studied for half-life extension, targeted deliver for cancer therapies, oral delivery and sustained activation of proinsulin (Brandsma et al., Biotechnol. Adv., 29: 230-238 (2011); Bai et al., Proc. Natl. Acad. Sci. USA 102:7292-7296 (2005); Kim et al., J. Pharmacol. Exp. Ther., 334:682-692 (2010); Wang et al., J. Controlled Release 155:386-392 (2011)).

In some embodiments, the transferrin sequence is flanked at the C-terminus, the N-terminus, or both termini, by a Gly-Ser peptide linker sequence. In some embodiments, the Gly-Ser peptide linker is Gly₄Ser (SEQ ID NO:191). In other embodiments, the Gly-Ser peptide linker is (Gly₄Ser)₂ (SEQ ID NO:192).

In certain aspects, a recombinant FVIII protein comprises at least one heterologous moiety inserted into the permissive loops of the A domains *(e.g.,* A1-1, A1-2, A2-1, A2-2, A3-1, or A3-2 as described above) or into the a3 region, wherein one or more amino acids in at least one of the permissive loops of the A domains or the a3 region are substituted or deleted and wherein at least one of the heterologous moieties is a transferrin sequence. In some aspects, two of the heterologous moieties are transferrin sequences. In some aspects, three of the heterologous moieties are transferrin sequences. In some aspects, four of the heterologous moieties are transferrin sequences. In some aspects, five of the heterologous moieties are transferrin sequences. In some aspects, six or more of the heterologous moieties are transferrin sequences.

In some aspects, a recombinant FVIII protein comprises one or more transferrin sequences in an insertion site within a permissive loop, *e.g.,* A1-1, A1-2, A2-1, A2-2, A3-1, A3-2, a3, or any combinations thereof, wherein one or more amino acids in at least one of the permissive loop or the a3 region are substituted or deleted. In one embodiment, the one or more transferrin sequences are inserted within A1-1, wherein one or more amino acids in A1-1 are substituted or deleted. It is disclosed that the one or more transferrin sequences are inserted within A1-2, wherein one or more amino acids in A1-2 are substituted or deleted. It is disclosed that the one or more transferrin sequences are inserted within A2-1, wherein one or more amino acids in A2-1 are substituted or deleted. It is disclosed that the one or more transferrin sequences are inserted within A2-2, wherein one or more amino acids in A2-2 are substituted or deleted. It is disclosed that the one or more transferrin sequences are inserted within A3-1, wherein one or more amino acids in A3-1 are substituted or deleted. It is disclosed that the one or more transferrin sequences are inserted within A3-2, wherein one or more amino acids in A3-2 are substituted or deleted. It is disclosed that the one or more transferrin sequences are inserted within the a3 region, wherein one or more amino acids in the a3 region are substituted or deleted.

In certain aspects, a recombinant FVIII protein comprises one transferrin sequence inserted at an insertion site listed in TABLE 10, wherein one or more amino acids in A1-1, A1-2, A2-1, A2-2, A3-1, or A3-2, or in the a3 region are substituted or deleted. In other aspects, a recombinant FVIII protein comprises two transferrin sequences inserted in two insertion sites listed in TABLE 10, wherein one or more amino acids in A1-1, A1-2, A2-1, A2-2, A3-1, or A3-2, or in the a3 region are substituted or deleted. In a particular embodiment, the two transferrin sequences are inserted in two insertion sites listed in TABLE 11, wherein one or more amino acids in A1-1, A1-2, A2-1, A2-2, A3-1, or A3-2, or in the a3 region are substituted or deleted. In still other aspects, a recombinant FVIII protein comprises three transferrin sequences inserted in three insertion sites listed in TABLE 10, wherein one or more amino acids in A1-1, A1-2, A2-1, A2-2, A3-1, or A3-2, or in the a3 region are substituted or deleted. In a specific aspect, the three transferrin sequences are inserted in three insertion sites listed in TABLE 12, TABLE 13 or both tables, wherein one or more amino acids in A1-1, A1-2, A2-1, A2-2, A3-1, or A3-2, or in the a3 region are substituted or deleted. In yet other aspects, a recombinant FVIII protein comprises four transferrin sequences inserted in four insertion sites listed in TABLE 10, wherein one or more amino acids in A1-1, A1-2, A2-1, A2-2, A3-1, or A3-2, or in the a3 region are substituted or deleted. In a particular aspect, the four transferrin sequences are inserted in four insertion sites listed in TABLE 14, TABLE 15, or both, wherein one or more amino acids in A1-1, A1-2, A2-1, A2-2, A3-1, or A3-2, or in the a3 region are substituted or deleted. In some aspects, a recombinant FVIII protein comprises five transferrin sequences inserted in five insertion sites listed in TABLE 10, wherein one or more amino acids in A1-1, A1-2, A2-1, A2-2, A3-1, or A3-2, or in the a3 region are substituted or deleted. In a particular aspect, the five transferrin sequences are inserted in five insertion sites listed in TABLE 16, wherein one or more amino acids in A1-1, A1-2, A2-1, A2-2, A3-1, or A3-2, or in the a3 region are substituted or deleted. In certain aspects, a recombinant FVIII protein comprises six transferrin sequences inserted in six insertion sites listed in TABLE 10, wherein one or more amino acids in A1-1, A1-2, A2-1, A2-2, A3-1, or A3-2, or in the a3 region are substituted or deleted. In a particular embodiment, the six transferrin sequences are inserted in six insertion sites listed in TABLE 17, wherein one or more amino acids in A1-1, A1-2, A2-1, A2-2, A3-1, or A3-2, or in the a3 region are substituted or deleted. In some aspects, all the inserted transferrin sequences are identical. In other aspects, at least one of the inserted transferrin sequences is different from the rest of inserted transferrin sequences.

In some aspects, a recombinant FVIII protein comprises one transferrin sequence inserted immediately downstream of an amino acid position corresponding to amino acid 26 of SEQ ID NO:1 with a substitution or deletion of amino acids 27 to 40 of SEQ ID NO: 1 or a portion thereof, amino acid 403 of SEQ ID NO:1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional transferrin sequence inserted immediately downstream of an amino acid corresponding to amino acid 1656 of SEQ ID NO:1. In some aspects, a recombinant FVIII protein comprises two transferrin sequences inserted immediately downstream of two amino acid positions corresponding to amino acid 26 of SEQ ID NO: 1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO: 1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional transferrin sequence inserted immediately downstream of an amino acid corresponding to amino acid 1656 of SEQ ID NO:1. In some aspects, a recombinant FVIII protein comprises three transferrin sequences inserted immediately downstream of three amino acid positions corresponding to amino acid 26 of SEQ ID NO: 1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO: 1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional transferrin sequence inserted immediately downstream of an amino acid corresponding to amino acid 1656 of SEQ ID NO:1.

### 3.1.8 PEG

In certain aspects, a recombinant FVIII protein of the invention comprises at least one attachment site for a non-polypeptide heterologous moiety or fragment, variant, or derivative thereof inserted into a permissive loop or into the a3 region, or both, wherein the recombinant FVIII protein has procoagulant activity and can be expressed *in vivo* or *in vitro* in a host cell. For example, a recombinant FVIII protein of the invention can include one or more polyethylene glycol (PEG) moieties attached in one or more permissive loops or in the a3 region, wherein the recombinant FVIII protein has procoagulant activity and can be expressed *in vivo* or *in vitro* in a host cell.

PEGylated FVIII can refer to a conjugate formed between FVIII and at least one polyethylene glycol (PEG) molecule. PEG is commercially available in a large variety of molecular weights and average molecular weight ranges. Typical examples of PEG average molecular weight ranges include, but are not limited to, about 200, about 300, about 400, about 600, about 1000, about 1300-1600, about 1450, about 2000, about 3000, about 3000-3750, about 3350, about 3000-7000, about 3500-4500, about 5000-7000, about 7000-9000, about 8000, about 10000, about 8500-11500, about 16000-24000, about 35000, about 40000, about 60000, and about 80000 daltons. These average molecular weights are provided merely as examples and are not meant to be limiting in any way.

A recombinant FVIII protein of the invention can be PEGylated to include mono-or poly-(e.g., 2-4) PEG moieties. PEGylation can be carried out by any of the PEGylation reactions known in the art. Methods for preparing a PEGylated protein product will generally include (i) reacting a polypeptide with polyethylene glycol (such as a reactive ester or aldehyde derivative of PEG) under conditions whereby the peptide of the invention becomes attached to one or more PEG groups; and (ii) obtaining the reaction product(s). In general, the optimal reaction conditions for the reactions will be determined case by case based on known parameters and the desired result.

There are a number of PEG attachment methods available to those skilled in the art, for example Malik F et al., Exp. Hematol. 20:1028-35 (1992); Francis, Focus on Growth Factors 3(2):4-10 (1992); European Pat. Pub. Nos. EP0401384, EP0154316, and EP0401384; and International Pat. Appl. Pub. Nos. WO92/16221 and WO95/34326. As a non-limiting example, FVIII variants can contain cysteine substitutions in one or more permissive loops as described herein, and the cysteines can be further conjugated to PEG polymer. See Mei et al., Blood 116:270-279 (2010) and U.S. Patent No. 7,632,921.

In certain aspects, a recombinant FVIII protein comprises at least one heterologous moiety inserted into the permissive loops of the A domains *(e.g.,* A1-1, A1-2, A2-1, A2-2, A3-1, or A3-2 as described above) or into the a3 region, wherein one or more amino acids in at least one of the permissive loops of the A domains or the a3 region are substituted or deleted and wherein at least one of the heterologous moieties is a PEG molecule. In some aspects, two of the heterologous moieties are PEGs. In some aspects, three of the heterologous moieties are PEGs. In some aspects, four of the heterologous moieties are PEGs. In some aspects, five of the heterologous moieties are PEGs. In some aspects, six or more of the heterologous moieties are PEGs.

In some aspects, a recombinant FVIII protein comprises one or more PEGs in an insertion site within a permissive loop, *e.g.,* A1-1, A1-2, A2-1, A2-2, A3-1, A3-2, a3, or any combinations thereof, wherein one or more amino acids in at least one of the permissive loop or the a3 region are substituted or deleted. In one embodiment, the one or more PEGs are inserted within A1-1, wherein one or more amino acids in A1-1 are substituted or deleted It is disclosed that the one or more PEGs are inserted within A1-2, wherein one or more amino acids in A1-2 are substituted or deleted. In other embodiments, the one or more PEGs are inserted within A2-1, wherein one or more amino acids in A2-1 are substituted or deleted. It is disclosed that the one or more PEGs are inserted within A2-2, wherein one or more amino acids in A2-2 are substituted or deleted. It is disclosed that the one or more PEGs are inserted within A3-1, wherein one or more amino acids in A3-1 are substituted or deleted. It is disclosed that the one or more PEGs are inserted within A3-2, wherein one or more amino acids in A3-2 are substituted or deleted. It is disclosed that the one or more PEGs are inserted within the a3 region, wherein one or more amino acids in the a3 region are substituted or deleted.

In certain aspects, a recombinant FVIII protein comprises one PEG inserted at an insertion site listed in TABLE 10, wherein one or more amino acids in A1-1, A1-2, A2-1, A2-2, A3-1, or A3-2, or in the a3 region are substituted or deleted. In other aspects, a recombinant FVIII protein comprises two PEGs inserted in two insertion sites listed in TABLE 10, wherein one or more amino acids in A1-1, A1-2, A2-1, A2-2, A3-1, or A3-2, or in the a3 region are substituted or deleted. In a particular embodiment, the two PEGs are inserted in two insertion sites listed in TABLE 11, wherein one or more amino acids in A1-1, A1-2, A2-1, A2-2, A3-1, or A3-2, or in the a3 region are substituted or deleted. In still other aspects, a recombinant FVIII protein comprises three PEGs inserted in three insertion sites listed in TABLE 10, wherein one or more amino acids in A1-1, A1-2, A2-1, A2-2, A3-1, or A3-2, or in the a3 region are substituted or deleted. In a specific aspect, the three PEGs are inserted in three insertion sites listed in TABLE 12, TABLE 13 or both tables, wherein one or more amino acids in A1-1, A1-2, A2-1, A2-2, A3-1, or A3-2, or in the a3 region are substituted or deleted. In yet other aspects, a recombinant FVIII protein comprises four PEGs inserted in four insertion sites listed in TABLE 10, wherein one or more amino acids in A1-1, A1-2, A2-1, A2-2, A3-1, or A3-2, or in the a3 region are substituted or deleted. In a particular aspect, the four PEGs are inserted in four insertion sites listed in TABLE 14, TABLE 15, or both, wherein one or more amino acids in A1-1, A1-2, A2-1, A2-2, A3-1, or A3-2, or in the a3 region are substituted or deleted. In some aspects, a recombinant FVIII protein comprises five PEGs inserted in five insertion sites listed in TABLE 10, wherein one or more amino acids in A1-1, A1-2, A2-1, A2-2, A3-1, or A3-2, or in the a3 region are substituted or deleted. In a particular aspect, the five PEGs are inserted in five insertion sites listed in TABLE 16, wherein one or more amino acids in A1-1, A1-2, A2-1, A2-2, A3-1, or A3-2, or in the a3 region are substituted or deleted. In certain aspects, a recombinant FVIII protein comprises six PEGs inserted in six insertion sites listed in TABLE 10, wherein one or more amino acids in A1-1, A1-2, A2-1, A2-2, A3-1, or A3-2, or in the a3 region are substituted or deleted. In a particular embodiment, the six PEGs are inserted in six insertion sites listed in TABLE 17, wherein one or more amino acids in A1-1, A1-2, A2-1, A2-2, A3-1, or A3-2, or in the a3 region are substituted or deleted. In some aspects, all the inserted PEGs are identical. In other aspects, at least one of the inserted PEGs is different from the rest of inserted PEGs.

In some aspects, a recombinant FVIII protein comprises one PEG inserted immediately downstream of an amino acid position corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO: 1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional PEG inserted immediately downstream of an amino acid corresponding to amino acid 1656 of SEQ ID NO:1. In some aspects, a recombinant FVIII protein comprises two PEGs inserted immediately downstream of two amino acid positions corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO: 1 with a substitution or deletion of amino acids 1901 to 1910 of SEQ ID NO: 1 or a portion thereof, and an additional PEG inserted immediately downstream of an amino acid corresponding to amino acid 1656 of SEQ ID NO:1. In some aspects, a recombinant FVIII protein comprises three PEGs inserted immediately downstream of three amino acid positions corresponding to amino acid 26 of SEQ ID NO: 1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO: 1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional PEG inserted immediately downstream of an amino acid corresponding to amino acid 1656 of SEQ ID NO:1.

### 3.1.9 HES

In certain aspects, a recombinant FVIII protein of the invention comprises at least one hydroxyethyl starch (HES) polymer conjugated in one or more permissive loops or in the a3 region, wherein the recombinant FVIII protein has procoagulant activity and can be expressed *in vivo* or *in vitro* in a host cell. HES is a derivative of naturally occurring amylopectin and is degraded by alpha-amylase in the body. HES exhibits advantageous biological properties and is used as a blood volume replacement agent and in hemodilution therapy in the clinics. See, *e.g.,* Sommermeyer et al., Krankenhauspharmazie 8:271-278 (1987); and Weidler et al., Arzneim.-Forschung/Drug Res. 41: 494-498 (1991).

HES is mainly characterized by the molecular weight distribution and the degree of substitution. HES has a mean molecular weight (weight mean) of from 1 to 300 kD, from 2 to 200kD, from 3 to 100 kD, or from 4 to 70kD. Hydroxyethyl starch can further exhibit a molar degree of substitution of from 0.1 to 3, from 0.1 to 2, from 0.1 to 0.9, or from 0.1 to 0.8, and a ratio between C2:C6 substitution in the range of from 2 to 20 with respect to the hydroxyethyl groups. HES with a mean molecular weight of about 130 kD is VOLUVEN^{®} from Fresenius. VOLUVEN^{®} is an artificial colloid, employed, *e.g.,* for volume replacement used in the therapeutic indication for therapy and prophylaxis of hypovolaemia. There are a number of HES attachment methods available to those skilled in the art, *e.g.,* the same PEG attachment methods described above.

In certain aspects, a recombinant FVIII protein comprises at least one heterologous moiety inserted into the permissive loops of the A domains *(e.g.,* A1-1, A1-2, A2-1, A2-2, A3-1, or A3-2 as described above) or into the a3 region, wherein one or more amino acids in at least one of the permissive loops of the A domains or the a3 region are substituted or deleted and wherein at least one of the heterologous moieties is a HES sequence. In some aspects, two of the heterologous moieties are HES sequences. In some aspects, three of the heterologous moieties are HES sequences. In some aspects, four of the heterologous moieties are HES sequences. In some aspects, five of the heterologous moieties are HES sequences. In some aspects, six or more of the heterologous moieties are HES sequences.

In some aspects, a recombinant FVIII protein comprises one or more HES sequences in an insertion site within a permissive loop, *e.g.,* A1-1, A1-2, A2-1, A2-2, A3-1, A3-2, a3, or any combinations thereof, wherein one or more amino acids in at least one of the permissive loop or the a3 region are substituted or deleted. In one embodiment, the one or more HES sequences are inserted within A1-1, wherein one or more amino acids in A1-1 are substituted or deleted. It is disclosed that the one or more HES sequences are inserted within A1-2, wherein one or more amino acids in A1-2 are substituted or deleted. It is disclosed that the one or more HES sequences are inserted within A2-1, wherein one or more amino acids in A2-1 are substituted or deleted. It is disclosed that the one or more HES sequences are inserted within A2-2, wherein one or more amino acids in A2-2 are substituted or deleted. It is disclosed that the one or more HES sequences are inserted within A3-1, wherein one or more amino acids in A3-1 are substituted or deleted. It is disclosed that the one or more HES sequences are inserted within A3-2, wherein one or more amino acids in A3-2 are substituted or deleted. It is disclosed that the one or more HES sequences are inserted within the a3 region, wherein one or more amino acids in the a3 region are substituted or deleted.

In certain aspects, a recombinant FVIII protein comprises one HES sequence inserted at an insertion site listed in TABLE 10, wherein one or more amino acids in A1-1, A1-2, A2-1, A2-2, A3-1, or A3-2, or in the a3 region are substituted or deleted. In other aspects, a recombinant FVIII protein comprises two HES sequences inserted in two insertion sites listed in TABLE 10, wherein one or more amino acids in A1-1, A1-2, A2-1, A2-2, A3-1, or A3-2, or in the a3 region are substituted or deleted. In a particular embodiment, the two HES sequences are inserted in two insertion sites listed in TABLE 11, wherein one or more amino acids in A1-1, A1-2, A2-1, A2-2, A3-1, or A3-2, or in the a3 region are substituted or deleted. In still other aspects, a recombinant FVIII protein comprises three HES sequences inserted in three insertion sites listed in TABLE 10, wherein one or more amino acids in A1-1, A1-2, A2-1, A2-2, A3-1, or A3-2, or in the a3 region are substituted or deleted. In a specific aspect, the three HES sequences are inserted in three insertion sites listed in TABLE 12, wherein one or more amino acids in A1-1, A1-2, A2-1, A2-2, A3-1, or A3-2, or in the a3 region are substituted or deleted, TABLE 13 or both tables, wherein one or more amino acids in A1-1, A1-2, A2-1, A2-2, A3-1, or A3-2, or in the a3 region are substituted or deleted. In yet other aspects, a recombinant FVIII protein comprises four HES sequences inserted in four insertion sites listed in TABLE 10, wherein one or more amino acids in A1-1, A1-2, A2-1, A2-2, A3-1, or A3-2, or in the a3 region are substituted or deleted. In a particular aspect, the four HES sequences are inserted in four insertion sites listed in TABLE 14, TABLE 15, or both, wherein one or more amino acids in A1-1, A1-2, A2-1, A2-2, A3-1, or A3-2, or in the a3 region are substituted or deleted. In some aspects, a recombinant FVIII protein comprises five HES sequences inserted in five insertion sites listed in TABLE 10, wherein one or more amino acids in A1-1, A1-2, A2-1, A2-2, A3-1, or A3-2, or in the a3 region are substituted or deleted. In a particular aspect, the five HES sequences are inserted in five insertion sites listed in TABLE 16, wherein one or more amino acids in A1-1, A1-2, A2-1, A2-2, A3-1, or A3-2, or in the a3 region are substituted or deleted. In certain aspects, a recombinant FVIII protein comprises six HES sequences inserted in six insertion sites listed in TABLE 10, wherein one or more amino acids in A1-1, A1-2, A2-1, A2-2, A3-1, or A3-2, or in the a3 region are substituted or deleted. In a particular embodiment, the six HES sequences are inserted in six insertion sites listed in TABLE 17, wherein one or more amino acids in A1-1, A1-2, A2-1, A2-2, A3-1, or A3-2, or in the a3 region are substituted or deleted. In some aspects, all the inserted HES sequences are identical. In other aspects, at least one of the inserted HES sequences is different from the rest of inserted HES sequences.

In some aspects, a recombinant FVIII protein comprises one HES sequence inserted immediately downstream of an amino acid position corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO: 1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional HES sequence inserted immediately downstream of an amino acid corresponding to amino acid 1656 of SEQ ID NO:1. In some aspects, a recombinant FVIII protein comprises two HES sequences inserted immediately downstream of two amino acid positions corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO: 1 with a substitution or deletion of amino acids 1901 to 1910 of SEQ ID NO: 1 or a portion thereof, and an additional HES sequence inserted immediately downstream of an amino acid corresponding to amino acid 1656 of SEQ ID NO:1. In some aspects, a recombinant FVIII protein comprises three HES sequences inserted immediately downstream of three amino acid positions corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional HES sequence inserted immediately downstream of an amino acid corresponding to amino acid 1656 of SEQ ID NO:1.

### 3.1.10 PSA

In certain aspects, a recombinant FVIII protein of the invention comprises at least one polysialic acid (PSA) polymer conjugated in one or more permissive loops or in the a3 region, wherein the recombinant FVIII protein has procoagulant activity and can be expressed *in vivo* or *in vitro* in a host cell. PSAs are naturally occurring unbranched polymers of sialic acid produced by certain bacterial strains and in mammals in certain cells. See, *e.g.,* Roth J. et al. (1993) in Polysialic Acid: From Microbes to Man, eds. Roth J., Rutishauser U., Troy F. A. (BirkhäuserVerlag, Basel, Switzerland), pp. 335-348. PSAs can be produced in various degrees of polymerization from n=about 80 or more sialic acid residues down to n=2 by limited acid hydrolysis or by digestion with neuraminidases, or by fractionation of the natural, bacterially derived forms of the polymer. There are a number of PSA attachment methods available to those skilled in the art, *e.g.,* the same PEG attachment methods described above. In certain aspects, an activated PSA can also be attached to a cysteine amino acid residue on FVIII. See, *e.g.,* U.S. Patent No. 5846951.

In certain aspects, a recombinant FVIII protein comprises at least one heterologous moiety inserted into the permissive loops of the A domains *(e.g.,* A1-1, A1-2, A2-1, A2-2, A3-1, or A3-2 as described above) or into the a3 region, wherein one or more amino acids in at least one of the permissive loops of the A domains or the a3 region are substituted or deleted and wherein at least one of the heterologous moieties is a PSA sequence. In some aspects, two of the heterologous moieties are PSA sequences. In some aspects, three of the heterologous moieties are PSA sequences. In some aspects, four of the heterologous moieties are PSA sequences. In some aspects, five of the heterologous moieties are PSA sequences. In some aspects, six or more of the heterologous moieties are PSA sequences.

In some aspects, a recombinant FVIII protein comprises one or more PSA sequences in an insertion site within a permissive loop, *e.g.,* A1-1, A1-2, A2-1, A2-2, A3-1, A3-2, a3, or any combinations thereof, wherein one or more amino acids in at least one of the permissive loops of the A domains or the a3 region are substituted or deleted. In one embodiment, the one or more PSA sequences are inserted within A1-1, wherein one or more amino acids in A1-1 are substituted or deleted. It is disclosed that the one or more PSA sequences are inserted within A1-2, wherein one or more amino acids in A1-2 are substituted or deleted. It is disclosed that the one or more PSA sequences are inserted within A2-1, wherein one or more amino acids in A2-1 are substituted or deleted. It is disclosed that the one or more PSA sequences are inserted within A2-2, wherein one or more amino acids in A2-2 are substituted or deleted. It is disclosed that the one or more PSA sequences are inserted within A3-1, wherein one or more amino acids in A3-1 are substituted or deleted. It is disclosed that the one or more PSA sequences are inserted within A3-2, wherein one or more amino acids in A3-1 are substituted or deleted. It is disclosed that the one or more PSA sequences are inserted within a3, wherein one or more amino acids in the a3 region are substituted or deleted.

In certain aspects, a recombinant FVIII protein comprises one PSA sequence inserted at an insertion site listed in TABLE 10, wherein one or more amino acids in A1-1, A1-2, A2-1, A2-2, A3-1, or A3-2, or in the a3 region are substituted or deleted. In other aspects, a recombinant FVIII protein comprises two PSA sequences inserted in two insertion sites listed in TABLE 10, wherein one or more amino acids in A1-1, A1-2, A2-1, A2-2, A3-1, or A3-2, or in the a3 region are substituted or deleted. In a particular embodiment, the two PSA sequences are inserted in two insertion sites listed in TABLE 11, wherein one or more amino acids in A1-1, A1-2, A2-1, A2-2, A3-1, or A3-2, or in the a3 region are substituted or deleted. In still other aspects, a recombinant FVIII protein comprises three PSA sequences inserted in three insertion sites listed in TABLE 10, wherein one or more amino acids in A1-1, A1-2, A2-1, A2-2, A3-1, or A3-2, or in the a3 region are substituted or deleted. In a specific aspect, the three PSA sequences are inserted in three insertion sites listed in TABLE 12, TABLE 13 or both tables, wherein one or more amino acids in A1-1, A1-2, A2-1, A2-2, A3-1, or A3-2, or in the a3 region are substituted or deleted. In yet other aspects, a recombinant FVIII protein comprises four PSA sequences inserted in four insertion sites listed in TABLE 10, wherein one or more amino acids in A1-1, A1-2, A2-1, A2-2, A3-1, or A3-2, or in the a3 region are substituted or deleted. In a particular aspect, the four PSA sequences are inserted in four insertion sites listed in TABLE 14, TABLE 15, or both, wherein one or more amino acids in A1-1, A1-2, A2-1, A2-2, A3-1, or A3-2, or in the a3 region are substituted or deleted. In some aspects, a recombinant FVIII protein comprises five PSA sequences inserted in five insertion sites listed in TABLE 10, wherein one or more amino acids in A1-1, A1-2, A2-1, A2-2, A3-1, or A3-2, or in the a3 region are substituted or deleted. In a particular aspect, the five PSA sequences are inserted in five insertion sites listed in TABLE 16, wherein one or more amino acids in A1-1, A1-2, A2-1, A2-2, A3-1, or A3-2, or in the a3 region are substituted or deleted. In certain aspects, a recombinant FVIII protein comprises six PSA sequences inserted in six insertion sites listed in TABLE 10, wherein one or more amino acids in A1-1, A1-2, A2-1, A2-2, A3-1, or A3-2, or in the a3 region are substituted or deleted. In a particular embodiment, the six PSA sequences are inserted in six insertion sites listed in TABLE 17, wherein one or more amino acids in A1-1, A1-2, A2-1, A2-2, A3-1, or A3-2, or in the a3 region are substituted or deleted. In some aspects, all the inserted PSA sequences are identical. In other aspects, at least one of the inserted PSA sequences is different from the rest of inserted PSA sequences.

In some aspects, a recombinant FVIII protein comprises one PSA sequence inserted immediately downstream of an amino acid position corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO: 1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional PSA sequence inserted immediately downstream of an amino acid corresponding to amino acid 1656 of SEQ ID NO:1. In some aspects, a recombinant FVIII protein comprises two PSA sequences inserted immediately downstream of two amino acid positions corresponding to amino acid 26 of SEQ ID NO: 1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO: 1 with a substitution or deletion of amino acids 1901 to 1910 of SEQ ID NO: 1 or a portion thereof, and an additional PSA sequence inserted immediately downstream of an amino acid corresponding to amino acid 1656 of SEQ ID NO:1. In some aspects, a recombinant FVIII protein comprises three PSA sequences inserted immediately downstream of three amino acid positions corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO:1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional PSA sequence inserted immediately downstream of an amino acid corresponding to amino acid 1656 of SEQ ID NO:1.

### 3.1.11 Clearance Receptors

In certain aspects, the half-life of a recombinant FVIII protein of the invention can be extended where the recombinant FVIII protein comprises at least one fragment of a FVIII clearance receptor or FVIII-binding fragment, variant, or derivative thereof inserted into a permissive loop or into the a3 region, or both, and wherein the recombinant FVIII protein has procoagulant activity and can be expressed *in vivo* or *in vitro* in a host cell. Insertion of soluble forms of clearance receptors, such as the low density lipoprotein-related protein receptor LRP1, or fragments thereof, can block binding of FVIII to clearance receptors and thereby extend its half-life, *e.g., in vivo* half-life. LRP1 is a 600 kDa integral membrane protein that is implicated in the receptor-mediate clearance of a variety of proteins, including FVIII. See, *e.g.,* Lenting et al., Haemophilia 16:6-16 (2010). Other suitable FVIII clearance receptors are, *e.g.,* LDLR (low-density lipoprotein receptor), VLDLR (very low-density lipoprotein receptor), and megalin (LRP-2), or fragments thereof. See, *e.g.,* Bovenschen et al., Blood 106:906-912 (2005); Bovenschen, Blood 116:5439-5440 (2010); Martinelli et al., Blood 116:5688-5697 (2010).

In some embodiments, the clearance receptor sequence is flanked at the C-terminus, the N-terminus, or both termini, by a Gly-Ser peptide linker sequence. In some embodiments, the Gly-Ser peptide linker is Gly₄Ser (SEQ ID NO:191). In other embodiments, the Gly-Ser peptide linker is (Gly₄Ser)₂ (SEQ ID NO:192).

In certain aspects, a recombinant FVIII protein comprises at least one heterologous moiety inserted into the permissive loops of the A domains *(e.g.,* A1-1, A1-2, A2-1, A2-2, A3-1, or A3-2 as described above) or into the a3 region, wherein one or more amino acids in at least one of the permissive loops of the A domains or the a3 region are substituted or deleted and wherein at least one of the heterologous moieties is a fragment of a FVIII clearance receptor or FVIII-binding fragment, variant, or derivative thereof. In some aspects, two of the heterologous moieties are fragments of a FVIII clearance receptor or FVIII-binding fragments, variants, or derivatives thereof. In some aspects, three of the heterologous moieties are fragments of a FVIII clearance receptor or FVIII-binding fragments, variants, or derivatives thereof. In some aspects, four of the heterologous moieties are fragments of a FVIII clearance receptor or FVIII-binding fragments, variants, or derivatives thereof. In some aspects, five of the heterologous moieties are fragments of a FVIII clearance receptor or FVIII-binding fragments, variants, or derivatives thereof. In some aspects, six or more of the heterologous moieties are fragments of a FVIII clearance receptor or FVIII-binding fragments, variants, or derivatives thereof.

In some aspects, a recombinant FVIII protein comprises one or more fragments of a FVIII clearance receptor or FVIII-binding fragments, variants, or derivatives thereof in an insertion site within a permissive loop, *e.g.,* A1-1, A1-2, A2-1, A2-2, A3-1, A3-2, a3, or any combinations thereof, wherein one or more amino acids in at least one of the permissive loops of the A domains or the a3 region are substituted or deleted. In one embodiment, the one or more fragments of a FVIII clearance receptor or FVIII-binding fragments, variants, or derivatives thereof are inserted within A1-1, wherein one or more amino acids in A1-1 are substituted or deleted. In another embodiment, the one or more fragments of a FVIII clearance receptor or FVIII-binding fragments, variants, or derivatives thereof are inserted within A1-2, wherein one or more amino acids in A1-2 are substituted or deleted. In other embodiments, the one or more fragments of a FVIII clearance receptor or FVIII-binding fragments, variants, or derivatives thereof are inserted within A2-1, wherein one or more amino acids in A2-1 are substituted or deleted. In still other embodiments, the one or more fragments of a FVIII clearance receptor or FVIII-binding fragments, variants, or derivatives thereof are inserted within A2-2, wherein one or more amino acids in A2-2 are substituted or deleted. In yet other embodiments, the one or more fragments of a FVIII clearance receptor or FVIII-binding fragments, variants, or derivatives thereof are inserted within A3-1, wherein one or more amino acids in A3-1 are substituted or deleted. In some embodiments, the one or more fragments of a FVIII clearance receptor or FVIII-binding fragments, variants, or derivatives thereof are inserted within A3-2, wherein one or more amino acids in A3-2 are substituted or deleted. In certain embodiments, the one or more fragments of a FVIII clearance receptor or FVIII-binding fragments, variants, or derivatives thereof are inserted within the a3 region, wherein one or more amino acids in the a3 region are substituted or deleted.

In certain aspects, a recombinant FVIII protein comprises one fragment of a FVIII clearance receptor or FVIII-binding fragment, variant, or derivative thereof inserted at an insertion site listed in TABLE 10, wherein one or more amino acids in A1-1, A1-2, A2-1, A2-2, A3-1, or A3-2, or in the a3 region are substituted or deleted. In other aspects, a recombinant FVIII protein comprises two fragments of a FVIII clearance receptor or FVIII-binding fragments, variants, or derivatives thereof inserted in two insertion sites listed in TABLE 10, wherein one or more amino acids in A1-1, A1-2, A2-1, A2-2, A3-1, or A3-2, or in the a3 region are substituted or deleted. In a particular embodiment, the two fragments of a FVIII clearance receptor or FVIII-binding fragments, variants, or derivatives thereof are inserted in two insertion sites listed in TABLE 11, wherein one or more amino acids in A1-1, A1-2, A2-1, A2-2, A3-1, or A3-2, or in the a3 region are substituted or deleted. In still other aspects, a recombinant FVIII protein comprises three fragments of a FVIII clearance receptor or FVIII-binding fragments, variants, or derivatives thereof inserted in three insertion sites listed in TABLE 10, wherein one or more amino acids in A1-1, A1-2, A2-1, A2-2, A3-1, or A3-2, or in the a3 region are substituted or deleted. In a specific aspect, the three fragments of a FVIII clearance receptor or FVIII-binding fragments, variants, or derivatives thereof are inserted in three insertion sites listed in TABLE 12, TABLE 13 or both tables, wherein one or more amino acids in A1-1, A1-2, A2-1, A2-2, A3-1, or A3-2, or in the a3 region are substituted or deleted. In yet other aspects, a recombinant FVIII protein comprises four fragments of a FVIII clearance receptor or FVIII-binding fragments, variants, or derivatives thereof inserted in four insertion sites listed in TABLE 10, wherein one or more amino acids in A1-1, A1-2, A2-1, A2-2, A3-1, or A3-2, or in the a3 region are substituted or deleted. In a particular aspect, the four fragments of a FVIII clearance receptor or FVIII-binding fragments, variants, or derivatives thereof are inserted in four insertion sites listed in TABLE 14, TABLE 15, or both, wherein one or more amino acids in A1-1, A1-2, A2-1, A2-2, A3-1, or A3-2, or in the a3 region are substituted or deleted. In some aspects, a recombinant FVIII protein comprises five fragments of a FVIII clearance receptor or FVIII-binding fragments, variants, or derivatives thereof inserted in five insertion sites listed in TABLE 10, wherein one or more amino acids in A1-1, A1-2, A2-1, A2-2, A3-1, or A3-2, or in the a3 region are substituted or deleted. In a particular aspect, the five fragments of a FVIII clearance receptor or FVIII-binding fragments, variants, or derivatives thereof are inserted in five insertion sites listed in TABLE 16, wherein one or more amino acids in A1-1, A1-2, A2-1, A2-2, A3-1, or A3-2, or in the a3 region are substituted or deleted. In certain aspects, a recombinant FVIII protein comprises six fragments of a FVIII clearance receptor or FVIII-binding fragments, variants, or derivatives thereof inserted in six insertion sites listed in TABLE 10, wherein one or more amino acids in A1-1, A1-2, A2-1, A2-2, A3-1, or A3-2, or in the a3 region are substituted or deleted. In a particular embodiment, the six fragments of a FVIII clearance receptor or FVIII-binding fragments, variants, or derivatives thereof are inserted in six insertion sites listed in TABLE 17, wherein one or more amino acids in A1-1, A1-2, A2-1, A2-2, A3-1, or A3-2, or in the a3 region are substituted or deleted. In some aspects, all the inserted fragments of a FVIII clearance receptor or FVIII-binding fragments, variants, or derivatives thereof are identical. In other aspects, at least one of the inserted fragments of a FVIII clearance receptor or FVIII-binding fragments, variants, or derivatives thereof is different from the rest of inserted fragments of a FVIII clearance receptor or FVIII-binding fragments, variants, or derivatives thereof.

In some aspects, a recombinant FVIII protein comprises one fragment of a FVIII clearance receptor or FVIII-binding fragment, variant, or derivative thereof inserted immediately downstream of an amino acid position corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO: 1 with a substitution or deletion of amino acids 1721 to 1724 of SEQ ID NO: 1 or a portion thereof, or amino acid 1900 of SEQ ID NO:1 in mature native human FVIII with a substitution or deletion of amino acids 1901 to 1910 of SEQ ID NO: 1 or a portion thereof, and an additional fragment of a FVIII clearance receptor or FVIII-binding fragment, variant, or derivative thereof inserted immediately downstream of an amino acid corresponding to amino acid 1656 of SEQ ID NO:1. In some aspects, a recombinant FVIII protein comprises two fragments of a FVIII clearance receptor or FVIII-binding fragments, variants, or derivatives thereof inserted immediately downstream of two amino acid positions corresponding to amino acid 26 of SEQ ID NO:1 with a substitution or deletion of amino acids 27 to 40 of SEQ ID NO: 1 or a portion thereof, amino acid 403 of SEQ ID NO: 1 with a substitution or deletion of amino acids 404 to 417 of SEQ ID NO: 1 or a portion thereof, amino acid 1720 of SEQ ID NO: 1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional fragment of a FVIII clearance receptor or FVIII-binding fragment, variant, or derivative thereof inserted immediately downstream of an amino acid corresponding to amino acid 1656 of SEQ ID NO:1. In some aspects, a recombinant FVIII protein comprises three fragments of a FVIII clearance receptor or FVIII-binding fragments, variants, or derivatives thereof inserted immediately downstream of three amino acid positions corresponding to amino acid 26 of SEQ ID NO: 1 with a substitution or deletion of amino acids 27 to 40 of SEQ ID NO: 1 or a portion thereof, amino acid 403 of SEQ ID NO:1 with a substitution or deletion of amino acids 404 to 417 of SEQ ID NO: 1 or a portion thereof, amino acid 1720 of SEQ ID NO: 1 with a substitution or deletion of amino acids 1721 to 1724 of SEQ ID NO: 1 or a portion thereof, or amino acid 1900 of SEQ ID NO:1 with a substitution or deletion of amino acids 1901 to 1910 of SEQ ID NO: 1 or a portion thereof, and an additional fragment of a FVIII clearance receptor or FVIII-binding fragment, variant, or derivative thereof inserted immediately downstream of an amino acid corresponding to amino acid 1656 of SEQ ID NO:1

### 3.2 Visualization and Location

In certain aspects a heterologous moiety facilitates visualization or localization of the recombinant FVIII protein of the invention. Myriad peptides and other moieties for insertion or conjugation into a recombinant protein which facilitate visualization or localization are known in the art. Such moieties can be used to facilitate visualization or localization *in vitro, in vivo, ex vivo* or any combination thereof.

Non-limiting examples of peptides or polypeptides which enable visualization or localization include biotin acceptor peptides which can facilitate conjugation of avidin- and streptavidin-based reagents, lipoic acid acceptor peptides which can facilitate conjugation of thiol-reactive probes to bound lipoic acid or direct ligation of fluorescent lipoic acid analogs, fluorescent proteins, *e.g.,* green fluorescent protein (GFP) and variants thereof *(e.g.,* EGFP, YFP such as EYFP, mVenus, YPet or Citrine, or CFP such as Cerulean or ECFP) or red fluorescent protein (RFP), cysteine-containing peptides for ligation of biarsenical dyes such as 4',5'-bis(1,3,2-dithioarsolan-2-yl)fluorescein (FlAsH), or for conjugating metastable technetium, peptides for conjugating europium clathrates for fluorescence resonance energy transfer (FRET)-based proximity assays, any variants, thereof, and any combination thereof.

In some embodiments, the peptide or polypeptide which enables visualization (*e.g*., a GFP such as EGFP) is flanked at the C-terminus, the N-terminus, or both termini, by a Gly-Ser peptide linker sequence. In some embodiments, the Gly-Ser peptide linker is Gly₄Ser (SEQ ID NO: 191). In other embodiments, the Gly-Ser peptide linker is (Gly₄Ser)₂ (SEQ ID NO: 192).

Recombinant FVIII proteins labeled by these techniques can be used, for example, for 3-D imaging of pathological thrombus formation and dissolution, tumor imaging in procoagulant malignancies, flow cytometric quantitation and characterization of procoagulant microparticles in blood and plasma, monitoring of thrombus formation by intravital microscopy.

### 4. Pharmaceutical Compositions and Methods of Treatment

Disclosed is a method for treating a bleeding condition in a human subject using a pharmaceutical composition comprising a recombinant FVIII protein of the invention. An exemplary method comprises administering to the subject in need thereof a therapeutically effective amount of a pharmaceutical composition/formulation comprising a recombinant FVIII protein of the invention. In other aspects, composition comprising a DNA encoding the recombinant protein of the invention can be administered to a subject in need thereof. In certain aspects of the invention, a cell expressing a recombinant FVIII protein of the invention can be administered to a subject in need thereof. In certain aspects of the invention, the pharmaceutical composition comprises (i) a recombinant FVIII protein, (ii) an isolated nucleic acid encoding a recombinant FVIII protein, (iii) a vector comprising a nucleic acid encoding, (iv) a cell comprising an isolated nucleic acid encoding a recombinant FVIII protein and/or a vector comprising a nucleic encoding a recombinant FVIII protein, or (v) a combination thereof, and the pharmaceutical compositions further comprises an acceptable excipient or carrier.

The bleeding condition can be caused by a blood coagulation disorder. A blood coagulation disorder can also be referred to as a coagulopathy. In one example, the blood coagulation disorder, which can be treated with a pharmaceutical composition of the current disclosure, is hemophilia or von Willebrand disease (vWD). In another example, the blood coagulation disorder, which can be treated with a pharmaceutical composition of the present disclosure is hemophilia A.

It is disclosed that the type of bleeding associated with the bleeding condition is selected from hemarthrosis, muscle bleed, oral bleed, hemorrhage, hemorrhage into muscles, oral hemorrhage, trauma, trauma capitis, gastrointestinal bleeding, intracranial hemorrhage, intra-abdominal hemorrhage, intrathoracic hemorrhage, bone fracture, central nervous system bleeding, bleeding in the retropharyngeal space, bleeding in the retroperitoneal space, and bleeding in the illiopsoas sheath.

It is disclosed that the subject suffering from bleeding condition is in need of treatment for surgery, including, *e.g*., surgical prophylaxis or peri-operative management. In one example, the surgery is selected from minor surgery and major surgery. Exemplary surgical procedures include tooth extraction, tonsillectomy, inguinal herniotomy, synovectomy, craniotomy, osteosynthesis, trauma surgery, intracranial surgery, intra-abdominal surgery, intrathoracic surgery, joint replacement surgery (*e.g*., total knee replacement, hip replacement, and the like), heart surgery, and caesarean section.

In another example, the subject is concomitantly treated with Factor IX. Because the compounds of the invention are capable of activating FIXa, they could be used to pre-activate the FIXa polypeptide before administration of the FIXa to the subject.

The methods of the invention may be practiced on a subject in need of prophylactic treatment or on-demand treatment.

Pharmaceutical compositions comprising a recombinant FVIII protein of the invention may be formulated for any appropriate manner of administration, including, for example, topical (*e.g*., transdermal or ocular), oral, buccal, nasal, vaginal, rectal or parenteral administration.

The term parenteral as used herein includes subcutaneous, intradermal, intravascular (*e.g.*, intravenous), intramuscular, spinal, intracranial, intrathecal, intraocular, periocular, intraorbital, intrasynovial and intraperitoneal injection, as well as any similar injection or infusion technique. The composition can be also for example a suspension, emulsion, sustained release formulation, cream, gel or powder. The composition can be formulated as a suppository, with traditional binders and carriers such as triglycerides.

In one example, the pharmaceutical formulation is a liquid formulation, *e.g.*, a buffered, isotonic, aqueous solution. In another example, the pharmaceutical composition has a pH that is physiologic, or close to physiologic. In other examples, the aqueous formulation has a physiologic or close to physiologic osmolarity and salinity. It can contain sodium chloride and/or sodium acetate. In some examples, the composition of the present invention is lyophilized.

### 5. Polynucleotides, Vectors, Host cells, and Methods of Making

The present invention further provides an isolated nucleic acid encoding a recombinant FVIII protein described herein, an expression vector comprising the nucleic acid, a host cell comprising the nucleic acid or the vector, or methods of making the recombinant FVIII protein.

The disclosure includes a method of making a recombinant FVIII protein comprising substituting or deleting one or more amino acids in an identified permissive loop location, the a3 region or both and inserting a heterologous moiety in the permissive location, the a3 region, or both as described herein, wherein the recombinant FVIII protein exhibits procoagulant activity.

The disclosure includes a method of increasing half-life of a FVIII protein without eliminating or reducing procoagulant activity of the FVIII protein, comprising substituting or deleting one or more amino acids in an identified permissive loop location, the a3 region or both and/or inserting a heterologous moiety in the permissive location, the a3 region, or both as described herein, wherein the recombinant FVIII protein exhibits procoagulant activity and increased half-life compared to the FVIII protein without the heterologous moiety.

The disclosure provides a method of constructing a recombinant FVIII protein comprising designing a nucleotide sequence encoding the recombinant FVIII protein as described herein.

The present disclosure includes a method of increasing expression of a recombinant FVIII protein comprising substituting or deleting one or more amino acids in an identified permissive loop location, the a3 region or both and/or inserting a heterologous moiety in the permissive location, the a3 region, or both as described herein, wherein the recombinant FVIII protein exhibits procoagulant activity

The disclosure provides a method of retaining procoagulant activity of a recombinant FVIII protein, comprising substituting or deleting one or more amino acids in an identified permissive loop location, the a3 region or both and/or inserting a heterologous moiety in an identified permissive location, the a3 region, or both as described herein, wherein the recombinant FVIII protein exhibits procoagulant activity.

The disclosure includes a method of manufacturing a recombinant FVIII protein comprising culturing a host cell comprising a nucleotide sequence encoding the recombinant FVIII protein described elsewhere herein. The FVIII protein manufactured by the method is a single chain FVIII. In other embodiments, the FVIII protein is not capable of binding to Von Willebrand Factor and thus has a deletion of all or substantially all of amino acids 745 to 1685 of SEQ ID NO: 1. In still other embodiments, the FVIII protein comprises a heterologous moiety in a permissive loop, wherein one or more amino acids in A1-1, are substituted or deleted. In yet other embodiments, a heterologous moiety comprises an element that increases *in vivo* half-life of the recombinant FVIII protein.

In some embodiments, the nucleic acid, vector, or host cell further comprises an additional nucleotide which encodes a protein convertase. The protein convertase can be selected from the group consisting of proprotein convertase subtilisin/kexin type 5 (PCSK5 or PC5), proprotein convertase subtilisin/kexin type 7 (PCSK7 or PC5), a yeast Kex 2, proprotein convertase subtilisin/kexin type 3 (PACE or PCSK3), and two or more combinations thereof. In some embodiments, the protein convertase is PACE, PC5, or PC7. In a specific embodiment, the protein convertase is PC5 or PC7. See International Appl. Publ. No. WO 2012/006623. In another embodiment, the protein convertase is PACE/Furin.

As used herein, an expression vector refers to any nucleic acid construct which contains the necessary elements for the transcription and translation of an inserted coding sequence, or in the case of an RNA viral vector, the necessary elements for replication and translation, when introduced into an appropriate host cell. Expression vectors can include plasmids, phagemids, viruses, and derivatives thereof.

A gene expression control sequence as used herein is any regulatory nucleotide sequence, such as a promoter sequence or promoter-enhancer combination, which facilitates the efficient transcription and translation of the coding nucleic acid to which it is operably linked. The gene expression control sequence may, for example, be a mammalian or viral promoter, such as a constitutive or inducible promoter. Constitutive mammalian promoters include, but are not limited to, the promoters for the following genes: hypoxanthine phosphoribosyl transferase (HPRT), adenosine deaminase, pyruvate kinase, beta-actin promoter, and other constitutive promoters. Exemplary viral promoters which function constitutively in eukaryotic cells include, for example, promoters from the cytomegalovirus (CMV), simian virus (*e.g.*, SV40), papilloma virus, adenovirus, human immunodeficiency virus (HIV), Rous sarcoma virus, cytomegalovirus, the long terminal repeats (LTR) of Moloney leukemia virus, and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

Examples of viral vectors include, but are not limited to, nucleic acid sequences from the following viruses: retrovirus, such as Moloney murine leukemia virus, Harvey murine sarcoma virus, murine mammary tumor virus, and Rous sarcoma virus; adenovirus, adeno-associated virus; SV40-type viruses; polyomaviruses; Epstein-Barr viruses; papilloma viruses; herpes virus; vaccinia virus; polio virus; and RNA virus such as a retrovirus. One can readily employ other vectors well-known in the art. Certain viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Retroviruses have been approved for human gene therapy trials. Most useful are those retroviruses that are replication-deficient *(i.e.,* capable of directing synthesis of the desired proteins, but incapable of manufacturing an infectious particle). Such genetically altered retroviral expression vectors have general utility for the high-efficiency transduction of genes *in vivo.* Standard protocols for producing replication-deficient retroviruses (including the steps of incorporation of exogenous genetic material into a plasmid, transfection of a packaging cell line with plasmid, production of recombinant retroviruses by the packaging cell line, collection of viral particles from tissue culture media, and infection of the target cells with viral particles) are provided in Kriegler, M., Gene Transfer and Expression, A Laboratory Manual, W.H. Freeman Co., New York (1990) and Murry, E. J., Methods in Molecular Biology, Vol. 7, Humana Press, Inc., Cliffton, N.J. (1991).

The expression vector or vectors are then transfected or co-transfected into a suitable target cell, which will express the polypeptides. Transfection techniques known in the art include, but are not limited to, calcium phosphate precipitation (Wigler et al. (1978) Cell 14:725), electroporation (Neumann et al. (1982) EMBO J 1:841), and liposome-based reagents. A variety of host-expression vector systems may be utilized to express the proteins described herein including both prokaryotic and eukaryotic cells. These include, but are not limited to, microorganisms such as bacteria *(e.g., E. coli)* transformed with recombinant bacteriophage DNA or plasmid DNA expression vectors containing an appropriate coding sequence; yeast or filamentous fungi transformed with recombinant yeast or fungi expression vectors containing an appropriate coding sequence; insect cell systems infected with recombinant virus expression vectors (*e.g*., baculovirus) containing an appropriate coding sequence; plant cell systems infected with recombinant virus expression vectors (*e.g*., cauliflower mosaic virus or tobacco mosaic virus) or transformed with recombinant plasmid expression vectors *(e.g.,* Ti plasmid) containing an appropriate coding sequence; or animal cell systems, including mammalian cells *(e.g.,* HEK 293, CHO, Cos, HeLa, HKB11, and BHK cells).

In one embodiment, the host cell is a eukaryotic cell. As used herein, a eukaryotic cell refers to any animal or plant cell having a definitive nucleus. Eukaryotic cells of animals include cells of vertebrates, *e.g.,* mammals, and cells of invertebrates, *e.g.,* insects. Eukaryotic cells of plants specifically can include, without limitation, yeast cells. A eukaryotic cell is distinct from a prokaryotic cell, *e.g.,* bacteria.

In certain embodiments, the eukaryotic cell is a mammalian cell. A mammalian cell is any cell derived from a mammal. Mammalian cells specifically include, but are not limited to, mammalian cell lines. In one embodiment, the mammalian cell is a human cell. In another embodiment, the mammalian cell is a HEK 293 cell, which is a human embryonic kidney cell line. HEK 293 cells are available as CRL-1533 from American Type Culture Collection, Manassas, VA, and as 293-H cells, Catalog No. 11631-017 or 293-F cells, Catalog No. 11625-019 from Invitrogen (Carlsbad, Calif.). In some embodiments, the mammalian cell is a PER.C6^{®} cell, which is a human cell line derived from retina. PER.C6^{®} cells are available from Crucell (Leiden, The Netherlands). In other embodiments, the mammalian cell is a Chinese hamster ovary (CHO) cell. CHO cells are available from American Type Culture Collection, Manassas, VA. *(e.g.,* CHO-K1; CCL-61). In still other embodiments, the mammalian cell is a baby hamster kidney (BHK) cell. BHK cells are available from American Type Culture Collection, Manassas, Va. *(e.g.,* CRL-1632). In some embodiments, the mammalian cell is a HKB11 cell, which is a hybrid cell line of a HEK293 cell and a human B cell line. Mei et al., Mol. Biotechnol. 34(2): 165-78 (2006).

In still other embodiments, transfected cells are stably transfected. These cells can be selected and maintained as a stable cell line, using conventional techniques known to those of skill in the art.

Host cells containing DNA constructs of the protein are grown in an appropriate growth medium. As used herein, the term "appropriate growth medium" means a medium containing nutrients required for the growth of cells. Nutrients required for cell growth may include a carbon source, a nitrogen source, essential amino acids, vitamins, minerals, and growth factors. Optionally, the media can contain one or more selection factors. Optionally the media can contain bovine calf serum or fetal calf serum (FCS). In one embodiment, the media contains substantially no IgG. The growth medium will generally select for cells containing the DNA construct by, for example, drug selection or deficiency in an essential nutrient which is complemented by the selectable marker on the DNA construct or co-transfected with the DNA construct. Cultured mammalian cells are generally grown in commercially available serum-containing or serum-free media (*e.g*., MEM, DMEM, DMEM/F12). In one embodiment, the medium is CD293 (Invitrogen, Carlsbad, CA.). In another embodiment, the medium is CD17 (Invitrogen, Carlsbad, CA.). Selection of a medium appropriate for the particular cell line used is within the level of those ordinary skilled in the art.

In certain aspects, the present invention relates to the recombinant FVIII protein produced by the methods described herein.

*In vitro* production allows scale-up to give large amounts of the desired altered polypeptides of the invention. Techniques for mammalian cell cultivation under tissue culture conditions are known in the art and include homogeneous suspension culture, *e.g.* in an airlift reactor or in a continuous stirrer reactor, or immobilized or entrapped cell culture, *e.g.* in hollow fibers, microcapsules, on agarose microbeads or ceramic cartridges. If necessary and/or desired, the solutions of polypeptides can be purified by the customary chromatography methods, for example gel filtration, ion-exchange chromatography, hydrophobic interaction chromatography (HIC, chromatography over DEAE-cellulose or affinity chromatography.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. *See,* for example, Molecular Cloning A Laboratory Manual, 2nd Ed., Sambrook et al., ed., Cold Spring Harbor Laboratory Press: (1989); Molecular Cloning: A Laboratory Manual, Sambrook et al., ed., Cold Springs Harbor Laboratory, New York (1992), DNA Cloning, D. N. Glover ed., Volumes I and II (1985); Oligonucleotide Synthesis, M. J. Gait ed., (1984); Mullis et al. U.S. Pat. No: 4,683,195; Nucleic Acid Hybridization, B. D. Hames & S. J. Higgins eds. (1984); Transcription And Translation, B. D. Hames & S. J. Higgins eds. (1984); Culture Of Animal Cells, R. I. Freshney, Alan R. Liss, Inc., (1987); Immobilized Cells And Enzymes, IRL Press, (1986); B. Perbal, A Practical Guide To Molecular Cloning (1984); the treatise, Methods In Enzymology, Academic Press, Inc., N.Y.; Gene Transfer Vectors For Mammalian Cells, J. H. Miller and M. P. Calos eds., Cold Spring Harbor Laboratory (1987); Methods In Enzymology, Vols. 154 and 155 (Wu et al. eds.); Immunochemical Methods In Cell And Molecular Biology, Mayer and Walker, eds., Academic Press, London (1987); Handbook Of Experimental Immunology, Volumes I-IV, D. M. Weir and C. C. Blackwell, eds., (1986); Manipulating the Mouse Embryo, Cold Spring Harbor Laboratory Press, Cold Spring Harbor, N.Y., (1986); and in Ausubel et al., Current Protocols in Molecular Biology, John Wiley and Sons, Baltimore, Maryland (1989).

Standard reference works setting forth general principles of immunology include Current Protocols in Immunology, John Wiley & Sons, New York; Klein, J., Immunology: The Science of Self-Nonself Discrimination, John Wiley & Sons, New York (1982); Roitt, I., Brostoff, J. and Male D., Immunology, 6th ed. London: Mosby (2001); Abbas A., Abul, A. and Lichtman, A., Cellular and Molecular Immunology, Ed. 5, Elsevier Health Sciences Division (2005); and Harlow and Lane, Antibodies: A Laboratory Manual, Cold Spring Harbor Press (1988).

Having now described the present invention in detail, the same will be more clearly understood by reference to the following examples, which are included herewith for purposes of illustration only and are not intended to be limiting of the invention.

### Examples

### Example 1: Construction and Manipulation of Factor VIII Base Vector, Cloning, Transfection and Expression

In general, the practice of the present invention employs, unless otherwise indicated, conventional techniques of chemistry, biophysics, molecular biology, recombinant DNA technology, and standard techniques in electrophoresis. See, *e.g.*, Sambrook, Fritsch and Maniatis, Molecular Cloning: Cold Spring Harbor Laboratory Press (1989). The coding sequence of human Factor VIII (Genbank Accession Number NM_000132), including its native signal sequence, was obtained by reverse transcription-polymerase chain reactions (RT-PCR) from human liver polyA RNA. Due to the large size of FVIII, the coding sequence was obtained in several sections from separate RT-PCR reactions, and assembled through a series of PCR reactions, restriction digests and ligations into an intermediate cloning vector containing a B domain deleted (BDD) FVIII coding region with a fusion of serine 743 (S743) to glutamine 1638 (Q1638), eliminating 2682 bp from the B domain of full length FVIII (SEQ ID NO:3).The BDD FVIII polypeptide coding sequence was ligated into expression vector pcDNA4/myc-His C (Invitrogen, Carlsbad, CA) between the *Hind*III and *Xho*I sites following introduction of a Kozak translation initiation sequence (GCCGCCACC immediately 5' to the ATG codon encoding the start Met residue. To facilitate the insertion of polypeptide encoding sequences into the base vector, two unique restriction sites (*Nhe*I and *Cla*I) were introduced by standard PCR-based mutagenesis methods such that the resulting protein sequence of BDD-FVIII remained unchanged. The *Nhe*I site (encoding Ala-Ser) was introduced at nucleotide positions 850-855, and the *Cla*I site (encoding Ile-Asp) was introduced at nucleotide positions 4984-4989. FIG. 1 (panels A to G) shows the domain structure of the Factor VIII construct and the location of the introduced *Nhe*I and *Cla*I sites (protein sequence, SEQ ID NO:2; DNA sequence, SEQ ID NO:3).

The resulting plasmid was designated pBC0102. Plasmid pBC102 was subsequently modified to generate plasmid pBC01 14 by introducing sequences encoding linker peptides comprising Ala, Glu, Gly, Pro, Ser, and Thr residues between the C-terminus of the Factor VIII sequence and the Myc epitope tag (-Glu-Gln-Lys-Leu-Ile-Ser-Glu-Glu-Asp-Leu-) and between the Myc epitope tag and the C-terminal hexa-histidine tag. FIG. 2 shows the topology of base vector pBC01 14.

HEK293F cells (Invitrogen, Carlsbad, CA) were transfected with the plasmid pBC0114 using polyethyleneimine (PEI, Polysciences Inc. Warrington, PA) or LIPOFECTAMINE^{®} transfection reagent (Invitrogen, Carlsbad, CA). The transiently transfected cells were grown in 293 Free Style medium or a mixture of 293 Free Style and CD OPTICHO^{®} media (Invitrogen, Carlsbad, CA).

The cell culture medium was harvested 3-5 days after transfection and analyzed for FVIII expression by chromogenic FVIII activity assay and FVIII ELISA. The concentrated conditioned media containing recombinant FVIII were used for initial pharmacokinetics studies.

### Example 2A: Potential Permissive Loop Site Selection - Method 1

Biocomputational methods were used to predict the location of specific sites in Factor VIII wherein the insertion of a heterologous moiety would not result in the loss of procoagulant activity. Structural analyses were performed on X-ray crystallographic coordinates 3CDZ (Ngo et al., Structure 16: 597-606 (2008)) and 2R7E (Shen et al., Blood 111:1240-1247 (2008)) deposited in the Protein Data Bank maintained by the Research Collaboratory for Structural Bioinformatics (RCSB; *www.rcsb.org*/*pdb*), as well as on atomic coordinates PM0076106 for the predicted refined FVIII structure derived from a molecular dynamics simulation study (Venkateswarlu, BMC Struct. Biol. 10:7 (2010)) deposited in the Protein Model Database *(mi.caspur.it*/*PMDB*/*main.php)* maintained by Consorzio Interuniversitario per le Applicazioni di Supercalcolo per Università e Riserca (CASPUR) and the Department of Biochemical Sciences of the University of Rome.

The accessible surface area (ASA) for each individual amino acid residue was calculated by using the ASAView algorithm (Ahmad S et al., BMC Bioinformatics 5: 51 (2004)) for the 2R7E, 3CDZ, and PM0076106 datasets and also by using the GETAREA algorithm (Fraczkiewicz R. & Braun W., J. Comp. Chem., 19, 319-333 (1998)) for the 2R7E and 3CDZ datasets. Graphical ASAView outputs for structural datasets 2R7E, 3CDZ, and PM0076106 are depicted in FIG. 3.

For the same structural datasets, the GETAREA algorithm produced substantially equivalent results. Regions of moderate to high predicted ASA (0.5-1) were further evaluated by comparing to atomic positional fluctuation (APF) data presented in Venkateswarlu *et al.* Sequence positions corresponding to those with ASA values of 0.5 or greater and APF values of 40Å² were considered for further analysis.

The surface exposure of residues comprising this resulting subset was then evaluated by manual inspection of 3-D structural depiction of 2R7E, 3CDZ, and PM0076106 by using PYMOL^{®} molecular visualization software (Schrödinger). Residues that were surface exposed and not located in defined secondary structural elements such as β-sheets or α-helices were considered for further evaluation.

The resulting subset of residues was further evaluated based on proximity in linear amino acid sequence to residues for which mutation is known to be causative for hemophilia A (HAMSTeRS database; hadb.org.uk). Sites within five residues of known hemophilia A mutation sites were eliminated from further consideration.

Based on this analysis, sites were chosen for insertion of heterologous moieties, and this group of sites was designated Batch 1.

### Example 2B: Potential Permissive Loop Site Selection - Method 2

Computational methods were used to predict the location of specific sites in Factor VIII wherein the insertion of a heterologous moiety would not result in the loss of procoagulant activity. First, sequence analyses of Factor VIII across different species were carried out using the Basic Local Alignment Search Tool (BLAST; blast.ncbi.nlm.nih.gov/Blast.cgi). A total of 18 FVIII polypeptide sequences from ten different vertebrate species were selected for variation analysis. The species used were human *(Homo sapiens)* (gi:31499, emb:CAA25619.1, SEQ ID NO:63; gi:182803, gb:AAA52484.1, SEQ ID NO:64; gi:182383, gb:AAA52420.1, SEQ ID NO:65; gi:119593053, gb:EAW72647.1, SEQ ID NO:78), chimpanzee *(Pan troglodytes)* (gi:332862036, ref:XP_003317837.1, SEQ ID NO:65), gibbon *(Nomascus leucogenys)* (gi:332260614, ref:XP_003279379.1, SEQ ID NO:66), rabbit *(Oryctolagus cuniculus)* (gi:284005234, ref:NP_001164742.1, SEQ ID NO:69), dog *(Canis lupus familiaris)* (gi:50978978, ref:NP_001003212.1, SEQ ID NO:70), cattle (*Bos taurus)* (gi:296471126, gb:DAA13241.1, SEQ ID NO:71; gi:224458398, ref:NP_001138980.1, SEQ ID NO:72), sheep *(Ovis aries)* (gi:289191358, ref:NP_001166021.1, SEQ ID NO:75), mouse *(Mus musculus)* (gi:238624182, ref:NP_001154845.1, SEQ ID NO:73; gi:238624180, ref:NP_032003.2, SEQ ID NO:74; gi:238624184, ref:NP_001154846.1, SEQ ID NO: 76), pig *(Sus scrofa)* (gi:47523422, ref:NP_999332.1, SEQ ID NO:77), rat *(Rattus norvegicus)* (gi:34328534, ref:NP_899160.1, SEQ ID NO:78; gi:316995315, gb:ADU79113.1, SEQ ID NO:79; gi:316995313, gb:ADU79112.1, SEQ ID NO:80). Sites with more than three (≥4) different amino acids were considered hypervariable.

Molecular Dynamics (MD) analyses were performed using X-ray crystallographic structure 2R7E (Shen et al., Blood 111: 1240-1247(2008)) deposited in the Protein Data Bank maintained by the Research Collaboratory for Structural Bioinformatics (RCSB; www.rcsb.org/pdb). The MD simulation was performed in the presence 43717 explicit water molecules using NAMD (www.ks.uiuc.edu/Research/namd/), and 1000 snapshots were collected during a 1 nanosecond simulation. The Root Mean Square Distance (RMSD) of Cα of each residue was calculated using the collected snapshots in VMD (www.ks.uiuc.edu/Research/vmd/) to estimate residue flexibility, and residues with RMSD value greater than 4 Å were designated highly flexible.

By combining these two methods, surface sites designated as both hypervariable and highly flexible were considered for further evaluation. Of these potential sites, those that were within 5 residues in the linear polypeptide sequence of sites identified by Method 1 (Example 2A, above) were excluded from further evaluation. The resulting subset of residues was further evaluated based on proximity in linear sequence to residues for which mutation is known to be causative for hemophilia A (HAMSTERS database; hadb.org.uk/). Sites within five residues of known hemophilia A mutation sites were eliminated from further consideration.

Based on this analysis, sites were chosen for insertion of heterologous moieties, and this group of sites was designated Batch 2.

### Example 3: XTEN AE42-4 Insertion

To demonstrate that FVIII can tolerate insertion of peptides of variable length and composition within individual structural domains without loss of cofactor function, a 42 amino acid long XTEN peptide (XTEN AE42-4, SEQ ID NO:13) was first inserted by standard recombinant DNA techniques. The XTEN AE42-4 DNA sequence (SEQ ID NO:14) encodes the amino acids Gly (G), Ala (A), Pro (P), Ser (S), Thr (T), and Glu (E) and is flanked by a 5' *Asc*I restriction site (ggcgcgcc) and a 3' *Xho*I site (ctcgag), neither of which is present in the sequence of the base vector pBC0114.

The XTEN AE42-4 DNA sequence was chemically synthesized and inserted such that the resulting DNA construct would encode a FVIII fusion protein in which the XTEN AE42-4 protein sequence is inserted immediately after the residue indicated in the site selection.

Thus, where residue X designates the site of insertion and residue Z designates the next residue in the native FVIII polypeptide sequence, the polypeptide resulting from insertion of XTEN AE42 would contain the sequence:

X-GAPGSPAGSPTSTEEGTSESATPESGPGSEPATSGSETPASS-Z

X-(SEQ ID NO:13)-Z

In addition, insertion of the corresponding DNA sequence at this position also introduces *Asc*I and *Xho*I restriction sites flanking the XTEN encoding sequence that are unique in the base vector and which can subsequently be used to excise the intervening sequence and introduce longer XTEN sequences, XTEN sequences of different composition and sequence, or unrelated heterologous sequences.

A total of 16 different sites in the FVIII sequence were selected for XTEN AE42 insertion based on the methods described above in Example 2A, and these were designed Batch 1. An additional 21 sites were selected for XTEN AE42 insertion based on the methods described above in Example 2B, and these were designed Batch 2. The location of these Batch 1 and Batch 2 insertion sites is summarized in TABLE IB.

Collectively, the Batch 1 and Batch 2 sites represent 12 sites in the A1 domain, 7 sites in the A2 domain, 10 sites in the A3 domain, 4 sites in the C1 domain, and 3 sites in the C2 domain. Locations of Batch 1 and 2 sites in the 3-D structure of FVIII are depicted in FIG. 4.

**TABLE 1B. Location of XTEN AE42-4 insertion sites.**

| Construct | Batch | Domain | Insertion Site | Upstream sequence |
|---|---|---|---|---|
| pBC0126 | 1 | A1 | 3 | CFS |
| pBC0165 | 2 | A1 | 18 | YMQ |
| pBC0183 | 2 | A1 | 22 | DLG |
| pBC0184 | 2 | A1 | 26 | LPV |
| pBC0166 | 2 | A1 | 40 | FPF |
| pBC0185 | 2 | A1 | 60 | LFN |
| pBC0167 | 2 | A1 | 116 | YDD |
| pBC0128 | 1 | A1 | 130 | VFP |
| pBC0168 | 2 | A1 | 188 | KEK |
| pBC0129 | 1 | A1 | 216 | NSL |
| pBC0169 | 2 | A1 | 230 | WPK |
| pBC0130 | 1 | A1 | 333 | EEP |
| pBC0131 | 1 | A2 | 375 | SVA |
| pBC0132 | 1 | A2 | 403 | APD |
| pSD0033 | | | | |
| pBC0170 | 2 | A2 | 442 | EAI |
| pBC0133 | 1 | A2 | 490 | RRL |
| pBC0171 | 2 | A2 | 518 | TVE |
| pBC0134 | 1 | A2 | 599 | NPA |
| pBC0172 | 2 | A2 | 713 | CDK |
| pBC0138 | 1 | A3 | 1720 | LRN |
| pBC0139 | 1 | A3 | 1796 | EDQ |
| pBC0140 | 1 | A3 | 1802 | AEP |
| pBC0173 | 2 | A3 | 1827 | PTK |
| pBC0174 | 2 | A3 | 1861 | HTN |
| pBC0175 | 2 | A3 | 1896 | NME |
| pBC0176 | 2 | A3 | 1900 | NCR |
| pBC0177 | 2 | A3 | 1904 | PCN |
| pBC0178 | 2 | A3 | 1937 | AQD |
| pBC0141 | 1 | A3 | 2019 | YSN |
| pBC0179 | 2 | C1 | 2068 | EPF |
| pBC0180 | 2 | C1 | 2111 | GKK |
| pBC0142 | 1 | C1 | 2120 | NST |
| pBC0143 | 1 | C1 | 2171 | CDL |
| pBC0181 | 2 | C2 | 2188 | SDA |
| pBC0182 | 2 | C2 | 2227 | NPK |
| pBC0144 | 1 | C2 | 2277 | FQN |

### Expression of FVIII-XTEN Variants

The FVIII variants with AE42-4 XTEN insertions were transfected into HEK293F cells (Invitrogen, Carlsbad, CA) using polyethyleneimine (PEI, Polysciences Inc. Warrington, PA) or LIPOFECTAMINE^{®} transfection reagent (Invitrogen, Carlsbad, CA). The transiently transfected cells were grown in 293 Free Style medium or a mixture of 293 Free Style and CD OPTICHO^{®} media (Invitrogen, Carlsbad, CA) and the recombinant Factor VIII protein was analyzed by chromogenic assay for FVIII activity and ELISA (enzyme linked immunosorbent assay) for FVIII expression.

### In vitro assays

To assess FVIII tolerability to XTEN AE42-4 insertion, the FVIII activity in culture media samples from FVIII-XTEN cell cultures was analyzed using a FVIII chromogenic assay. Antigen expression levels were analyzed by FVIII-HC (FVIII heavy chain) and FVIII-LC (FVIII light chain) ELISA.

### FVIII Activity Measurement by Chromogenic Assay

The FVIII activity was measured using the COATEST^{®} SP FVIII kit from DiaPharma (lot# N089019) and all incubations were performed on a 37°C plate heater with shaking. Cell culture harvests from transient transfection media of FVIII-XTEN AE42-4 variants from 6 well plates were diluted to the desired FVIII activity range using 1x FVIII COATEST^{®} buffer. FVIII standards were prepared in 1x FVIII COATEST^{®} buffer containing mock transfection media with matching culture media concentration as the testing sample. The range of recombinant Factor VIII (rFVIII) standard was from 100 mIU/mL to 0.78 mIU/mL. The standards, diluted cell culture samples, and a pooled normal human plasma assay control were added to IMMULON^{®} 2HB 96-well plates in duplicates (25 µL/well).

Freshly prepared IXa/FX/Phospholipid mix (50 µL), 25 µL of 25mM CaCl₂, and 50 µL of FXa substrate were added sequentially into each well, with 5 minutes incubation between each addition. After incubating with the substrate, 25 µL of 20% acetic acid was added to terminate the color reaction, and the absorbance at 405 nm was measured with a SPECTRAMAX^{®} plus (Molecular Devices) instrument.

Data analysis was performed using SOFTMAX^{®} Pro software (version 5.2). The Lowest Level of Quantification (LLOQ) was 39 mIU/mL.

### Expression Measurement by FVIII-HC and FVIII-LC ELISA

Expression of variants was quantified using ELISA. The FVIII antigen expression levels of DNA constructs corresponding to XTEN insertions in the A1 and A2 domains of FVIII were analyzed by FVIII-LC ELISA. The FVIII antigen expression levels of DNA constructs corresponding to XTEN insertions in the A3, C1 and C2 domains of FVIII were analyzed by FVIII-HC ELISA.

FVIII-XTEN antigens in cell culture media after harvest were captured by GMA011 antibodies (Green Mountain Antibodies) for FVIII-LC ELISA) or by GMA016 antibodies (Green Mountain Antibodies) for FVIII-HC ELISA. IMMULON^{®} 2HB 96-well plates were coated with 100µl/well of anti-FVIII antibody (2µg/ml) by overnight incubation at 4°C. Plates were then washed four times with Phosphate Buffer saline with TWEEN-20^{®} (PBST) and blocked with blocking buffer (PBST with 10% heat inactivated horse serum) for 1 hour at room temperature.

Cell culture harvests from transient transfection media of FVIII-XTEN variants from a 6-well plate were diluted to the desired FVIII antigen range using 1x blocking buffer. FVIII standards were prepared in 1x FVIII blocking buffer containing mock transfection media with matching media concentration as the testing samples. The range of rFVIII standard was from 50 ng/mL to 0.39 ng/mL.

Standards, diluted cell culture samples, and a pooled normal human plasma assay control were added into IMMULON^{®} 2HB 96-well plates in duplicates (100 µL/well) and incubated at 37°C for 2 hours. Following four times washing with PBST, 100 µl of HRP-sheep anti-hFVIII antibody (Affinity Biologicals, F8C-EIC-D) were added into each well and plates were incubated for 1 hour at 37°C. After another four washes with PBST, 100 µl of TMB Super Sensitive Substrate (BioFX) were added to each well, followed by 5-10 min color development. To terminate the color reaction, 50 µL of H₂SO₄ were added to each well, and the absorbance of at 450 nm was measured with a SPECTRAMAX^{®} plus (Molecular Devices) instrument.

Data analysis was performed using SOFTMAX^{®} Pro software (version 5.4). The Lowest Level of Quantification (LLOQ) was 0.0039 µg/mL. The results are shown in TABLE 2.

**TABLE 2. Summary of Activity and Expression Data for FVIII variants with XTEN insertions**

| DNA Construct | FVIII Domain | | Insertion Site | | Upstream Sequence | | FVIII Activity (IU/ml) | FVIII Antigen (ug/ml) |
|---|---|---|---|---|---|---|---|---|
| pBC0126 | | A1 | | 3 | | CFS | <LLOQ | <LLOQ |
| pBC0165 | | A1 | | 18 | | YMQ | 0.82 | 0.088 |
| pBC0183 | | A1 | | 22 | | DLG | 0.85 | 0.168 |
| pBC0184 | | A1 | | 26 | | LPV | 0.42 | 0.082 |
| pBC0166 | | A1 | | 40 | | FPF | 0.64 | 0.072 |
| pBC0185 | | A1 | | 60 | | LFN | <LLOQ | <LLOQ |
| pBC0167 | | A1 | | 116 | | YDD | <LLOQ | <LLOQ |
| pBC0128 | | A1 | | 130 | | VFP | <LLOQ | <LLOQ |
| pBC0168 | | A1 | | 188 | | KEK | 0.29 | 0.045 |
| pBC0129 | | A1 | | 216 | | NSL | 0.179 | 0.038 |
| pBC0169 | | A1 | | 230 | | WPK | <LLOQ | <LLOQ |
| pBC0130 | | A1 | | 333 | | EEP | 0.75 | 0.61 |
| pBC0131 | | A2 | | 375 | | SVA | <LLOQ | 0.25 |
| pBC0132 | | A2 | | 403 | | APD | 1.65 | 0.25 |
| pSD0033 | | A2 | | 409 | | YKS | 0.936 | 0.089 |
| pBC0170 | | A2 | | 442 | | EAI | 0.26 | 0.064 |
| pBC0133 | | A2 | | 490 | | RRL | 0.22 | 0.19 |
| pBC0171 | | A2 | | 518 | | TVE | <LLOQ | 0.009 |
| pBC0134 | | A2 | | 599 | | NPA | 0.74 | 0.16 |
| pBC0172 | | A2 | | 713 | | CDK | 0.116 | 0.289 |
| pBC0138 | | A3 | | 1720 | | LRN | 2.4 | 0.41 |
| pBC0139 | | A3 | | 1796 | | EDQ | 0.157 | 0.096 |
| pBC0140 | | A3 | | 1802 | | AEP | 0.134 | 0.127 |
| pBC0173 | | A3 | | 1827 | | PTK | <LLOQ | <LLOQ |
| pBC0174 | | A3 | | 1861 | | HTN | <LLOQ | <LLOQ |
| pBC0175 | | A3 | | 1896 | | NME | <LLOQ | <LLOQ |
| pBC0176 | | A3 | | 1900 | | NCR | 0.973 | 0.242 |
| pBC0177 | | A3 | | 1904 | | PCN | 0.0689 | 0.016 |
| pBC0178 | | A3 | | 1937 | | AQD | <LLOQ | <LLOQ |
| pBC0141 | | A3 | | 2019 | | YSN | <LLOQ | 0.04 |
| pBC0179 | | C1 | | 2068 | | EPF | 0.34 | 0.271 |
| pBC0180 | | C1 | | 2111 | | GKK | <LLOQ | <LLOQ |
| pBC0142 | | C1 | | 2120 | | NST | <LLOQ | 0.07 |
| pBC0143 | | C1 | | 2171 | | CDL | 0.66 | 0.52 |
| pBC0181 | | C2 | | 2188 | | SDA | <LLOQ | <LLOQ |
| pBC0182 | | C2 | | 2227 | | NPK | 0.416 | 0.173 |
| pBC0144 | | C2 | | 2277 | | FQN | 0.251 | 0.062 |

Permissive sites into which heterologous moieties were inserted without eliminating procoagulant activity of the recombinant protein, or the ability of the recombinant proteins to be expressed in the host cell were clustered within loops in each of the three A domains of FVIII. FIG. 8 shows the location of insertion sites in the recombinant FVIII proteins that showed FVIII activity on domains A1, A2 and A3. FIG. 5 shows a structural representation depicting the location of insertion sites in the recombinant FVIII proteins that showed FVIII activity.

The permissive sites clustered in solvent exposed, highly flexible surface loops (permissive loops). The A1 domain loops were located in a region corresponding approximately to amino acid positions 15 to 45, and 201 to 232, respectively, in the sequence of mature human FVIII (SEQ ID NO: 1). The A2 domain loops were located in a region corresponding approximately to amino acid positions 395 to 421, and 577 to 635, respectively, in the sequence of mature human FVIII (SEQ ID NO: 1). The A3 domain loops were located in a region corresponding approximately to amino acid positions 1705 to 1732, and 1884 to 1917, respectively, in the sequence of mature human FVIII (SEQ ID NO: 1). FIGS. 9A and 9B show the location of the permissive loops relative to secondary structure elements in the tridimensional structure of FVIII.

### Example 4: XTEN 144 Insertion

Analysis of the preliminary data presented above (Example 3B) suggested the existence of defined regions within the linear polypeptide sequences and 3-D structures of the FVIII A domains that can accommodate the insertion of heterologous polypeptide sequences. To test this hypothesis and further define the boundaries of putative regions that can accommodate the insertion of heterologous polypeptides without loss of FVIII activity, 23 additional insertion sites not present in either Batch 1 or 2 were chosen and designated Batch 3.

Batch 3 constructs were generated by the insertion of a 144 residue XTEN AE polypeptide, comprising amino acid residues Gly (G), Ala (A), Pro (P), Ser (S), Thr (T), and Glu (E), or a 144 residue XTEN AG polypeptide, comprising amino acid residues Gly (G), Ala (A), Pro (P), Ser (S), and Thr (T). Five different version of the 144 residue AE polypeptide were generated and designated XTEN-AE144-2A (SEQ ID NO:15), XTEN-AE144-3B (SEQ ID NO: 17), XTEN-AE144-4A (SEQ ID NO: 19), XTEN-AE144-5A (SEQ ID NO:21), XTEN-AE144-6B (SEQ ID NO:23. Five different versions of the 144 residue polypeptide were generated and designated XTEN-AG144-1 (SEQ ID NO:25), XTEN-AG144-A (SEQ ID NO:27), XTEN-AG144-B (SEQ ID NO:29), XTEN-AG144-C (SEQ ID NO:31), and XTEN-AG144-F (SEQ ID NO:33).

The 144 residue XTEN encoding DNA sequence was introduced by the chemical synthesis of DNA segments (GENEART^{®} Gene Synthesis, Invitrogen, Carlsbad, CA) spanning the nearest unique restriction sites within the base vector on either side of the site of insertion.

The DNA sequences corresponding to the XTEN 144 peptides were inserted such that the resulting DNA construct would encode a FVIII fusion protein in which the XTEN 144 protein sequence is inserted immediately after the residue indicated in the site selection, and flanked by *Asc*I and *Xho*I sites.

In addition to these sites, those sites from Batch 1 and 2 at which insertion of the XTEN AE42 polypeptide did not abolish FVIII procoagulant activity were modified by excision of the AE42 polypeptide encoding DNA segment with restriction enzymes *Asc*I and *Xho*I*,* and introduction of XTEN AE144 and XTEN AG144 coding sequences at the same sites. The location of these Batch 1, Batch 2 and Batch insertion sites is summarized in TABLE 3. FIG. 6 presents a structural representation of FVIII showing the location of the XTEN 144 insertion sites.

**TABLE 3. Location of insertion sites.**

| Construct | Domain | | Insertion Site | | Upstream Sequence | | XTEN Type | Batch |
|---|---|---|---|---|---|---|---|---|
| pSD0045 | | A1 | | 18 | | YMQ | AE144-5A | 2 |
| pSD0046 | | A1 | | 18 | | YMQ | AG144-F | 2 |
| pSD0047 | | A1 | | 22 | | DLG | AE144-5A | 2 |
| pSD0048 | | A1 | | 22 | | DLG | AG144-F | 2 |
| pSD0049 | | A1 | | 26 | | LPV | AE144-5A | 2 |
| pSD0050 | | A1 | | 26 | | LPV | AG144-F | 2 |
| pSD0051 | | A1 | | 40 | | FPF | AE144-5A | 2 |
| pSD0052 | | A1 | | 40 | | FPF | AG144-F | 2 |
| pSD0023 | | A1 | | 65 | | KPR | AE144_5A | 3 |
| pSD0024 | | A1 | | 81 | | EVY | AE144_5A | 3 |
| pSD0025 | | A1 | | 119 | | QTS | AG144_F | 3 |
| pSD0026 | | A1 | | 211 | | HSE | AG144_F | 3 |
| pSD0053 | | A1 | | 216 | | NSL | AE144-2A | 1 |
| pSD0054 | | A1 | | 216 | | NSL | AG144-A | 1 |
| pSD0027 | | A1 | | 220 | | QDR | AG144_F | 3 |
| pSD0028 | | A1 | | 224 | | AAS | AG144_F | 3 |
| pSD0029 | | A1 | | 336 | | QLR | AG144_F | 3 |
| pSD0030 | | A1 | | 339 | | MKN | AG144_F | 3 |
| pSD0055 | | A2 | | 375 | | SVA | AG144-A | 1 |
| pSD0031 | | A2 | | 378 | | KKH | AE144_2A | 3 |
| pSD0032 | | A2 | | 399 | | PLV | AE144_2A | 3 |
| pSD0001 | | A2 | | 403 | | APD | AE144_2A | 1 |
| pSD0003 | | A2 | | 403 | | APD | AG144_A | 1 |
| pSD0034 | | A2 | | 416 | | NGP | AE144_2A | 3 |
| pSD0056 | | A2 | | 442 | | EAI | AE144-A2 | 2 |
| pSD0057 | | A2 | | 442 | | EAI | AG144-A | 2 |
| pSD0035 | | A2 | | 487 | | PLY | AE144_2A | 3 |
| pSD0036 | | A2 | | 494 | | PKG | AE144_2A | 3 |
| pSD0037 | | A2 | | 500 | | LKD | AE144_2A | 3 |
| pSD0002 | | A2 | | 599 | | NPA | AE144_2A | 1 |
| pSD0004 | | A2 | | 599 | | NPA | AG144_A | 1 |
| pSD0038 | | A2 | | 603 | | VQL | AG144_A | 3 |
| pSD0039 | a3 region | | | 1656 | | TLQ | AG144_C | 3 |
| pSD0040 | | A3 | | 1711 | | YGM | AE144_4A | 3 |
| pSD0009 | | A3 | | 1720 | | LRN | AE144_4A | 1 |
| pSD0010 | | A3 | | 1720 | | LRN | AG144_C | 1 |
| pSD0041 | | A3 | | 1725 | | QSG | AE144_4A | 3 |
| pSD0042 | | A3 | | 1749 | | LYR | AE144_4A | 3 |
| pSD0058 | | A3 | | 1796 | | EDQ | AE144-4A | 1 |
| pSD0059 | | A3 | | 1796 | | EDQ | AG144-C | 1 |
| pSD0060 | | A3 | | 1802 | | AEP | AE144-4A | 1 |
| pSD0061 | | A3 | | 1802 | | AEP | AG144-C | 1 |
| pSD0062 | | A3 | | 1900 | | NCR | AE144_4A | 3 |
| pSD0063 | | A3 | | 1900 | | NCR | AG144_C | 3 |
| pSD0043 | | A3 | | 1905 | | CNI | AG144_C | 3 |
| pSD0044 | | A3 | | 1910 | | EDP | AG144_C | 3 |
| pSD0011 | | C1 | | 2171 | | CDL | AE144_5A | 1 |
| pSD0012 | | C1 | | 2171 | | CDL | AG144_F | 1 |

### Expression of FVIII-XTEN 144 Variants

FVIII variants with XTEN 144 insertions were transfected into HEK293F cells (Invitrogen, Carlsbad, CA) using polyethyleneimine (PEI, Polysciences Inc. Warrington, PA) or LIPOFECTAMINE^{®} transfection reagent (Invitrogen, Carlsbad, CA). The transiently transfected cells were grown in 293 Free Style medium or a mixture of 293 Free Style and CD OPTICHO^{®} media (Invitrogen, Carlsbad, CA). The cell culture medium was harvested 3-5 days after transfection and analyzed for FVIII expression by chromogenic FVIII activity assay and FVIII ELISA as discussed in Example 3.

Cell culture media from transient transfection were concentrated 10-fold in CENTRICON^{®} spin columns (30 kDa MW cut-off). Concentrated material was then flash frozen and stored at -80°C for future *in vitro* analysis and *in vivo* PK studies.

### In vitro assays

To assess FVIII tolerability to insertions, the FVIII activity in culture media samples from cell cultures was analyzed using a FVIII chromogenic assay. Antigen expression levels were analyzed by FVIII-HC (FVIII heavy chain) and FVIII-LC (FVIII light chain) ELISA (see Example 3).

### FVIII Activity Measurement by Chromogenic Assay and Expression Measurement by FVIII-HC and FVIII-LC ELISA

Chromogenic and ELISA assays were conducted as described in Example 3. The results obtained are summarized in TABLE 4.

**TABLE 4. Location of insertion sites and expression/activity**

| Construct | Domain | Insertion Site | Upstream Sequence | XTEN Type | Batch | FVIII Activity (IU/mL) | FVIII Antigen (ug/ml) |
|---|---|---|---|---|---|---|---|
| pSD0045 | A1 | 18 | YMQ | AE144-5A | 2 | 0.171 | 0.032 |
| pSD0046^{∗} | A1 | 18 | YMQ | AG144-F | 2 | <LLOQ | <LLOQ |
| pSD0047^{∗} | A1 | 22 | DLG | AE144-5A | 2 | <LLOQ | <LLOQ |
| pSD0048^{∗} | A1 | 22 | DLG | AG144-F | 2 | <LLOQ | <LLOQ |
| pSD0049 | A1 | 26 | LPV | AE144-5A | 2 | 0.374 | 0.076 |
| pSD0050 | A1 | 26 | LPV | AG144-F | 2 | 0.952 | 0.203 |
| pSD0051 | A1 | 40 | FPF | AE144-5A | 2 | 0.043 | 0.009 |
| pSD0052 | A1 | 40 | FPF | AG144-F | 2 | 1.18 | 0.244 |
| pSD0023 | A1 | 65 | KPR | AE144_5A | 3 | <LLOQ | <LLOQ |
| pSD0024 | A1 | 81 | EVY | AE144_5A | 3 | <LLOQ | <LLOQ |
| pSD0025 | A1 | 119 | QTS | AG144_F | 3 | <LLOQ | <LLOQ |
| pSD0026 | A1 | 211 | HSE | AG144_F | 3 | 0.055 | 0.013 |
| pSD0053 | A1 | 216 | NSL | AE144-2A | 1 | <LLOQ | <LLOQ |
| pSD0054 | A1 | 216 | NSL | AG144-A | 1 | <LLOQ | <LLOQ |
| pSD0027 | A1 | 220 | QDR | AG144_F | 3 | 0.1 | 0.012 |
| pSD0028 | A1 | 224 | AAS | AG144_F | 3 | 0.108 | 0.023 |
| pSD0029 | A1 | 336 | QLR | AG144_F | 3 | 0.289 | 0.214 |
| pSD0030 | A1 | 339 | MKN | AG144_F | 3 | 0.374 | 0.181 |
| pSD0055 | A2 | 375 | SVA | AG144-A | 1 | <LLOQ | 0.221 |
| pSD0031 | A2 | 378 | KKH | AE144_2A | 3 | <LLOQ | 0.166 |
| pSD0032 | A2 | 399 | PLV | AE144_2A | 3 | 0.427 | 0.043 |
| pSD0001 | A2 | 403 | APD | AE144_2A | 1 | 0.287 | 0.047 |
| pSD0003 | A2 | 403 | APD | AG144_A | 1 | 0.364 | 0.057 |
| pSD0034 | A2 | 416 | NGP | AE144_2A | 3 | 0.067 | 0.009 |
| pSD0056 | A2 | 442 | EAI | AE144-A2 | 2 | <LLOQ | <LLOQ |
| pSD0057 | A2 | 442 | EAI | AG144-A | 2 | <LLOQ | <LLOQ |
| pSD0035 | A2 | 487 | PLY | AE144_2A | 3 | <LLOQ | 0.052 |
| pSD0036 | A2 | 494 | PKG | AE144_2A | 3 | <LLOQ | 0.021 |
| pSD0037 | A2 | 500 | LKD | AE144_2A | 3 | <LLOQ | 0.007 |
| pSD0002 | A2 | 599 | NPA | AE144_2A | 1 | 0.116 | 0.021 |
| pSD0004 | A2 | 599 | NPA | AG144_A | 1 | 0.114 | 0.021 |
| pSD0038 | A2 | 603 | VQL | AG144_A | 3 | 0.1 | 0.013 |
| pSD0039 | a3 region | 1656 | TLQ | AG144_C | 3 | 1.67 | 0.382 |
| pSD0040 | A3 | 1711 | YGM | AE144_4A | 3 | 0.132 | 0.02 |
| pSD0009 | A3 | 1720 | LRN | AE144_4A | 1 | 0.079 | 0.02 |
| pSD0010 | A3 | 1720 | LRN | AG144_C | 1 | 0.223 | 0.053 |
| pSD0041 | A3 | 1725 | QSG | AE144_4A | 3 | 0.255 | 0.031 |
| pSD0042 | A3 | 1749 | LYR | AE144_4A | 3 | <LLOQ | <LLOQ |
| pSD0058 | A3 | 1796 | EDQ | AE144-4A | 1 | <LLOQ | <LLOQ |
| pSD0059 | A3 | 1796 | EDQ | AG144-C | 1 | 0.044 | 0.028 |
| pSD0060 | A3 | 1802 | AEP | AE144-4A | 1 | <LLOQ | 0.011 |
| pSD0061 | A3 | 1802 | AEP | AG144-C | 1 | <LLOQ | <LLOQ |
| pSD0062 | A3 | 1900 | NCR | AE144_4A | 3 | 0.559 | 0.063 |
| pSD0063 | A3 | 1900 | NCR | AG144_C | 3 | 1.09 | 0.134 |
| pSD0043 | A3 | 1905 | CNI | AG144_C | 3 | 0.253 | 0.032 |
| pSD0044 | A3 | 1910 | EDP | AG144_C | 3 | 0.198 | 0.026 |
| pSD0011 | C2 | 2171 | CDL | AE144_5A | 1 | <LLOQ | <LLOQ |
| pSD0012 | C2 | 2171 | CDL | AG144_F | 1 | <LLOQ | <LLOQ |

| | | | | | | | |
|---|---|---|---|---|---|---|---|
| ^{∗} Cell culture supernatants resulting from transfection with DNA constructs pSD0046, pSD0047, and pSD0047 exhibited no detectable activity or antigen levels. This result was subsequently ascribed to a lack of DNA in these preparations due to degradation. | | | | | | | |

Permissive sites into which heterologous moieties were inserted without eliminating procoagulant activity of the recombinant protein, or the ability of the recombinant proteins to be expressed in the host cell clustered within loops in each of the three A domains of FVIII. The same permissive loop regions tolerating the shorter heterologous moieties inserted were found to tolerate the insertion of the longer heterologous sequences. FIG. 10 shows the location of XTEN 144 insertion sites within domains A1, A2, and A3 that showed FVIII activity in the resulting recombinant FVIII proteins. FIG. 7 shows a structural representation depicting the location of insertion sites in the recombinant FVIII proteins that showed FVIII activity.

These observation indicate that two regions within each of the A domains of FVIII are able to accommodate insertion of heterologous polypeptides without loss of FVIII cofactor activity. A structural depiction of these so-called permissive loops (FIGS. 11 and 12) demonstrate that they occupy structurally analogous positions in each of the A domains and project from one face of the FVIII molecule. The identified permissive loops correspond to highly flexible loops located between beta strands in the three-dimensional structures of the A1, A2, and A3 domains, as shown in FIGS. 9A and 9B.

### In vivo Evaluation of XTEN 144 insertions on FVIII Half-life Extension

### Cell Culture Media PK in HemA Mice

HemA mice (8-12 weeks old) were dosed with cell culture concentrate at 100-300 IU/kg (n=3/group). Plasma samples were collected at 5 minutes, 24 hours and 48 hours post dosing by retro orbital blood collection from the same set of mice. The FVIII activities of plasma samples and cell culture concentrates were analyzed by FVIII chromogenic assay as previously described. The PK profiles of FVIII XTEN 144 variants were analyzed using WINNONLIN^{®} (Pharsight Corp., Mountain View, CA).

The PK profile of two FVIII variants with XTEN 144 intra domain insertions (pSD0050 and pSD0062, *see* TABLE 3) were compared with B domain-deleted (BDD)-FVIII by cell culture PK in HemA mice *(see* FIG. 13, panel A; and TABLE 5). Comparable initial recovery for the three tested FVIII molecules was observed, as show in FIG. 13, panel A. Both FVIII XTEN 144 variants exhibited a half-life two-fold longer when compared to wild-type BDD-FVIII.

### Cell Culture Media PK in FVIII-VWF DKO Mice

Male FVIII/VWF double knock-out (DKO) mice, 8-12 weeks old, were treated with a single intravenous administration of cell culture concentrates containing either recombinant BDD-FVIII, pSD0050 or pSD062 at 100-300 IU/kg (n=3/group). At 5 min, 8 hr and 16 hr post infusion, blood samples were collected via retro orbital bleeds from the same set of mice. The FVIII activity of plasma samples and cell culture concentrates were analyzed by a FVIII chromogenic assay, and the PK profile of rBDD FVIII and FVIII XTEN 144 variants were analyzed using the WINNONLIN^{®} program.

The PK profile of the two FVIII XTEN 144 intradomain insertion variants pSD0050 and pSD0062 and rBDD-FVIII in FVIII/VWF DKO mice is shown in FIG. 13, panel B, and TABLE 5. Because of the loss of VWF protection, rBDD-FVIII had only a 15 min plasma half-life. In the case of the two XTEN insertion variants, however, half-life was extended to 3.15 hr and 3.83 hr, respectively. Under the experimental conditions, the study results demonstrate that intradomain insertion of an XTEN with 144 amino acid residues at these permissive loop regions not only preserved FVIII activity, but also provided extension of FVIII half-life.

**TABLE 5. Pharmacokinetic parameters of CFXTEN in HemA and FVIII/VWF DKO mice**

| Mouse Strain | Treatme nt | 5 min Recove ry (%) ry (%) | t_{1/2} (hr) | MRT (hr) | Cl (mL/hr/k g) | Vss (mL/kg) | AUC _D (hr^{∗}kg^{∗}mI U/ml/mIU ) | t_{1/2} Fold Increase^{∗} |
|---|---|---|---|---|---|---|---|---|
| HemA | pSD005 0 | 40 | 14.12 | 14.25 | 5.27 | 75.03 | 0.19 | 2.3 |
| | pSD006 2 | 43 | 12.96 | 14.79 | 4.24 | 62.67 | 0.24 | 2.1 |
| | rBDD-FVIII | 47 | 6.19 | 2.62 | 6.35 | 16.62 | 0.16 | |
| | | | | | | | | |
| FVIII/ VWF DKO | pSD005 0 | 34 | 3.15 | 2.59 | 21.73 | 56.28 | 0.05 | ~12 |
| | pSD006 2 | 35 | 3.83 | 3.71 | 18.51 | 68.69 | 0.05 | ~15 |
| | rBDD-FVIII | 23 | ~0.25 | | | | | |

| | | | | | | | | |
|---|---|---|---|---|---|---|---|---|
| ^{∗} Compared to rBDD-FVIII | | | | | | | | |

### Example 5: Multiple XTEN Insertion

After demonstrating that FVIII can tolerate the insertion of 42 and 144 amino acid long XTEN sequences in permissive sites without loss of cofactor function, variants containing two XTEN peptides were designed. These FVIII variants contained two XTEN 144 insertions, two XTEN 288 insertions, or one XTEN 144 and one XTEN 288 insertion. Ten 144 amino acid residues-long XTEN sequences were selected for insertion at multiple locations in FVIII: XTEN-AE144-2A (SEQ ID NO:15), XTEN-AE144-3B (SEQ ID NO:17), XTEN-AE144-4A (SEQ ID NO:19), XTEN-AE144-5A (SEQ ID NO:21), XTEN-AE144-6B (SEQ ID NO:23), XTEN-AG144-1 (SEQ ID NO:25), XTEN-AG144-A (SEQ ID NO:27), XTEN-AG144-B (SEQ ID NO:29), XTEN-AG144-C (SEQ ID NO:31), and XTEN-AG144-F (SEQ ID NO:33). Three different 288 amino acid residues-long XTEN sequences were selected for insertion at multiple locations in FVIII: XTEN-AE288_1 (SEQ ID NO:45), XTEN-AG228-2 (SEQ ID NO:46), and XTEN-AG228-1 (SEQ ID NO:47). Insertion sites were selected as described in Examples 2A and 3B, above. The locations of the insertion sites, XTENs inserted, and additional mutations introduced in the FVIII variants are summarized in TABLE 4.

The DNA sequences corresponding to the XTEN 144 and 288 peptides were inserted such that the resulting DNA construct would encode an FVIII fusion protein in which the XTEN 144 protein sequence is inserted immediately after the residue indicated in the site selection.

### Expression of FVIII-XTEN Double Variants

FVIII variants with XTEN 144 and XTEN 288 insertions were transfected into HEK293F cells (Invitrogen, Carlsbad, CA) using polyethyleneimine (PEI, Polysciences Inc. Warrington, PA) or LIPOFECTAMINE^{®} transfection reagent (Invitrogen, Carlsbad, CA). The transiently transfected cells were grown in 293 Free Style medium or a mixture of 293 Free Style and CD OPTICHO^{®} media (Invitrogen, Carlsbad, CA). The cell culture medium was harvested 3-5 days after transfection and analyzed for FVIII expression by chromogenic FVIII activity assay and FVIII ELISA.

FVIII-XTEN double variant cell culture media from transient transfection were concentrated 10-fold in CENTRICON^{®} spin columns (30 kDa MW cut-off). Concentrated material was then flash frozen and stored at -80°C for future *in vitro* analysis and *in vivo* PK studies.

### In vitro assays

To assess FVIII tolerability to XTEN 144 insertions, the FVIII activity in culture media samples from FVIII-XTEN cell cultures was analyzed using a FVIII chromogenic assay as previously described. Antigen expression levels will be analyzed by FVIII-HC (FVIII heavy chain) and FVIII-LC (FVIII light chain) ELISA.

### FVIII-XTEN Variant Activity Measurement by Chromogenic Assay

Chromogenic assays were conducted as described in Example 3. The results obtained are summarized in TABLE 4.

**TABLE 4. Cell culture results for two XTEN insertion constructs**

| Library | DNA Construct | XTEN Insertion 1 | XTEN Insertion 2 | Additional Modifications | FVIII Activity (IU/ml) |
|---|---|---|---|---|---|
| L01 | LSD0001.002 | 0745_AE288_1 | 2332_AE144_6B | | 2.346 |
| | LSD0001.013 | 0745_AE288_1 | 2332_AE144_6B | R1648A | 1.865 |
| | LSD0001.005 | 0745_AE144_3B | 2332_AE144_6B | | 1.730 |
| | LSD0001.012 | 0745_AE144_3B | 2332_AE144_6B | R1648A | 2.565 |
| | LSD0001.011 | 0745_AG144_B | 2332_AE144_6B | | 2.816 |
| | LSD0001.006 | 0745_AG144_B | 2332_AE144_6B | R1648A | 3.988 |
| | LSD0001.021 | 0745_AG288_2 | 2332_AE144_6B | | 2.223 |
| | LSD0001.016 | 0745_AG288_2 | 2332_AE144_6B | R1648A | 3.272 |
| | | | | | |
| | LSD0002.001 | 0745_AE288_1 | 2332_AG144_1 | | 1.188 |
| | LSD0002.014 | 0745_AE288_1 | 2332_AG144_1 | R1648A | 3.528 |
| | LSD0002.002 | 0745_AG288_2 | 2332_AG144_1 | | 0.984 |
| | LSD0002.013 | 0745_AG288_2 | 2332_AG144_1 | R1648A | 2.299 |
| | LSD0002.005 | 0745_AG144_B | 2332_AG144_1 | | 3.159 |
| | LSD0002.025 | 0745_AE144_3B | 2332_AG144_1 | | 3.161 |
| | | | | | |
| | LSD0003.005 | 0745_AE288_1 | 2332_AE288_1 | | 0.511 |
| | LSD0003.004 | 0745_AE288_1 | 2332_AE288_1 | R1648A | 2.072 |
| | LSD0003.009 | 0745_AE144_3B | 2332_AE288_1 | | 2.307 |
| | LSD0003.006 | 0745_AE144_3B | 2332_AE288_1 | R1648A | 2.484 |
| | LSD0003.014 | 0745_AG288_2 | 2332_AE288_1 | R1648A | 0.061 |
| | LSD0003.016 | 0745_AG144_B | 2332_AE288_1 | | 2.570 |
| | LSD0003.025 | 0745_AG144_B | 2332_AE288_1 | R1648A | 2.139 |
| | | | | | |
| | LSD0004.010 | 0745_AE288_1 | 2332_AG288_1 | | 1.160 |
| | LSD0004.016 | 0745_AE288_1 | 2332_AG288_1 | R1648A | 0.224 |
| | LSD0004.022^{∗} | 0745_AG288_2 | 2332_AG288_1 | | |
| | LSD0004.014 | 0745_AG288_2 | 2332_AG288_1 | R1648A | 0.275 |
| | LSD0004.011 | 0745_AG144_B | 2332_AG288_1 | | 0 |
| | LSD0004.025 | 0745_AE144_3B | 2332_AG288_1 | | 1.083 |
| | | | | | |
| L02 | LSD0005.002 | 0026_AG_144_F | 0403_AE144_2A | | 0.765 |
| | LSD0005.004 | 0026_AE144_5A | 0403_AE144_2A | | 0.410 |
| | LSD0005.005 | 0040_AG_144F | 0403_AE144_2A | | 0.688 |
| | LSD0005.011 | 0040_AE144_5A | 0403_AE144_2A | | 0.380 |
| | LSD0005.018 | 0018_AE144_5A | 0403_AE144_2A | | 0.770 |
| | | | | | |
| | LSD0006.002 | 0026_AE144_5A | 0599_AE144_2A | | 0.161 |
| | LSD0006.005 | 0040_AG_144F | 0599_AE144_2A | | 0.450 |
| | LSD0006.007 | 0026_AG_144F | 0599_AE144_2A | | 0.432 |
| | LSD0006.011 | 0018_AG_144F | 0599_AE144_2A | | 0.975 |
| | | | | | |
| | LSD0007.002 | 0040_AG_144F | 0403_AG144_A | | 1.377 |
| | LSD0007.004 | 0026_AG_144F | 0403_AG144_A | | 1.308 |
| | LSD0007.013 | 0026_AE144_5A | 0403_AG144_A | | 0.726 |
| | | | | | |
| | LSD0008.001 | 0026_AG_144F | 0599_AG144_A | | 0.528 |
| | LSD0008.002 | 0040_AG_144F | 0599_AG144_A | | 0.426 |
| | LSD0008.006 | 0026_AE144_5A | 0599_AG144_A | | 0.274 |
| | LSD0008.009 | 0018_AE144_5A | 0599_AG144_A | | 0.445 |
| | LSD0008.017 | 0040_AE144_5A | 0599_AG144_A | | 0.222 |
| | | | | | |
| L03 | LSD0044.002 | 1720_AG144_C | 1900_AE144_4A | | <LLOQ |
| | LSD0044.005 | 1725_AE144_4A | 1900_AE144_4A | | <LLOQ |
| | LSD0044.039 | 1720_AG144_C | 1900_AG144_C | | <LLOQ |
| | LSD0044.022 | 1711_AE144_4A | 1905_AG144_C | | <LLOQ |
| | LSD0044.003 | 1720_AG144_C | 1905_AG144_C | | <LLOQ |
| | LSD0044.001 | 1725_AE144_4A | 1905_AG144_C | | <LLOQ |
| | LSD0038.001 | 1656_AG144_C | 0026_AG144_F | | 0.504 |
| | LSD0038.003 | 1656_AG144_C | 0018_AE144_5A | | 0.662 |
| | LSD0038.008 | 1656_AG144_C | 0018_AG144_F | | 1.119 |
| | LSD0038.012 | 1656_AG144_C | 0040_AE144_5A | | 0.402 |
| | LSD0038.013 | 1656_AG144_C | 0040_AG144_F | | 0.764 |
| | LSD0038.015 | 1656_AG144_C | 0026_AE144_5A | | 0.420 |
| | LSD0039.001 | 1656_AG144_C | 0399_AE144_2A | | 0.266 |
| | LSD0039.003 | 1656_AG144_C | 0403_AG144_A | | 0.503 |
| | LSD0039.010 | 1656_AG144_C | 0403_AE144_2A | | 0.344 |
| | LSD0045.001 | 1656_AG144_C | 1725_AE144_4A | | 0.165 |
| | LSD0045.002 | 1656_AG144_C | 1720_AG144_C | | 0.396 |
| | LSD0042.014 | 1900_AE144_4A | 0018_AE144_5A | | 0.106 |
| | LSD0042.023 | 1900_AE144_4A | 0018_AG144_F | | 0.097 |
| | LSD0042.006 | 1900_AE144_4A | 0026_AE144_5A | | 0.043 |
| | LSD0042.013 | 1900_AE144_4A | 0026_AG144_F | | 0.083 |
| | LSD0042.001 | 1900_AE144_4A | 0040_AG144_F | | 0.142 |
| | LSD0042.039 | 1900_AG144_C | 0040_AG144_F | | 0.163 |
| | LSD0042.047 | 1900_AG144_C | 0026_AG144_F | | 0.167 |
| | LSD0042.003 | 1905_AG144_C | 0018_AG144_F | | 0.102 |
| | LSD0042.004 | 1905_AG144_C | 0040_AG144_F | | <LLOQ |
| | LSD0042.008 | 1905_AG144_C | 0026_AG144_F | | <LLOQ |
| | LSD0042.038 | 1905_AG144_C | 0026_AE144_5A | | <LLOQ |
| | LSD0042.082 | 1905_AG144_C | 0040_AE144_5A | | <LLOQ |
| | LSD0042.040 | 1910_AG144_C | 0026_AG144_F | | <LLOQ |
| | LSD0037.002 | 0018_AG144_F | 0399_AE144_2A | | 0.448 |
| | LSD0037.009 | 0026_AG144_F | 0399_AE144_2A | | 0.124 |
| | LSD0037.011 | 0040_AG144_F | 0399_AE144_2A | | 0.092 |
| | LSD0047.002 | 0018_AG144_F | 0403_AE144_2A | | 0.463 |
| | LSD0047.005 | 0018_AG144_F | 0403_AG144_A | | 0.235 |
| | LSD0048.007 | 0018_AE144_5A | 0403_AG144_A | | 0.148 |
| | LSD0046.001 | 1656_AG144_C | 1900_AG144_C | | 0.302 |
| | LSD0046.002 | 1656_AG144_C | 1900_AE144_4A | | 0.123 |
| | LSD0046.003 | 1656_AG144_C | 1905_AG144_C | | 0.072 |
| | LSD0040.011 | 1711_AE144_4A | 0040_AG144_F | | <LLOQ |
| | LSD0040.042 | 1711_AE144_4A | 0026_AE144_5A | | <LLOQ |
| | LSD0040.002 | 1720_AG144_C | 0026_AG144_F | | 0.085 |
| | LSD0040.008 | 1720_AG144_C | 0040_AG144_F | | 0.078 |
| | LSD0040.021 | 1720_AG144_C | 0018_AE144_5A | | 0.075 |
| | LSD0040.037 | 1720_AG144_C | 0026_AE144_5A | | <LLOQ |
| | LSD0040.046 | 1720_AG144_C | 0018_AG144_F | | 0.155 |
| | LSD0040.003 | 1725_AE144_4A | 0026_AE144_5A | | <LLOQ |
| | LSD0040.006 | 1725_AE144_4A | 0040_AG144_F | | <LLOQ |
| | LSD0040.007 | 1725_AE144_4A | 0026_AG144_F | | <LLOQ |
| | LSD0040.010 | 1725_AE144_4A | 0018_AE144_5A | | <LLOQ |
| | LSD0040.039 | 1725_AE144_4A | 0040_AE144_5A | | <LLOQ |
| | LSD0040.052 | 1725_AE144_4A | 0018_AG144_F | | 0.046 |
| | LSD0041.001 | 1720_AG144_C | 0403_AG144_A | | 0.046 |
| | LSD0041.004 | 1720_AG144_C | 0399_AE144_2A | | <LLOQ |
| | LSD0041.006 | 1711_AE144_4A | 0403_AG144_A | | <LLOQ |
| | LSD0041.008 | 1720_AG144_C | 0403_AE144_2A | | <LLOQ |
| | LSD0041.010 | 1725_AE144_4A | 0403_AG144_A | | <LLOQ |
| | LSD0041.014 | 1725_AE144_4A | 0403_AE144_2A | | <LLOQ |
| | LSD0041.016 | 1725_AE144_4A | 0399_AE144_2A | | <LLOQ |
| | LSD0041.035 | 1711_AE144_4A | 0403_AE144_2A | | <LLOQ |
| | LSD0043.001 | 1900_AG144_C | 0399_AE144_2A | | <LLOQ |
| | LSD0043.002 | 1900_AG144_C | 0403_AG144_A | | <LLOQ |
| | LSD0043.005 | 1905_AG144_C | 0403_AG144_A | | <LLOQ |
| | LSD0043.006 | 1900_AE144_4A | 0399_AE144_2A | | <LLOQ |
| | LSD0043.007 | 1900_AE144_4A | 0403_AG144_A | | <LLOQ |
| | LSD0043.008 | 1900_AE144_4A | 0403_AE144_2A | | <LLOQ |
| | LSD0043.015 | 1905_AG144_C | 0399_AE144_2A | | <LLOQ |
| | LSD0043.029 | 1905_AG144_C | 0403_AE144_2A | | <LLOQ |
| | LSD0043.043 | 1910_AG144_C | 0403_AG144_A | | <LLOQ |

The "XTEN Insertion 1" and "XTEN Insertion 2" columns indicate the location and type of insertion, e.g., "1910_AG144_C" corresponds to the insertion of XTEN AG144-C at amino acid position 1910 of mature human FVIII. The "Additional Modifications" column indicates the location and type of additional mutations, e.g., "R1648A" indicated the mutation of the Arginine at amino acid position 1648 of mature human FVIII to Alanine.

### FVIII-XTEN Variant Expression Measurement by FVIII-HC and FVIII-LC ELISA

ELISA assays are conducted as described in Example 3.

### In vivo Evaluation of Multiple XTEN Insertions on FVIII Half-life Extension

### Cell Culture Media PK in HemA Mice

HemA mice (8-12 weeks old) are dosed with cell culture concentrate at 100-300 IU/kg (n=3/group). Plasma samples are collected at 5 minutes, 24 hours and 48 hours post dosing by retro orbital blood collection from the same set of mice. The FVIII activities of plasma samples and cell culture concentrates are analyzed by FVIII chromogenic assay as previously described. The PK profiles of FVIII variants with two XTEN insertions are analyzed using WINNONLIN^{®} (Pharsight Corp., Mountain View, CA).

The PK profile of FVIII variants with two XTEN intra domain insertions are compared with B domain-deleted (BDD)-FVIII by cell culture PK in HemA mice.

### Cell Culture Media PK in FVIII-VWF DKO Mice

FVIII-VWF DKO mice (8-12 weeks old) are dosed with cell culture concentrate at ~100 IU/kg (n=3/group). A blood sample is collected at 5 minutes post dosing to evaluate initial recovery, and another two blood collections from the same set of mice are performed for half-life evaluation (up to 96 hours post dosing). The FVIII activity of plasma samples and cell culture concentrates are analyzed by FVIII chromogenic assay as previously described. The PK profile of FVIII variants variants with two XTEN insertions are analyzed using WINNONLIN^{®} (Pharsight Corp., Mountain View, CA). The PK profile of FVIII variants with two XTEN intra domain insertions are compared with B-Domain Deleted (BDD)-FVIII by cell culture PK in FVIII-VWF DKO Mice.

### Example 6: GFP Insertion

Green fluorescent protein (GFP) is a ~30 kDa protein with intrinsic fluorescent properties and a compact 3-D structure in which the N- and C-termini are in close proximity (Shimomura et al., J. Cell Comp. Physiol. 59:223-39 (1962); Ormo et al., Science 273:1392-95 (1996); the crystal structure of GFP is available under the identifier PDB ID:1EMA at the Protein Data Bank). GFP (see, *e.g.,* SEQ ID NO:48), or variants thereof that exhibit distinct spectral properties and stability profiles (Davidson and Campbell, Nat. Methods 6:713-717 (2009); Rizzo et al. (2010). Fluorescent protein tracking and detection. In Live Cell Imaging: A Laboratory Manual (ed. Goldman, R. D., Spector, D. L. and Swedlow, J. R.), pp. 3-34. Cold Spring Harbor: Cold Spring Harbor Laboratory Press) is introduced within permissive loops and the a3 segment of the FVIII molecule by standard molecular biology techniques employing a DNA segment comprising a 5' *Asc*I restriction site, the coding sequence of GFP or variants thereof, and a 3' *Xho*I restriction site to enable insertion. The resulting recombinant FVIII protein is tested for procoagulant activity and can be used to visualize the location of the recombinant FVIII protein by methods known in the art.

GFP is inserted into at least one the locations disclosed in TABLES 1B or 3, other suitable insertion sites in at least one of permissive loops A1-1, A1-2, A2-1, A2-2, A3-1 or A3-2, or into the a3 region, or both. FVIII variants with GFP insertions are transfected and transiently expressed into HEK293F cells as described above. Cell culture media from transient transfection are concentrated 10-fold in CENTRICON^{®} spin columns (30 kDa MW cut-off). Concentrated material is then flash frozen and stored at -80°C for future *in vitro* analysis and *in vivo* PK studies. The FVIII activity in culture media samples from cell cultures expressing FVIII variants comprising GFP is analyzed using a FVIII chromogenic assay. Antigen expression levels are analyzed by FVIII-HC (FVIII heavy chain) and FVIII-LC (FVIII light chain) ELISA. The PK of FVIII variants comprising GFP is analyzed in HemA mice and FVIII-VWF DKO mice as described above.

The resulting recombinant FVIII protein is also tested to characterize its fluorescent properties and used to visualize the location of the recombinant FVIII protein using methods known in the art, *e.g.,* flow cytometry or microscopy, such as confocal microscopy.

### Example 7: Insertion of Heterologous Moieties increasing Half-Life

### Example 7.1 - Insertion of Fc region of IgG

Fusion of an Fc region of IgG confers an increase in circulating half-life to both coagulation factors IX and VIII when the Fc region is fused to the C-terminus of either protein (Dumont et al., Blood (2012), published online before print, doi: 10.1182/blood-2011-08-367813; Peters et al., Blood 115:2056-64 (2010); Shapiro et al., Blood 119:666-72 (2012)). As an alternative approach, a single-chain Fc (scFc) region, comprising identical Fc polypeptide sequences separated by a flexible glycine- and serine-containing linker (see, *e.g.,* SEQ ID NO:49) is introduced within permissive loops of the FVIII molecule by standard molecular biology techniques. This scFc region may additionally include terminal flexible linker sequences to enable insertion into permissive loops without structural distortion of the FVIII molecule. The DNA segment to be inserted comprises a 5' *Asc*I restriction site, the coding sequence of scFc, and a 3' *Xho*I restriction site to enable facile insertion into permissive loop sites. The resulting recombinant FVIII protein is tested for procoagulant activity and for extended half-life.

The Fc sequence is inserted into at least one the locations disclosed in TABLES 1B or 3, other suitable insertion sites in at least one of permissive loops A1-1, A1-2, A2-1, A2-2, A3-1 or A3-2, or into the a3 region, or both. FVIII variants with Fc insertions are transfected and transiently expressed into HEK293F cells as described above. Cell culture media from transient transfection are concentrated 10-fold in CENTRICON^{®} spin columns (30 kDa MW cut-off). Concentrated material is then flash frozen and stored at -80°C for future *in vitro* analysis and *in vivo* PK studies. The FVIII activity in culture media samples from cell cultures expressing FVIII variants comprising an Fc heterologous moiety is analyzed using a FVIII chromogenic assay. Antigen expression levels are analyzed by FVIII-HC (FVIII heavy chain) and FVIII-LC (FVIII light chain) ELISA. The PK of FVIII variants comprising an Fc heterologous moiety are analyzed in HemA mice and FVIII-VWF DKO mice as described above.

### Example 7.2 - Insertion of Albumin or Albumin-Binding Moieties

### Example 7.2.1 - Albumin Insertion

The circulating half-lives of recombinant coagulation factors can be extended by recombinant fusion of an albumin polypeptide to a protein terminus. See Schulte, Thromb. Res. 128(Suppl. 1):29-S12 (2011). As an alternative to this approach, the albumin polypeptide, either with or without flexible linker segments appended to its termini, can be introduced within the permissive loops and a3 segment of the FVIII molecule by standard molecular biology techniques. The DNA segment to be inserted comprises a 5' *Asc*I restriction site, the protein coding sequence of albumin (SEQ ID NO:50), an albumin variant or an albumin fragment, and a 3' *Xho*I restriction site to enable insertion into permissive loop sites. The resulting recombinant FVIII protein is tested for procoagulant activity and extended half-life.

The albumin sequence is inserted into at least one the locations disclosed in TABLES 1B or 3, other suitable insertion sites in at least one of permissive loops A1-1, A1-2, A2-1, A2-2, A3-1 or A3-2, or into the a3 region, or both. FVIII variants with albumin insertions are transfected and transiently expressed into HEK293F cells as described above. Cell culture media from transient transfection are concentrated 10-fold in CENTRICON^{®} spin columns (30kDa MW cut-off). Concentrated material is then flash frozen and stored at -80°C for future *in vitro* analysis and *in vivo* PK studies. The FVIII activity in culture media samples from cell cultures expressing FVIII variants comprising an albumin heterologous moiety is analyzed using a FVIII chromogenic assay. Antigen expression levels are analyzed by FVIII-HC (FVIII heavy chain) and FVIII-LC (FVIII light chain) ELISA. The PK of FVIII variant comprising an albumin heterologous moiety is analyzed in HemA mice and FVIII-VWF DKO mice as described above.

### Example 7.2.2: Insertion of Peptide Albumin-Binding Moieties

One or more polypeptide albumin-binding moieties such as RLIEDICLPRWGCLWEDD (SEQ ID NO: 52), QRLMEDICLPRWGCLWEDDF (SEQ ID NO:53), QGLIGDICLPRWGCLWGDSVK (SEQ ID NO:54) or GEWWEDICLPRWGCLWEEED (SEQ ID NO:55) is inserted into a permissive loop or into the a3 region of FVIII, or both, by standard molecular biology techniques. One approach is to synthesize a degenerative nucleotide sequence of the albumin-binding peptide, create appropriate restriction endonuclease sites, and then insert the albumin-binding moiety by restriction enzyme digestion and plasmid DNA ligation. A linker sequence, (GGGS)n, (Denise et al. J. Biol. Chem. 277:35035-35043 (2002)) where n can be 0, 1, 2, 3, 4, or more (SEQ ID NO: 51), can be added at the N-terminal and/or C-terminal of the albumin-binding peptide before inserting it to FVIII. The resulting recombinant FVIII protein is tested for procoagulant activity and extended half-life.

The sequence of the polypeptide albumin-binding moiety is inserted into at least one the locations disclosed in TABLES 1B or 3, other suitable insertion sites in at least one of permissive loops A1-1, A1-2, A2-1, A2-2, A3-1 or A3-2, or into the a3 region, or both. FVIII variants with inserted peptide albumin-binding moieties are transfected and transiently expressed into HEK293F cells as described above. Cell culture media from transient transfection are concentrated 10-fold in CENTRICON^{®} spin columns (100 kDa MW cut-off). Concentrated material is then flash frozen and stored at -80°C for future *in vitro* analysis and *in vivo* PK studies. The FVIII activity in culture media samples from cell cultures expressing FVIII variants with inserted peptide albumin-binding moieties is analyzed using a FVIII chromogenic assay. Antigen expression levels are analyzed by FVIII-HC (FVIII heavy chain) and FVIII-LC (FVIII light chain) ELISA. The PK of FVIII variants with inserted peptide albumin-binding moieties is analyzed in HemA mice and FVIII-VWF DKO mice as described above.

### Example 7.2.3 - Insertion of Small Molecule Albumin-Binding Moieties

In addition to peptide albumin-binding moieties, one or more small molecules that possess albumin-binding capability can also be attached within one or more of the permissive loops or the a3 region of FVIII. As FVIII does not have free cysteine at its surface based on crystal structure (PDB:2R7E, Shen et al., Blood 111:1240 (2008); PDB:3CDZ, Ngo, Structure, 16:597-606 (2008)), one approach is to insert a cysteine containing peptide *(e.g.,* GGGSGCGGGS) (SEQ ID NO:56) into a permissive loop or a3 region of FVIII. An albumin-binding 2-(3-maleimideopropananmido)-6-(4-(4-iodophenyl)butanamido)hexanoate can then be conjugated specifically to the cysteine introduced on FVIII. Briefly, the FVIII containing the Cys insertion can be constructed by standard molecular technology, and the FVIII expressed in mammalian expression system (e.g., HEK293, CHO, BHK21, PER.C6, CAP cells) can be purified via affinity and ion exchange chromatography.

The purified recombinant FVIII protein is reduced by Tris(2-carboxyethyl)phosphine (TCEP) to expose the thiol group of the introduced cysteine and then reacted with 2-(3-maleimideopropananmido)-6-(4-(4-iodophenyl)butanamido)hexanoate. The unconjugated recombinant FVIII protein can be removed by HSA affinity chromatography as the conjugated recombinant FVIII protein will bind the HAS affinity resin. The resulting recombinant FVIII protein is tested for procoagulant activity and extended half-life.

The small molecule albumin-binding moiety sequence is attached at least one the locations disclosed in TABLES 1B or 3, other suitable insertion sites in at least one of permissive loops A1-1, A1-2, A2-1, A2-2, A3-1 or A3-2, or into the a3 region, or both. The FVIII activity of FVIII variants with small molecule albumin-binding moieties is analyzed using a FVIII chromogenic assay. The PK of FVIII variants with small molecule albumin-binding moiety is analyzed in HemA mice and FVIII-VWF DKO mice as described above.

### Example 7.3 PEGylation

One or more polyethylene glycol (PEG) molecules can be attached within one or more of the permissive loops or the a3 region of FVIII. As FVIII does not have a free cysteine at its surface based on crystal structure (PDB:2R7E, Shen et al., Blood 111:1240 (2008); PDB:3CDZ, Ngo, Structure, 16:597-606 (2008)), one approach is to insert a cysteine containing peptide (e.g., GGGSGCGGGS) (SEQ ID NO: 56) into a permissive loop or the a3 region of FVIII. PEG molecules containing maleimide can then be conjugated specifically to the cysteine introduced on the recombinant FVIII protein. Briefly, the recombinant FVIII protein containing the Cys insertion can be constructed by standard molecular technology, and the recombinant FVIII protein expressed in mammalian expression system (e.g., HEK293, CHO, BHK21, PER.C6, CAP cells) can be purified via affinity and ion exchange chromatography. The purified recombinant FVIII protein is reduced by Tris(2-carboxyethyl)phosphine (TCEP) to expose the thiol group of the introduced cysteine and then reacted with maleimide PEG. The resulting recombinant FVIII protein is tested for procoagulant activity and extended half-life.

PEG is attached to at least one the locations disclosed in TABLES 1B or 3, other suitable insertion sites in at least one of permissive loops A1-1, A1-2, A2-1, A2-2, A3-1 or A3-2, or into the a3 region, or both. The FVIII activity of the PEGylated recombinant FVIII protein is analyzed using a FVIII chromogenic assay. The PK of the PEGylated recombinant FVIII protein is analyzed in HemA mice and FVIII-VWF DKO mice as described above.

### Example 7.4 - Insertion of the Carboxyl-Terminal Peptide of Human Chorionic Gonadotropin β-subunit (CTP)

Fusion of the 29 residue C-terminal peptide of human chorionic gonadotropin beta subunit has been demonstrated to enhance the pharmacokinetic properties of recombinant proteins (Fares et al., Proc. Natl. Acad. Sci. USA 89:4304-7 (1992)). CTP (DSSSSKAPPPSLPSPSRLPGPSDTPILPQ) (SEQ ID NO:62), or concatenated versions thereof, can be introduced within the permissive loops and a3 segment of the FVIII molecule by standard molecular biology techniques. The DNA segment to be inserted comprises a 5' AscI restriction site, the protein coding sequence CTP, and a 3' *Xho*I restriction site to enable insertion into permissive loop sites. The resulting recombinant FVIII protein is tested for procoagulant activity and extended half-life.

The CTP sequence is inserted into at least one the locations disclosed in TABLES 1B or 3, other suitable insertion sites in at least one of permissive loops A1-1, A1-2, A2-1, A2-2, A3-1 or A3-2, or into the a3 region, or both. FVIII with CTP insertions are transfected and transiently expressed into HEK293F cells as described above. Cell culture media from transient transfection are concentrated 10-fold in CENTRICON^{®} spin columns (30 kDa MW cut-off). Concentrated material is then flash frozen and stored at -80°C for future *in vitro* analysis and *in vivo* PK studies. The FVIII activity in culture media samples from cell cultures expressing recombinant FVIII proteins comprising a CTP heterologous moiety is analyzed using a FVIII chromogenic assay. Antigen expression levels are analyzed by FVIII-HC (FVIII heavy chain) and FVIII-LC (FVIII light chain) ELISA. The PK of recombinant FVIII proteins comprising a CTP heterologous moiety is analyzed in HemA mice and FVIII-VWF DKO mice as described above.

### Example 7.5 - Fusion to Clearance Receptor LRP1

Lipoprotein Receptor-related Protein-1 (LRP1) is a 600 kDa integral membrane protein that is implicated in the receptor-mediate clearance of a variety of proteins, including FVIII (Lenting et al., Haemophilia 16:6-15 (2010)). See SEQ ID NO:57 (human LRP1 sequence, comprising signal peptide)

The fusion of LRP1 to FVIII can result in intramolecular shielding of FVIII, thereby protecting FVIII from normal clearance by LRP1 and increasing its circulating half-life. The 4404 amino acid extracellular region of LRP1, or discrete domains or fragments thereof, can be introduced within the permissive loops or into an a3 segment of the FVIII molecule by standard molecular biology techniques. The DNA segment to be inserted comprises a 5' *AscI* restriction site, the protein coding sequence of LRP1 (or discrete domains or fragments thereof), and a 3' *Xho*I restriction site to enable insertion into permissive loop sites. The resulting recombinant FVIII protein is tested for procoagulant activity and extended half-life.

The LRP1 sequence (or discrete domains or fragments thereof) is inserted into at least one the locations disclosed in TABLES 1B or 3, other suitable insertion sites in at least one of permissive loops A1-1, A1-2, A2-1, A2-2, A3-1 or A3-2, or into the a3 region, or both. FVIII with LRP1 insertions are transfected and transiently expressed into HEK293F cells as described above. Cell culture media from transient transfection are concentrated 10-fold in CENTRICON^{®} spin columns (30 kDa MW cut-off). Concentrated material is then flash frozen and stored at -80°C for future *in vitro* analysis and *in vivo* PK studies. The FVIII activity in culture media samples from cell cultures expressing recombinant FVIII proteins comprising an LRP1 heterologous moiety is analyzed using a FVIII chromogenic assay. Antigen expression levels are analyzed by FVIII-HC (FVIII heavy chain) and FVIII-LC (FVIII light chain) ELISA. The PK of recombinant FVIII proteins comprising an LRP1 heterologous moiety is analyzed in HemA mice and FVIII-VWF DKO mice as described above.

### Example 7.6 - PASylation

FVIII PASylation refers to the recombinant fusion of FVIII with one or more polypeptides primarily composed of three amino acids, Alanine, Serine and Proline (See European Pat. Pub. No. EP2173890).

Exemplary PAS polypeptides can contain one or many repeats of the sequence ASPAAPAPASPAAPAPSAPA (SEQ ID NO:37), AAPASPAPAAPSAPAPAAPS (SEQ ID NO:38), APSSPSPSAPSSPSPASPSS (SEQ ID NO:39), APSSPSPSAPSSPSPASPS (SEQ ID NO:40), SSPSAPSPSSPASPSPSSPA (SEQ ID NO:41), AASPAAPSAPPAAASPAAPSAPPA (SEQ ID NO:42), or ASAAAPAAASAAASAPSAAA (SEQ ID NO:43). One or more PAS polypeptides can be inserted into a permissive loop or into the a3 region of FVIII, or both, by standard molecular biology techniques. One approach is to synthesize a degenerative nucleotide sequence of the PAS polypeptides, create appropriate restriction endonuclease sites, and insert the PAS polypeptides by restriction enzyme digestion and plasmid DNA ligation. A linker sequence such as (GGGS)ₙ, where n can be 0, 1, 2, 3, 4, or more, can be added at N-terminal and/or C-terminal of PAS polypeptide before inserting it to FVIII. The resulting recombinant FVIII protein is tested for procoagulant activity and extended half-life.

PAS sequences are inserted into at least one the locations disclosed in TABLES 1B or 3, other suitable insertion sites in at least one of permissive loops A1-1, A1-2, A2-1, A2-2, A3-1 or A3-2, or into the a3 region, or both. FVIII with PAS polypeptide insertions are transfected and transiently expressed into HEK293F cells as described above. Cell culture media from transient transfection are concentrated 10-fold in CENTRICON^{®} spin columns (30 kDa MW cut-off). Concentrated material is then flash frozen and stored at -80°C for future *in vitro* analysis and *in vivo* PK studies. The FVIII activity in culture media samples from cell cultures expressing recombinant FVIII proteins comprising a PAS polypeptide heterologous moiety are analyzed using a FVIII chromogenic assay. Antigen expression levels are analyzed by FVIII-HC (FVIII heavy chain) and FVIII-LC (FVIII light chain) ELISA. The PK of recombinant FVIII proteins comprising a PAS polypeptide heterologous moiety is analyzed in HemA mice and FVIII-VWF DKO mice as described above.

### Example 7.7 - HAPylation

FVIII HAPylation refers to the recombinant fusion of one or more polypeptides primarily composed of glycine rich homo-amino-acid polymer (HAP) to FVIII. Examples of HAP polypeptides can contain one (Gly₄Ser)ₙ module, where n can be 1, 2, and up to 400 (SEQ ID NO:60). One or more HAP polypeptides can be inserted into a permissive loop or into the a3 region of FVIII, or both, by standard molecular biology techniques. One approach is to synthesize a degenerative nucleotide sequence of the HAP polypeptide, create appropriate restriction enzyme sites, and insert the HAP polypeptide by restriction enzyme digestion and plasmid DNA ligation. The resulting recombinant FVIII protein is tested for procoagulant activity and extended half-life.

The HAP sequence is inserted into at least one the locations disclosed in TABLES 1B or 3, other suitable insertion sites in at least one of permissive loops A1-1, A1-2, A2-1, A2-2, A3-1 or A3-2, or into the a3 region, or both. FVIII with HAP polypeptide insertions are transfected and transiently expressed into HEK293F cells as described above. Cell culture media from transient transfection are concentrated 10-fold in CENTRICON^{®} spin columns (30 kDa MW cut-off). Concentrated material is then flash frozen and stored at -80°C for future *in vitro* analysis and *in vivo* PK studies. The FVIII activity in culture media samples from cell cultures expressing recombinant FVIII proteins comprising a HAP polypeptide heterologous moiety is analyzed using a FVIII chromogenic assay. Antigen expression levels are analyzed by FVIII-HC (FVIII heavy chain) and FVIII-LC (FVIII light chain) ELISA. The PK of FVIII variants comprising a HAP polypeptide heterologous moiety are analyzed in HemA mice and FVIII-VWF DKO mice as described above.

### Example 7.8 - HESylation

One or more hydroxyethyl starch (HES) molecules can be attached within one or more of the permissive loops or to the a3 region of FVIII. As FVIII does not have free cysteines at its surface based on its crystal structure (PDB:2R7E, Shen et al., Blood 111:1240 (2008); PDB:3CDZ, Ngo, Structure, 16:597-606 (2008)), one approach is to insert a cysteine containing peptide (e.g., GGGSGCGGGS) (SEQ ID NO:56) into a permission loop or a3 region of FVIII, HES molecules containing maleimide can then be conjugated specifically to the cysteine introduced on FVIII. Briefly, the recombinant FVIII protein containing a Cys insertion is constructed by standard molecular technology, the recombinant FVIII protein is expressed in a mammalian expression system (e.g., HEK293, CHO, BHK21, PER.C6, CAP cells), and then purified via affinity and ion exchange chromatography. The purified recombinant FVIII protein is reduced by Tris(2-carboxyethyl)phosphine (TCEP) to expose the thiol group of the introduced cysteine and then reacted with maleimide HES. The resulting recombinant FVIII proteins are tested for procoagulant activity and extended half-life.

The HES molecule is attached to at least one the locations disclosed in TABLES 1B or 3, other suitable insertion sites in at least one of permissive loops A1-1, A1-2, A2-1, A2-2, A3-1 or A3-2, or into the a3 region, or both. The FVIII activity of recombinant FVIII proteins comprising an HES heterologous moiety is analyzed using a FVIII chromogenic assay. The PK of recombinant FVIII proteins comprising an HES heterologous moiety is analyzed in HemA mice and FVIII-VWF DKO mice as described above.

### Example 7.9 - Transferrin fusion

One or more transferrin molecules or fragments or variants thereof can be inserted into a permissive loop or into the a3 region of FVIII, or both, by standard molecular biology techniques. One approach is to synthesize degenerative nucleotide sequences of the transferrin-peptide, create appropriate restriction endonuclease sites, and insert the transferrin by restriction enzyme digestion and plasmid DNA ligation. A linker sequence, (GGGS)ₙ(SEQ ID NO:51), where n can be 0, 1, 2, 3, 4, or more, can be added at N-terminal and/or C-terminal of the transferrin peptide before inserting it to FVIII. Alternative linkers such as PEAPTDPEAPTD (SEQ ID NO:61) can also be employed in place of GGGS linker (Kim et al., J. Pharmacol. Exp. Ther., 2010, 334, 682-692). The resulting recombinant FVIII protein is tested for procoagulant activity and extended half-life.

The transferrin sequence is inserted into at least one the locations disclosed in TABLES 1B or 3, other suitable insertion sites in at least one of permissive loops A1-1, A1-2, A2-1, A2-2, A3-1 or A3-2, or into the a3 region, or both. FVIII with transferrin insertions are transfected and transiently expressed into HEK293F cells as described above. Cell culture media from transient transfection are concentrated 10-fold in CENTRICON^{®} spin columns (30 kDa MW cut-off). Concentrated material is then flash frozen and stored at -80°C for future *in vitro* analysis and *in vivo* PK studies. The FVIII activity in culture media samples from cell cultures expressing recombinant FVIII proteins comprising a transferrin heterologous moiety is analyzed using a FVIII chromogenic assay. Antigen expression levels are analyzed by FVIII-HC (FVIII heavy chain) and FVIII-LC (FVIII light chain) ELISA. The PK of recombinant FVIII proteins comprising a transferrin heterologous moiety is analyzed in HemA mice and FVIII-VWF DKO mice as described above.

### Example 8: Insertion of Heterologous Moieties for Visualization

### Example 8.1 - Biotin Acceptor Peptide (BAP)

Biotin Acceptor Peptide (BAP) is a 13-residue peptide (LNDIFEAQKIEWH) (SEQ ID NO:58) identified by random peptide display methods that serves as a substrate *for E. coli* biotin ligase. *E. coli* biotin ligase catalyzes the covalent linkage of biotin to the amino group of the single lysine residue within the peptide (Schatz, Biotechnology 11:1138-43 (1993)). In this manner, fusion proteins to which BAP has been appended can be covalently labeled with biotin, thereby facilitating purification, secondary labeling, and immobilization with (strept)avidin-based reagents. In addition, mammalian cell-based expression systems have been developed to enable the site-specific enzymatic biotinylation of recombinant target proteins bearing the BAP sequence (Mize et al., Protein Expr. Purif. 576:280-89 (2008); Kulman et al., Protein Expr. Purif. 52:320-28 (2007). The resulting recombinant FVIII proteins can be used for visualization or location.

The BAP encoding sequence flanked by a 5' *AscI* restriction site and a 3' *Xho*I restriction site is introduced within the permissive loops or a3 region of the FVIII molecule by standard molecular biology techniques at permissive loop insertion sites or a3 region.

The BAP sequence is inserted into at least one the locations disclosed in TABLES 1B or 3, other suitable insertion sites in at least one of permissive loops A1-1, A1-2, A2-1, A2-2, A3-1 or A3-2, or into the a3 region, or both. Recombinant FVIII proteins with BAP insertions are transfected and transiently expressed into HEK293F cells as described above. Cell culture media from transient transfection are concentrated 10-fold in CENTRICON^{®} spin columns (30 kDa MW cut-off). Concentrated material is then flash frozen with liquid nitrogen and stored at -80°C for future *in vitro* analysis and *in vivo* PK studies. The FVIII activity in culture media samples from cell cultures expressing recombinant FVIII proteins comprising a BAP heterologous moiety is analyzed using a FVIII chromogenic assay. Antigen expression levels are analyzed by FVIII-HC (FVIII heavy chain) and FVIII-LC (FVIII light chain) ELISA. The PK of FVIII variants comprising a BAP heterologous moiety are analyzed in HemA mice and FVIII-VWF DKO mice as described above.

### Example 8.2 - Lipoate Acceptor Peptide (LAP)

The 13 residue Lipoate Acceptor Peptide 2 (LAP2; GFEIDKVWYDLDA) (SEQ ID NO:59) is one of a class of peptidyl substrates identified by yeast peptide display methods that can serve as a substrate *for E. coli* lipoic acid ligase (Puthenveetil et al., J. Am. Chem. Soc. 131:16430-38 (2009)). A variant of LplA in which tryptophan 37 is replaced with alanine (W37ALplA) possesses altered substrate specificity such that it catalyzes the covalent conjugation of fluorescent 7-hydroxycoumarin derivatives, and not lipoic acid, to LAP2 either *in vitro* or in live cells (Uttamapinant et al., Proc. Natl. Acad. Sci. USA 107:10914-19 (2010)).

The LAP2 sequence flanked by a 5' *AscI* restriction site and a 3' *Xho*I restriction site is introduced within the permissive loops and a3 segment of the FVIII molecule by standard molecular biology techniques at sites located in permissive loops, thereby enabling the direct and covalent site-specific fluorescent labeling of the recombinant FVIII protein. The resulting recombinant FVIII protein can be used for visualization.

The LAP sequence is inserted into at least one the locations disclosed in TABLES 1B or 3, other suitable insertion sites in at least one of permissive loops A1-1, A1-2, A2-1, A2-2, A3-1 or A3-2, or into the a3 region, or both. FVIII with LAP insertions are transfected and transiently expressed into HEK293F cells as described above. Cell culture media from transient transfection are concentrated 10-fold in CENTRICON^{®} spin columns (30 kDa MW cut-off). Concentrated material is then flash frozen and stored at -80°C for future *in vitro* analysis and *in vivo* PK studies. The FVIII activity in culture media samples from cell cultures expressing recombinant FVIII proteins comprising a LAP heterologous moiety is analyzed using a FVIII chromogenic assay. Antigen expression levels are analyzed by FVIII-HC (FVIII heavy chain) and FVIII-LC (FVIII light chain) ELISA. The PK of FVIII variants comprising a LAP heterologous moiety are analyzed in HemA mice and FVIII-VWF DKO mice as described above.

### Example 9: Rescue or Enhancement of FVIII Expression by Insertion of an XTEN Sequence within the a3 Acidic Peptide Region of FVIII

Adherent HEK293 cells were transfected as described in Example 5 with FVIII-XTEN DNA constructs in which the coding sequence of a B domain-deleted FVIII contained 2 to 4 XTEN insertions of 144 amino acid residues each. The composition of the constructs and insert locations are indicated in TABLE 7, below. At 5 days post-transfection, cell culture supernatants were assayed for FVIII activity by the chromogenic assay as described in Example 3. Results are shown in TABLE 7.

**TABLE 7. Expression levels of FVIII activity by FVIII variants containing an XTEN at amino acid position 1720 and one, two, or three additional XTEN insertions.**

| Construct Name | Domain, Position, and Type of XTEN Insertion | | | | | Activity (mIU/mL ) |
|---|---|---|---|---|---|---|
| | A1-1 | A2-1 | a3 region | A3-1 | A3-2 | |
| LSD0040.00 2 | 26_AG14 4 | | | 1720_AG14 4 | | 175 |
| LSD0041.008 | | 403_AE14 4 | | 1720_AG14 4 | | 279 |
| LSD0045.002 | | | 1656_AG14 4 | 1720_AG14 4 | | 2598 |
| pSD080.002 | 26_AG14 4 | | 1656_AG14 4 | 1720_AG14 4 | | 1081 |
| pSD083.001 | | 403_AE14 4 | 1656_AG14 4 | 1720_AG14 4 | | 789 |
| pSD082.001 | 26_AG14 4 | | | 1720_AG14 4 | 1900_AE14 4 | <LLOQ |
| pSD090.003 | 26_AG14 4 | | 1656_AG14 4 | 1720_AG14 4 | 1900_AE14 4 | 316 |

For the purpose of comparison, all FVIII-XTEN constructs had an AG144 XTEN insertion at position 1720, numbered relative to mature native FVIII, within the A3 domain. Expression levels were determined by the chromogenic assay and expressed in units of mIU/mL. Constructs with a single additional XTEN insertion at either position 26 in the A1 domain (LSD0040.002) or position 403 in the A2 domain (LSD0041.008) yielded expression levels of 175 and 279 mIU/mL, respectively. In contrast, a construct with a single additional XTEN insertion at position 1656 within the a3 acidic peptide yielded an expression level of 2598 mIU/mL, demonstrating enhancement of expression levels for the a3 XTEN insertion construct relative to the A1 and A2 insertion constructs.

In addition, in comparison to the FVIII-XTEN construct with XTEN insertions at positions 26 in the A1 domain and 1720 in the A3 domain (LSD0040.002), the construct with an additional XTEN insertion at position 1656 within the a3 acidic peptide region (pSD080.002) yielded significantly higher expression (175 and 1081 mIU/mL, respectively). Consistent with these findings, the construct with XTEN insertions at positions 403 in the A2 domain and 1720 in the A3 domain (LSD0041.008) yielded an expression level of 279 mIU/mL, whereas an additional XTEN insertion at position 1656 within the a3 acidic peptide region (PSD083.001) resulted in an increase in the expression level to 789 mIU/mL.

Lastly, the FVIII-XTEN construct with an XTEN insertion at position 26 within the A1 domain and two XTEN insertions at positions 1720 and 1900 within the A3 domain (PSD082.001) did not yield activity above the lower limit of quantitation. However, the FVIII-XTEN construct with an additional XTEN insertion within the a3 acidic peptide region (PSD090.003) resulted in detectable activity, demonstrating that inclusion of an XTEN sequence within the a3 region can result in recovery of expression (as measured by activity) in FVIII-XTEN constructs that are otherwise expressed at levels below the lower limit of quantitation (LLOQ). Under the conditions of the experiment, the results support the conclusion that insertion of XTEN at the 1656 position and, by extension, within the a3 region, results in enhanced expression of procoagulant FVIII-XTEN compositions.

### Example 10: Effect of XTEN Insertion on FVIII Activity Measured by aPTT

A one stage activated partial prothrombin (aPTT) coagulation assay was employed in addition to the chromogenic assay (as described in Example 3) to determine FVIII activity of various FVIII-XTEN fusion proteins.

Method: The FVIII-XTEN aPTT activity was measured using the SYSMEX^{®} CA-1500 instrument (Siemens Healthcare Diagnostics Inc., Tarrytown, NY). To create a standard curve for the assay, WHO factor VIII standard was diluted with 2% mock transfection media to 100 mU/mL and a two-fold serial dilution series was then performed, with the last standard being 0.78 mU/mL. FVIII-XTEN cell culture samples were first diluted at 1:50 with aPTT assay buffer, further dilutions were made with 2% mock transfection media when needed.

After dilution, the aPTT assay was performed using the SYSMEX^{®} instrument as follow: 50 µl of diluted standards and samples were mixed with 50 µl human FVIII deficient plasma and then 50 µl of aPTT reagent. The mixture was incubated at 37°C for 4 min, and following incubation, 50 µl of CaCl₂ was added to the mixture, and the clotting time was measured immediately.

To determine test samples FVIII activity, the clotting times of the standards were plotted using a semi-log scale (Clotting time: Linear; Standard concentration: Log) to extrapolate the equation between clotting time and FVIII activity, and FVIII-XTEN activity was then calculated against the standard curve. The sensitivity of the assay was 40 mU/mL Factor VIII.

Results: The results are summarized in FIGS. 14 to 16. When single XTEN 144 or 288 amino acids long were inserted into FVIII, all of the FVIII-XTEN fusion proteins exhibiting activity in the chromogenic assay were also active in an aPTT assay. The aPTT activity followed the trend observed in the chromogenic assay, for example, those molecules that showed low FVIII activity in the chromogenic assay also had low aPTT values.

Generally, aPTT results for the fusion proteins were lower than those obtained by the chromogenic assay, with a chromogenic to aPTT ratio of 1.1 up to 2.2, as illustrated in FIG. 14, for the single XTEN insertions. The FVIII-XTEN fusion proteins with multiple XTEN insertions generally showed further reductions in aPTT activity in comparison to the activity observed via chromogenic assay. Assays of FVIII-XTEN with two XTEN insertions showed activity with all constructs, but with chromogenic/aPTT ratios approaching 4 in some instances (FIG. 15). Assays of FVIII-XTEN with three XTEN insertions also showed activity in both assays, with chromogenic/aPTT ratios approaching 5 in some instances (FIG. 16), while the ratios for the BDD FVIII control were more comparable (right side of FIG 16).

Additionally, the site of XTEN insertion appeared to contribute to the differences seen between aPTT and chromogenic activities. For example, while some molecules with 2 XTEN insertions resulted in up to 4-fold lower aPTT activity than chromogenic values, the aPTT activity values for other FVIII molecules with 2 XTEN insertions were fairly comparable to chromogenic activity (FIG. 15). Some molecules with 3 XTEN insertions showed aPTT activities up to 5 -fold lower than chromogenic activities, whereas other FVIII molecules with 3 XTEN had aPTT activities that were less than 2-fold lower than their corresponding chromogenic activities (FIG. 15).

Under the conditions of the experiment, the results support the conclusion that FVIII-XTEN fusion protein constructs do retain procoagulant activity, but that the chromogenic assay generally provides higher activity levels than those observed in the aPTT assay system employed in the study.

### Example 11: Evaluations of the Effect of XTEN Insertion Site on FVIII Half-life Extension

Methods: Six FVIII-XTEN fusion proteins with single XTEN AG-144 insertions at defined locations were tested in FVIII/VWF DKO mice (as generally described in Example 4) to evaluate the effect of XTEN insertion site on FVIII half-life. Six representative FVIII variants (pSD-0050, pSD-0003, pSD-0039, pSD-0010, and pSD-0063 listed in TABLE 4; and pSD-0014, comprising a single AG-144 insertion at position 2332, *i.e.,* the carboxy-terminal) with XTEN insertion in either within A1-1, A2-1, a3, A3-1, A3-2, or at the C-terminus were selected for this study, and BDD FVIII generated from the base vector was used as the control.

FVIII/VWF DKO mice were treated with a single intravenous administration of transient transfection cell culture media concentrate from the six FVIII-XTEN constructs (or positive control media) at 100-200 IU/kg, and plasma samples were subsequently collected at 5 minutes, 7 hours and 16 hours post-dosing. Plasma FVIII activity was tested using the FVIII chromogenic assay and FVIII-XTEN half-life was estimated using the WINNONLIN^{®} program. The study data are summarized in TABLE 8 and FIG 17.

Results: A significantly longer half-life was observed for all FVIII-XTEN variants tested compared to BDD-FVIII control, but the degree of the half-life increase varied, with the variant with XTEN at the 403 insertion site conferring the least half-life extension at 10-fold (in comparison to control), while the 1900 insertion variant conferred the most half-life extension at 18-fold. The differences of XTEN insertion site on FVIII half-life extension may reflect the roles of different FVIII domains in FVIII clearance *in vivo.*

**TABLE 8: FVIII-XTEN single AG-144 insertion variants PK in FVIII/VWF DKO mice**

| Treatment | BDD-FVIII | pSD-0050 | pSD-0003 | pSD-0039 | pSD-0010 | pSD-0063 | pSD-0014 |
|---|---|---|---|---|---|---|---|
| Insertion site | None | 26 | 403 | 1656 | 1720 | 1900 | CT |
| Recovery | 21.3 | 33.8 | 34.8 | 36.0 | 33.6 | 39.6 | 32.4 |
| t_{1/2} (hr) | 0.25 | 3.15 | 2.4 | 3.3 | 4.28 | 4.54 | 3.91 |
| t_{1/2} Increase (fold) | | 13 | 10 | 13 | 17 | 18 | 16 |

### Example 12: Evaluations of the Additive Effect of XTEN Insertions on FVIII Half-life Extension

Methods: To evaluate the effects of multiple XTEN insertions on the half-lives of FVIII-XTEN fusion protein, the half-lives of FVIII-XTEN variants with 1 to 3 XTEN insertions were determined in FVIII-XTEN DKO mice using the cell culture concentrate from five constructs (as generally described in Example 4). Five FVIII-XTEN variants were tested in the study: pSD-0062, with AE144 insertion at position 1900 (numbered relative to full-length factor VIII); pSD-0005 with AE144 in the FVIII B domain (B domain amino acid position 745); pSD-0019 with AE288 at the FVIII C-terminus (CT); LSD-0003.006 with AE144 inserted in the B domain and AE288 inserted at the C-terminus, and LSD-0055.021 with three XTEN of AE144, AE144, and AE288 inserted at position 1900, with the B domain and at the C-terminus. The FVIII-XTEN half-life values were estimated using the WINNONLIN^{®} program.

Results: The study results are summarized in TABLE 9, and the PK curves are shown in FIG. 18. The study results demonstrated the additive effect of multiple XTEN insertions on FVIII half-life extension. With single XTEN insertions, the half-life of FVIII was extended from 0.25 hours to 3.2-4.0 hours, *i.e.,* a 13 to16-fold increase. When the B and CT XTEN insertions were combined together, the FVIII half-life was further extended to 10.6 hours, *i.e.,* a 42-fold prolongation. Finally, in the case of a third XTEN insertion added at position 1900 to the B/CT construct, the half-life reached 16 hours in the FVIII-VWF DKO mice, *i.e.,* a 64-fold increase.

**TABLE 9: Effect of XTEN insertions on FVIII t_{1/2} in FVIII/VWF DKO mice**

| Treatment | BDD-FVIII | pSD -062 | pSD-0005 | pSD-0019 | LSD-0003.006 | LSD-0055.021 |
|---|---|---|---|---|---|---|
| XTEN Insertion site | None | 1900 | B | CT | B/CT | 1900/B/CT |
| Recovery | 21.3 | 35.3 | 44.9 | 33.3 | 39.0 | 37.2 |
| t_{1/2} (hr) | 0.25 | 3.8 | 3.2 | 4.0 | 10.6 | 16.0 |
| t_{1/2} Increase (fold) | | 15 | 13 | 16 | 42 | 64 |

### Example 13: Additional FVIII variants containing XTEN Insertions

The data presented in TABLES 10 to 18 corresponds to additional FVIII variant expression constructs which contained from one to six XTEN insertions. The methods used to generate the constructs, the method to determine expression levels using ELISA, and the method to determine procoagulant activity using the chromogenic assay are described above in detail.

The results presented in TABLE 10 were obtained using FVIII variants with XTEN inserted in single sites selected on the basis of criteria described herein. The pBC00114 FVIII positive control showed good expression and FVIII activity.

**TABLE 10: Results of Coagulation Activity Assays for FVIII Variants Comprising One XTEN Insertion**

| **Insertion Site** | **Domain** | **Construct** | **Activity*** | **Expression ELISA** |
|---|---|---|---|---|
| pBC0114 | | | +++ | +++ |
| 3 | A1 | pBC0126 | LLOQ^{∗} | LLOQ |
| 3 | A1 | pBC0127 | + | + |
| 18 | A1 | pBC0165 | ++ | ++ |
| 22 | A1 | pBC0183 | +++ | ++ |
| 26 | A1 | pBC0184 | ++ | ++ |
| 40 | A1 | pBC0166 | ++ | ++ |
| 60 | A1 | pBC0185 | LLOQ | LLOQ |
| 116 | A1 | pBC0167 | LLOQ | LLOQ |
| 130 | A1 | pBC0128 | LLOQ | LLOQ |
| 188 | A1 | pBC0168 | ++ | ++ |
| 216 | A1 | pBC0129 | ++ | ++ |
| 230 | A1 | pBC0169 | LLOQ | LLOQ |
| 333 | A1 | pBC0130 | ++ | ++ |
| 375 | A2 | pBC0131 | LLOQ | +++ |
| 403 | A2 | pBC0132 | ++ | ++ |
| 442 | A2 | pBC0170 | ++ | ++ |
| 490 | A2 | pBC0133 | + | ++ |
| 518 | A2 | pBC0171 | LLOQ | + |
| 599 | A2 | pBC0134 | ++ | ++ |
| 713 | A2 | pBC0172 | + | +++ |
| 1720 | A3 | pBC0138 | +++ | +++ |
| 1796 | A3 | pBC0139 | + | ++ |
| 1802 | A3 | pBC0140 | + | ++ |
| 1827 | A3 | pBC0173 | LLOQ | LLOQ |
| 1861 | A3 | pBC0174 | LLOQ | LLOQ |
| 1896 | A3 | pBC0175 | LLOQ | LLOQ |
| 1900 | A3 | pBC0176 | +++ | +++ |
| 1904 | A3 | pBC0177 | + | + |
| 1937 | A3 | pBC0178 | LLOQ | LLOQ |
| 2019 | A3 | pBC0141 | LLOQ | + |
| 403 | A2 | pSD0001 | +++ | +++ |
| 599 | A2 | pSD0002 | + | + |
| 403 | A2 | pSD0003 | +++ | +++ |
| 599 | A2 | pSD0004 | + | + |
| 1720 | A3 | pSD0009 | + | + |
| 1720 | A3 | pSD0010 | ++ | ++ |
| 65 | A1 | pSD0023 | LLOQ | LLOQ |
| 81 | A1 | pSD0024 | LLOQ | LLOQ |
| 119 | A1 | pSD0025 | LLOQ | LLOQ |
| 211 | A1 | pSD0026 | + | + |
| 220 | A1 | pSD0027 | + | + |
| 224 | A1 | pSD0028 | + | + |
| 336 | A1 | pSD0029 | ++ | +++ |
| 339 | A1 | pSD0030 | ++ | +++ |
| 378 | A2 | pSD0031 | LLOQ | ++ |
| 399 | A2 | pSD0032 | ++ | ++ |
| 409 | A2 | pSD0033 | ++ | ++ |
| 416 | A2 | pSD0034 | + | + |
| 487 | A2 | pSD0035 | LLOQ | + |
| 494 | A2 | pSD0036 | LLOQ | + |
| 500 | A2 | pSD0037 | LLOQ | + |
| 603 | A2 | pSD0038 | + | + |
| 1656 | a3 region | pSD0039 | +++ | +++ |
| 1656 | a3 region | pNL009^{∗∗} | ++++ | ND |
| 1711 | A3 | pSD0040 | ++ | + |
| 1725 | A3 | pSD0041 | LLOQ | ++ |
| 1749 | A3 | pSD0042 | LLOQ | LLOQ |
| 1905 | A3 | pSD0043 | ++ | ++ |
| 1910 | A3 | pSD0044 | + | + |
| 1900 | A3 | pSD0062 | ++ | ++ |
| 1900 | A3 | pSD0063 | +++ | ++ |
| 18 | A1 | pSD0045 | +++ | +++ |
| 18 | A1 | pSD0046 | +++ | +++ |
| 22 | A1 | pSD0047 | LLOQ | LLOQ |
| 22 | A1 | pSD0048 | LLOQ | LLOQ |
| 26 | A1 | pSD0049 | +++ | +++ |
| 26 | A1 | pSD0050 | +++ | +++ |
| 40 | A1 | pSD0051 | +++ | +++ |
| 40 | A1 | pSD0052 | +++ | +++ |
| 216 | A1 | pSD0053 | LLOQ | LLOQ |
| 216 | A1 | pSD0054 | LLOQ | LLOQ |
| 375 | A2 | pSD0055 | LLOQ | + |
| 442 | A2 | pSD0056 | LLOQ | LLOQ |
| 442 | A2 | pSD0057 | LLOQ | LLOQ |
| 1796 | A3 | pSD0058 | LLOQ | LLOQ |
| 1796 | A3 | pSD0059 | + | + |
| 1802 | A3 | pSD0060 | + | + |
| 1802 | A3 | pSD0061 | LLOQ | LLOQ |

| | | | | |
|---|---|---|---|---|
| ^{∗}LLOQ: below the limits of quantitation ^{∗∗} pNL009 includes a deletion of 745-1656 | | | | |

The results of the single insertion site data guided the creation of FVIII-XTEN variant constructs with 2 XTEN insertions, the results of which are presented in TABLE 11.

**TABLE 11: Results of Coagulation Activity Assays for FVIII Variants Comprising Two XTEN Insertions**

| Insertion 1 | | Insertion 2 | | | |
|---|---|---|---|---|---|
| Insertion Site | Domain | Insertion Site | Domain | Construct | Activity |
| 26 | A1 | 403 | A2 | LSD0005.002 | ++ |
| 26 | A1 | 403 | A2 | LSD0005.004 | ++ |
| 40 | A1 | 403 | A2 | LSD0005.005 | ++ |
| 40 | A1 | 403 | A2 | LSD0005.011 | ++ |
| 18 | A1 | 403 | A2 | LSD0005.018 | ++ |
| 26 | A1 | 599 | A2 | LSD0006.002 | + |
| 40 | A1 | 599 | A2 | LSD0006.005 | ++ |
| 40 | A1 | 599 | A2 | LSD0006.007 | ++ |
| 40 | A1 | 599 | A2 | LSD0006.011 | +++ |
| 40 | A1 | 403 | A2 | LSD0007.002 | + |
| 40 | A1 | 403 | A2 | LSD0007.004 | + |
| 26 | A1 | 403 | A2 | LSD0007.013 | ++ |
| 26 | A1 | 599 | A2 | LSD0008.001 | ++ |
| 40 | A1 | 599 | A2 | LSD0008.002 | ++ |
| 26 | A1 | 599 | A2 | LSD0008.006 | + |
| 18 | A1 | 599 | A2 | LSD0008.009 | ++ |
| 40 | A1 | 599 | A2 | LSD0008.017 | + |
| 26 | A1 | 403 | A2 | LSD0007.008 | ++ |
| 1720 | A3 | 1900 | A3 | LSD0044.002 | LLOQ |
| 1725 | A3 | 1900 | A3 | LSD0044.005 | LLOQ |
| 1720 | A3 | 1900 | A3 | LSD0044.039 | LLOQ |
| 1711 | A3 | 1905 | A3 | LSD0044.022 | LLOQ |
| 1720 | A3 | 1905 | A3 | LSD0044.003 | LLOQ |
| 1725 | A3 | 1905 | A3 | LSD0044.001 | LLOQ |
| 1656 | a3 region | 26 | A1 | LSD0038.001 | ++ |
| 1656 | a3 region | 18 | A1 | LSD0038.003 | ++ |
| 1656 | a3 region | 18 | A1 | LSD0038.008 | +++ |
| 1656 | a3 region | 40 | A1 | LSD0038.012 | ++ |
| 1656 | a3 region | 40 | A1 | LSD0038.013 | ++ |
| 1656 | a3 region | 26 | A1 | LSD0038.015 | ++ |
| 1656 | a3 region | 399 | A2 | LSD0039.001 | + |
| 1656 | a3 region | 403 | A2 | LSD0039.003 | ++ |
| 1656 | a3 region | 403 | A2 | LSD0039.010 | ++ |
| 1656 | a3 region | 1725 | A3 | LSD0045.001 | + |
| 1656 | a3 region | 1720 | A3 | LSD0045.002 | ++ |
| 1900 | A3 | 18 | A1 | LSD0042.014 | + |
| 1900 | A3 | 18 | A1 | LSD0042.023 | + |
| 1900 | A3 | 26 | A1 | LSD0042.006 | + |
| 1900 | A3 | 26 | A1 | LSD0042.013 | ++ |
| 1900 | A3 | 40 | A1 | LSD0042.001 | + |
| 1900 | A3 | 40 | A1 | LSD0042.039 | + |
| 1900 | A3 | 26 | A1 | LSD0042.047 | + |
| 1905 | A3 | 18 | A1 | LSD0042.003 | + |
| 1905 | A3 | 40 | A1 | LSD0042.004 | LLOQ |
| 1905 | A3 | 26 | A1 | LSD0042.008 | LLOQ |
| 1905 | A3 | 26 | A1 | LSD0042.038 | LLOQ |
| 1905 | A3 | 40 | A1 | LSD0042.082 | LLOQ |
| 1910 | A3 | 26 | A1 | LSD0042.040 | LLOQ |
| 18 | A1 | 399 | A2 | LSD0037.002 | ++ |
| 26 | A1 | 399 | A2 | LSD0037.009 | + |
| 40 | A1 | 399 | A2 | LSD0037.011 | ++ |
| 18 | A1 | 403 | A2 | LSD0047.002 | ++ |
| 18 | A1 | 403 | A2 | LSD0047.005 | + |
| 18 | A1 | 403 | A2 | LSD0048.007 | + |
| 1656 | a3 region | 1900 | A3 | LSD0046.001 | ++ |
| 1656 | a3 region | 1900 | A3 | LSD0046.002 | + |
| 1656 | a3 region | 1905 | A3 | LSD0046.003 | + |
| 1711 | A3 | 40 | A1 | LSD0040.011 | LLOQ |
| 1711 | A3 | 26 | A1 | LSD0040.042 | LLOQ |
| 1720 | A3 | 26 | A1 | LSD0040.002 | + |
| 1720 | A3 | 40 | A1 | LSD0040.008 | + |
| 1720 | A3 | 18 | A1 | LSD0040.021 | + |
| 1720 | A3 | 26 | A1 | LSD0040.037 | LLOQ |
| 1720 | A3 | 18 | A1 | LSD0040.046 | + |
| 1725 | A3 | 26 | A1 | LSD0040.003 | LLOQ |
| 1725 | A3 | 40 | A1 | LSD0040.006 | LLOQ |
| 1725 | A3 | 26 | A1 | LSD0040.007 | LLOQ |
| 1725 | A3 | 18 | A1 | LSD0040.010 | LLOQ |
| 1725 | A3 | 40 | A1 | LSD0040.039 | LLOQ |
| 1725 | A3 | 18 | A1 | LSD0040.052 | + |
| 1720 | A3 | 403 | A2 | LSD0041.001 | + |
| 1720 | A3 | 399 | A2 | LSD0041.004 | LLOQ |
| 1711 | A3 | 403 | A2 | LSD0041.006 | LLOQ |
| 1720 | A3 | 403 | A2 | LSD0041.008 | LLOQ |
| 1725 | A3 | 403 | A2 | LSD0041.010 | LLOQ |
| 1725 | A3 | 403 | A2 | LSD0041.014 | LLOQ |
| 1725 | A3 | 399 | A2 | LSD0041.016 | LLOQ |
| 1711 | A3 | 403 | A2 | LSD0041.035 | LLOQ |
| 1900 | A3 | 399 | A2 | LSD0043.001 | LLOQ |
| 1900 | A3 | 403 | A2 | LSD0043.002 | LLOQ |
| 1905 | A3 | 403 | A2 | LSD0043.005 | LLOQ |
| 1900 | A3 | 399 | A2 | LSD0043.006 | LLOQ |
| 1900 | A3 | 403 | A2 | LSD0043.007 | LLOQ |
| 1900 | A3 | 403 | A2 | LSD0043.008 | LLOQ |
| 1905 | A3 | 399 | A2 | LSD0043.015 | LLOQ |
| 1905 | A3 | 403 | A2 | LSD0043.029 | LLOQ |
| 1910 | A3 | 403 | A2 | LSD0043.043 | LLOQ |

The results of the foregoing data guided the creation of FVIII-XTEN variant constructs with 3 XTEN insertions, the results of which are presented in TABLE 12. Additional FVIII variants comprising 3 XTEN insertions are shown in TABLE 13.

**TABLE 12: Results of Coagulation Activity Assays for FVIII Variants Comprising Three XTEN Insertions**

| Insertion 1 | | Insertion 2 | | Insertion 3 | | | |
|---|---|---|---|---|---|---|---|
| Insertion Site | Domain | Insertion Site | Domain | Insertion Site | Domain | Construct | Activity |
| 26 | A1 | 403 | A2 | 1656 | a3 region | pSD0077 | +++ |
| 26 | A1 | 403 | A2 | 1720 | A3 | pSD0078 | ++ |
| 26 | A1 | 403 | A2 | 1900 | A3 | pSD0079 | ++ |
| 26 | A1 | 1656 | a3 region | 1720 | A3 | pSD0080 | +++ |
| 26 | A1 | 1656 | a3 region | 1900 | A3 | pSD0081 | LLOQ |
| 26 | A1 | 1720 | A3 | 1900 | A3 | pSD0082 | + |
| 403 | A2 | 1656 | a3 region | 1720 | A3 | pSD0083 | +++ |
| 403 | A2 | 1656 | a3 region | 1900 | A3 | pSD0084 | +++ |
| 403 | A2 | 1720 | A3 | 1900 | A3 | pSD0085 | + |
| 1656 | a3 region | 1720 | A3 | 1900 | A3 | pSD0086 | +++ |
| 18 | A1 | 745 | B | 2332 | CT | LSD0049.002 | +++ |
| 26 | A1 | 745 | B | 2332 | CT | LSD0049.008 | +++ |
| 26 | A1 | 745 | B | 2332 | CT | LSD0049.011 | +++ |
| 40 | A1 | 745 | B | 2332 | CT | LSD0049.012 | +++ |
| 40 | A1 | 745 | B | 2332 | CT | LSD0049.020 | +++ |
| 18 | A1 | 745 | B | 2332 | CT | LSD0049.021 | +++ |
| 40 | A1 | 745 | B | 2332 | CT | LSD0050.002 | +++ |
| 18 | A1 | 745 | B | 2332 | CT | LSD0050.003 | +++ |
| 26 | A1 | 745 | B | 2332 | CT | LSD0050.007 | LLOQ |
| 18 | A1 | 745 | B | 2332 | CT | LSD0050.010 | +++ |
| 26 | A1 | 745 | B | 2332 | CT | LSD0050.012 | +++ |
| 40 | A1 | 745 | B | 2332 | CT | LSD0050.014 | +++ |
| 403 | A2 | 745 | B | 2332 | CT | LSD0051.002 | +++ |
| 399 | A2 | 745 | B | 2332 | CT | LSD0051.003 | +++ |
| 403 | A2 | 745 | B | 2332 | CT | LSD0052.001 | +++ |
| 399 | A2 | 745 | B | 2332 | CT | LSD0052.003 | +++ |
| 1725 | A3 | 745 | B | 2332 | CT | LSD0053.021 | LLOQ |
| 1720 | A3 | 745 | B | 2332 | CT | LSD0053.022 | +++ |
| 1711 | A3 | 745 | B | 2332 | CT | LSD0053.024 | +++ |
| 1720 | A3 | 745 | B | 2332 | CT | LSD0054.021 | +++ |
| 1711 | A3 | 745 | B | 2332 | CT | LSD0054.025 | ++ |
| 1725 | A3 | 745 | B | 2332 | CT | LSD0054.026 | +++ |
| 1900 | A3 | 745 | B | 2332 | CT | LSD0055.021 | +++ |
| 1905 | A3 | 745 | B | 2332 | CT | LSD0055.022 | +++ |
| 1900 | A3 | 745 | B | 2332 | CT | LSD0055.026 | +++ |
| 1900 | A3 | 745 | B | 2332 | CT | LSD0056.021 | +++ |
| 1900 | A3 | 745 | B | 2332 | CT | LSD0056.024 | +++ |
| 1910 | A3 | 745 | B | 2332 | CT | LSD0056.025 | +++ |

**TABLE 13: FVIII Variants Comprising Three XTEN Insertions**

| XTEN insertion 1 | XTEN insertion2 | XTEN insertion3 | Additional mutations | Construct ID | |
|---|---|---|---|---|---|
| 0745 | 1900 | 2332 | R1648A | pBC0294 | |
| 0745 | 1900 | 2332 | R1648A | pBC0295 | |
| 0745 | 1900 | 2332 | R1648A | pBC0296 | |
| 0745 | 1900 | 2332 | R1648A | pBC0297 | |
| 0745 | 1900 | 2332 | R1648A | pBC0298 | |
| 0745 | 1900 | 2332 | R1648A | pBC0299 | |
| 0745 | 1900 | 2332 | R1648A | pBC0300 | |
| 0745 | 1900 | 2332 | R1648A | pBC0301 | |
| 0745 | 1900 | 2332 | R1648A | pBC0302 | |
| 0745 | 1900 | 2332 | R1648A | pBC0303 | |
| 0745 | 1900 | 2332 | R1648A | pBC0304 | |
| 0745 | 1900 | 2332 | R1648A | pBC0305 | |
| 0745 | 1900 | 2332 | R1648A | pBC0306 | |
| 0745 | 1900 | 2332 | R1648A | pBC0307 | |
| 0745 | 1900 | 2332 | R1648A | pBC0308 | |
| 0745 | 1900 | 2332 | R1648A | pBC0309 | |
| 0745 | 1900 | 2332 | R1648A | pBC0310 | |
| 0745 | 1900 | 2332 | R1648A | pBC0311 | |
| 0745 | 1900 | 2332 | R1648A | pBC0312 | |
| 0745 | 1900 | 2332 | R1648A | pBC0313 | |
| 0745 | 1900 | 2332 | R1648A | pBC0314 | |
| 0745 | 1900 | 2332 | R1648A | pBC0315 | |
| 0745 | 1900 | 2332 | R1648A | pBC0316 | |
| 0745 | 1900 | 2332 | R1648A | pBC0317 | |
| 0745 | 1900 | 2332 | R1648A | pBC0318 | |
| 0745 | 1900 | 2332 | R1648A | pBC0319 | |
| 0745 | 1900 | 2332 | R1648A | pBC0320 | |
| 0018 | 0745 | 2332 | R1648A | pBC0269 | |
| 0403 | 0745 | 2332 | R1648A | pBC0270 | |
| 1720 | 0745 | 2332 | R1648A | pBC0271 | |
| 1900 | 0745 | 2332 | R1648A | pBC0272 | |
| 0403 | 0745 | 2332 | R1648A | pBC0273 | |
| 1720 | 0745 | 2332 | R1648A | pBC0274 | |
| 1900 | 0745 | 2332 | R1648A | pBC0275 | |
| 0018 | 0745 | 2332 | R1648A | pBC0276 | |
| 0403 | 0745 | 2332 | R1648A | pBC0277 | |
| 1720 | 0745 | 2332 | R1648A | pBC0278 | |
| 1900 | 0745 | 2332 | R1648A | pBC0279 | |

A number of constructs with 4 XTEN insertions were created and assayed, with most of the molecules exhibiting FVIII activity (TABLE 14 and TABLE 15), suggesting that FVIII with insertion of multiple XTEN can still retain FVIII activity.

**TABLE 14: Results of Coagulation Activity Assays for FVIII Variants Comprising Four XTEN Insertions**

| Insertion 1 | | Insertion 2 | | Insertion 3 | | Insertion 4 | | | |
|---|---|---|---|---|---|---|---|---|---|
| Insertion Site | Domain | Insertion Site | Domain | Inserti on Site | Domain | Insertion Site | Domain | Construct | Activit y |
| 26 | A1 | 403 | A2 | 1656 | a3 region | 1720 | A3 | pSD0087 | +++ |
| 26 | A1 | 403 | A2 | 1656 | a3 region | 1900 | A3 | pSD0088 | +++ |
| 26 | A1 | 403 | A2 | 1720 | A3 | 1900 | A3 | pSD0089 | LLO Q |
| 26 | A1 | 1656 | a3 region | 1720 | A3 | 1900 | A3 | pSD0090 | ++ |
| 403 | A2 | 1656 | a3 region | 1720 | A3 | 1900 | A3 | pSD0091 | ++ |

**TABLE 15: Results of Coagulation Activity Assays for Additional FVIII Variants Comprising Four XTEN insertions**

| XTEN insertion 1 | XTEN insertion2 | XTEN insertion3 | XTEN insertion4 | Additional mutations | Construct ID | Activity |
|---|---|---|---|---|---|---|
| 0040 | 0403 | 745 | 2332 | R1648A | LSD0057.001 | ++ |
| 0040 | 0403 | 745 | 2332 | R1648A | LSD0058.006 | ++ |
| 0018 | 0409 | 745 | 2332 | R1648A | LSD0059.002 | + |
| 0040 | 0409 | 745 | 2332 | R1648A | LSD0059.006 | + |
| 0040 | 0409 | 745 | 2332 | R1648A | LSD0060.001 | + |
| 0018 | 0409 | 745 | 2332 | R1648A | LSD0060.003 | + |
| 0040 | 1720 | 745 | 2332 | R1648A | LSD0061.002 | + |
| 0026 | 1720 | 745 | 2332 | R1648A | LSD0061.007 | ++ |
| 0018 | 1720 | 745 | 2332 | R1648A | LSD0061.008 | ++ |
| 0018 | 1720 | 745 | 2332 | R1648A | LSD0061.012 | ++ |
| 0018 | 1720 | 745 | 2332 | R1648A | LSD0062.001 | ++ |
| 0026 | 1720 | 745 | 2332 | R1648A | LSD0062.002 | ++ |
| 0018 | 1720 | 745 | 2332 | R1648A | LSD0062.006 | ++ |
| 0018 | 1900 | 745 | 2332 | R1648A | LSD0063.001 | ++ |
| 0018 | 1900 | 745 | 2332 | R1648A | LSD0064.017 | ++ |
| 0026 | 1900 | 745 | 2332 | R1648A | LSD0064.020 | ++ |
| 0040 | 1900 | 745 | 2332 | R1648A | LSD0064.021 | ++ |
| 0040 | 1905 | 745 | 2332 | R1648A | LSD0065.001 | + |
| 0018 | 1905 | 745 | 2332 | R1648A | LSD0065.014 | + |
| 0040 | 1905 | 745 | 2332 | R1648A | LSD0066.001 | + |
| 0026 | 1905 | 745 | 2332 | R1648A | LSD0066.002 | + |
| 0018 | 1905 | 745 | 2332 | R1648A | LSD0066.009 | ++ |
| 0018 | 1905 | 745 | 2332 | R1648A | LSD0066.011 | ++ |
| 0018 | 1910 | 745 | 2332 | R1648A | LSD0067.004 | ++ |
| 0018 | 1910 | 745 | 2332 | R1648A | LSD0067.005 | + |
| 0040 | 1910 | 745 | 2332 | R1648A | LSD0067.006 | + |
| 0026 | 1910 | 745 | 2332 | R1648A | LSD0067.008 | + |
| 0018 | 1910 | 745 | 2332 | R1648A | LSD0068.001 | + |
| 0026 | 1910 | 745 | 2332 | R1648A | LSD0068.002 | + |
| 0040 | 1910 | 745 | 2332 | R1648A | LSD0068.005 | + |
| 0018 | 1910 | 745 | 2332 | R1648A | LSD0068.010 | ++ |
| 0409 | 1720 | 745 | 2332 | R1648A | LSD0069.004 | + |
| 0403 | 1720 | 745 | 2332 | R1648A | LSD0069.008 | + |
| 0409 | 1720 | 745 | 2332 | R1648A | LSD0070.003 | + |
| 0403 | 1720 | 745 | 2332 | R1648A | LSD0070.004 | ++ |
| 0403 | 1720 | 745 | 2332 | R1648A | LSD0070.005 | ++ |
| 0403 | 1900 | 745 | 2332 | R1648A | LSD0071.001 | ++ |
| 0403 | 1900 | 745 | 2332 | R1648A | LSD0071.002 | + |
| 0409 | 1900 | 745 | 2332 | R1648A | LSD0071.008 | ++ |
| 0403 | 1900 | 745 | 2332 | R1648A | LSD0072.001 | ++ |
| 0403 | 1900 | 745 | 2332 | R1648A | LSD0072.002 | + |
| 0409 | 1900 | 745 | 2332 | R1648A | LSD0072.003 | + |
| 0409 | 1905 | 745 | 2332 | R1648A | LSD0073.002 | + |
| 0403 | 1905 | 745 | 2332 | R1648A | LSD0073.004 | + |
| 0403 | 1905 | 745 | 2332 | R1648A | LSD0073.006 | + |
| 0403 | 1905 | 745 | 2332 | R1648A | LSD0074.007 | ++ |
| 0409 | 1905 | 745 | 2332 | R1648A | LSD0074.010 | + |
| 0403 | 1905 | 745 | 2332 | R1648A | LSD0074.011 | + |
| 0409 | 1910 | 745 | 2332 | R1648A | LSD0075.004 | + |
| 0403 | 1910 | 745 | 2332 | R1648A | LSD0075.007 | + |
| 0403 | 1910 | 745 | 2332 | R1648A | LSD0076.002 | + |
| 0403 | 1910 | 745 | 2332 | R1648A | LSD0076.003 | + |
| 0403 | 1910 | 745 | 2332 | R1648A | pSD0093 | + |
| 1720 | 1900 | 745 | 2332 | R1648A | pSD0094 | ++ |
| 1720 | 1905 | 745 | 2332 | R1648A | pSD0095 | + |
| 1720 | 1910 | 745 | 2332 | R1648A | pSD0097 | + |
| 1720 | 1910 | 745 | 2332 | R1648A | pSD0098 | + |
| 0403 | 1656 | 1720 | 2332 | | pNL0022 | + |
| 0403 | 1656 | 1900 | 2332 | | pNL0023 | + |
| 0403 | 1720 | 1900 | 2332 | | pNL0024 | LLOQ |
| 1656 | 1720 | 1900 | 2332 | | pNL0025 | + |
| 0018 | 0403 | 1656 | 2332 | | pBC0247 | ++ |
| 0018 | 0403 | 1720 | 2332 | | pBC0248 | + |
| 0018 | 0403 | 1900 | 2332 | | pBC0249 | + |
| 0018 | 1656 | 1720 | 2332 | | pBC0250 | + |
| 0018 | 1656 | 1900 | 2332 | | pBC0251 | ++ |
| 0018 | 1720 | 1900 | 2332 | | pBC0252 | LLOQ |
| 0018 | 0403 | 0745 | 2332 | | LSD57.005 | ++ |
| 0018 | 0745 | 1720 | 2332 | | LSD62.001 | ++ |
| 0018 | 0745 | 1900 | 2332 | | pBC0262 | ++ |
| 0403 | 0745 | 1720 | 2332 | | LSD70.004 | + |
| 0403 | 0745 | 1900 | 2332 | | pBC0266 | + |
| 0745 | 1720 | 1900 | 2332 | | pBC0268 | + |
| 0188 | 1900 | 0745 | 2332 | R1648A | pCS0001 | ND |
| 0599 | 1900 | 0745 | 2332 | R1648A | pCS0002 | ND |
| 2068 | 1900 | 0745 | 2332 | R1648A | pCS0003 | ND |
| 2171 | 1900 | 0745 | 2332 | R1648A | pCS0004 | ND |
| 2227 | 1900 | 0745 | 2332 | R1648A | pCS0005 | ND |
| 2277 | 1900 | 0745 | 2332 | R1648A | pCS0006 | ND |

A limited number of FVIII variant constructs with 4 XTEN inserted in the A1, A2, B, A3 domains and C-terminus were created and assayed, with 6 out of 9 molecules exhibiting FVIII activity (TABLE 16). At the meantime, 2 FVIII variants with 6 XTEN insertions each were also created and they did not exhibit FVIII activity in this chromogenic assay (TABLE 17), suggesting that number and site of XTEN insertions are important to retain FVIII activity.

**TABLE 16: Results of Coagulation Activity Assays for FVIII Variants Comprising Five XTEN Insertions**

| XTEN Insertion 1 | XTEN insertion 2 | XTEN Insertion 3 | XTEN Insertion 4 | XTEN Insertion 5 | Construct ID | Activity |
|---|---|---|---|---|---|---|
| 0403 | 1656 | 1720 | 1900 | 2332 | pNL0030 | LLOQ |
| 0018 | 0403 | 1656 | 1720 | 2332 | pBC0253 | + |
| 0018 | 0403 | 1656 | 1900 | 2332 | pBC0254 | + |
| 0018 | 0403 | 1720 | 1900 | 2332 | pBC0255 | LLOQ |
| 0018 | 1656 | 1720 | 1900 | 2332 | pBC0256 | + |
| 0018 | 0403 | 0745 | 1720 | 2332 | pBC0259 | + |
| 0018 | 0403 | 0745 | 1900 | 2332 | pBC0260 | + |
| 0018 | 0745 | 1720 | 1900 | 2332 | pBC0263 | + |
| 0403 | 0745 | 1720 | 1900 | 2332 | pBC0267 | LLOQ |

**TABLE 17: Results of Coagulation Activity Assays for FVIII Variants Comprising Six XTEN Insertions**

| XTEN Insertion 1 | XTEN insertion 2 | XTEN Insertion 3 | XTEN Insertion 4 | XTEN Insertion 5 | XTEN Insertion 6 | Construct ID | Activity |
|---|---|---|---|---|---|---|---|
| 0018 | 0403 | 1656 | 1720 | 1900 | 2332 | pBC0257 | LLOQ |
| 0018 | 0403 | 0745 | 1720 | 1900 | 2332 | pBC0264 | LLOQ |

The results presented supported the notion that, under the conditions of the experiments, the criteria used to select XTEN insertion sites were valid, that the insertion of one or more XTEN into the selected sites of FVIII more likely than not resulted in retention of procoagulant activity of the resulting XTEN molecule, and that insertion of three XTENs appeared to result in a greater proportion of fusion proteins retaining high levels of FVIII procoagulant activity compared to single or double XTEN insertion constructs.

### Example 14: Insertion of CTP1 at Representative Sites within Permissive Loops

To demonstrate that FVIII can tolerate insertion of peptides of variable length and composition within individual structural domains without loss of cofactor function, a 45 amino acid long peptide encompassing a 29 amino acid long peptide derived from the carboxy terminus of human chorionic gonadotropin (CTP1, SEQ ID NO:81) was inserted by standard recombinant DNA techniques. The CTP-1 DNA sequence (SEQ ID NO:82) encodes a polypeptide comprising the human chorionic gonadotropin-derived peptide (SEQ ID NO:62) flanked by the amino acid sequence Gly-Gly-Gly-Gly-Ser (SEQ ID NO:191), terminally flanked by a 5' *Asc*I restriction site (ggcgcgcc) and a 3' *Xho*I site (ctcgag), neither of which is present in the sequence of the base vector pBC0114.

The CTP-1 DNA sequence was chemically synthesized, digested with *Asc*I and *Xho*I*,* and inserted into an appropriate FVIII expression plasmid into which the unique *Asc*I and *Xho*I sites had been inserted immediately downstream of the designated insertion site, such that the resulting DNA construct encoded a FVIII fusion protein in which the CTP1 protein sequence was inserted immediately after the residue indicated in the site selection.

Thus, where residue X designates the site of insertion and residue Z designates the next residue in the native FVIII polypeptide sequence, the polypeptide resulting from insertion of CTP1 contained the sequence:

X-GAPGGGGSDSSSSKAPPPSLPSPSRLPGPSDTPILPQGGGGSASS-Z
X-(SEQ ID NO:81)-Z

In addition, insertion of the corresponding DNA sequence at this position retained the *Asc*I and *Xho*I restriction sites flanking the CTP1 encoding sequence that are unique in the base vector and which can subsequently be used to excise the intervening CTP1 sequence and introduce sequences that differ in composition, length, and primary sequence.

A total of 12 different insertion sites in the FVIII sequence were selected for CTP1 insertion. For each A domain of FVIII one site was selected in each of the permissive loops *(i.e.,* in loops A1-1, A1-2, A2-1, A2-2, A3-1, and A3-2) as well as one site within the a3 acidic peptide region. The locations of these CTP1 insertion sites are summarized in TABLE 18 (see also TABLE 24).

**TABLE 18: Location of CTP1 Insertion Sites.**

| Construct | Domain | Loop | Insertion Site | Upstream Sequence | Downstream Sequence |
|---|---|---|---|---|---|
| FVIII-0026-CTP1 | A1 | A1-1 | 26 | LPV | DAR |
| FVIII-0116-CTP1 | A1 | | 116 | YDD | QTS |
| FVIII -0216-CTP1 | A1 | A1-2 | 216 | NSL | MQD |
| FVIII-0403-CTP1 | A2 | A2-1 | 403 | APD | DRS |
| FVIII-0518-CTP1 | A2 | | 518 | TVE | DGP |
| FVIII-0599-CTP1 | A2 | A2-2 | 599 | NPA | GVQ |
| FVIII-1656-CTP1 | a3 | | 1656 | TLQ | SDQ |
| FVIII-1720-CTP1 | A3 | A3-1 | 1720 | RAQ | RAQ |
| FVIII-1861-CTP1 | A3 | | 1861 | HTN | TLN |
| FVIII -1900-CTP1 | A3 | A3-2 | 1900 | NCR | APC |
| FVIII-2111-CTP1 | C1 | | 2111 | GKK | WQT |
| FVIII-2188-CTP1 | C2 | | 2188 | SDA | QIT |

FVIII variants with CTP1 insertions were used to transfect HEK293F cells (Life Technologies, Carlsbad, CA) using polyethyleneimine (PEI, Polysciences Inc. Warrington, PA). The transiently transfected cells were grown in a mixture of FREESTYLE^{®} F17 medium and CD OPTICHO^{®} media (Life Technologies). Five days post-transfection, the activities of recombinant FVIII-CTP1 variants in culture medium were analyzed by chromogenic FVIII assay to assess the tolerability of FVIII to CTP1 insertion at these sites.

The FVIII activity was measured using the COATEST^{®} SP FVIII kit from DiaPharma, and all incubations were performed on a 37°C plate heater with shaking. Harvests cell culture medium from transient transfection of FVIII-CTP1 variants were diluted to the desired FVIII activity range using 1x FVIII COATEST^{®} buffer. FVIII standards were prepared in 1x FVIII COATEST^{®} buffer containing medium from mock transfected cells a concentrations matching those of the test samples. The range of recombinant Factor VIII (rFVIII) standard was from 100 mIU/mL to 0.78 mIU/mL. The standards, diluted cell culture samples, and a pooled normal human plasma assay control were added to IMMULON^{®} 2HB 96-well plates in duplicate with 25 µL/well. Freshly prepared IXa/FX/phospholipid mix (50 µL), 25 µL of 25mM CaCl₂, and 50 µL of FXa substrate were added sequentially to each well, with a 5 minute incubation between each addition. After incubation with the substrate, 25 µL of 20% acetic acid was added to terminate color development, and the absorbance at 405 nm was measured with a SPECTRAMAX^{®} plus (Molecular Devices) instrument. Data analysis was performed using SOFTMAX^{®} Pro software (version 5.2). The Lowest Level of Quantification (LLOQ) was 39 mIU/mL. The results of the chromogenic FVIII assay are shown in FIG. 19.

The results depicted in FIG. 19 show that FVIII is able to accommodate the insertion of the CTP1 peptide at representative sites within permissive loops A1-1, A1-2, A2-1, A2-2, A3-1, and A3-2, as well as within the a3 region, without abrogation of the cofactor activity of FVIII. Insertion of the CTP1 peptide at positions 518 in the A2 domain, 1861 in the A3 domain, 2111 in the C1 domain, and 2188 in the C2 domain, resulted in FVIII activity levels that were below the limit of quantitation (BLOQ). Insertion of the CTP1 peptide at position 116 in the A1 domain yielded low but detectable FVIII activity relative to that observed for CTP1 insertion at representative sites within permissive loops or within the a3 region. These results support the conclusion that the tolerability of FVIII to peptidyl insertion at these permissive sites is an intrinsic property of FVIII that is not strictly dependent on the composition of the inserted element.

### Example 15: Insertion of CTP1 at Additional Sites within Permissive Loops

To demonstrate that FVIII can tolerate individual insertions of exogenous peptidyl elements at various sites within permissive loops without loss of cofactor function, a 45 amino acid long peptide encompassing a 29 amino acid long peptide derived from the carboxy terminus of human chorionic gonadotropin (CTP1, SEQ ID NO:81) was inserted by standard recombinant DNA techniques. The CTP1 DNA sequence (SEQ ID NO:82) encodes a polypeptide comprising the human chorionic gonadotropin-derived peptide (SEQ ID NO:62) flanked by the amino acid sequence Gly-Gly-Gly-Gly-Ser (SEQ ID NO:191), terminally flanked by a 5' *Asc*I restriction site (ggcgcgcc) and a 3' *Xho*I site (ctcgag), neither of which is present in the sequence of the base vector pBC0114.

The CTP1 DNA sequence was chemically synthesized, digested with *Asc*I and *Xho*I*,* and inserted into an appropriate FVIII expression plasmid into which the unique *Asc*I and *Xho*I sites had been inserted immediately downstream of the designated insertion site, such that the resulting DNA construct encoded a FVIII fusion protein in which the CTP1 protein sequence was inserted immediately after the residue indicated in the site selection.

Thus, where residue X designates the site of insertion and residue Z designates the next residue in the native FVIII polypeptide sequence, the polypeptide resulting from insertion of CTP1 contained the sequence:

X-GAPGGGGSDSSSSKAPPPSLPSPSRLPGPSDTPILPQGGGGSASS-Z
X-(SEQ ID NO:81)-Z

In addition, insertion of the corresponding DNA sequence at this position retained the *Asc*I and *Xho*I restriction sites flanking the CTP1 encoding sequence that are unique in the base vector and which subsequently was used to excise the intervening CTP1 sequence and introduce sequences that differ in composition, length, and primary sequence.

A total of 14 insertion sites in the FVIII sequence were selected for CTP1 insertion. For each A domain of FVIII one site was selected in each of the permissive loops *(i.e.,* in loops A1-1, A1-2, A2-1, A2-2, A3-1, and A3-2) as well as one site within the a3 acidic peptide region. The locations of these CTP1 insertion sites are summarized in TABLE 19 (see also TABLE 24).

**TABLE 19. Location of CTP1 insertion sites.**

| Construct | Domain | Loop | Insertion Site | Upstream Sequence | Downstream Sequence |
|---|---|---|---|---|---|
| FVIII-0018-CTP1 | A1 | A1-1 | 18 | YMQ | SDL |
| FVIII-0022-CTP1 | A1 | A1-1 | 22 | DLG | ELP |
| FVIII-0026-CTP1 | A1 | A1-1 | 26 | LPV | DAR |
| FVIII-0040-CTP1 | A1 | A1-1 | 40 | PFP | NTS |
| FVIII-0216-CTP1 | A1 | A1-2 | 216 | NSL | MQD |
| FVIII-0399-CTP1 | A2 | A2-1 | 399 | PLV | LAP |
| FVIII-0403-CTP1 | A2 | A2-1 | 403 | APD | DRS |
| FVIII-0599-CTP1 | A2 | A2-2 | 599 | NPA | GVQ |
| FVIII-1656-CTP1 | a3 region | | 1656 | TLQ | SDQ |
| FVIII-1711-CTP1 | A3 | A3-1 | 1711 | YGM | SSS |
| FVIII-1720-CTP1 | A3 | A3-1 | 1720 | RAQ | RAQ |
| FVIII-1900-CTP1 | A3 | A3-2 | 1900 | NCR | APC |
| FVIII-1905-CTP1 | A3 | A3-2 | 1905 | CNI | QME |
| FVIII-1910-CTP1 | A3 | A3-2 | 1910 | EDP | TFK |

FVIII variants with CTP1 insertions were used to transfect HEK293F cells (Life Technologies, Carlsbad, CA) using polyethyleneimine (PEI, Polysciences Inc. Warrington, PA). The transiently transfected cells were grown in a mixture of FREESTYLE^{®} F17 medium and CD OPTICHO^{®} media (Life Technologies). Five days post-transfection, the activities of recombinant FVIII-CTP1 variants in culture medium were analyzed by chromogenic FVIII assay to assess the tolerability of FVIII to CTP1 insertion.

The FVIII activity was measured using the COATEST^{®} SP FVIII kit from DiaPharma, and all incubations were performed on a 37°C plate heater with shaking. Harvests cell culture medium from transient transfection of FVIII-CTP1 variants were diluted to the desired FVIII activity range using 1x FVIII COATEST^{®} buffer. FVIII standards were prepared in 1x FVIII COATEST^{®} buffer containing medium from mock transfected cells a concentrations matching those of the test samples. The range of recombinant Factor VIII (rFVIII) standard was from 100 mIU/mL to 0.78 mIU/mL. The standards, diluted cell culture samples, and a pooled normal human plasma assay control were added to IMMULON^{®} 2HB 96-well plates in duplicate with 25 µL/well. Freshly prepared IXa/FX/phospholipid mix (50 µL), 25 µL of 25mM CaCl₂, and 50 µL of FXa substrate were added sequentially to each well, with a 5 minute incubation between each addition. After incubation with the substrate, 25 µL of 20% acetic acid was added to terminate color development, and the absorbance at 405 nm was measured with a SPECTRAMAX^{®} plus (Molecular Devices) instrument. Data analysis was performed using SOFTMAX^{®} Pro software (version 5.2). The Lowest Level of Quantification (LLOQ) was 39 mIU/mL. The results of the chromogenic FVIII assay are shown in FIG. 20.

The results depicted in FIG. 20 show that FVIII is able to accommodate the insertion of the CTP1 peptide at other sites within permissive loops A1-1, A2-1, A3-1, and A3-2 in addition to the single representative sites in each permissive loop depicted in FIG. 19, without abrogation of the cofactor activity of FVIII. These results support the conclusion that the tolerability of FVIII to peptidyl insertion is a general property of each permissive loop, and not restricted to specific positions within surface loops.

### Example 16: Insertion of albumin-binding peptide ABP1 at Representative Sites within Permissive Loops

To demonstrate that FVIII can tolerate individual insertions of exogenous peptidyl elements at various sites within permissive loops without loss of cofactor function, a 44 amino acid long peptide encompassing an 18 amino acid long albumin-binding peptide (ABP1, SEQ ID NO:83) was inserted by standard recombinant DNA techniques. The ABP1 DNA sequence (SEQ ID NO:84) encodes a polypeptide comprising the albumin-binding peptide (SEQ ID NO:52) flanked by two repeats of the amino acid sequence Gly-Gly-Gly-Gly-Ser (SEQ ID NO: 191), terminally flanked by a 5' *Asc*I restriction site (ggcgcgcc) and a 3' *Xho*I site (ctcgag), neither of which is present in the sequence of the base vector pBC0114.

The ABP1 DNA sequence was chemically synthesized, digested with *Asc*I and *Xho*I*,* and inserted into an appropriate FVIII expression plasmid into which the unique *Asc*I and *Xho*I sites had been inserted immediately downstream of the designated insertion site, such that the resulting DNA construct encoded a FVIII fusion protein in which the ABP1 protein sequence was inserted immediately after the residue indicated in the site selection.

Thus, where residue X designates the site of insertion and residue Z designates the next residue in the native FVIII polypeptide sequence, the polypeptide resulting from insertion of ABP1 contained the sequence:

X-GAPGGGGSGGGGSRLIEDICLPRWGCLWEDDGGGGSGGGGSASS-Z
X-(SEQ ID NO:83)-Z

In addition, insertion of the corresponding DNA sequence at this position retained the *Asc*I and *Xho*I restriction sites flanking the ABP1 encoding sequence that are unique in the base vector and which are subsequently used to excise the intervening ABP1 sequence and introduce sequences that differ in composition, length, and primary sequence.

For each A domain of FVIII one ABP1 insertion site was selected in each of the permissive loops (*i.e.,* in loops A1-1, A1-2, A2-1, A2-2, A3-1, and A3-2) as well as one site within the a3 acidic peptide region. The locations of these ABP1 insertion sites are summarized in TABLE 20 (see also TABLE 24).

**TABLE 20. Location of ABP1 insertion sites.**

| Construct | Domain | Loop | Insertion Site | Upstream Sequence | Downstream Sequence |
|---|---|---|---|---|---|
| FVIII-0026- ABP 1 | A1 | A1-1 | 26 | LPV | DAR |
| FVIII-0116-ABP1 | A1 | | 116 | YDD | QTS |
| FVIII-0216-ABP1 | A1 | A1-2 | 216 | NSL | MQD |
| FVIII-0403-ABP1 | A2 | A2-1 | 403 | APD | DRS |
| FVIII-0518-ABP1 | A2 | | 518 | TVE | DGP |
| FVIII-0599-ABP1 | A2 | A2-2 | 599 | NPA | GVQ |
| FVIII-1656-ABP1 | a3 region | | 1656 | TLQ | SDQ |
| FVIII-1720-ABP1 | A3 | A3-1 | 1720 | RAQ | RAQ |
| FVIII-1861-ABP1 | A3 | | 1861 | HTN | TLN |
| FVIII-1900-ABP1 | A3 | A3-2 | 1900 | NCR | APC |
| FVIII-2111-ABP1 | C1 | | 2111 | GKK | WQT |
| FVIII-2188-ABP1 | C2 | | 2188 | SDA | QIT |

FVIII variants with ABP1 insertions were used to transfect HEK293F cells (Life Technologies, Carlsbad, CA) using polyethyleneimine (PEI, Polysciences Inc. Warrington, PA). The transiently transfected cells were grown in a mixture of FREESTYLE^{®} F17 medium and CD OPTICHO^{®} media (Life Technologies). Five days post-transfection, the activities of recombinant FVIII-ABP1 variants in culture medium were analyzed by chromogenic FVIII assay to assess the tolerability of FVIII to ABP1 insertion.

The FVIII activity was measured using the COATEST^{®} SP FVIII kit from DiaPharma, and all incubations were performed on a 37°C plate heater with shaking. Harvests cell culture medium from transient transfection of FVIII-ABP1 variants were diluted to the desired FVIII activity range using 1× FVIII COATEST^{®} buffer. FVIII standards were prepared in 1x FVIII COATEST^{®} buffer containing medium from mock transfected cells a concentrations matching those of the test samples. The range of recombinant Factor VIII (rFVIII) standard was from 100 mIU/mL to 0.78 mIU/mL. The standards, diluted cell culture samples, and a pooled normal human plasma assay control were added to IIMMULON^{®} 2HB 96-well plates in duplicate with 25 µL/well. Freshly prepared IXa/FX/phospholipid mix (50 µL), 25 µL of 25mM CaCl₂, and 50 µL of FXa substrate were added sequentially to each well, with a 5 minute incubation between each addition. After incubation with the substrate, 25 µL of 20% acetic acid was added to terminate color development, and the absorbance at 405 nm was measured with a SPECTRAMAX^{®} plus (Molecular Devices) instrument. Data analysis was performed using SOFTMAX^{®} Pro software (version 5.2). The Lowest Level of Quantification (LLOQ) was 39 mIU/mL. The results of the chromogenic FVIII assay are shown in FIG. 21.

The results depicted in FIG. 21 show that FVIII is able to accommodate the insertion of the ABP1 peptide at representative sites within permissive loops A1-1, A1-2, A2-1, A2-2, A3-1, and A3-2, as well as within the a3 region, without abrogation of the cofactor activity of FVIII. Insertion of the ABP1 peptide at positions 116 in the A1 domain, 518 in the A2 domain, 1861 in the A3 domain, 2111 in the C1 domain, and 2188 in the C2 domain, resulted in FVIII activity levels that were below the limit of quantitation (BLOQ). In general, individual APB1 insertions yielded lower FVIII activity than did corresponding CTP1 insertions, indicating that the composition of the inserted peptidyl element may modulate the activity of the resulting construct. However, these results support the conclusion that the overall tolerability of FVIII to peptidyl insertion at these permissive sites is an intrinsic property of FVIII that is not strictly dependent on the composition of the inserted element.

### Example 17: Insertion of a Gly-Ser Repeat at Representative Sites within Permissive Loops

To demonstrate that FVIII can tolerate individual insertions of exogenous peptidyl elements at various sites within permissive loops without loss of cofactor function, a 41 amino acid long peptide encompassing an 35 residue Gly-Ser repeat (HAP1, SEQ ID NO:85) was inserted by standard recombinant DNA techniques. The HAP1 DNA sequence (SEQ ID NO:86) encodes a polypeptide comprising seven tandem repeats of the amino acid sequence Gly-Gly-Gly-Gly-Ser (SEQ ID NO:191), terminally flanked by a 5' *Asc*I restriction site (ggcgcgcc) and a 3' *Xho*I site (ctcgag), neither of which is present in the sequence of the base vector pBC0114.

The HAP1 DNA sequence was chemically synthesized, digested with *Asc*I and *Xho*I*,* and inserted into an appropriate FVIII expression plasmid into which the unique *Asc*I and *Xho*I sites had been inserted immediately downstream of the designated insertion site, such that the resulting DNA construct encoded a FVIII fusion protein in which the HAP1 protein sequence was inserted immediately after the residue indicated in the site selection.

Thus, where residue X designates the site of insertion and residue Z designates the next residue in the native FVIII polypeptide sequence, the polypeptide resulting from insertion of HAP1 contained the sequence:

X-GAPGGGGSGGGGSGGGGSGGGGSGGGGSGGGGSGGGGSASS-Z
X-(SEQ ID NO:85)-Z

In addition, insertion of the corresponding DNA sequence at this position retained the *Asc*I and *Xho*I restriction sites flanking the HAP1 encoding sequence that are unique in the base vector and which was subsequently used to excise the intervening HAP1 sequence and introduce sequences that differ in composition, length, and primary sequence.

For each A domain of FVIII one HAP1 insertion site was selected in each of the permissive loops (*i.e.,* in loops A1-1, A1-2, A2-1, A2-2, A3-1, and A3-2) as well as one site within the a3 acidic peptide region. The locations of these HAP1 insertion sites are summarized in TABLE 21 (see also TABLE 24).

**TABLE 21. Location of HAP1 insertion sites.**

| Construct | Domain | Loop | Insertion Site | Upstream Sequence | Downstream Sequence |
|---|---|---|---|---|---|
| FVIII-0026-HAP1 | A1 | A1-1 | 26 | LPV | DAR |
| FVIII-0116-HAP1 | A1 | | 116 | YDD | QTS |
| FVIII -0216- HAP 1 | A1 | A1-2 | 216 | NSL | MQD |
| FVIII-0403-HAP1 | A2 | A2-1 | 403 | APD | DRS |
| FVIII-0518-HAP1 | A2 | | 518 | TVE | DGP |
| FVIII-0599-HAP1 | A2 | A2-2 | 599 | NPA | GVQ |
| FVIII-1656-HAP1 | a3 region | | 1656 | TLQ | SDQ |
| FVIII-1720-HAP1 | A3 | A3-1 | 1720 | RAQ | RAQ |
| FVIII-1861-HAP1 | A3 | | 1861 | HTN | TLN |
| FVIII -1900- HAP 1 | A3 | A3-2 | 1900 | NCR | APC |
| FVIII-2111-HAP1 | C1 | | 2111 | GKK | WQT |
| FVIII-2188-HAP1 | C2 | | 2188 | SDA | QIT |

FVIII variants with HAP1 insertions were used to transfect HEK293F cells (Life Technologies, Carlsbad, CA) using polyethyleneimine (PEI, Polysciences Inc. Warrington, PA). The transiently transfected cells were grown in a mixture of FREESTYLE^{®} F17 medium and CD OPTICHO^{®} media (Life Technologies). Five days post-transfection, the activities of recombinant FVIII-HAP1 variants in culture medium were analyzed by chromogenic FVIII assay to assess the tolerability of FVIII to HAP1 insertion.

The FVIII activity was measured using the COATEST^{®} SP FVIII kit from DiaPharma, and all incubations were performed on a 37°C plate heater with shaking. Harvests cell culture medium from transient transfection of FVIII-HAP1 variants were diluted to the desired FVIII activity range using 1x FVIII COATEST^{®} buffer. FVIII standards were prepared in 1× FVIII COATEST^{®} buffer containing medium from mock transfected cells a concentrations matching those of the test samples. The range of recombinant Factor VIII (rFVIII) standard was from 100 mIU/mL to 0.78 mIU/mL. The standards, diluted cell culture samples, and a pooled normal human plasma assay control were added to IMMULON^{®} 2HB 96-well plates in duplicate with 25 µL/well. Freshly prepared IXa/FX/phospholipid mix (50 µL), 25 µL of 25mM CaCl₂, and 50 µL of FXa substrate were added sequentially to each well, with a 5 minute incubation between each addition. After incubation with the substrate, 25 µL of 20% acetic acid was added to terminate color development, and the absorbance at 405 nm was measured with a SPECTRAMAX^{®} plus (Molecular Devices) instrument. Data analysis was performed using SOFTMAX^{®} Pro software (version 5.2). The Lowest Level of Quantification (LLOQ) was 39 mIU/mL. The results of the chromogenic FVIII assay are shown in FIG. 22.

The results depicted in FIG. 22 show that FVIII is able to accommodate the insertion of the HAP1 peptide at representative sites within permissive loops A1-1, A1-2, A2-1, A2-2, A3-1, and A3-2, as well as within the a3 region, without abrogation of the cofactor activity of FVIII. Insertion of the HAP 1 peptide at positions 518 in the A2 domain, 2111 in the C1 domain, and 2188 in the C2 domain, resulted in FVIII activity levels that were below the limit of quantitation (BLOQ). Insertion of the HAP1 peptide at position 116 in the A1 domain and position 1861 in the A3 domain, yielded low but detectable FVIII activity relative to that observed for HAP1 insertion at representative sites within permissive loops or within the a3 region. These results support the conclusion that the tolerability of FVIII to peptidyl insertion at these permissive sites is an intrinsic property of FVIII that is not strictly dependent on the composition of the inserted element.

### Example 18: Insertion of a Green Fluorescent Protein at Representative Sites within Selected Permissive Loops

To demonstrate that FVIII can tolerate within permissive loops the insertion of a protein known to adopt a defined 3-dimensional structure without loss of cofactor function, a 265 amino acid long polypeptide encompassing the 239 amino acid residue sequence of enhanced green fluorescent protein (EGFP1, SEQ ID NO:87) was inserted by standard recombinant DNA techniques. The EGFP1 DNA sequence (SEQ ID NO:89) encodes the EGFP polypeptide (SEQ ID NO:88) flanked by two tandem repeats of the amino acid sequence Gly-Gly-Gly-Gly-Ser (SEQ ID NO:191) and terminally flanked by a 5' *Asc*I restriction site (ggcgcgcc) and a 3' *Xho*I site (ctcgag), neither of which is present in the sequence of the base vector pBC0114.

The EGFP1 DNA sequence was chemically synthesized, digested with *Asc*I and *Xho*I*,* and inserted into an appropriate FVIII expression plasmid into which the unique *Asc*I and *Xho*I sites had been inserted immediately downstream of the designated insertion site, such that the resulting DNA construct encoded a FVIII fusion protein in which the EGFP1 protein sequence was inserted immediately after the residue indicated in the site selection.

Thus, where residue X designates the site of insertion and residue Z designates the next residue in the native FVIII polypeptide sequence, the polypeptide resulting from insertion of EGFP1 contained the sequence:

X-GAPGGGGSGGGGSMVSKGEELFTGVVPILVELDGDVNGHKFSVSGEGEGD
ATYGKLTLKFICTTGKLPVPWPTLVTTLTYGVQCFSRYPDHMKQHDFFKSAMP
EGYVQERTIFFKDDGNYKTRAEVKFEGDTLVNRIELKGIDFKEDGNILGHKLE
YNYNSHNVYIMADKQKNGIKVNFKIRHNIEDGSVQLADHYQQNTPIGDGPVL
LPDNHYLSTQSALSKDPNEKRDHMVLLEFVTAAGITLGMDELYKGGGGSGGG
GSASS–Z
X-(SEQ ID NO:87)-Z

In addition, insertion of the corresponding DNA sequence at this position retained the AscI and XhoI restriction sites flanking the EGFP1 encoding sequence that are unique in the base vector and which can subsequently be used to excise the intervening EGFP1 sequence and introduce sequences that differ in composition, length, and primary sequence.

An EGFP1 insertion site was selected within each of the permissive loops A1-1, A2-1, A3-1, and A3-2, as well as within the a3 acidic peptide region. The locations of these EGFP1 insertion sites are summarized in TABLE 22 (see also TABLE 24).

**TABLE 22. Location of EGFP1 insertion sites.**

| Construct | Domain | Loop | Insertion Site | Upstream Sequence | Downstream Sequence |
|---|---|---|---|---|---|
| FVIII-0026-EGFP1 | A1 | A1-1 | 26 | LPV | DAR |
| FVIII-0403-EGFP1 | A2 | A2-1 | 403 | APD | DRS |
| FVIII-1656-EGFP1 | a3 region | | 1656 | TLQ | SDQ |
| FVIII-1720-EGFP1 | A3 | A3-1 | 1720 | RAQ | RAQ |
| FVIII-1900-EGFP1 | A3 | A3-2 | 1900 | NCR | APC |

FVIII variants with EGFP1 insertions were used to transfect HEK293F cells (Life Technologies, Carlsbad, CA) using polyethyleneimine (PEI, Polysciences Inc. Warrington, PA). The transiently transfected cells were grown in a mixture of FREESTYLE^{®} F17 medium and CD OPTICHO^{®} media (Life Technologies). Five days post-transfection, the activities of recombinant FVIII-EGFP1 variants in culture medium were analyzed by chromogenic FVIII assay to assess the tolerability of FVIII to EGFP1 insertion.

The FVIII activity was measured using the COATEST^{®} SP FVIII kit from DiaPharma, and all incubations were performed on a 37°C plate heater with shaking. Harvests cell culture medium from transient transfection of FVIII-EGFP1 variants were diluted to the desired FVIII activity range using 1x FVIII COATEST^{®} buffer. FVIII standards were prepared in 1x FVIII COATEST^{®} buffer containing medium from mock transfected cells a concentrations matching those of the test samples. The range of recombinant Factor VIII (rFVIII) standard was from 100 mIU/mL to 0.78 mIU/mL. The standards, diluted cell culture samples, and a pooled normal human plasma assay control were added to IMMULON^{®} 2HB 96-well plates in duplicate with 25 µL/well. Freshly prepared IXa/FX/phospholipid mix (50 µL), 25 µL of 25mM CaCl₂, and 50 µL of FXa substrate were added sequentially to each well, with a 5 minute incubation between each addition. After incubation with the substrate, 25 µL of 20% acetic acid was added to terminate color development, and the absorbance at 405 nm was measured with a SPECTRAMAX^{®} plus (Molecular Devices) instrument. Data analysis was performed using SOFTMAX^{®} Pro software (version 5.2). The Lowest Level of Quantification (LLOQ) was 39 mIU/mL. The results of the chromogenic FVIII assay are shown in FIG. 23.

The results depicted in FIG. 23 show that FVIII is able to accommodate the insertion of EGFP1 at selected representative sites within permissive loops A1-1, A2-1, A3-1, and A3-2, as well as within the a3 region, without abrogation of the cofactor activity of FVIII. Although EGFP1 insertion at each of these sites yielded detectable FVIII activity, the degree of activity observed was dependent upon the site of the insertion, with EGFP1 insertion within the a3 region yielding an activity comparable to that from the non-modified base vector, EGFP1 insertion within permissive loops A2-1 and A3-1 yielding moderate FVIII activity, and EGFP1 insertion within permissive loops A1-1 and A3-2 yielding low FVIII activity. Thus, FVIII exhibits significant variability in the extent of its tolerability to the insertion of EGFP, a protein known to adopt a defined 3-dimensional structure, as a function of the site of insertion.

### Example 19: Insertion of a Cys-containing Peptide and Chemical PEG Modification

To demonstrate that FVIII can tolerate insertion of an exogenous peptidyl element within a permissive loop and subsequent covalent conjugation to a cysteine residue contained within that element without loss of FVIII cofactor function, a 41 amino acid long peptide encompassing an 35 residue Gly-Ser repeat sequence containing a single Cys residue (CCP1, SEQ ID NO:90) was inserted by standard recombinant DNA techniques. The CCP1 DNA sequence (SEQ ID NO:91) encodes a polypeptide comprising seven tandem repeats of the amino acid sequence Gly-Gly-Gly-Gly-Ser (SEQ ID NO:191) with a Cys residue substituted at position 21, terminally flanked by a 5' *Asc*I restriction site (ggcgcgcc) and a 3' *Xho*I site (ctcgag), neither of which is present in the sequence of the base vector pBC01 14.

The CCP1 DNA sequence was chemically synthesized, digested with *Asc*I and *Xho*I*,* and inserted between the *Asc*I and *Xho*I sites of plasmid pBC0184, such that the resulting DNA construct, FVIII-0026-CCP1, encodes a FVIII fusion protein in which the CCP1 protein sequence is inserted immediately after residue 26.

X-GAPGGGGSGGGGSGGGGSGGCGSGGGGSGGGGSGGGGSASS -Z
X-(SEQ ID NO: 90)-Z

In addition, insertion of the corresponding DNA sequence at this position retained the *Asc*I and *Xho*I restriction sites flanking the CCPP1 encoding sequence that are unique in the base vector and which can subsequently be used to excise the intervening CCP1 sequence and introduce sequences that differ in composition, length, and primary sequence.

Plasmid FVIII-0026-CCP1 was used for large-scale transient transfection of HEK293F cells (Life Technologies, Carlsbad, CA) using polyethyleneimine (PEI, Polysciences Inc. Warrington, PA). The transiently transfected cells were grown in a mixture of FREESTYLE^{®} F17 medium and CD OPTICHO^{®} media (Life Technologies).

Conditioned cell culture medium was harvested five days post-transfection and the FVIII-0026-CCP1 protein was purified to a high degree by sequential immunoaffinity chromatography and ion-exchange chromatography steps.

Covalent conjugation of PEG to FVIII-0026-CCP1 was achieved by mild reduction of FVIII-0026-CCP1 with tris(2-carboxyethyl)phosphine (TCEP), purification of the reduced product by ion exchange chromatography, and incubation of purified reduced FVIII-0026-CCP1 with 60 kDa PEG-maleimide. PEGylated and non-PEGylated FVIII-0026-CCP1 were resolved by ion exchange chromatography.

To confirm that PEG conjugation had occurred specifically on the A1 domain of FVIII-0026-CCP1, both the PEGylated and non-PEGylated species were digested with thrombin and analyzed by non-reducing SDS-PAGE along with their non-thrombin-treated counterparts. Thrombin digestion of FVIII is well known to generate bands corresponding to the A1 domain, the A2 domain, and the residual FVIII light chain that are clearly distinguishable when the products are resolved by SDS-PAGE. Application of this method to FVIII-0026-CCP1 samples that were untreated or subjected to chemical PEGylation therefore enables verification that the PEG moiety has been appended to the A1 domain.

To confirm that FVIII-0026-CCP1 had been quantitatively PEGylated, non-modified FVIII-0026-CCP1 and PEGylated FVIII-0026-CCP1 were analyzed by size-exclusion chromatography (SEC) on a Tosoh G3000 SWxl column, and the absorbance of the eluent was monitored at 214 nm as a function of time. The resulting chromatograms were overlaid with a reference chromatogram generated with protein molecular weight standards of 17, 44, 158, and 670 kDa to enable determination of the apparent molecular weights of both non-modified and PEGylated FVIII-0026-CCP1 and assessment of the efficiency of the PEGylation reaction.

The FVIII activities of non-modified FVIII-0026-CCP1 and PEGylated FVIII-0026-CCP1 were measured using the COATEST^{®} SP FVIII kit from DiaPharma, and all incubations were performed on a 37°C plate heater with shaking. Samples of purified FVIII-0026-CCP1 and mono-PEGylated FVIII-0026-CCP1 were diluted to the desired FVIII activity range using 1x FVIII COATEST^{®} buffer. FVIII standards were prepared in 1x FVIII COATEST^{®} buffer. The range of recombinant Factor VIII (rFVIII) standard was from 100 mIU/mL to 0.78 mIU/mL. The standards and a pooled normal human plasma assay control were added to IMMULON^{®} 2HB 96-well plates in duplicate with 25 µL/well. Freshly prepared IXa/FX/phospholipid mix (50 µL), 25 µL of 25mM CaCl₂, and 50 µL of FXa substrate were added sequentially to each well, with a 5 minute incubation between each addition. After incubation with the substrate, 25 µL of 20% acetic acid was added to terminate color development, and the absorbance at 405 nm was measured with a SPECTRAMAX^{®} plus (Molecular Devices) instrument. Data analysis was performed using SOFTMAX^{®} Pro software (version 5.2). The Lowest Level of Quantification (LLOQ) was 39 mIU/mL. Activity data for the non-PEGylated and PEGylated constructs is shown in TABLE 23:

**TABLE 23: Activity Before and After PEGylation**

| Sample | mg/mL | IU/mL | IU/mg | Activity % |
|---|---|---|---|---|
| Before PEGylation | 0.1 | 792 | 7920 | 100 |
| After PEGylation | 0.13 | 1050 | 8077 | 101 |

The results presented in FIG. 24 show that the purified FVIII-0026-CCP1 preparation contained a single chain species (SC FVIII) and a two-chain species comprising a heavy chain (HC) and a light chain (LC). Protein bands corresponding to non-modified SC FVIII and HC (lane 1) shifted up as a result of PEGylation (lane 2) due to decreased electrophoretic mobility. Bands corresponding to PEGylated SC FVIII and HC, but not LC were observed by PEG staining (lane 7). Thrombin treatment of non-modified FVIII-0026-CCP1 resulted in the expected pattern of cleavage products with bands corresponding to the a3-deleted light chain (LCΔa3), the A1 domain, and the A2 domain (lane 4). Of these, only the band corresponding to the A1 domain shifted upwards upon PEGylation (lane 3) giving rise to a single band with reduced electrophoretic mobility that was detected by PEG staining (lane 8). These results demonstrate that FVIII-0026-CCP1 was specifically and PEGylated on the A1 domain.

As illustrated in FIG. 25, PEGylated and non-PEGylated FVIII-0026-CCP1 were further analyzed by size exclusion chromatography (SEC). The major peak corresponding to non-PEGylated FVIII-0026-CCP1 eluted with a retention time similar to that of the 158 kDa molecular weight standard, whereas the major peak corresponding to PEGylated FVIII-0026-CCP1 eluted with a retention time slightly less than that of the 670 kDa molecular weight standard, indicating that PEGylation significantly increased the hydrodynamic radius of FVIII-0026-CCP1. The chromatograms for both non-PEGylated and PEGylated FVIII-0026-CCP1 indicate that both protein species are >90% pure. Consequently, the FVIII activity data presented in TABLE 23 can be interpreted to conclude that the chemical conjugation of PEG to the cysteine-containing CCP1 peptide inserted after residue 26 of FVIII does not significantly alter the specific activity of the resulting molecule relative to non-modified FVIII-0026-CCP1. In addition, the observed specific activities of both FVIII-0026-CCP1 and PEGylated FVIII-0026-CCP1 are similar to those observed for non-modified B domain-deleted (BDD) FVIII, indicating that insertion of the CCP1 peptide at residue 26 within permissive loop A1-1 of FVIII does not, of itself, contribute to a reduction in the specific activity of FVIII, whether the inserted CCP1 peptide is PEGylated or not.

**TABLE 24: Sequence identification numbers for DNA and protein FVIII constructs described in Examples 14-19.**

| Construct | DNA Sequence SEQ ID NO | Protein Sequence SEQ ID NO |
|---|---|---|
| FVIII-0018-CTP1 | 92 | 93 |
| FVIII-0022-CTP1 | 94 | 95 |
| FVIII-0026-CTP1 | 96 | 97 |
| FVIII-0040-CTP1 | 98 | 99 |
| FVIII-0116-CTP1 | 100 | 101 |
| FVIII-0216-CTP 1 | 102 | 103 |
| FVIII-0399-CTP1 | 104 | 105 |
| FVIII-0403-CTP1 | 106 | 107 |
| FVIII-0518-CTP1 | 108 | 109 |
| FVIII-0599-CTP1 | 110 | 111 |
| FVIII-1656-CTP1 | 112 | 113 |
| FVIII-1711-CTP1 | 114 | 115 |
| FVIII-1720-CTP1 | 116 | 117 |
| FVIII-1861-CTP1 | 118 | 119 |
| FVIII-1900-CTP1 | 120 | 121 |
| FVIII-1905-CTP1 | 122 | 123 |
| FVIII-1910-CTP1 | 124 | 125 |
| FVIII-2111-CTP1 | 126 | 127 |
| FVIII-2188-CTP1 | 128 | 129 |
| FVIII-0026-ABP1 | 130 | 131 |
| FVIII-0116-ABP1 | 132 | 133 |
| FVIII-0216-ABP1 | 134 | 135 |
| FVIII-0403-ABP1 | 136 | 137 |
| FVIII-0518-ABP1 | 138 | 139 |
| FVIII-0599-ABP1 | 140 | 141 |
| FVIII-1656-ABP1 | 142 | 143 |
| FVIII-1720-ABP1 | 144 | 145 |
| FVIII-1861-ABP1 | 146 | 147 |
| FVIII-1900-ABP1 | 148 | 149 |
| FVIII-2111-ABP1 | 150 | 151 |
| FVIII-2188-ABP1 | 152 | 153 |
| FVIII-0026-HAP1 | 154 | 155 |
| FVIII-0116-HAP1 | 156 | 157 |
| FVIII-0216-HAP1 | 158 | 159 |
| FVIII-0403-HAP1 | 160 | 161 |
| FVIII-0518-HAP1 | 162 | 163 |
| FVIII-0599-HAP1 | 164 | 165 |
| FVIII-1656-HAP1 | 166 | 167 |
| FVIII-1720-HAP1 | 168 | 169 |
| FVIII-1861-HAP1 | 170 | 171 |
| FVIII-1900-HAP 1 | 172 | 173 |
| FVIII-2111-HAP1 | 174 | 175 |
| FVIII-2188-HAP1 | 176 | 177 |
| FVIII-0026-EGFP1 | 179 | 180 |
| FVIII-0403-EGFP1 | 181 | 182 |
| FVIII-1656-EGFP1 | 183 | 184 |
| FVIII-1720-EGFP1 | 185 | 186 |
| FVIII-1900-EGFP1 | 187 | 188 |
| FVIII-0026-CCP1 | 189 | 190 |

### Example 20: A Combinatorial Library Approach to Generate FVIII Variants

XTEN is a polypeptide comprising unstructured repeats that has been shown to increase the circulating half-lives of a number of proteins. The impact of XTEN on the clearance and function of payload molecules can be optimized by varying the location, composition, length and number of XTEN insertions, all of which can be achieved by recombinant technology. With the identification of permissive loops in FVIII that can accommodate intra-domain insertion of XTEN, a multivariate approach towards XTEN modification of FVIII was explored to develop FVIII-XTEN variants with half-life extension beyond 2-fold as observed with current clinical candidates. Accordingly, the effects of multiple XTEN insertions on the activity and pharmacokinetics of FVIII was evaluated.

**Methods:** FVIII-XTEN combinatorial libraries were constructed comprising over 400 BDD-FVIII variants with 2 to 6 XTEN insertions within permissive loops in the A domains, at the B domain junction, and at the C-terminus. Variants were expressed in HEK293 cells by small-scale transient transfection, and FVIII activity in conditioned medium was measured by FVIII chromogenic assay. The pharmacokinetic (PK) properties of variants with ≥0.3 IU/mL FVIII activity in culture medium were evaluated in FVIII knockout (HemA) and FVIII/VWF double-knockout (DKO) mice by monitoring plasma FVIII activity over time. DKO mice were used for initial ranking purposes to eliminate the influence of endogenous VWF on half-life. Concentrated conditioned medium or partially purified FVIII-XTEN preparations were used in PK studies to increase the throughput of PK screening. Similar PK profiles were observed using either conditioned medium or purified proteins.

**Results:** FVIII variants retained activity with up to 5 XTEN insertions. In DKO mice, which lack the protective benefit of VWF, the half-life improvement conferred by XTEN was insertion site-dependent, with single XTEN insertions in the A3 domain extending half-lives up to 4.5 hours, and those in the A2 domain up to 2.5 hours, versus 0.25 hours for unmodified BDD-FVIII. For intra-domain insertions, an XTEN length of 144 residues was optimal with regard to activity in cell culture and half-life extension, and the effects on PK of multiple XTEN insertions was additive when insertion sites were in different domains. FVIII with 3 XTEN insertions achieved a half-life of 16 hours in DKO mice, representing a 64-fold increase relative to BDD FVIII, but the introduction of additional XTENs resulted in only a nominal increase to 18 hours, indicating that half-life extension with XTENs is additive but saturable. Selected FVIII-XTEN variants that had exhibited half-lives of 3-18 h in DKO mice all had similar half-lives in HemA mice (~14 hours).

### Example 21: Complete or Partial Replacement of Permissive Loops with Various Peptidyl Elements

As illustrated in Table 2, Table 4, and Figure 20, FVIII can accommodate the insertion of XTEN AE42, XTEN 144, and CTP1 immediately after positions 18, 22, 26, and 40 without abrogation of its procoagulant activity. This region, spanning residues 18 through 40 in the primary sequence of mature FVIII has been denoted as permissive loop A1-1. Since the insertion of XTEN AE42, XTEN 144 or CTP1 peptidyl elements within this loop do not abrogate FVIII activity, we reasoned that this loop may be dispensable for the function of FVIII and that replacement of part or all of these loops with AE42 XTEN may result in a FVIII variant that retains procoagulant activity. An example of the method used to replace all or part of loop A1-1 by using restriction endonuclease fragment exchanged between two plasmids with insertions, described above, is provided in Figure 26. Here a plasmid encoding FVIII with a peptidyl element inserted immediately after residue 18 and a plasmid encoding FVIII with a peptidyl element inserted immediately after residue 40 are each digested with the restriction endonuclease AscI, which cleaves at the 5' end of the peptidyl element in both plasmids, corresponding to the unique site 1 in Figure 26, and with the restriction endonuclease AfIII, which cleaves downstream (3') of the peptidyl element at a site that is unique within each plasmid. The resulting DNA fragments are then ligated such that the peptidyl element replaces amino acid residues from position 19 to 40. While the method, as described here, is used to replace the entire A1-1 loop, it could, by logical extension, be used to replace smaller fragments of the A1-1 loop, such as those spanning residue 19-22, 19-26, 19-32, 23-26, 23-32, 23-40, 27-32, 27-40, and 33-40, as described in Table 25.

This method could be more broadly applied to replace analogous regions of FVIII permissive loop A1-1 with peptidyl elements of different lengths and compositions. Constructs pDC001 through pDC006 in Table 25 represent FVIII variants in which corresponding segments of the A1-1 loop are replaced with a CTP1 peptidyl element. Alternatively, partial or complete loop replacement within loop A1-1with XTEN AE42 or XTEN 144 could be achieved by a similar method, as described for constructs pOM001 through pOM020. For replacement of all or part of the A1-1 permissive loop with CTP1, XTEN AE42 or XTEN 144, both the acceptor and donor plasmids are digested with AscI and AflII, the latter being a the unique restriction site that is closest to the A1-1 loop in the 3' direction.

This general method could similarly be applied to replace all or part of permissive loops A2-1, A3-1, and A3-2. As indicated in Table 25, each of these permissive loops differs from one another with regard to the identity of the nearest unique restriction site in the 3' direction from the site p insertion. Thus, while AscI is used to cleave at the 5' end of peptidyl insertions all permissive loops, different restriction enzymes are used to cleave at the downstream site of each permissive loop, namely, BamHI for permissive loop A2-1, PflMI for permissive loop A3-1, and ApaI for permissive loop A3-2.

**TABLE 25: FVIII Constructs in which Part or All of Individual Permissive Loops are Replaced by Different Peptidyl Elements by Subcloning Between Plasmids with Two Different Insertions**

| | Loop Replacement | | | Acceptor (Plasmid) | | | Donor (Insert) | | | Restriction Sites | |
|---|---|---|---|---|---|---|---|---|---|---|---|
| Construct | Loop | Deletion | Insertion | Plasmid | Site | XTEN | Plasmid | Site | XTEN | 5' | 3' |
| pDO001 | A1-1 | 19-22 | CTP1 | pFVIII-0018-CTP1 | 18 | CTP1 | pFVIII-0022-CTP1 | 22 | CTP1 | *Asc*I | *Afl*II |
| pDC002 | A1-1 | 19-26 | CTP1 | pFVIII-0018-CTP1 | 18 | CTP1 | pFVIII-0026-CTP1 | 26 | CTP1 | *Asc*I | *Afl*II |
| _{P}DCOD3 | Al-1 | 19-40 | CTP1 | pFVIII-0018-CTP1 | 18 | CTP1 | pFVIII-0040-CTP1 | 40 | CTP1 | *Asc* I | *Afl*II |
| pDO004 | A1-1 | 23-26 | CTP1 | pFVIII-0022-CTP1 | 22 | CTP1 | pFVIII-0026-CrP1 | 26 | CTP1 | *Asc I* | *Afl* II |
| pDO005 | Al-1 | 23-40 | CTP1 | pFVIII-0022-CTP1 | 22 | CTP1 | pFVIII-0040-CTP1 | 40 | CTP1 | *Asc*I | *Afl*II |
| pDO006 | A1-1 | 27-40 | CTP1 | pFVIII-0026-CTP1 | 26 | CTP1 | pFVIII-0040-CTP1 | 40 | CTP1 | *Asc I* | *Afl*II |
| pDO007 | A2-1 | 400-403 | CTP1 | pFVIII-0399-CTP1 | 399 | CTP1 | pFVIII-0403-CTP1 | 403 | CTP1 | *Asc* I | *Bam* HI |
| pDO008 | A3-1 | 1712-1720 | CTP1 | pFVIII-1711-CTP1 | 1711 | CTP1 | pFVIII-1720-CTP1 | 1720 | CTP1 | *Asc I* | *Pfl* MI |
| pDO009 | A3-2 | 1901-1905 | CTP1 | pFVIII-1900-CTP1 | 1900 | CTP1 | pFVIII-1905-CTP1 | 1905 | CTP1 | *Asc* I | *Apa* I |
| pDO010 | A3-2 | 1901-1910 | CTP1 | pFVIII-1900-CrP1 | 1900 | CTP1 | pFVIII-1910-CTP1 | 1910 | CTP1 | *Asc* I | *Apa I* |
| pDCOll | A3-2 | 1906-1910 | CTP1 | pFVIII-1905-CTR1 | 1905 | CTP1 | pFVIII-1910-CTP1 | 1910 | CTP1 | *Asc* I | *Apa* I |
| pOM001 | A1-1 | 19-22 | AE42 | pBC0155 | 18 | AE42 | pBC0183 | 22 | AE42 | *Asc* I | *Afl*II |
| pOM002 | A1-1 | 19-26 | AE42 | pBC0165 | 18 | AE42 | pBC0184 | 26 | AE42 | *Asc* I | *Afl*II |
| pOM003 | A1-1 | 19-32 | AE42 | pBC0155 | 18 | AE42 | pNL0081.001 | 32 | AE42 | *Asc I* | *Afl*II |
| pOM004 | Al-1 | 19-40 | AE42 | pBC0155 | 18 | AE42 | pBC0166 | 40 | AE42 | *Asc I* | *Afl*II |
| pOM005 | A1-1 | 23-26 | AE42 | pBC0183 | 22 | AE42 | pBC0184 | 26 | AE42 | *Asc* I | *Afl*II |
| pOM006 | Al-1 | 23-32 | AE42 | pBC0183 | 22 | AE42 | pNL0081.001 | 32 | AE42 | *Asc* I | *Afl*II |
| pDM007 | Al-1 | 23-40 | AE42 | pBC0183 | 22 | AE42 | pBC0166 | 40 | AE42 | *Asc I* | *Afl*II |
| pOM008 | A1-1 | 27-32 | AE42 | pBC0184 | 26 | AE42 | pNL0081.001 | 32 | AE42 | *Asc I* | *Afl*II |
| pOM009 | Al-1 | 27-40 | AE42 | pBC0184 | 26 | AE42 | pBC0166 | 40 | AE42 | *Asc*I | *Afl*II |
| pOM010 | A1-1 | 33-40 | AE42 | pNL0081.001 | 32 | AE42 | pBC0166 | 40 | AE42 | *Asc* I | *Afl*II |
| pOM011 | A1-1 | 19-22 | AE144_5A | pBC165 | 18 | AE42 | pSD0047 | 22 | AE144_5A | *Asc* I | *Afl*II |
| pOM012 | Al-1 | 19-26 | AE144_5A | pBC165 | 18 | AE42 | pSD0049 | 26 | AE144_5A | *Asc* I | *Afl*II |
| pOM013 | A1-1 | 19-32 | AE144_5A | pBC165 | 18 | AE42 | pSD0022 | 32 | AE144_5A | *Asc* I | *Afl*II |
| pOM014 | Al-1 | 19-40 | AE144_5A | pBC165 | 18 | AE42 | pSD0051 | 40 | AE144_5A | *Asc*I | *Afl*II |
| pOM015 | A1-1 | 23-26 | AE144_5A | pBC0183 | 22 | AE42 | pSD0049 | 26 | AE144_5A | *Asc* I | *Afl*II |
| pOM016 | A1-1 | 23-32 | AE144_5A | pBC0183 | 22 | AE42 | pSD0022 | 32 | AE144_5A | *Asc* I | *Afl*II |
| pOM017 | A1-1 | 23-40 | AE144_5A | pBC0183 | 22 | AE42 | pSD0051 | 40 | AE144_5A | *Asc I* | *Afl*II |
| pOM018 | A1-1 | 27-32 | AE144_5A | pBC0184 | 26 | AE42 | pSD0022 | 32 | AE144_5A | *Asc* I | *Afl*II |
| pOM019 | Al-1 | 27-40 | AE144_5A | pBC0184 | 26 | AE42 | pSD0051 | 40 | AE144_5A | *Asc* I | *Afl* II |
| pOM020 | Al-1 | 33-40 | AE144_5A | pNL0081.001 | 32 | AE42 | pSD0051 | 40 | AE144_5A | *Asc*I | *Afl*II |
| pOM021 | A2-1 | 400-403 | AE42 | pNL0091.002 | 399 | AE42 | pBC0132 | 403 | AE42 | *Asc I* | *Bam* HI |
| pOM022 | A2-1 | 400-403 | AE144_2A | pNL0091.002 | 399 | AE42 | pSD0001 | 403 | AE144_2A | *Asc* I | *Bam* HI |
| pOM023 | A3-1 | 1712-1720 | AE42 | pNL0097.001 | 1711 | AE42 | pBC0138 | 1720 | AE42 | *Asc I* | *Pfl* MI |
| pOM024 | A3-1 | 1712-1725 | AE42 | pNL0097.001 | 1711 | AE42 | pNL0098.001 | 1725 | AE42 | *Asc* I | *Pft* MI |
| pOM025 | A3-1 | 1721-1725 | AE42 | pBC0138 | 1720 | AE42 | pNL0098.001 | 1725 | AE42 | *Asc* I | *Pfl* MI |
| p0M026 | A3-1 | 1712-1720 | AE144_4A | pNL0097.001 | 1711 | AE42 | pSD0009 | 1720 | AE144_4A | *Asc* I | *Pfl* MI |
| pOM027 | A3-1 | 1712-1725 | AE144_4A | pNL0097.001 | 1711 | AE42 | pSD0041 | 1725 | AE144_4A | *Asc*I | *Pfi* MI |
| pOM028 | A3-1 | 1721-1725 | AE144_4A | pEC0138 | 1720 | AE42 | pSD0041 | 1725 | AE144_4A | *Asc* I | *Pfl* MI |
| pOM029 | A3-2 | 1901-1905 | AE42 | pBC0176 | 1900 | AE42 | pNL0101.001 | 1905 | AE42 | *Asc* I | *Apa* I |
| pOM030 | A3-2 | 1901-1910 | AE42 | pBC0176 | 1900 | AE42 | pNL0102.001 | 1910 | AE42 | *Asc* I | *Apa* I |
| pOM031 | A3-2 | 1906-1910 | AE42 | pNL0101.001 | 1905 | AE42 | pNL0102.001 | 1910 | AE42 | *Asc I* | *Apa* I |
| pOM032 | A3-2 | 1901-1905 | AE144_1A | pBC0176 | 1900 | AE42 | pNL0002 | 1905 | AE144_1A | *Asc* I | *Apa I* |
| pOM033 | A3-2 | 1901-1910 | AE144_1A | pBC0176 | 1900 | AE42 | pNL0003 | 1910 | AE144_1A | *Asc* I | *Apa* I |
| pOM034 | A3-2 | 1906-1910 | AE144_1A | pNL0101.001 | 1905 | AE42 | pNL0003 | 1910 | AE144_1A | *Asc* I | *Apa* I |

### Example 22: Complete or Partial Replacement of Permissive Loops with Heterologous Peptidyl Elements or Complete or Partial Deletion of Permissive Loops

As depicted in Figure 2, the FVIII expression construct has several restriction endonuclease sites that are unique with respect to the entire FVIII expression plasmid. Thus, any pair of unique restriction sites that flank a particular permissive loop can be used to facilitate the insertion of a synthetic DNA constructs that encodes the intervening FVIII sequence with a complete or partial deletion of that particular permissive loop either with or without replacement of the deleted sequence with a heterologous peptidyl element. For example, to replace residues 19-40 in permissive loop A1-1, a DNA fragment would be synthesized that comprises, from its 5' and to its 3' end, the native sequence of the FVIII expression construct from the BsiWI site to the DNA sequence that encodes residue 18, a DNA sequence encoding a heterologous peptidyl element, the native sequence of the FVIII expression construct beginning with the DNA sequence that encodes residue 40 up to and including the unique *Afl*II restriction site. This synthetic DNA construct would be excised from its host plasmid with BsiWI and AflII and inserted into the corresponding sites of the FVIII expression construct that had been digested with the same restriction enzymes. Examples of heterologous peptidyl elements include CTP1, HAP1, ABP1, and EGFP1. These insertions could incorporate flanking unique restriction sites flanking the heterologous moiety insertion for easier subcloning. Alternatively, this method could be used to simply delete part or all of a permissive loop without the introduction of a heterologous peptidyl element by omitting the region encoding the heterologous peptidyl element from the synthetic DNA fragment. Alternatively, these synthetic DNA sequences could incorporate amino acid substitutions in the FVIII sequence, including substitutions of non-native amino acid sequences (mutations), in the permissive loops, with or without a heterologous peptidyl element. Alternatively, the heterologous insertions could be either adjacent or nonadjacent to the deletion or substitution within a loop. Examples of partial and complete deletions of FVIII permissive loops A1-1, A1-2, A2-1, A2-2, A3-1, and A3-2 as well as their replacement with heterologous peptidyl elements CTP1, HAP1, ABP1, and EGFP1 are given in Table 25. Examples of pairs of unique restriction enzymes that are appropriate for generation of these constructs are also given in Table 26. Multiple insertions and replacements could likewise be incorporated into a single FVIII construct.

**TABLE 26. FVIII Constructs in which Part or All of Individual Permissive Loops are Replaced by Different Peptidyl Elements by Cloning Synthetic DNA Sequences into Unique FVIII Restriction Enzyme Sites**

| | Loop Replacement | | | Restriction Sites | |
|---|---|---|---|---|---|
| Construct | Loop | Deletion | Insertion | 5' | 3' |
| pWF001 | A1-1 | 19-22 | CTP1 | *Bsi*WI | *Afl* II |
| pWF002 | A1-1 | 19-26 | CTP1 | *Bsi*WI | *Afl* II |
| pWF003 | A1-1 | 19-32 | CTP1 | *Bsi*WI | *Afl* II |
| pWF004 | A1-1 | 19-40 | CTP1 | *Bsi*WI | *Afl* II |
| pWF005 | A1-1 | 23-26 | CTP1 | *Bsi*WI | *Afl*II |
| pWF006 | A1-1 | 23-32 | CTP1 | *Bsi*WI | *Afl*II |
| pWF007 | A1-1 | 23-40 | CTP1 | *Bsi*WI | *Afl*II |
| pWF008 | A1-1 | 27-32 | CTP1 | *Bsi*WI | *Afl*II |
| pWF009 | A1-1 | 27-40 | CTP1 | *Bsi*WI | *Afl*II |
| pWF010 | A1-1 | 33-40 | CTP1 | *Bsi*WI | *Afl*II |
| pWF011 | A1-1 | 19-22 | HAP1 | *Bsi*WI | *Afl*II |
| pWF012 | A1-1 | 19-26 | HAP1 | *Bsi*WI | *Afl*II |
| pWF013 | A1-1 | 19-32 | HAP1 | *Bsi*WI | *Afl*II |
| pWF014 | A1-1 | 19-40 | HAP1 | *Bsi*WI | *Afl*II |
| pWF015 | A1-1 | 23-26 | HAP1 | *Bsi*WI | *Afl*II |
| pWF016 | A1-1 | 23-32 | HAP1 | *Bsi*WI | *Afl*II |
| pWF017 | A1-1 | 23-40 | HAP1 | *Bsi*WI | *Afl*II |
| pWF018 | A1-1 | 27-32 | HAP1 | *Bsi*WI | *Afl*II |
| pWF019 | A1-1 | 27-40 | HAP1 | *Bsi*WI | *Afl*II |
| pWF020 | A1-1 | 33-40 | HAP1 | *Bsi*WI | *Afl*II |
| pWF021 | A1-1 | 19-22 | ABP1 | *Bsi*WI | *Afl*II |
| pWF022 | A1-1 | 19-26 | ABP1 | *Bsi*WI | *Afl*II |
| pWF023 | A1-1 | 19-32 | ABP1 | *Bsi*WI | *Afl*II |
| pWF024 | A1-1 | 19-40 | ABP1 | *Bsi*WI | *Afl*II |
| pWF025 | A1-1 | 23-26 | ABP1 | *Bsi*WI | *Afl*II |
| pWF026 | A1-1 | 23-32 | ABP1 | *Bsi*WI | *Afl*II |
| pWF027 | A1-1 | 23-40 | ABP1 | *Bsi*WI | *Afl*II |
| pWF028 | A1-1 | 27-32 | ABP1 | *Bsi*WI | *Afl*II |
| pWF029 | A1-1 | 27-40 | ABP1 | *Bsi*WI | *Afl*II |
| pWF030 | A1-1 | 33-40 | ABP1 | *Bsi*WI | *Afl*II |
| pWF031 | A1-1 | 19-22 | EGFP1 | *Bsi*WI | *Afl*II |
| pWF032 | A1-1 | 19-26 | EGFP1 | *Bsi*WI | *Afl*II |
| pWF033 | A1-1 | 19-32 | EGFP1 | *Bsi*WI | *Afl*II |
| pWF034 | A1-1 | 19-40 | EGFP1 | *Bsi*WI | *Afl*II |
| pWF035 | A1-1 | 23-26 | EGFP1 | *Bsi*WI | *Afl*II |
| pWF036 | A1-1 | 23-32 | EGFP1 | *Bsi*WI | *Afl*II |
| pWF037 | A1-1 | 23-40 | EGFP1 | *Bsi*WI | *Afl*II |
| pWF038 | A1-1 | 27-32 | EGFP1 | *Bsi*WI | *Afl*II |
| pWF039 | A1-1 | 27-40 | EGFP1 | *Bsi*WI | *Afl*II |
| pWF040 | A1-1 | 33-40 | EGFP1 | *Bsi*WI | *Afl*II |
| pWF041 | A1-1 | 19-22 | No Insert | *Bsi*WI | *Afl*II |
| pWF042 | A1-1 | 19-26 | No Insert | *Bsi*WI | *Afl*II |
| pWF043 | A1-1 | 19-32 | No Insert | *Bsi*WI | *Afl*II |
| pWF044 | A1-1 | 19-40 | No Insert | *Bsi*WI | *Afl*II |
| pWF045 | A1-1 | 23-26 | No Insert | *Bsi*WI | *Afl*II |
| pWF046 | A1-1 | 23-32 | No Insert | *Bsi*WI | *Afl*II |
| pWF047 | A1-1 | 23-40 | No Insert | *Bsi*WI | *Afl*II |
| pWF048 | A1-1 | 27-32 | No Insert | *Bsi*WI | *Afl*II |
| pWF049 | A1-1 | 27-40 | No Insert | *Bsi*WI | *Afl*II |
| pWF050 | A1-1 | 33-40 | No Insert | *Bsi*WI | *Afl*II |
| pWF051 | A1-2 | 218-229 | CTP1 | *Afl*II | *Nhe*I |
| pWF052 | A1-2 | 218-229 | HAP1 | *Afl*II | *Nhe*I |
| pWF053 | A1-2 | 218-229 | ABP1 | *Afl*II | *Nhe*I |
| pWF054 | A1-2 | 218-229 | EGFP1 | *Afl*II | *Nhe*I |
| pWF055 | A1-2 | 218-229 | No Insert | *Afl*II | *Nhe*I |
| pWF056 | A2-1 | 400-403 | CTP1 | *Nhe*I | *Bam*HI |
| pWF057 | A2-1 | 400-403 | HAP1 | *Nhe*I | *Bam*HI |
| pWF058 | A2-1 | 400-403 | ABP1 | *Nhe*I | *Bam*HI |
| pWF059 | A2-1 | 400-403 | EGFP1 | *Nhe*I | *Bam*HI |
| pWF060 | A2-1 | 400-403 | No Insert | *Nhe*I | *Bam*HI |
| pWF061 | A2-2 | 595-607 | CTP1 | *Nhe*I | *Cla*I |
| pWF062 | A2-2 | 595-607 | HAP1 | *Nhe*I | *Cla*I |
| pWF063 | A2-2 | 595-607 | ABP1 | *Nhe*I | *Cla*I |
| pWF064 | A2-2 | 595-607 | EGFP1 | *Nhe*I | *Cla*I |
| pWF065 | A2-2 | 595-607 | No Insert | *Nhe*I | *Cla*I |
| pWF066 | A3-1 | 1712-1720 | CTP1 | *Cla*I | *Pfl*MI |
| pWF067 | A3-1 | 1712-1725 | CTP1 | *Cla*I | *Pfl*MI |
| pWF068 | A3-1 | 1721-1725 | CTP1 | *Cla*I | *Pfl*MI |
| pWF069 | A3-1 | 1712-1720 | HAP1 | *Cla*I | *Pfl*MI |
| pWF070 | A3-1 | 1712-1725 | HAP1 | *Cla*I | *Pfl*MI |
| pWF071 | A3-1 | 1721-1725 | HAP1 | *Cla*I | *Pfl*MI |
| pWF072 | A3-1 | 1712-1720 | ABP1 | *Cla*I | *Pfl*MI |
| pWF073 | A3-1 | 1712-1725 | ABP1 | *Cla*I | *Pfl*MI |
| pWF074 | A3-1 | 1721-1725 | ABP1 | *Cla*I | *Pfl*MI |
| pWF075 | A3-1 | 1712-1720 | EGFP1 | *Cla*I | *Pfl*MI |
| pWF076 | A3-1 | 1712-1725 | EGFP1 | *Cla*I | *Pfl*MI |
| pWF077 | A3-1 | 1721-1725 | EGFP1 | *Cla*I | *Pfl*MI |
| pWF078 | A3-1 | 1712-1720 | No Insert | *Cla*I | *Pfl*MI |
| pWF079 | A3-1 | 1712-1725 | No Insert | *Cla*I | *Pfl*MI |
| pWF080 | A3-1 | 1721-1725 | No Insert | *Cla*I | *Pfl*MI |
| pWF081 | A3-2 | 1901-1905 | CTP1 | *Pfl*MI | *Apa*I |
| pWF082 | A3-2 | 1901-1910 | CTP1 | *Pfl*MI | *Apa*I |
| pWF083 | A3-2 | 1906-1910 | CTP1 | *Pfl*MI | *Apa*I |
| pWF084 | A3-2 | 1901-1905 | HAP1 | *Pfl*MI | *Apa*I |
| pWF085 | A3-2 | 1901-1910 | HAP1 | *Pfl*MI | *Apa*I |
| pWF086 | A3-2 | 1906-1910 | HAP1 | *Pfl*MI | *Apa*I |
| pWF087 | A3-2 | 1901-1905 | ABP1 | *Pfl*MI | *Apa*I |
| pWF088 | A3-2 | 1901-1910 | ABP1 | *Pfl*MI | *Apa*I |
| pWF089 | A3-2 | 1906-1910 | ABP1 | *Pfl*MI | *Apa*I |
| pWF090 | A3-2 | 1901-1905 | EGFP1 | *Pfl*MI | *Apa*I |
| pWF091 | A3-2 | 1901-1910 | EGFP1 | *Pfl*MI | *Apa*I |
| pWF092 | A3-2 | 1906-1910 | EGFP1 | *Pfl*MI | *Apa*I |
| pWF093 | A3-2 | 1901-1905 | No Insert | *Pfl*MI | *Apa*I |
| pWF094 | A3-2 | 1901-1910 | No Insert | *Pfl*MI | *Apa*I |
| pWF095 | A3-2 | 1906-1910 | No Insert | *Pfl*MI | *Apa*I |

### Example 23: FVIII Activity Following Complete or Partial Replacement of a Permissive Loop with a Heterologous Peptidyl Elements

To determine the effect of partial or complete permissive loop replacement on recombinant FVIII activity, selected expression plasmids shown in Table 25 were assayed assayed for FVIII activity. HEK 293 cells were transiently transfected with expression plasmids encoding factor VIII (FVIII) variants in which permissive loop sequences were substituted with 42 or 144 amino acid XTEN sequences. FVIII activity in conditioned medium was determined five days post-transfection. Figure 27 and Table 27 show that replacement of a permissive loop with a heterologous moiety, e.g., XTENs, does not negatively affect that activity of the recombinant FVIII protein. In fact, several replacements made within the A1-1 permissive loop resulted in enhanced activity relative to the positive control, pBC0114, which encodes B domain-deleted (BDD) FVIII bearing a carboxy terminal epitope tag but no XTEN insertion. Assay of the positive control was repeated in triplicate, and the mean FVIII activity +/- standard deviation is shown with the hatched bar in Figure 27. Transfection with a negative control plasmid, pBC0185, which encodes a FVIII protein in which a 42 residue XTEN was inserted after residue 60, yielded no activity.

**TABLE 27. Activities of FVIII Constructs in HEK 293 Cells Transiently Transfected with Expression Plasmids Encoding FVIII Variants in which Part or All of Individual Permissive Loops are Replaced by Different Heterologous Moieties**

| **Tube / well #** | **Construct Name** | **XTEN1** | **Other Mutations** | **Activities IU/mL** |
|---|---|---|---|---|
| 1 | pOM001.001 | 0018 AE42 1 | Del 19-22 | 7.7 |
| 2 | pOM002.002 | 0018 AE42 1 | Del 19-26 | 7.8 |
| 3 | pOM003.001 | 0018 AE42 1 | Del 19-32 | 4.3 |
| 4 | pOM004.001 | 0018 AE42 1 | Del 19-40 | 8.9 |
| 5 | pOM005.001 | 0022 AE42 1 | Del 23-26 | 6.3 |
| 6 | pOM006.001 | 0022 AE42 1 | Del 23-32 | 4.8 |
| 7 | pOM007.004 | 0022 AE42 1 | Del 23-40 | 5.3 |
| 8 | pOM008.001 | 0026 AE42 1 | Del 27-32 | 4.2 |
| 9 | pOM009.001 | 0026_AE42 1 | Del 27-40 | 9.3 |
| 10 | pOM010.001 | 0032 AE42 1 | Del 33-40 | 9.9 |
| 11 | pOM011.001 | 0018 AE144 5A | Del 19-22 | 5.4 |
| 12 | pOM012.001 | 0018 AE144 5A | Del 19-26 | 5.2 |
| 13 | pOM013.002 | 0018 AE144 5A | Del 19-32 | 4.5 |
| 14 | pOM014.002 | 0018 AE144 5A | Del 19-40 | 7.6 |
| 15 | pOM015.001 | 0022 AE144 5A | Del 23-26 | 1.5 |
| 16 | pOM016.001 | 0022 AE144 5A | Del 23-32 | 2.1 |
| 17 | pOM017.001 | 0022 AE144 5A | Del 23-40 | 2.9 |
| 18 | pOM018.001 | 0026_AE144 5A | Del 27-32 | 2.5 |
| 19 | pOM019.001 | 0026_AE144 5A | Del 27-40 | 2.6 |
| 20 | pOM020.001 | 0032 AE144 5A | Del 33-40 | 2.8 |
| 21 | pOM021.001 | 0399 AE42 1 | Del 400-403 | 4.9 |
| 22 | pOM022.002 | 0399 AE144 2A | Del 400-403 | 3.1 |
| 23 | pOM023.001 | 1711 AE42 1 | Del 1712-1720 | 2.5 |
| 24 | pOM024.002 | 1711 AE42 1 | Del 1712-1725 | 2.0 |
| 25 | pOM025.004 | 1720 AE42 1 | Del 1721-1725 | 3.4 |
| 26 | pOM026.002 | 1711 AE144 4A | Del 1712-1720 | 0.2 |
| 27 | pOM027.001 | 1711 AE144 4A | Del 1712-1725 | 0.2 |
| 28 | pOM028.001 | 1720 AE144 4A | Del 1721-1725 | 0.9 |
| 29 | pOM029.002 | 1900 AE42 1 | Del 1901-1905 | 1.7 |
| 30 | pOM030.001 | 1900 AE42 1 | Del 1901-1910 | 2.1 |
| 31 | pOM031.001 | 1905 AE42 1 | Del 1906-1910 | 1.9 |
| 32 | pOM032.002 | 1900 AE144 1A | Del 1901-1905 | 0.3 |
| 33 | pOM033.001 | 1900 AE144 1A | Del 1901-1910 | 0.4 |
| 34 | pOM034.002 | 1905 AE144 1A | Del 1906-1910 | 0.8 |
| 35 | pBC0185 (neg con) | | | BLD |
| 36 | pBC114 a | | | 5.4 |
| 37 | pBC114 b | | | 7.4 |
| 38 | pBC114 c | | | 4.8 |

The present invention has been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present invention should not be limited by any of the above-described exemplary embodiments, but should be defined only in accordance with the following claims. Other embodiments of the invention will be apparent to those skilled in the art from consideration of the specification and practice of the invention disclosed herein.

This application claims the benefit of priority of U.S. Provisional Patent Application No. 61/866,017 filed on August 14, 2013.

## Claims

1. A recombinant FVIII protein comprising: a first polypeptide comprising Formula I: (Al)-a1- (A2)- a2- [B]; and a second polypeptide comprising Formula II: a3- (A3)- (Cl); wherein the first polypeptide and the second polypeptide are fused or associated as a heterodimer; wherein
a) Al is an Al domain of FVIII;
b) A2 is an A2 domain of FVIII;
c) [B] is optionally present and is a B domain of FVIII, or a fragment thereof;
d) A3 is an A3 domain of FVIII;
e) Cl is a Cl domain of FVIII; and
f) al, a2, and a3 are acidic spacer regions of FVIII;
wherein one or more amino acids in a permissive loop-1 region in the Al domain (Al-l) are substituted or deleted;
wherein A1-1 corresponds to a region in native mature human FVIII from amino acid 15 to 45;
wherein Al-2 corresponds to a region in native mature human FVIII from amino acid 201 to amino acid 232;
wherein A2-l corresponds to a region in native mature human FVIII from amino acid 395 to amino acid 421;
wherein A2-2 corresponds to a region in native mature human FVIII from amino acid 577 to amino acid 635;
wherein A3-1 corresponds to a region in native mature human FVIII from amino acid 1705 to amino acid 1732; and/or
wherein A3-2 corresponds to a region in native mature human FVIII from amino acid 1884 to amino acid 1917;
wherein a heterologous moiety is inserted in at least Al-l, wherein the heterologous moiety is a polypeptide;
wherein the one or more amino acids substituted or deleted in Al-l comprise amino acids 19 to 22, amino acids 19 to 26, or amino acids 19 to 40, corresponding to SEQ ID NO: 1; and
wherein the recombinant FVIII protein exhibits procoagulant activity.

2. The recombinant FVIII protein of claim 1, wherein the first polypeptide and the second polypeptide form a single polypeptide chain comprising the formula (Al)-a1- (A2)-a2-[B]-[a3]-(A3)-(Cl).

3. The recombinant FVIII protein of claim 1 or 2, wherein A1-1 corresponds to a region in native mature human FVIII from amino acid 18 to amino acid 41 of SEQ ID NO: 1.

4. The recombinant FVIII protein of any one of claims 1 to 3, wherein the heterologous moiety is inserted immediately downstream of amino acid 18, amino acid 22, amino acid 26 or amino acid 32 corresponding to SEQ ID NO: 1.

5. The recombinant FVIII protein of claim 4, wherein amino acids 19 to 40 corresponding to SEQ ID NO: 1 are deleted, and the heterologous moiety is inserted immediately downstream of amino acid 18 corresponding to SEQ ID NO: 1.

6. The recombinant FVIII protein of any one of claims 1 to 5, wherein the Al-l is completely substituted or deleted, and wherein the heterologous moiety is inserted at the point of deletion.

7. The recombinant FVIII protein of any one of claims 1 to 6, wherein the heterologous moiety comprises an element which increases the *in vivo* half-life of the protein, wherein the element which increases the in vivo half-life of the recombinant FVIII protein comprises albumin, albumin-binding polypeptide, Fe, PAS, the C-terminal peptide (CTP) of the subunit of human chorionic gonadotropin, polyethylene glycol (PEG), hydroxyethyl starch (HES), albumin-binding small molecules, a clearance receptor or a fragment thereof, or combinations thereof.

8. A nucleic acid or a set of nucleic acids comprising a sequence encoding the recombinant FVIII protein of any one of claims 1 to 7.

9. An expression vector or a set of expression vectors comprising the nucleic acid or the set of nucleic acids of claim 8.

10. A host cell comprising the nucleic acid or the set of nucleic acids of claim 8 or the expression vector or the set of expression vectors of claim 9.

11. A composition comprising the recombinant FVIII protein of any one of claims 1 to 7, the nucleic acid or the set of nucleic acids of claim 8, the expression vector or the set of expression vectors of claim 9, or the host cell of claim 10 and a pharmaceutically acceptable excipient.

12. The composition of claim 11 for use in preventing, treating, ameliorating, or managing a clotting disease or condition in a patient in need thereof.

## Patentansprüche

1. Rekombinantes FVIII-Protein, umfassend: ein erstes Polypeptid, das die Formel I: (A1)-a1-(A2)-a2-[B] umfasst; und ein zweites Polypeptid, das die Formel II: a3-(A3)-(C1) umfasst; wobei das erste Polypeptid und das zweite Polypeptid als ein Heterodimer fusioniert oder assoziiert sind; wobei
a) A1 für eine A1-Domäne von FVIII steht;
b) A2 für eine A2-Domäne von FVIII steht;
c) [B] gegebenenfalls vorliegt und für eine B-Domäne von FVIII oder ein Fragment davon steht;
d) A3 für eine A3-Domäne von FVIII steht;
e) C1 für eine C1-Domäne von FVIII steht; und
f) a1, a2 und a3 für saure Spacerregionen von FVIII stehen;
wobei eine oder mehrere Aminosäuren in einer permissiven Loop-1-Region in der A1-Domäne (A1-1) substituiert oder deletiert sind;
wobei A1-1 einer Region in nativem reifem menschlichem FVIII von Aminosäure 15 bis 45 entspricht;
wobei Al-2 einer Region in nativem reifem menschlichem FVIII von Aminosäure 201 bis Aminosäure 232 entspricht;
wobei A2-1 einer Region in nativem reifem menschlichem FVIII von Aminosäure 395 bis Aminosäure 421 entspricht;
wobei A2-2 einer Region in nativem reifem menschlichem FVIII von Aminosäure 577 bis Aminosäure 635 entspricht;
wobei A3-1 einer Region in nativem reifem menschlichem FVIII von Aminosäure 1705 bis Aminosäure 1732 entspricht; und/oder
wobei A3-2 einer Region in nativem reifem menschlichem FVIII von Aminosäure 1884 bis Aminosäure 1917 entspricht;
wobei eine heterologe Gruppierung in wenigstens A1-1 inseriert ist, wobei es sich bei der heterologen Gruppierung um ein Polypeptid handelt;
wobei die eine oder mehreren in A1-1 substituierten oder deletierten Aminosäuren Aminosäuren 19 bis 22, Aminosäuren 19 bis 26 oder Aminosäuren 19 bis 40, jeweils gemäß SEQ ID NO: 1, umfassen; und
wobei das rekombinante FVIII-Protein prokoagulatorische Aktivität zeigt.

2. Rekombinantes FVIII-Protein nach Anspruch 1, wobei das erste Polypeptid und das zweite Polypeptid eine einzige Polypeptidkette bilden, die die Formel (A1)-a1-(A2)-a2-[B]-[a3]-(A3)-(C1) umfasst.

3. Rekombinantes FVIII-Protein nach Anspruch 1 oder 2, wobei A1-1 einer Region in nativem reifem menschlichem FVIII von Aminosäure 18 bis Aminosäure 41 von SEQ ID NO: 1 entspricht.

4. Rekombinantes FVIII-Protein nach einem der Ansprüche 1 bis 3, wobei die heterologe Gruppierung unmittelbar stromabwärts von Aminosäure 18, Aminosäure 22, Aminosäure 26 oder Aminosäure 32 gemäß SEQ ID NO: 1 inseriert ist.

5. Rekombinantes FVIII-Protein nach Anspruch 4, wobei Aminosäuren 19 bis 40 gemäß SEQ ID NO: 1 deletiert sind und die heterologe Gruppierung unmittelbar stromabwärts von Aminosäure 18 gemäß SEQ ID NO: 1 inseriert ist.

6. Rekombinantes FVIII-Protein nach einem der Ansprüche 1 bis 5, wobei die A1-1 vollständig substituiert oder deletiert ist und wobei die heterologe Gruppierung am Deletionspunkt inseriert ist.

7. Rekombinantes FVIII-Protein nach einem der Ansprüche 1 bis 6, wobei die heterologe Gruppierung ein Element umfasst, das die In-vivo-Halbwertszeit des Proteins erhöht, wobei das Element, das die In-vivo-Halbwertszeit des rekombinanten FVIII-Proteins erhöht, Albumin, Albumin bindendes Polypeptid, Fe, PAS, das C-terminale Peptid (CTP) der Untereinheit von menschlichem Choriongonadotropin, Polyethylenglykol (PEG), Hydroxyethylstärke (HES), Albumin bindende kleine Moleküle, einen Clearance-Rezeptor oder ein Fragment davon oder Kombinationen davon umfasst.

8. Nukleinsäure oder Satz von Nukleinsäuren, umfassend eine Sequenz, die das rekombinante FVIII-Protein nach einem der Ansprüche 1 bis 7 codiert.

9. Expressionsvektor oder Satz von Expressionsvektoren, umfassend die Nukleinsäure bzw. den Satz von Nukleinsäuren nach Anspruch 8.

10. Wirtszelle, umfassend die Nukleinsäure bzw. den Satz von Nukleinsäuren nach Anspruch 8 oder den Expressionsvektor bzw. den Satz von Expressionsvektoren nach Anspruch 9.

11. Zusammensetzung, umfassend das rekombinante FVIII-Protein nach einem der Ansprüche 1 bis 7, die Nukleinsäure bzw. den Satz von Nukleinsäuren nach Anspruch 8, den Expressionsvektor bzw. den Satz von Expressionsvektoren nach Anspruch 9 oder die Wirtszelle nach Anspruch 10 sowie einen pharmazeutisch akzeptablen Exzipienten.

12. Zusammensetzung nach Anspruch 11 zur Verwendung beim Vorbeugen, Behandeln, Lindern von oder Zurechtkommen mit einer Gerinnungskrankheit bzw. einem Gerinnungsleiden bei einem dies benötigenden Patienten.

## Revendications

1. Protéine FVIII recombinante comprenant : un premier polypeptide comprenant la Formule I : (A1)-a1-(A2)-a2-[B] ; et un deuxième polypeptide comprenant la Formule II : a3-(A3)-(C1) ; le premier polypeptide et le deuxième polypeptide étant fusionnés ou associés sous forme d'hétérodimère ;
a) A1 étant un domaine A1 de FVIII ;
b) A2 étant un domaine A2 de FVIII ;
c) [B] étant éventuellement présent et étant un domaine B de FVIII, ou un fragment correspondant ;
d) A3 étant un domaine A3 de FVIII ;
e) C1 étant un domaine C1 de FVIII ; et
f) a1, a2 et a3 étant des régions d'espaceur acide de FVIII ;
un ou plusieurs acides aminés dans une région de boucle permissive 1 dans le domaine A1 (A1-1) étant substitués ou supprimés ;
A1-1 correspondant à une région dans la FVIII humaine mature native de l'acide aminé 15 à 45 ;
Al-2 correspondant à une région dans la FVIII humaine mature native de l'acide aminé 201 à l'acide aminé 232 ;
A2-1 correspondant à une région dans la FVIII humaine mature native de l'acide aminé 395 à l'acide aminé 421 ;
A2-2 correspondant à une région dans la FVIII humaine mature native de l'acide aminé 577 à l'acide aminé 635 ;
A3-1 correspondant à une région dans la FVIII humaine mature native de l'acide aminé 1 705 à l'acide aminé 1 732 ; et/ou
A3-2 correspondant à une région dans la FVIII humaine mature native de l'acide aminé 1 884 à l'acide aminé 1 917 ;
un groupement hétérologue étant inséré dans au moins A1-1, le groupement hétérologue étant un polypeptide ;
l'acide aminé ou les acides aminés substitués ou supprimés dans A1-1 comprenant les acides aminés 19 à 22, les acides aminés 19 à 26 ou les acides aminés 19 à 40, correspondant à SEQ ID NO: 1 ; et
la protéine FVIII recombinante présentant une activité procoagulante.

2. Protéine FVIII recombinante selon la revendication 1, le premier polypeptide et le deuxième polypeptide formant une unique chaîne polypeptidique comprenant la formule (A1)-a1-(A2)-a2-[B]-[a3]-(A3)-(C1).

3. Protéine FVIII recombinante selon la revendication 1 ou 2, A1-1 correspondant à une région dans la FVIII humaine mature native de l'acide aminé 18 à l'acide aminé 41 de SEQ ID NO : 1.

4. Protéine FVIII recombinante selon l'une quelconque des revendications 1 à 3, le groupement hétérologue étant inséré immédiatement en aval de l'acide aminé 18, de l'acide aminé 22, de l'acide aminé 26 ou de l'acide aminé 32 correspondant à SEQ ID NO : 1.

5. Protéine FVIII recombinante selon la revendication 4, les acides aminés 19 à 40 correspondant à SEQ ID NO : 1 étant supprimés, et le groupement hétérologue étant inséré immédiatement en aval de l'acide aminé 18 correspondant à SEQ ID NO : 1.

6. Protéine FVIII recombinante selon l'une quelconque des revendications 1 à 5, le A1-1 étant complètement substitué ou supprimé, et le groupement hétérologue étant inséré au point de suppression.

7. Protéine FVIII recombinante selon l'une quelconque des revendications 1 à 6, le groupement hétérologue comprenant un élément qui augmente la demi-vie *in vivo* de la protéine, l'élément qui augmente la demi-vie *in vivo* de la protéine recombinante FVIII comprenant l'albumine, un polypeptide de liaison à l'albumine, Fe, PAS, le peptide C-terminal (CTP) de la sous-unité de la gonadotrophine chorionique humaine, un polyéthylène glycol (PEG), un hydroxyéthyl-amidon (HES), des petites molécules de liaison à l'albumine, un récepteur de clairance ou un fragment correspondant, ou des combinaisons correspondantes.

8. Acide nucléique ou ensemble d'acides nucléiques comprenant une séquence codant pour la protéine FVIII recombinante selon l'une quelconque des revendications 1 à 7.

9. Vecteur d'expression ou ensemble de vecteurs d'expression comprenant l'acide nucléique ou l'ensemble d'acides nucléiques selon la revendication 8.

10. Cellule hôte comprenant l'acide nucléique ou l'ensemble d'acides nucléiques selon la revendication 8 ou le vecteur d'expression ou l'ensemble de vecteurs d'expression selon la revendication 9.

11. Composition comprenant la protéine FVIII recombinante selon l'une quelconque des revendications 1 à 7, l'acide nucléique ou l'ensemble d'acides nucléiques selon la revendication 8, le vecteur d'expression ou l'ensemble de vecteurs d'expression selon la revendication 9, ou la cellule hôte selon la revendication 10 et un excipient pharmaceutiquement acceptable.

12. Composition selon la revendication 11 pour une utilisation dans la prévention, le traitement, l'amélioration ou la gestion d'une maladie ou d'une affection de coagulation chez un patient qui en a besoin.
